# EUROPEAN PATENT APPLICATION

(11) **EP 0 992 240 A1**
(43) Date of publication of application: **12.04.2000**
(21) Application number: 98921878.9
(22) Date of filing: 29.05.1998
(51) Int. Cl.: A61K 31/435, A61K 31/12, A61K 31/165, A61K 31/235, A61K 31/40, A61K 31/415, A61K 31/44, A61K 31/505, A61K 31/52, A61K 38/08, A61K 45/00, C07D 471/00, C07D 471/04, C07D 471/06, C07D 471/14, C07D 209/08, C07D 209/88, C07D 213/62, C07D 213/72, C07D 217/02, C07D 217/24, C07D 235/06, C07D 235/08, C07D 235/24, C07D 239/34

(54) **THERAPEUTIC AGENT FOR ERECTION FAILURE**

(30) Priority: 29.05.1997 US 402; 29.11.1997 JP 34413497
(71) Applicant: MOCHIDA PHARMACEUTICAL CO., LTD., Shinjuku-ku Tokyo 160-8515 (JP)
(72) Inventor: NOTSU, Tatsuto, Mochida Pharmaceutical Co., Ltd., Shinjuku-ku, Tokyo 160-8515 (JP); OHZAWA, Nobuo, Mochida Pharmaceutical Co., Ltd., Shinjuku-ku, Tokyo 160-8515 (JP); NAKAI, Yasuhiro, Mochida Pharmaceutical Co., Ltd., Shinjuku-ku, Tokyo 160-8515 (JP)
(74) Representative: Grünecker, Kinkeldey, Stockmair & Schwanhäusser Anwaltssozietät
(86) International application number: JP9802378
(87) International publication number: WO9853819

(57) **Abstract**

A therapeutic agent for erection dysfunction and gynecoid incompetence, which is free from the problems involved in oral administration and direct injection, is safe, and exhibits excellent effect. A therapeutic agent for erection dysfunction and gynecoid incompetence for intranasal administration, percutaneous administration, or mucosal administration, such as oral mucosal administration or penis mucosal administration, characterized by containing as the active ingredient at least one compound having cyclic GMP phosphodiesterase inhibitory activity or salt thereof.

## Description

### REFERENCE TO RELATED APPLICATIONS

The subject application is a continuation-in-part of Serial No. 09/000402 which is a U.S. domestic application of an international patent application filed on May 29, 1997 claiming convention priority from Japanese Patent Application No. 160731/1996 filed on May 31, 1996. The specification of said U.S. application is herein incorporated by reference.

### TECHNICAL FIELD

The present invention relates to a pharmaceutical composition which strongly and selectively inhibits the phosphodiesterase (hereinafter abbreviated as PDE) of type V, exhibits a high safety and achieves an excellent effect for the therapy of erectile dysfunction and of female sexual dysfunction.

The present invention further relates to a pharmaceutical composition which affords a preferred absorption behavior that, unlike oral administration, it has no first-pass effect, that side effect of digestive system can be reduced and that its absorption is not affected by food and which also has a high clinical usefulness that, as compared with injection to cavernous body, it has no side effect such as pain of penis, prolonged erection and corporal fibrosis and that it can be injected by himself/herself whereby burden of patients and doctors is little.

### BACKGROUND ART

Impotence can be defined as a deficiency of ability of sexual intercourse in male and, to be more specific, impotence or erectile dysfunction can be defined as a state where hardness of penis or lasting time of erection which is sufficient for sexual intercourse cannot be achieved. Its occurring rate is said to be 2-7% of male population of until 50 years age and it has been reported that the rate further increases with an increase in age. It is presumed that, in Japan, there are about three millions of patients of erectile dysfunction. Most of them are not psychogenic but rather organic.

It has been known some pharmaceuticals induce erection of penis but the effect is merely achieved after a direct injection of a drug into penis such as cavernous body or after an infusion of a drug into urethra. Today's medical therapy is based upon injection of a vasoactive substance into cavernous body and it has been reported that phenoxybenzamine, phentolamine, papaverine and prostaglandin E1 derivative induce good erection solely or in combination. However, when some of those drugs are administered into cavernous body, pain of penis, prolonged erection and corporal fibrosis are resulted. Moreover, injection into cavernous body by a patient himself/herself is not allowed in Japan whereby such a means is not practical. Potassium channel openers and vasoactive intestinal peptide are known to be active by injection into cavernous body but they have not been actually used due to a problem of cost and safety. One substitute for the administration into cavernous body is the use of nitroglycerin patch applied to penis. Although this was confirmed to be effective, it results in a side effect to both patient and partner.

On the other hand, efficiency of preparations for oral administration is low and there has been almost no report for well-controlled clinical test for human being. Recently, there is a report on the clinical test result by oral administration of piperazine, 5-[2-ethoxy-5-(4-methylpiperazin-1-ylsulfonyl)phenyl]-1-methyl-3-propyl-6,7-dihydro-1H-pyrazolo[4,3-d]pyrimidin-7-one-citrate (hereinafter, abbreviated as sildenafil) which is an only agent subjected to a clinical test (cf. Drugs of the Future, volume 22, pages 138-143, 1997). However, side effects such as headache, flushing, dyspepsia, muscular pain and visual disability were reported and, in addition, it has been known that absorption of the drug into body is apt to be affected by food.

Moreover, with regard to intranasal or percutaneous administration, when compounds having vasodilating action are intranasally administered, congestion or engorgement of mucosa of nose usually takes place resulting in a so-called nasal congestion. Especially in the case of administration for long term, there is a danger of causing a permanent damage to nasal cell membrane and, therefore, its practical use is believed to be difficult. In addition, administration of compounds having a vasodilating action may cause redness of skin.

Further, in the case of percutaneous absorption, absorption of the drug is usually poor and it is necessary to take a drug delivery system such as iontophoresis or absorption promoter whereby percutaneous administrtion is not commonly used.

Among the compositions to be administered transmucomembranouslly are those which are administered in anticipation of absorption through the oral or penile mucous membrane. Commonly known dosage forms of compositions to be administered through the oral mucous membrane include, for example, sublingual preparations, buccal preparations, intraoral disintegrable preparations, chewable tablets, troches, jelly preparations, pastes preparations and patches to be applied to the oral mucosa. Intraoral disintegrable preparations are compositions that are rapidly disintegrable with saliva (a small amount of water) in the oral cavity; since they can be administered without water, intraoral disintegrable preparations are suitable for patients who are permitted to drink only limited amounts of water and even patients with dysphagia have no difficulty in taking the agents. In addition, intraoral disintegrable preparations can be dissolved and absorbed so efficiently that they are not affected by the first pass through the liver but instead have a potential for quick action.

Conventional intraoral disintegrable preparations include, for example, the preparation described in Unexamined Published Japanese Laid-Open Patent Publication Sho-53/44619 that has a drug solution cast into an open matrix reticular structure such as hydrolyzed gelatin or dextrin and which is subsequently freeze-dried; the preparation having a porous structure described in Unexamined Published Japanese Laid-Open Patent Publication Hei-05/271054 that contains saccharides such as sugar, starch sugar, lactose, honey, sugar alcohol and tetrose; the preparation described in Unexamined Published Japanese Laid-Open Patent Publication Hei-05/310558 that incorporates the particles of mannitol and/or lactose and sorbitol; the preparation described in International Patent Publication WO 95/20380 that has saccharides of low formability such as lactose, mannitol, glucose, sucrose and xylitol granulated with saccharides of high formability such as maltose, maltitol, sorbitol and oligosaccharide; the preparation described in Patent No. 2705787 that contains a sugar alcohol having a heat of solution of no more than -60 kJ/kg as exemplified by xylitol, sorbitol, mannitol or erythritol; the preparation described in Unexamined Published Japanese Laid-Open Patent Publication Hei-09/48726 that contains a saccharide, a sugar alcohol, a water-soluble polymer or the like as exemplified by glucose, fructose, sucrose, mannitol, sorbitol, maltitol, xylitol, erythritol, polyvinyl pyrrolidone, dextrin, hydroxyethyl cellulose and macrogol; the preparation described in Unexamined Published Japanese Laid-Open Patent Publication Hei-09/71523 that contains crystalline cellulose, hydroxypropyl cellulose of low substitution degree and a lubricant; the preparation described in Unexamined Published Japanese Laid-Open Patent Publication Hei-09/316006 that contains erythritol and a small amount of sourness presenting, solid organic acid; and the preparation described in Japanese Domestic Patent Announcement (kohyo) Hei-09/502622 that has a drug solution containing a solvent, gelatin and xanthan gum freeze-dried on a tray. However, no reports have been made to show that the intraoral disintegrable preparations are useful as therapeutics of erectile dysfunction or female sexual dysfunction.

Common dosage forms of compositions for administration through the penile mucous membrane include ointments, creams, jellies, gels, and so forth.

The mechanism of erection may be explained to be as follows. Thus, when cerebral nerve center is excited as a result of a sexual stimulation from eye or ear or of a direct stimulation to penis, such a stimulation is transmitted to the nerve of cavernous body of penis via a pelvic nerve which is a parasympathetic nerve. Then, in the cavernous body, acetylcholine, vasoactive intestinal peptide and nitric oxide (hereinafter, abbreviated as NO) are released whereupon relaxation of smooth muscle forming a valve structure of helical artery takes place. Artery blood supplied from back artery and deep artery of penis instantly flows into caves of cavernous body of penis whereupon the inner pressure of cavernous body of penis increases and the trabecula of cavernous body relaxed by vasoactive intestinal peptide and NO clogs the defluxing vein and volume of cavernous body caves themselves increases. In addition, stress of albuginea by the pressure compresses the vein which obliquely passes the albuginea whereby flowing out of blood is disturbed. Accordingly, blood is stored in the caves of cavernous body, albuginea becomes hard by stress and erection is resulted.

It has been clarified that NO expresses the relaxation of vein and cavernous body via an increase in cyclic GMP (hereinafter, abbreviated as cGMP). However, it is believed that a selective suppression of cGMP decomposing system also maintains the cGMP concentration whereby penis is erected. Thus, an inhibitor of PDE which is a enzyme specifically catalyzing the cGMP is expected as a new therapeutic agent without the above-mentioned side effect. As such, it is believed that, when PDE is inhibited, cGMP increases and that might be related to the therapy. At present, existence of at least seven isozymes was ascertained for PDE (cf. Physiological Reviews, volume 75, pages 725-748, 1995). Among them, five isozymes were widely distributed in many tissues. There are two isozymes which well decompose the cGMP and they are PDE type I (calmodulin-dependent PDE) and PDE type V (cGMP-PDE). On the other hand, PDE type III and PDE type IV decompose cAMP and PDE type II has no substrate selectivity. It is easily predicted that, when the last three isozymes are inhibited, cAMP increases whereby various side effects such as increase in contraction of myocardium, increase in heart rate and decrease of systemic blood pressure take place. It is especially well known that inhibition of PDE type III results in an increase in cAMP and in contraction of myocardium. Although there is a report that, when cGMP is increased in myocardium, contraction decreases, PDE type V is not distributed in myocardium Accordingly, when PDE type V is selectively inhibited, a selective action with little lowering effect on systemic blood pressure and little side effect on heart is expected.

Examples of the cGMP-PDE inhibitors disclosed up to now are pyrazolopyrimidine derivatives in the European Patent Publication No. 463,756 or No. 526,004; purinone derivatives in the Japanese Laid-Open Patent Publication Sho-63/196,585 and Hei-02/88,577, the International Publication WO 94/00,453 or the Japanese Laid-Open Patent Publication Hei-08/231,545, Hei-08/231,546 or Hei-09/124,648; quinazoline derivatives in the Japanese Laid-Open Patent Publication Sho-52/100,479, the International Publication WO95/06,648 or WO 96/26,940, or the Japanese Laid-Open Patent Publication Hei-08/104,679, Hei-07/126,255, Hei-06/192,235 or Hei-07/10,843; quinazolinone derivatives in the Japanese Laid-Open Patent Publication Hei-02/193,983, the International Publication WO 93/12,095 or the Japanese Laid-Open Patent Publication Hei-08/253,457; pyrimidine derivatives in the Japanese Laid-Open Patent Publication Sho-53/103,497 or Sho-61/236,778, U.S. Patent No. 5,294,612, the Japanese Laid-Open Patent Publication Hei-02/42,079, Hei-02/56,484, Hei-02/40,388, Hei-03/261,785, Hei-07/89,958, Hei-07/267,961, Hei-07/330,777 or Hei-08/143,571, U.S. Patent No. 5,525,604 or No. 5,541,187, the International Publication WO 96/28,429 or WO 96/28,448 or the Japanese Laid-Open Patent Publication Hei-08/253,484; pyrimidinone derivatives in the Japanese Laid-Open Patent Publication Hei-02/295,978 or the International Publication WO 93/06,104, WO 93/07,149 or WO 94/05,561; grizeol derivatives in the Japanese Laid-Open Patent Publication Sho-62/30,796; phenylpyridone derivatives in the Japanese Laid-Open Patent Publication Hei-01/311,067 or Hei-03/145,466; dihydropyridine derivatives in the Japanese Laid-Open Patent Publication Hei-02/223,580; polycyclic guanine derivatives in the International Publication WO 91/19,717 or WO 94/19,351; benzimidazole derivatives in the Japanese Laid-Open Patent Publication Hei-05/222,000 or the International Publication WO 97/24,334; fluorenone derivatives in the Japanese Laid-Open Patent Publication Hei-07/61,949; anthranilic acid derivatives in the Japanese Laid-Open Patent Publication Hei-08/188,563; pyridazine derivatives in the Japanese Laid-Open Patent Publication Hei-08/225,541; pyrazoloquinoline derivatives in the International Publication WO 96/28,446; pyridopyrazinone derivatives in the Japanese Laid-Open Patent Publication Hei-08/269,059; indole derivatives in the International Publication WO 96/32,379; dihydropyrazolone derivatives in the Japanese Laid-Open Patent Publication Hei-08/311,035; phthalazine derivatives in the International Publication WO 96/05,176; imidazoquinazoline derivatives in the Internatioal Patent Publication WO 98/08848; or thienopyrimidine derivatives in the International Publication WO 98/06722. However, there is no disclosure at all for a composition containing those compounds for the therapy of erectile dysfunction by intranasal administration.

With regard to pyridocarbazole derivatives, there has been no report either on their PDE inhibitory action or on the fact that they are effective to erectile dysfunction or female sexual dysfunction.

### DISCLOSURE OF INVENTION

An object of the present invention is to provide a novel composition for the treatment of erectile dysfunction or female sexual dysfunction that contains at least one compound having a highly enzyme selectivity and potent cGMP-PDE inhibitory action as an effective component and which presents high safety with reduced side effects, particularly one that is intended for intranasal administration, percutaneous administration or transmucomembranous administration as through the oral or penile mucous membrane.

Another object of the invention is to provide a novel intranasal composition that contains at least one compound having a highly enzyme selectivity and potent cGMP-PDE inhibitory action as an effective component and which presents high safety with reduced side effects.

Yet another object of the invention is to provide a novel transmucomembranous composition that contains at least one of the compounds represented by the formula (I) having a highly enzyme selectivity and potent cGMP-PDE inhibitory action, salts or solvates thereof as an effective component and which presents high safety with reduced side effects.

A further object of the invention is to provide a novel intranasal, percutaneous or transmucomembranous composition for the treatment of erectile dysfunction that contains at least one compound having a potent cGMP-PDE inhibitory action as an effective component and which presents high safety with reduced side effects.

The present invention is particularly intended to provide intranasal, percutaneous or transmucomembranous compositions for the treatment of erectile dysfunction and female sexual dysfunction that have solved at least one or more of the aforementioned problems in the prior art and which contain at least one compound having a highly enzyme selectivity and potent cGMP-PDE inhibitory action as an effective component.

The present inventors have conducted an intensive investigation for finding the pharmaceuticals inhibiting the PDE of the type V in a potent and selective manner and having a high safety and, as a result, they have found that novel pyridocarbazole derivatives and salts thereof exhibit a PDE-inhibiting activity in a potent and selective manner. It was also found that these compounds are useful as compositions for the treatment of erectile dysfunction and female sexual dysfunction. The present invention has been accomplished on the basis of these findings.

The present inventors also found that when the compounds having a cGMP-PDE inhibitory action were administered intranasally, percutaneously or transmucomembranouslly, there were obtained more preferred absorption dynamics than when they were administered perorally, as exemplified by the avoidance of the first pass effect, reduced side effects in the digestive tract, freedom of absorption from the effects of meals and potential rapidity in action, as well as higher clinical utility than when the compounds were injected into the cavernous tissue, as exemplified by the absence of side effects such as penile pain, prolonged erection and fibrosis, administrability by the patient himself with reduced burden on both the patient and the doctor. The present invention has been accomplished on the basis of this finding.

The first aspect of the present invention is a composition for remedy of erectile dysfunction wherein the composition contains at least one of the compounds represented by the following formula (I) and salts or solvates thereof as an effective component: 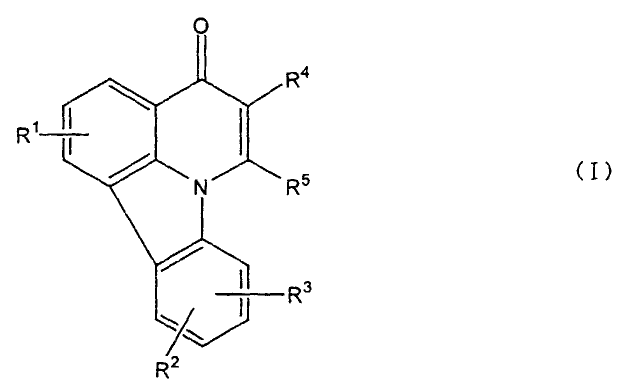 where R¹ represents a hydrogen atom, a halogen atom, a cyano group, an optionally protected carboxyl group, an optionally protected carboxymethyl group, an alkoxycarbonyl group having 1 - 4 carbon atoms, a carbamoyl group, an acetylamino group, a 3-carboxy-1-propenyl group, a 2-hydroxypentyloxy group, a 2,2-diethoxyethoxy group, an optionally protected hydroxyl group, an optionally protected mercapto group, a straight- or branched-chain alkanoyloxy group having 1 - 4 carbon atoms, a carbonyloxy group substituted by a phenyl group or a pyridyl group, a straight- or branched-chain alkyl group having 1 - 4 carbon atoms which may be substituted by one hydroxyl group, an amino group which may be mono- or disubstituted by an alkyl group having 1 - 4 carbon atoms, an alkylthio group having 1 - 3 carbon atoms which may be monosubstituted by any group selected from the group consisting of a hydroxyl group, a carboxyl group, a phenyl group and a pyridyl group, or represented by the following general formula (XXI):

- O - (CH₂)ₙ -Z (XXI)

(where Z is selected from the group consisting of a hydrogen atom, a carboxyl group, an alkoxy group having 1 or 2 carbon atoms which may be substituted by one hydroxyl group, an alkoxycarbonyl group having 1 - 6 carbon atoms, a carbamoyl group which may be mono- or disubstituted by a hydroxymethyl group or an alkyl group having 1 or 2 carbon atoms, an alkanoyl group having 1 - 4 carbon atoms which may be substituted by one hydroxyl group or mercapto group, a piperidinylcarbonyl group which may be substituted by one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted by a hydroxymethyl group or an acetoxymethyl group or an alkyl group having 1 - 4 carbon atoms or an alkoxycarbonyl group having 1 or 2 carbon atoms, a pyrazinyl group, a pyrimidinyl group, a furyl group, a thienyl group, an oxadiazolyl group and a 4-methoxyphenoxy group; n is 1 - 6); R² is selected from the group consisting of a hydrogen atom, a halogen atom, an optionally protected hydroxyl group, an optionally protected mercapto group, an optionally protected amino group, a cyano group, a nitro group, a trifluoromethyl group, a trifluoromethoxy group, an optionally protected carboxyl group, a 4-morpholylacetyl group, a straight- or branched-chain alkanoyloxy group having 1 - 4 carbon atoms, a straight- or branched-chain alkanoyl group having 1 - 4 carbon atoms, a straight- or branched-chain alkyl group having 1 - 4 carbon atoms, an alkylthio group having 1 - 3 carbon atoms which may be monosubstituted by any group selected from the group consisting of a hydroxyl group, a carboxyl group, a phenyl group and a pyridyl group and a straight- or branched-chain alkoxy group having 1 - 4 carbon atoms which may be substituted by one alkoxycarbonyl group having 1 - 4 carbon atoms; R³ is selected from the group consisting of a hydrogen atom, a halogen atom, an optionally protected hydroxyl group and a straight- or branched-chain alkoxy group having 1 - 4 carbon atoms; R⁴ is selected from the group consisting of a hydrogen atom, a halogen atom, an optionally protected carboxyl group, a phenoxy group, an anilino group, a N-methylanilino group, a 4-morpholylcarbonyl group, an alkyl group having 1 or 2 carbon atoms which may be substituted by a cyclic alkyl group having 3 - 6 carbon atoms, a benzyl group which may be mono- or disubstituted in the phenyl portion by any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 - 4 carbon atoms, an acetylamino group, a carboxyl group and an amino group, a pyridylmethyl group which may be substituted by an alkyl group having 1 - 4 carbon atoms, a morpholylmethyl group, a triazolylmethyl group, a furylmethyl group, a thienylmethyl group, a pyrimidinylmethyl group, a pyrazinylmethyl group, a pyrrolylmethyl group, an imidazolylmethyl group, a quinolylmethyl group, an indolylmethyl group, a naphthylmethyl group, a benzoyl group, an α-hydroxybenzyl group and an alkoxycarbonyl group having 1 or 2 carbon atoms; R⁵ represents a hydrogen atom or a methyl group; when R¹, R², R³ and R⁵ are a hydrogen atom at the same time, R⁴ is not a hydrogen atom, a benzyl group, a 4-diethylaminobenzyl group or a furylmethyl group.

The preferred substituents in the compounds represented by the general formula (I) or the preferred combinations thereof are shown below but the invention is by no means limited thereto.

Speaking of R¹, it is preferably substituted in 2-position and is preferably a hydroxyl group or represented by the following general formula (XXI):

- O - (CH₂)ₙ -Z (XXI)

where Z represents a hydrogen atom, a carboxyl group, an alkoxycarbonyl group having 1 - 6 carbon atoms, a carbamoyl group which may be mono- or disubstituted by a hydroxymethyl group or an alkyl group having 1 or 2 carbon atoms, an alkanoyl group having 1 - 4 carbon atoms which may be substituted by one hydroxyl group or mercapto group, a hydroxyl group, an amino group, a phenyl group, a pyridyl group which may be monosubstituted by a hydroxymethyl group or an acetoxymethyl group or an alkyl group having 1 - 4 carbon atoms or an alkoxycarbonyl group having 1 or 2 carbon atoms; n is 1 - 4.

More preferably, R¹ is substituted in 2-position and is either a hydroxyl group or represented by the following general formula (XXI):

- O - (CH₂)ₙ -Z (XXI)

where Z represents a hydrogen atom, a carboxyl group, a carbamoyl group which may be mono- or disubstituted by a hydroxymethyl group or an alkyl group having 1 or 2 carbon atoms, an alkanoyl group having 1 - 4 carbon atoms which may be substituted by one hydroxyl group or mercapto group, a hydroxyl group, a phenyl group, a pyridyl group, a pyrazinyl group or a pyrimidinyl group; n is 1 - 4.

Preferably, R² and R³ are not a hydrogen atom at the same time; it is preferred that R² is substituted in 9- or 10-position and is a hydrogen atom, a halogen atom, a hydroxyl group, a trifluoromethyl group or a straight- or branched-chain alkoxy group having 1 - 4 carbon atoms and that R³ is a hydrogen atom. Further, it is more preferred that R² is substituted in 9-position and is a halogen atom or a trifluoromethyl group and that R³ is a hydrogen atom.

Preferably, R⁴ is a hydrogen atom, an alkyl group having 1 or 2 carbon atoms, a pyrimidinylmethyl group or a pyridylmethyl group which may be substituted by a methyl group. Further, it is more preferred that R⁴ is a methyl group, a pyrimidinylmethyl group or a pyridylmethyl group. Preferably, R⁵ is a hydrogen atom.

The preferred combinations of the substituents are as follows: R¹ is substituted in 2-position and is either a hydroxyl group or represented by the following general formula (XXI):

- O - (CH₂)ₙ -Z (XXI)

where Z represents a hydrogen atom, a carboxyl group, a carbamoyl group which may be mono- or disubstituted by a hydroxylmethyl group or an alkyl group having 1 or 2 alkyl groups, an alkanoyl group having 1 - 4 carbon atoms which may be substituted by one hydroxyl group or mercapto group, a hydroxyl group, a phenyl group, a pyridyl group, a pyrazinyl group or a pyrimidinyl group; n is 1 - 4; R² is a halogen atom or a trifluoromethyl group which are substituted in 9-position; R³ is a hydrogen atom; R⁴ is a methyl group, a pyrimidinylmethyl group or a pyridylmethyl group; and R⁵ is a hydrogen atom.

The particularly preferred compounds are 9-bromo-2-(3-hydroxypropyloxy)-5-(3-pyridylmethyl)-4H-pyrido[3,2,1-jk]carbazol-4-one, and salts or solvates thereof.

The second aspect of the present invention is a composition for remedy of female sexual dysfunction wherein the composition contains at least one of the compounds represented by the above formula (I) and salts or solvates thereof as an effective component.

The third aspect of the present invention is a composition for remedy of erectile dysfunction or female sexual dysfunction by intranasal administration wherein the composition contains at least one of the compounds represented by the above formula (I) and salts or solvates thereof as an effective component.

The fourth aspect of the present invention is a composition for remedy of erectile dysfunction or female sexual dysfunction by percutaneous administration wherein the composition contains at least one of the compounds represented by the above formula (I) and salts or solvates thereof as an effective component.

The fifth aspect of the present invention is a composition for remedy of erectile dysfunction or female sexual dysfunction by transmucomembranous administration wherein the composition contains at least one of the compounds represented by the above formula (I) and salts or solvates thereof as an effective component.

In the above-mentioned second to the fifth aspect of the present invention, the preferred substituents or their preferred combinations in the compounds represented by the above formula (I) are the same as those in the first aspect.

The sixth aspect of the present invention is a composition for remedy of erectile dysfunction or female sexual dysfunction especially by intranasal administration, percutaneous administration or transmucomembranous administration wherein the composition contains 9-bromo-2-(3-hydroxypropyloxy)-5-(3-pyridylmethyl)-4H-pyrido[3,2,1-jk]carbazol-4-one or a salt or solvate thereof as an effective component.

The seventh aspect of the present invention is a composition for intranasal administration wherein the composition contains at least one of the compounds represented by the above formula (I) and salts or solvates thereof as an effective component. The preferred substituents or their preferred combinations in the compounds represented by the above formula (I) are the same as those in the first embodiment.

The eighth aspect of the present invention is a composition for intranasal administration wherein the composition contains 9-bromo-2-(3-hydroxypropyloxy)-5-(3-pyridylmethyl)-4H-pyrido[3,2,1-jk]carbazol-4-one or a salt or solvate thereof as an effective components.

The ninth aspect of the present invention is a composition for remedy of erectile dysfunction by intranasal administration wherein the composition contains at least one of the compounds having an inhibitory action to cyclic GMP phosphodiesterase and salts or solvates thereof as an effective component.

The tenth aspect of the present invention is a composition for remedy of erectile dysfunction by intranasal administration wherein the composition contains at least one of the compounds represented by the following formula (XXV) and salts or solvates thereof as an effective component: 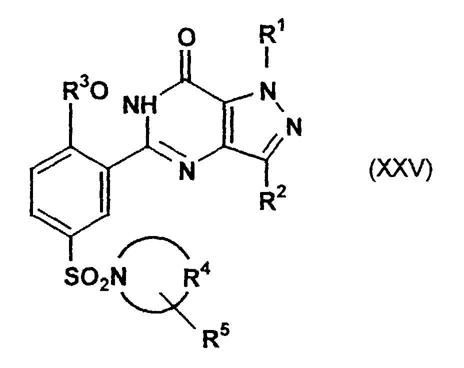 (where R¹ is a hydrogen atom, an alkyl group having 1 - 3 carbon atoms, a cycloalkyl group having 3 - 5 carbon atoms or a perfluoroalkyl group having 1 - 3 carbon atoms; R² is selected from the group consisting of a hydrogen atom, a hydroxyl group, an alkyl group having 1 - 6 carbon atoms which is substituted by an alkoxy group having 1 - 3 carbon atoms or an cycloalkyl group having 3 - 6 carbon atoms and a perfluoroalkyl group having 1 - 3 carbon atoms; R³ is selected from the group consisting of an alkyl group having 1 - 6 carbon atoms, an alkenyl group having 3 - 6 carbon atoms, an alkynyl group having 3 - 6 carbon atoms, a cycloalkyl group having 3 - 7 carbon atoms, a perfluoroakyl group having 1 - 6 carbon atoms and (C₃₋₆ cycloalkyl)-C₁₋₆ alkyl; R⁴ forms a group selected from the group consisting of pyrrolidinyl, piperidino, morpholino and 4-N-(R⁶)-piperazinyl together with a nitrogen atom to which R⁴ is bonded; R⁵ is selected from the group consisting of a hydrogen atom, an alkyl group having 1 - 4 carbon atoms, an alkoxy group having 1 - 3 carbon atoms, NR⁷R⁸ and CONR⁷R⁸; R⁶ is selected from the group consisting of a hydrogen atom, an alkyl group having 1 - 6 carbon atoms, (C₁₋₃ alkoxy)-C₂₋₆ alkyl, hydroxy-C₂₋₆ alkyl, (R⁷R⁸N)-C₂₋₆ alkyl, (R⁷R⁸NCO)-C₁₋₆ alkyl, CONR⁷R⁸, CSNR⁷R⁸ and C(NH)NR⁷R⁸; and R⁷ and R⁸ each independently is selected from the group consisting of a hydrogen atom, an alkyl group having 1 - 4 carbon atoms, (C₁₋₃ alkoxy)-C₂₋₄ alkyl and hydroxy-C₂₋₄ alkyl).

A preferred compound is sildenafil.

The eleventh aspect of the present invention is a composition for remedy of erectile dysfunction by intranasal administration wherein the composition contains at least one of 5-[2-ethoxy-5-(4-methylpiperazin-1-ylsulfonyl)phenyl]-1-methyl-3-propyl-6,7-dihydro-1H-pyrazolo[4,3-d]pyrimidin-7-one and salts thereof.

The twelfth aspect of the present invention is a composition for remedy of erectile dysfunction by intranasal administration wherein the composition contains at least one of the below-listed compounds having an inhibitory action to cGMP-PDE and salts or solvates thereof:
1) 2-piperazinyl-6,7-dimethoxyquinazoline derivatives mentioned in the Japanese Laid-Open Patent Publication Sho-52/100,479 (which is herein incorporated by reference) represented by the following formula (XXVI): 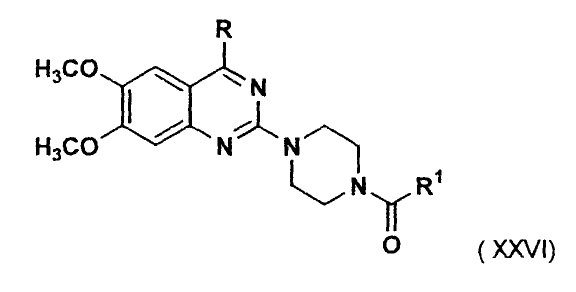 (where R is amino or hydrazino; and R¹ is cycloalkyl or methylcycloalkyl having 3 - 8 cyclic carbon atoms or cycloalkenyl having 4 - 8 cyclic carbon atoms);
2) heterocyclopyrimidine derivatives mentioned in the Japanese Laid-Open Patent Publication Sho-53/103,497 (which is herein incorporated by reference) represented by the following formula (XXVII): 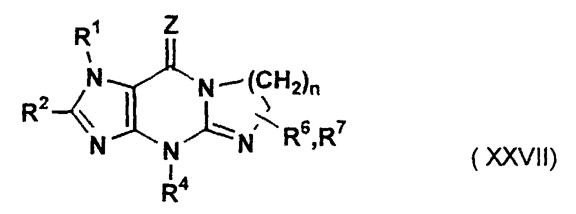 and the following formula (XXVIII): 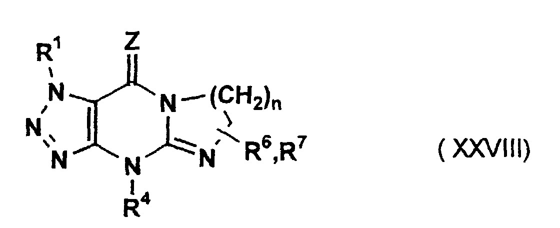 (where R¹ is hydrogen or a group A [where A is lower alkyl or lower alkenyl (each having carbon atoms up to 8), pyridylmethyl, arylalkyl having 7 - 12 carbon atoms, substituted arylalkyl having 7 - 12 carbon atoms, aryloxyalkyl having 8 - 12 carbon atoms or substituted aryloxyalkyl having 8 - 12 carbon atoms (where each of said arylalkyl and aryloxyalkyl has one or two substituents selected from halogen, alkoxy and alkyl and each of said alkoxy and alkyl has carbon atoms of up to 6)]; R² is hydrogen, trifluoromethyl, halogen, azido, cyano, amino, lower alkylamino, di-(lower alkyl)-amino or lower alkyl (where said lower alkyl has carbon atoms of up to 8); R⁴ is hydrogen, lower alkyl or lower alkenine (each having carbon atoms up to 8), pyridylmethyl, lower alkanoyl or lower alkenoyl (each having carbon atoms of up to 8), aroyl having 7 - 10 carbon atoms; substituted aroyl having 7 - 12 carbon atoms, arylalkyl having 7 - 12 carbon atoms, substituted arylalkyl having 7 - 12 carbon atoms, aryloxyalkyl having 8 - 12 carbon atoms or substituted aryloxyalkyl having 8 - 12 carbon atoms (where each of said substituted aroyl, substituted arylalkyl and substituted arloxyalkyl has one or two substituents selected from halogen, alkoxy and alkyl on the ring and each of said alkoxy and alkyl has carbon atoms of up to 6); R⁶ and R⁷ represent substituents bonded to carbon and selected from hydrogen, methyl and ethyl; n is an integer of 1, 2 or 3; and Z is oxo or imino);
3) 5-substituted compounds of pyrazolo[4,3-d]pyrimidin-7-ons mentioned in the Japanese Laid-Open Patent Publication Sho-61/236,778 (which is herein incorporated by reference) represented by the following formula (XXIX): 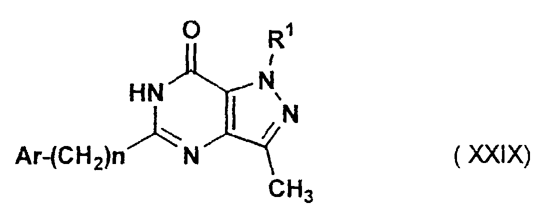 (where R¹ is lower alkyl having 1 - 6 carbon atoms, lower alkylene having 1 - 6 carbon atoms, lower hydroxylalkyl having 1 - 6 carbon atoms, lower hydroxyalkylene having 2 - 6 carbon atoms, lower aminoalkyl having 1 - 6 carbon atoms or lower aminoalkylene having 2 - 6 carbon atoms; n is 0 - 4; and Ar is represented by the following formula (XXX) or 2, 3 or 4-pyridyl 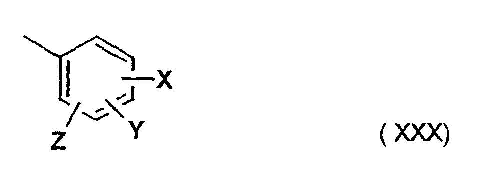 (where X, Y and Z each independently is hydrogen, lower alkyl having 1 - 6 carbon atoms, halogen, hydroxyl, lower alkoxy having 1 - 6 carbon atoms, nitro, amino, NR'R'' (R' and R'' each independently is hydrogen or lower alkyl having 1 - 6 carbon atoms which is optionally substituted by amino, morpholino or cycloalkyl having 5 - 7 carbon atoms), sulfonyl or the following formula (XXXI):

   ―SO₂NR'R'' (XXXI)

   (where R' and R'' are the same as those mentioned already) with a proviso that the case wherein all of X, Y and Z are nitro, amino or NR'R'' is excluded));
4) griseol derivatives mentioned in the Japanese Laid-Open Patent Publication Sho-62/30,796 (which is herein incorporated by reference) represented by the following formula (XXXII): 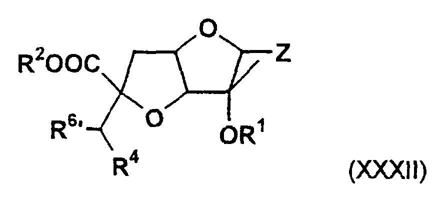 (where R¹ is a protective group for hydroxyl group or hydrogen atom; R² and R³ are same or different and each is a protective group for carboxyl group or hydrogen atom; R⁴ is hydrogen atom or an optionally protected hydroxyl group; and Z is represented by the following formula (XXXIII): 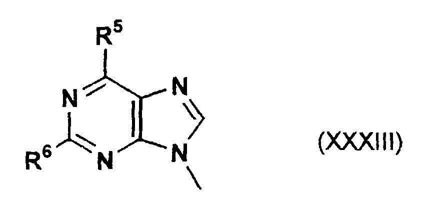 (where R⁵ and R⁶ are same or different and each is hydrogen atom, hydroxyl, optionally alkyl-substituted mercapto, optionally protected amino or halogen with a proviso that, when R⁶ is hydrogen, then R⁴ is hydrogen), represented by the following formula (XXXIV): 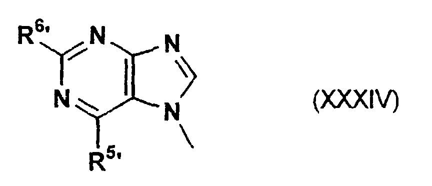 (where R^{5'} and R^{6'} are same or different and each is hydrogen, hydroxyl, optionally alkyl-substituted mercapto, optionally protected amino or halogen) or represented by the following formula (XXXV): 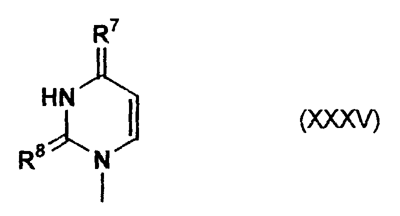 (where R⁷ and R⁸ are same or different and each is oxygen, sulfur or imino)); or grizeol derivatives mentioned in the Japanese Laid-Open Patent Publication Sho-62/30,782 (which is herein incorporated by reference) represented by the following formula (XXXVI): 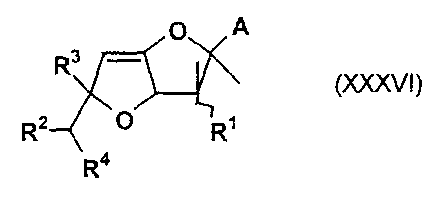 (where R¹ and R² are same or different and each is hydrogen, optionally protected hydroxyl, substituted hydroxyl or halogen; R³ and R⁴ are same or different and each is carbamoyl or optionally protected carboxyl; and A is a group represented by the following formula (XXXVII): 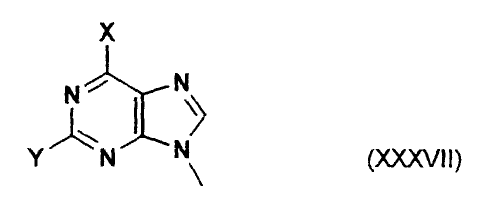 (where X and Y are same or different and each is optionally protected hydroxyl, substituted hydroxyl, optionally protected amino, substituted amino, hydrazino, optionally protected mercapto, optionally alkyl-substituted mercapto, substituted mercapto, or halogen), a group represented by the following formula (XXXVIII): 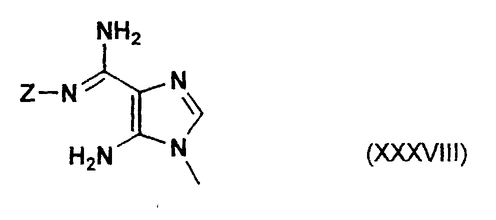 (where Z is hydrogen or optionally protected hydroxyl) or a group represented by the following formula (XXXIX): 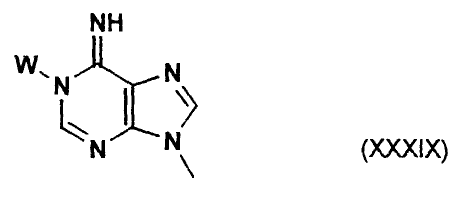 (where W is lower alkyloxy or aralkyloxy or may form an N-oxide together with nitrogen atom));
5) purinone derivatives mentioned in the Japanese Laid-Open Patent Publication Sho-63/196,585 (which is herein incorporated by reference) and represented by the following formula (XL): 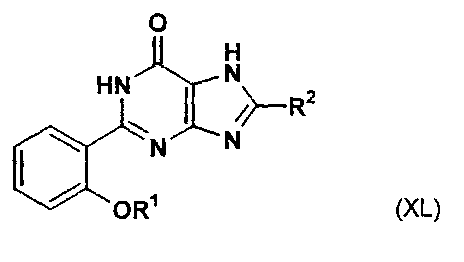 (where R¹ is alkyl having 1 - 6 carbon atoms or alkenyl having 2 - 6 carbon atoms; and R² is hydrogen or hydroxyl); or purinone derivatives mentioned in the Japanese Laid-Open Patent Publication Hei-02/88,577 (which is herein incorporated by reference) represented by the following formula (XLI): 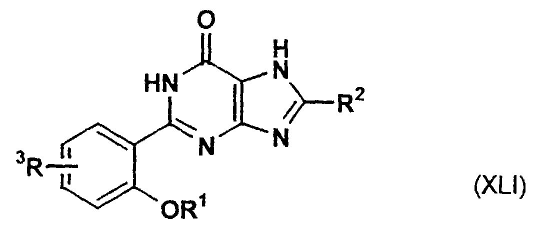 (where R¹ is alkyl having 1 - 6 carbons, alkenyl having 2 - 6 carbons, cycloalkyl(having 3 - 5 carbons)-alkyl (having 1 - 4 carbons), phenyl-alkyl having 1 - 4 carbons or alkyl having 1 - 4 carbons substituted with 1 - 6 fluoro groups; R² is hydrogen, hydroxyl, alkyl having 1 - 4 carbons, phenyl, mercapto, alkylthio having 1 - 4 carbons, CF₃ or amino; R³ is hydrogen, nitro, amino, alkoxy having 1 - 4 carbons, alkyl having 1 - 4 carbons, halogen, SO₂NR⁴R⁵, CONR⁴R⁵, cyano or alkyl-S(O)ₙ having 1 - 4 carbons; R⁴ and R⁵ independently is hydrogen or alkyl having 1 - 4 carbons; and n is 0, 1 or 2; with a proviso that, when R¹ is alkyl having 1 - 6 carbons or alkenyl having 2 - 6 carbons and R² is hydrogen or hydroxyl, then R³ is not hydrogen);
6) KS 506 mentioned in the Japanese Laid-Open Patent Publication Hei-02/1,463 (which is herein incorporated by reference) represented by the following formula (XLII): 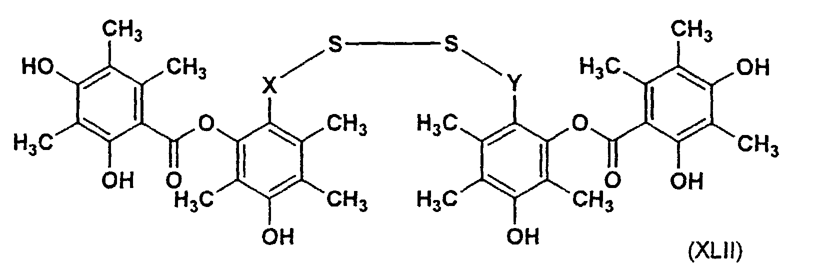 (where X and Y is a single bond or -CO-);
7) phenylpyridone derivatives mentioned in the Japanese Laid-Open Patent Publication Hei-01/311,067 (which is herein incorporated by reference) represented by the following formula (XLIII): 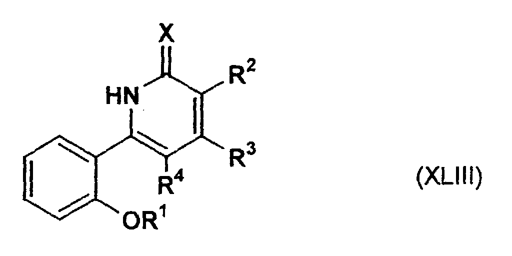 (where X is oxygen or sulfur; R¹ is alkyl having 1 - 6 carbons, alkenyl having 2 - 6 carbons, cycloalkyl(having 3 - 5 carbons)-alkyl(having 1 - 4 carbons) or alkyl having 1 - 4 carbons substituted with 1 - 6 fluoro groups; R² is hydrogen, -CN, -CONR⁵R⁶, -CO₂R⁷, 5-tetrazolyl, -NO₂, -NH₂ or -NHCOR⁸ where R⁵ to R⁸ independently is hydrogen or alkyl having 1 - 4 carbons; R³ is hydrogen or alkyl having 1 - 4 carbons; and R⁴ is hydrogen or alkyl having 1 - 4 carbons; with a proviso that R¹ is not methyl when R² is -CO₂H, -CO₂CH₂CH₃ or -CN; X is oxygen; R³ is hydrogen; and R⁴ is hydrogen or methyl); or phenylpyridone derivatives mentioned in the Japanese Laid-Open Patent Publication Hei-03/145,466 (which is herein incorporated by reference) represented by the following formula (XLIV): 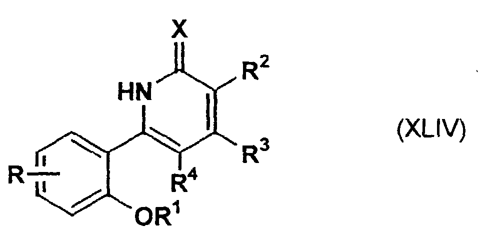 (where X is oxygen or sulfur; R¹ is alkyl having 1 - 6 carbons, alkenyl having 2 - 6 carbons, cycloalkyl(having 3 - 5 carbons)-alkyl(having 1 - 4 carbons) or alkyl having 1 - 4 carbons substituted with 1 - 3 fluoro groups; R² is hydrogen, -CN, -CONR⁵R⁶, -CO₂R⁷, 5-tetrazolyl, -NO₂, -NH₂ or -NHCOR⁸ where R⁵ to R⁸ independently is hydrogen or alkyl having 1 - 4 carbons; R³ is hydrogen or alkyl having 1 - 4 carbons; R⁴ is hydrogen or alkyl having 1 - 4 carbons; and R is halogen, alkyl having 1 - 4 carbons, alkoxy having 1 - 4 carbons, cyano, -CONR⁹R¹⁰, -CO₂R¹¹, S(O)ₙ-alkyl (the alkyl having 1-4 carbons), -NO₂, -NH₂, NHCOR¹² or SO₂NR¹³R¹⁴; where n is 0, 1 or 2 and R⁹ to R¹⁴ independently is hydrogen or alkyl having 1 - 4 carbons; with a proviso that R¹ is not methyl when R² is -CO₂H, -CO₂CH₂CH₃ or -CN; X is oxygen; R³ is hydrogen; R⁴ is hydrogen or methyl; and R⁶ is 6-methoxy);
8) 1,4-dihydropyridine derivatives mentioned in the Japanese Laid-Open Patent Publication Hei-02/223,580 (which is herein incorporated by reference) represented by the following formula (XLV): 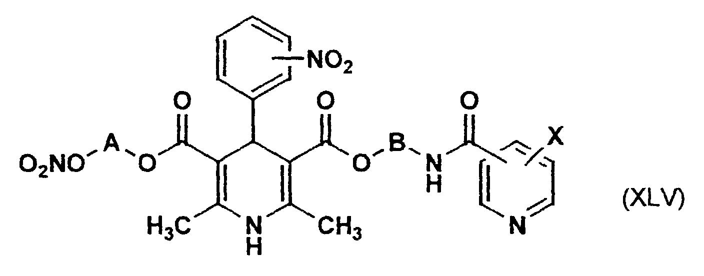 (where X is hydrogen or alkoxy having 1 - 4 carbons; and A and B are same or different and each is alkylene having 1 - 4 carbons);
9) condensed pyrimidine derivatives mentioned in the Japanese Laid-Open Patent Publication Hei-02/42,079 (which is herein incorporated by reference) represented by the following formula (XLVI): 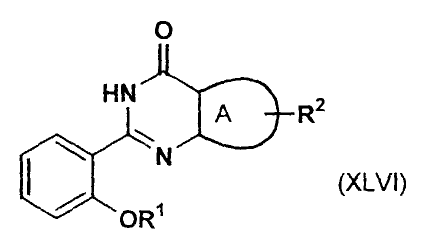 (where the following formula (XLVII): 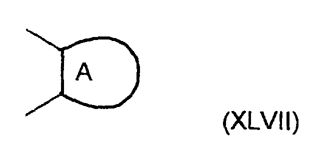 is a ring represented by the following formula (XLVIII): 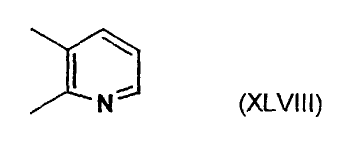 the following formula (XLIX): 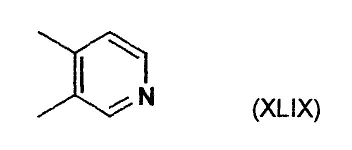 the following formula (L): 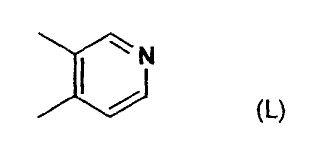 the following formula (LI): 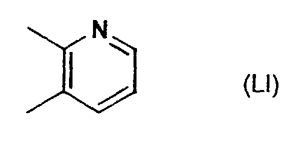 the following formula (LII): 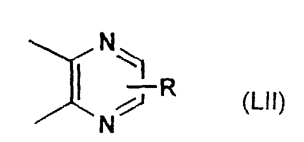 the following formula (LIII): 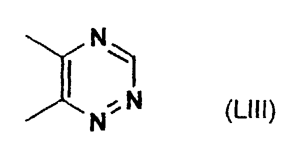 or the following formula (LIV): 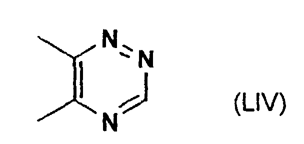 (where R¹ is alkyl having 1 - 6 carbon atoms, alkenyl having 2 - 6 carbons, cycloalkyl(having 3 - 5 carbons)-alkyl(having 1 - 6 carbons) or alkyl having 1 - 6 carbons substituted with 1 - 6 fluoro groups; R² is alkylthio having 1 - 6 carbons, alkylsulfonyl having 1 - 6 carbons, alkoxy having 1 - 6 carbons, hydroxyl, hydrogen, hydrazino, alkyl having 1 - 6 carbons, phenyl, -NHCOR³ or -NR⁴R⁵; R³ is hydrogen or alkyl having 1 - 6 carbons; R⁴ and R⁵ form a pyrrolidino, piperidino, hexahydroazepino, morpholino or pyrazino ring together with the nitrogen atoms bonded thereto or R⁴ and R⁵ each independently is hydrogen or cycloalkyl having 3 - 5 carbons or alkyl having 1 - 6 carbons which may be substituted with -CF₃, phenyl, -S(O)ₙ-alkyl (having 1-6 carbons) (where n is 0, 1 or 2), -OR⁶, -CO₂R⁷ or -NR⁸R⁹ (with a proviso that the carbon atom adjacent to the nitrogen atom is not substituted with -S(O)ₙ-alkyl (having 1-6 carbons), -OR⁶, or -NR⁸R⁹; R⁸ and R⁹ each independently may be hydrogen or alkyl having 1 - 6 carbons; and R is hydrogen or may be hydroxyl when R² is hydroxyl); or condensed pyrimidine derivatives mentioned in the Japanese Laid-Open Patent Publication Hei-02/56,484 (which is herein incorporated by reference) represented by the following formula (LV): 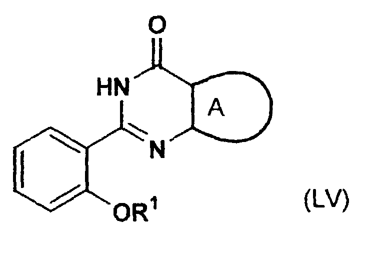 (where the following formula (LVI): 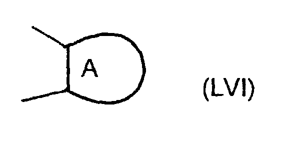 is a ring represented by the following formula (LVII): 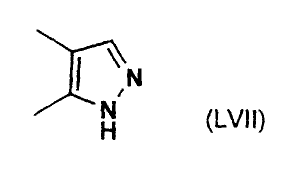 the following formula (LVIII): 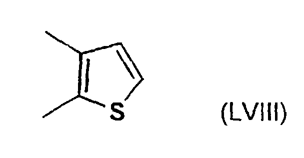 or the following formula (LIX): 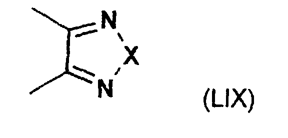 where X is oxygen or sulfur; and R¹ is alkyl having 1 - 6 carbons, alkenyl having 2 - 6 carbons, cycloalkyl(having 3 - 5 carbons)-alkyl(having 1 - 4 carbons) or alkyl having 1 - 4 carbons substituted with 1 - 6 fluoro groups);
10) pyrimidopyrimidine derivatives mentioned in the Japanese Laid-Open Patent Publication Hei-02/40,388 represented by the following formula (LX): 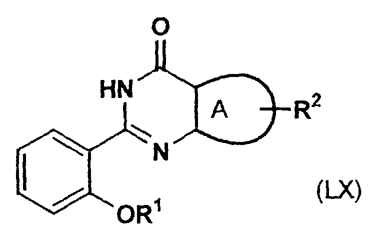 (where R¹ is alkyl having 1 - 6 carbons, alkenyl having 2 - 6 carbons, cycloalkyl(having 3 - 5 carbons)-alkyl(having 1 - 4 carbons) or alkyl having 1 - 6 carbons substituted with 1 - 6 fluoro groups; R² is alkylthio having 1 - 6 carbons, alkylsulfonyl having 1 - 6 carbons, alkoxy having 1 - 6 carbons, hydroxyl, hydrogen, hydrazino, alkyl having 1 - 6 carbons, phenyl, -NHCOR³ (R³ is hydrogen or alkyl having 1 - 6 carbons) or -NR⁴R⁵ (R⁴ and R⁵ may form a pyrrolidino, piperidino, hexahydroazepino, morpholino or pyrazino ring together with a nitrogen atom bonded thereto or they each independently is hydrogen or cycloalkyl having 3 - 5 carbons or alkyl having 1 - 6 carbons which is optionally substituted with -CF₃, phenyl, -S(O)ₙ-alkyl(having 1 - 6 carbons); where n is 0, 1 or 2, -OR⁶, -CO₂R⁷ or -NR⁸R⁹ (where R⁶ to R⁹ each independently is hydrogen or alkyl having 1 - 6 carbons) (with a proviso that the carbon atom adjacent to the nitrogen atom is not substituted with -S(O)ₙ-alkyl (having 1 - 6 carbons), -OR⁶ or -NR⁸R⁹); and the following formula (LXI): 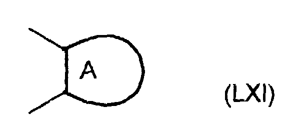 is a ring represented by the following formula (LXII): 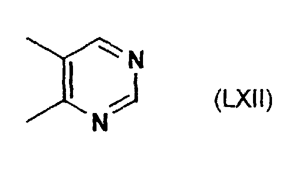 or the following formula (LXIII): 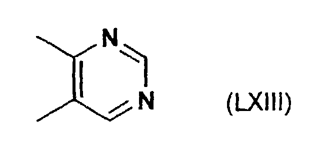 or pyrimidopyrimidine derivatives mentioned in the Japanese Laid-Open Patent Publication Hei-03/261,785 (which is herein incorporated by reference) represented by the following formula (LXIV): 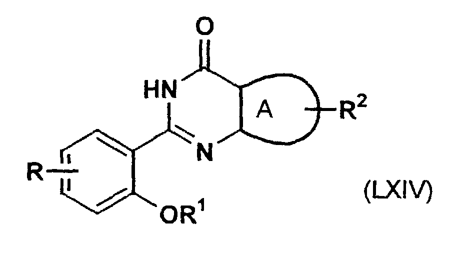 (where R¹ is alkyl having 1 - 6 carbons, alkenyl having 2 - 6 carbons, cycloalkyl(having 3 - 5 carbons)-alkyl(having 1 - 6 carbons) or alkyl having 1 - 6 carbons substituted with 1 - 6 fluorines; R² is alkylthio having 1 - 6 carbons, alkylsulfonyl having 1 - 6 carbons, alkoxy having 1 - 6 carbons, hydroxyl, hydrogen, hydrazino, alkyl having 1 - 6 carbons, phenyl, -NHCOR³ (R³ is hydrogen or alkyl having 1 - 6 carbons) or -NR⁴R⁵ (R⁴ and R⁵ may form a pyrrolidino, piperidino, hexahydroazepino, morpholino or pyrazino ring together with a nitrogen atom bonded thereto or they each independently is hydrogen, cycloalkyl having 3 - 5 carbons or alkyl having 1 - 6 carbons which is optionally substituted with -CF₃, phenyl, -S(O)ₙ-alkyl(having 1 - 6 carbons) (where n is 0, 1 or 2), -OR⁶, -CO₂R⁷ or -NR⁸R⁹ (where R⁶ to R⁹ each independently is hydrogen or alkyl having 1 - 6 carbons) (with a proviso that the carbon atom adjacent to the nitrogen atom is not substituted with -S(O)ₙ-alkyl(having 1 - 6 carbons), -OR⁶ or -NR⁸R⁹); R is halogen, alkyl having 1 - 4 carbons, alkoxy having 1 - 4 carbons, cyano, -CONR¹⁰R¹¹, CO₂R¹², alkyl (having 1 - 4 carbons)-S(O)ₙ, -NO₂, -NH₂, -NHCOR¹³ or SO₂NR¹⁴R¹⁵ (where n is 0, 1 or 2 and R¹⁰ to R¹⁵ each independently is hydrogen or alkyl having 1 - s4 carbons); and the following formula (LXV): 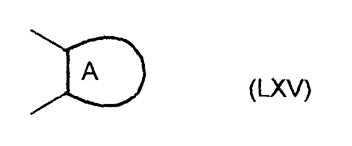 is a ring represented by the following formula (LXVI): 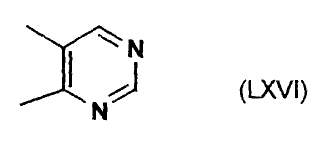 or the following formula (LXVII): 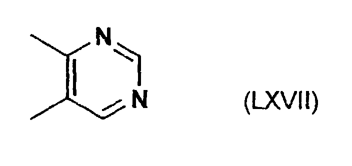
11) quinazolinone derivatives mentioned in the Japanese Laid-Open Patent Publication Hei-02/193,983 represented by the following formula (LXVIII): 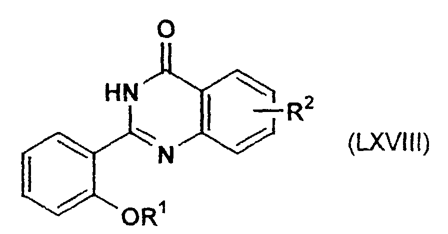 (where R¹ is alkyl having 1 - 6 carbon atoms, alkenyl having 2 - 6 carbon atoms, cycloalkyl(having 3 - 5 carbon atoms)-alkyl (having 1 - 4 carbon atoms), phenyl-alkyl (having 1 - 4 carbon atoms) or alkyl having 1 - 4 carbon atoms substituted with 1 - 6 fluoro groups; and R² is hydrogen, alkyl having 1 - 6 carbon atoms, alkylthio having 1 - 6 carbon atoms, alkoxy having 1 - 6 carbon atoms, nitro, or -NR³R⁴ where R³ and R⁴ each independently is hydrogen or alkyl having 1 - 4 carbons which is optionally substituted with hydroxyl (with a proviso that the carbon atom adjacent to the nitrogen atom is not substituted with hydroxyl although, when R² is hydrogen, then R¹ is neither methyl nor ethyl); or quinazolinone derivatives mentioned in the International Publication WO 93/12,095 (which is herein incorporated by reference) represented by the following formula (LXIX): 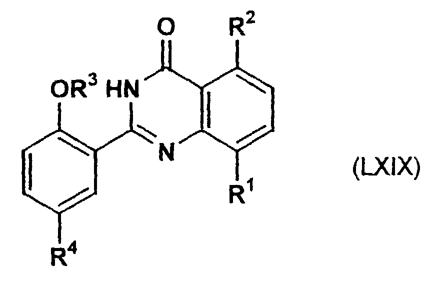 (where R¹ is hydrogen, alkyl having 1-4 carbon atoms, alkoxy having 1-6 carbon atoms or CONR⁵R⁶; R² is hydrogen or alkyl having 1-4 carbons; R³ is alkyl having 1-4 carbons; R⁴ is hydrogen, alkanoyl having 2-4 carbons optionally substituted with NR⁷R⁸, (hydroxy)alkyl (having 2-4 carbons) optionally substituted with NR⁷R⁸, CH=CHCO₂R⁹, CH=CHCONR⁷R⁸, CH₂CH₂CO₂R⁹, CH₂CH₂CONR⁷R⁸, SO₂NR⁷R⁸, SO₂NH(CH₂)ₙNR⁷R⁸ or imidazolyl; R⁵ and R⁶ each independently is hydrogen or alkyl having 1-4 carbons; R⁷ and R⁸ each independently is hydrogen or alkyl having 1-4 carbons or both may form a pyrrolidino, piperidino, morpholino or 4-(NR¹⁰)-1-piperazinyl ring (all of those rings may optionally be substituted with CONR⁵R⁶) together with a nitrogen atom to which they are bonded); R⁹ is hydrogen or alkyl having 1-4 carbons; R¹⁰ is hydrogen, alkyl having 1-4 carbons or (hydroxy)alkyl having 2-4 carbons ; n is 2, 3 or 4; and with a proviso that R² is not hydrogen when R¹ is hydrogen, alkyl having 1-4 carbons or alkoxy having 1-6 alkoxy);
12) phenylpyrimidinone derivatives mentioned in the Japanese Laid-Open Patent Publication Hei-02/295,977 (which is herein incorporated by reference) represented by the following formula (LXX): 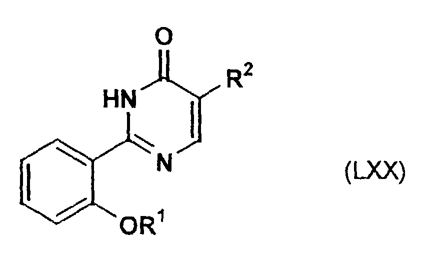 (where R¹ is alkyl having 1-6 carbons, alkenyl having 2-6 carbons, cycloalkyl(having 3-5 carbons)-alkyl(having 1-6 carbons), phenyl-alkyl(having 1-6 carbons) or alkyl having 1-6 carbons substituted with 1-6 fluoro groups; R² is hydrogen, amino, -NHCOR³ or -CONR⁴R⁵; R³ is alkyl having 1-6 carbons; R⁴ is alkyl having 1-6 carbons; and R⁵ is hydrogen or alkyl having 1-6 carbons); or phenylpyrimidinone derivatives mentioned in the Japanese Laid-Open Patent Publication Hei-02/295,978 (which is herein incorporated by reference) represented by the following formula (LXXI): 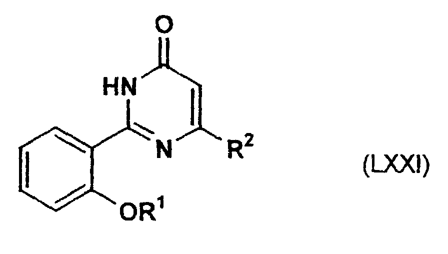 (where R¹ is alkyl having 1-6 carbon atoms, alkenyl having 2-6 carbon atoms, cycloalkyl(having 3-5 carbons)-alkyl (having 1-6 carbons), phenyl-alkyl(having 1-6 carbons) or alkyl having 1-6 carbons substituted with 1-6 fluoro groups; R² is alkyl having 1-6 carbons, phenyl, hydroxy, alkoxy having 1-6 carbons, halogen, -NHCOR³, -NHCONHR⁴⁵-tetrazolyl, -CO₂R⁵, cyano, -CONR⁷R⁸ or -NR⁸R⁹; and R⁸ and R⁹ each independently is hydrogen or alkyl having 1-6 carbons which is optionally substituted with hydroxy; with a proviso that the carbon atom adjacent to the nitrogen atom is not substituted with hydroxy);
13) polycyclic guanine derivatives mentioned in the International Publication WO 91/19,717 (which is herein incorporated by reference) represented by the following formula (LXXII): 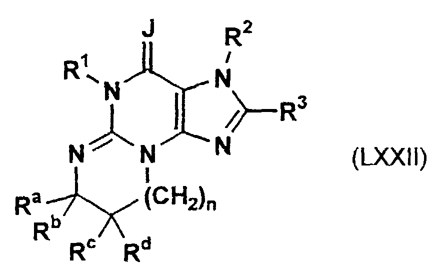 and the following formula (LXXIII): 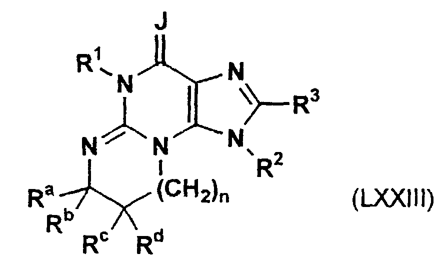 (where J is oxygen or sulfur; R¹ is alkyl or aryl- or hydroxy-substituted alkyl; R² is hydrogen, aryl, heteroaryl, cycloalkyl, alkyl, alkyl which is substituted with arylheteroaryl, hydroxy, alkoxy, amino, monoalkylamino or dialkylamino or -(CH₂)ₘTCOR²⁰ (where m is an integer of 1-6; T is oxygen or -NH-; R²⁰ is hydrogen, aryl, heteroaryl, alkyl or alkyl which is substituted with aryl or heteroaryl); R³ is hydrogen, halo, trifluoromethyl, alkoxy, alkylthio, alkyl, cycloalkyl, aryl, aminosulfonyl, amino, monoalkylamino, dialkylamino, hydroxyalkylamino, aminoalkylamino, carboxy, alkoxycarbonyl or aminocarbonyl or alkyl which is substituted with aryl, hydroxy, alkoxy, amino, monoalkylamino or dialkylamino; R^{a}, R^{b}, R^{c} and R^{d} each independently is hydrogen, alkyl, cycloalkyl or aryl, or (R^{a} and R^{b}) or (R^{c} and R^{d}) or (R^{b} and R^{c}) may form a saturated ring having 5-7 carbon atoms or each of (R^{a} and R^{b}) together and (R^{b} and R^{c}) together may form a saturated ring having 5-7 carbon atoms; with a proviso that each of the rings may optionally contain sulfur or oxygen atom and, in addition, said carbon atom may be substituted with one or more alkenyl, alkynyl, hydroxy, carboxy, alkoxycarbonyl, alkyl or alkyl which is substituted with hydroxycarboxy or alkoxycarbonyl; or such a saturated ring may have two adjacent carbon atoms together with the adjacent aryl ring; and n is 0 or 1);
14) pyrazolopyrimidinone derivatives mentioned in the International Publication WO 93/06,104 (which is herein incorporated by reference) represented by the following formula (LXXIV): 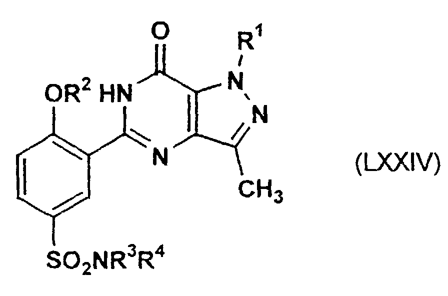 (where R¹ is methyl or ethyl; R² is ethyl or n-propyl; R³ and R⁴ each independently is hydrogen or alkyl having 1-6 carbons which may be substituted with cycloalkyl having 5-7 carbons or morpholino); or pyrazolopyrimidinone derivatives mentioned in the International Publication WO 93/07,149 (which is herein incorporated by reference) represented by the following formula (LXXV): 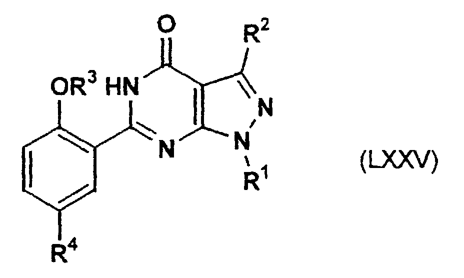 (where R¹ is alkyl having 1-6 carbons; R² is hydrogen, methyl or ethyl; R³ is alkyl having 2-4 carbons; R⁴ is alkyl having 1-4 carbons which may be substituted with NR⁵R⁶, CN, CONR⁵R⁶ or CO₂R⁷, alkenyl having 2-4 carbons which may be substituted with CN, CONR⁵R⁶ or CO₂R⁷, alkanoyl having 2-4 carbons which may be substituted with NR⁵R⁶, or SO₂NR⁵R⁶, CONR⁵R⁶, CO₂R⁷ or halo; R⁵ and R⁶ each independently is hydrogen or alkyl having 1-4 carbons or may form, together with a nitrogen atom bonded thereto, pyrrolidino, piperidino, morpholino, 4-(NR⁸)-1-piperazinyl or 1-imidazolyl which is optionally substituted with one or two alkyls having 1-4 carbons; R⁷ is hydrogen or alkyl having 1-4 carbons; and R⁸ is hydrogen, alkyl having 1-3 carbons or alkyl having 2-3 carbons);
15) nitrogen-containing heterocyclic compounds mentioned in the International Publication WO 93/07,124 (which is herein incorporated by reference) represented by the following formula (LXXVI): 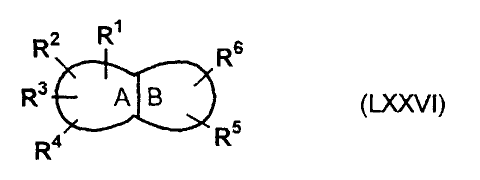 (where ring A is benzene ring, pyridine ring or cyclohexane; ring B is pyridine ring, pyrimidine ring or imidazole ring; where ring A and ring B hold two atoms in common and said commonly-held atoms may be either carbon or nitrogen; the ring A is represented by the following formula (LXXVII): 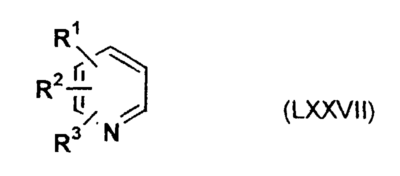 except the case where the nitrogen atom of a pyridine ring is held in common by the ring B when the ring A is a pyridine ring; R¹, R², R³ and R⁴ are same or different and each is hydrogen, halogen, lower alkyl which may be substituted with halogen, optionally substituted cycloalkyl, lower alkoxy, hydroxyalkyl, nitro, cyano, acylamino, optionally protected carboxyl, a group represented by the following formula (LXXVIII): 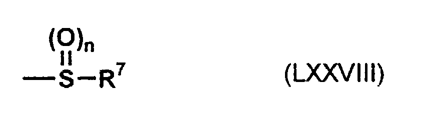 (where R⁷ is lower alkyl; and n is 0 or an integer of 1-2) or a group represented by the following formula (LXXIX): 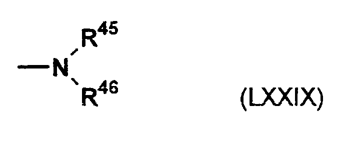 (where R⁴⁵ and R⁴⁶ are same or different and each is hydrogen or lower alkyl; two of R¹, R², R³ and R⁴ may form a methylenedioxy, ethylenedioxy or phenyl ring; R⁵ is hydrogen, halogen, hydroxy, hydrazino, lower alkyl, optionally substituted cycloalkyl, lower alkoxy, lower alkenyl, optionally protected carboxyalkyl, optionally protected carboxyalkenyl, hydroxyalkyl, optionally protected carboxyl, a group represented by the following formula (LXXX): 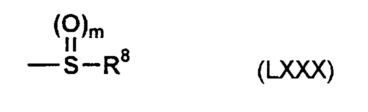 (where R⁸ is lower alkyl; and m is 0 or an integer of 1-2), a group represented by -OR⁹ (where R⁹ is optionally protected hydroxyalkyl, optionally protected carboxyalkyl or optionally substituted benzyl), a group represented by the following formula (LXXXI): 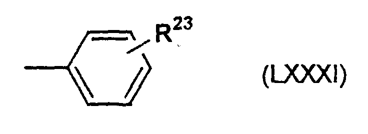 (where R²³ is hydroxyl, lower alkyl, lower alkoxy, hydroxyalkyl or hydroxyalkyloxy), optionally substituted heteroaryl, optionally substituted 1,3-benzdioxyl, optionally substituted 1,4-benzdioxyl, optionally substituted 1,3-benzdioxylylalkyl, optionally substituted 1,4-benzdioxylylalkyl, a group represented by the formula -C(R²⁴)=X (where X is oxygen, sulfur or a group of the formula =NR¹⁰ (where R¹⁰ is hydroxyl, cyano or optionally protected carboxyalkyloxy) and R²⁴ is hydrogen or lower alkyl), or a group represented by the formula -NR¹¹R¹² (where R¹¹ and R¹² are same or different and each is hydrogen, lower alkyl, hydroxyalkyl, aminoalkyl, optionally protected carboxyalkyl, alkylcarbamoyl, optionally substituted heteroarylalkyl, 1,3-benzoxolylalkyl or 1,4-benzdioxylylalkyl and said R¹¹ and R¹² may also form, together with a nitrogen atom bonded thereto, a ring containing other nitrogen or oxygen wherein said ring may be substituted; R⁶ is hydrogen, halogen, hydroxyl, amino, lower alkyl, lower alkoxy, lower alkenyl, 1,3-benzdioxolylalkyloxy or 1,4-benzdioxylalkyloxy, optionally substituted phenylalkyloxy, a group represented by the following formula (LXXXII): 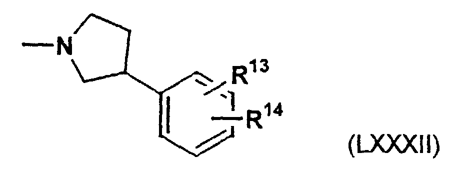 (where R¹³ and R¹⁴ are same or different and each is hydrogen, lower alkyl or lower alkoxy or R¹³ and R¹⁴ may form methylenedioxy or ethylenedioxy together), a group represented by the following formula (LXXXIII): 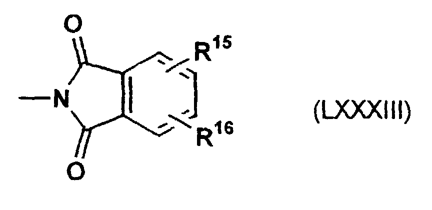 a group represented by the following formula (LXXXIV): 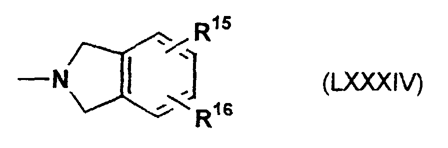 a group represented by the following formula (LXXXV): 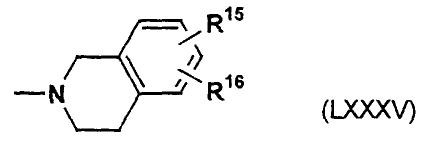 a group represented by the following formula (LXXXVI): 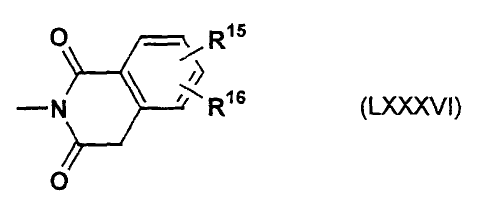 (where R¹⁵ and R¹⁶ are same or different and each is hydrogen, lower alkyl or lower alkoxy or R¹⁵ and R¹⁶ may form methylenedioxy or ethylenedioxy together), piperidine-4-spiro-2-dioxan-1-yl, a group represented by the following formula (LXXXVII): 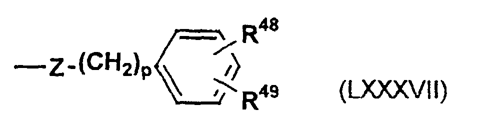 (where R⁴⁸ and R⁴⁹ are same or different and each is hydrogen, lower alkyl or lower alkoxy; R⁴⁸ and R⁴⁹ may form methylenedioxy or ethylenedioxy together; and Z is sulfur or oxygen), a group represented by the following formula (LXXXVIII): 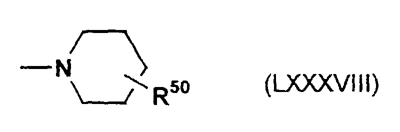 (where R⁵⁰ is hydroxyl, halogen, lower alkyl, lower alkoxy, optionally protected carboxyl, cyano, hydroxyalkyl or carboxyalkyl), a group represented by the following (LXXXIX): 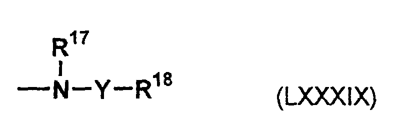 (where R¹⁷ is hydrogen, lower alkyl, acyl, lower alkoxyalkyl, optionally protected carboxyalkyl or hydroxyalkyl); Y is -(CH₂)_{q}- (where q is 0 or an integer of 1-8) or -CO-); when q is an integer of 1-8 in a group of the formula -(CH₂)_{q}-, each of the carbons may have 1-2 substituent(s); R¹⁸ is hydrogen, hydroxyl, optionally protected carboxyl, cyano, acyl, optionally substituted heteroaryl or optionally substituted cycloalkyl); or represented by the following formula (XC): 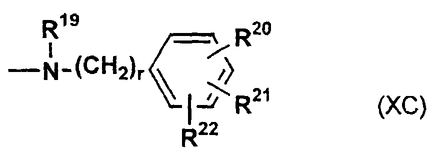 (where R¹⁹ is hydrogen, lower alkyl, lower alkoxyalkyl or hydroxyalkyl; R²⁰, R²¹ and R²² are same or different and each is hydrogen, halogen, hydroxyl, amino, nitro, lower alkyl, lower alkoxy, lower alkoxyalkyl, lower alkenyl, acyl, acylamino, alkylsulfonylamino, hydroxyiminoalkyl, alkyloxycarbonylamino, alkyloxycarbonyloxy or optionally substituted heteroaryl; two of R²⁰, R²¹ and R²² may together form a saturated or unsaturated ring which may contain nitrogen, sulfur or oxygen; and r is 0 or an integer of 1-8); or nitrogen-containing heterocylic compound mentioned in the Japanese Laid-Open Patent Publication Hei-07/10,843 (which is herein incorporated by reference) represented by the following formula (XCI): 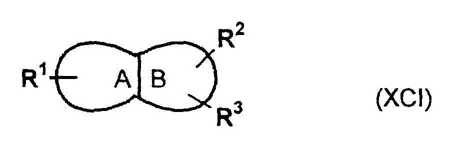 (where ring A is benzene ring, pyridine ring or cyclohexane ring; ring B is pyridine ring or imidazole ring; ring A and ring B hold two atoms in common and said commonly-held atoms may be carbon or nitrogen; R¹ is a group represented by the formula -NR⁴R⁵ (where R⁴ and R⁵ are same or different and each is hydrogen, lower alkyl, acyl or optionally protected carboxyl; and R⁴ and R⁵ may form a ring together with a nitrogen bonded thereto where said ring may be substituted) or a heteroaryl group which may have substituent containing 1-2 nitrogen atoms; R² is hydrogen, a group represented by the formula (XCII): 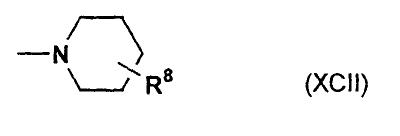 (where R⁸ is an optionally protected carboxyl or optionally protected tetrazolyl) or halogen; R³ is hydrogen or a group of the formula (XCIII): 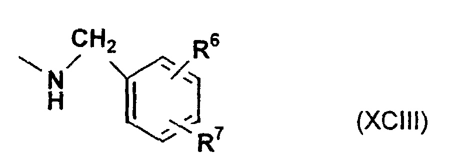 (where R⁶ and R⁷ each is hydrogen, halogen or lower alkoxy or R⁶ and R⁷ may form methylenedioxane or ethylenedioxane together);
16) benzimidazole derivatives mentioned in the Japanese Laid-Open Patent Publication Hei-05/222,000 (which is herein incorporated by reference) represented by the following formula (XCIV): 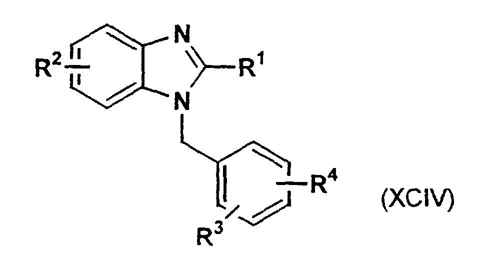 (where R¹ is lower alkyl or cyclo(lower)alkyl; R² is carboxyl, esterified carboxy or amidated carboxyl; and R³ and R⁴ each is hydrogen, halogen, carboxy, esterified carboxy or lower alkyl which may have 1-3 halogens;
17) WS 63967 mentioned in the Japanese Laid-Open Patent Publication Hei-05/301,857 (which is herein incorporated by reference) represented by the formula (XCV): 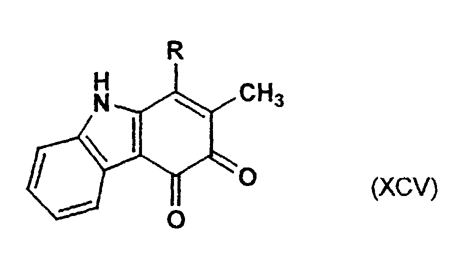 (where R is n-pentyl, 3-methyl-n-pentyl, n-hexyl, 5-methyl-n-hexyl, n-heptyl or 5-methyl-n-heptyl);
18) 4-aminoquinazoline derivatives mentioned in the Japanese Laid-Open Patent Publication Hei-06/192,235 or Hei-08/99,962, or U.S. Patent No. 5,436,233 or No. 5,439,895 (which is herein incorporated by reference) represented by the following formula (XCVI): 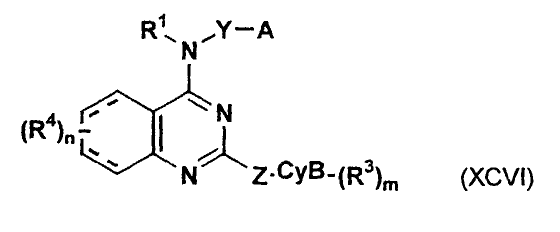 (where R¹ is hydrogen or alkyl having 1-4 carbons; Y is a single bond or alkylene having 1-6 carbons; A is (1) -CyA-(R²)l, (2) -O-R⁰ or -S(O)ₚR⁰ or (3) -NR¹⁶R¹⁷ (where R⁰ is hydrogen, C 1-4 alkyl, hydroxy C 1-4 alkyl or -CyA-(R²)l; CyA is (1) 3- to 7-membered monocyclic saturated or unsaturated carbon ring, (2) 4- to 7-membered monocyclic unsaturated or partially saturated heterocyclic ring having one nitrogen atom, (3) 4- to 7-membered monocyclic unsaturated or partially saturated hetero ring containing one nitrogen atom and one oxygen atom, (4) 4- to 7-membered monocyclic unsaturated or partially saturated hetero ring containing one nitrogen atom and two oxygen atoms, (5) 4- to 7-membered monocyclic unsaturated or partially saturated hetero ring containing two nitrogen atoms and one oxygen atom, (6) 4- to 7-membered monocyclic unsaturated or partially saturated hetero ring containing one or two sulfur atoms or (7) 4- to 7-membered monocyclic unsaturated, partially saturated or saturated hetero ring containing one or two oxygen atoms; R² is (1) hydrogen, (2) C 1-4 alkyl, (3) C 1-4 alkoxy, (4) -COOR⁵ (R⁵ is hydrogen or C 1-4 alkyl), (5) -NR⁶R⁷ (R⁶ and R⁷ each independently is hydrogen or c 1-4 alkyl), (6) -SO₂NR⁶R⁷ (where R⁶ and R⁷ have the same meanings as defined above), (7) halogen, (8) trifluoromethyl, (9) nitro or (10) trifluromethoxy; Z is a single bond, methylene, ethylene, vinylene or ethynylene; CyB is (1) 4- to 7-membered monocyclic unsaturated or partially saturated hetero ring containing one nitrogen atom, (2) 4- to 7-membered monocyclic unsaturated or partially saturated hetero ring containing two nitrogen atoms, (3) 4- to 7-membered monocyclic unsaturated or partially saturated hetero ring containing three nitrogen atoms or (4) 4- to 7-membered monocyclic unsaturated or partially saturated hetero ring containing one or two oxygen atoms; R³ is hydrogen, C 1-4 alkyl, C 1-4 alkoxy, halogen or trufluoromethyl; R⁴ is (1) hydrogen, (2) C 1-4 alkyl, (3) C 1-4 alkoxy, (4) -COOR⁸ (where R⁸ is hydrogen or C 1-4 alkyl ), (5) -NR⁹R¹⁰ (where R⁹ is hydrogen, C 1-4 alkyl or phenyl-(C 1-4 alkyl) and R¹⁰ is hydrogen or C 1-4 alkyl), (6) -NHCOR¹¹ (where R¹¹ is C 1-4 alkyl), (7) -NHSO₂R¹¹ (R¹¹ has the same meaning as defined above), (8) -SO₂NR⁹R¹⁰ (R⁹ and R¹⁰ have the same meanings as defined above), (9) -OCOR¹¹ (R¹¹ has the same meaning as defined above), (10) halogen, (11) trifluoromethyl, (12) hydroxy, (13) nitro, (14) cyano, (15) -SO₂N=CHNR¹²R¹³ (R¹² is hydrogen or C 1-4 alkyl and R¹³ is C 1-4 alkyl), (16) -CONR¹⁴R¹⁵ (R¹⁴ is hydrogen or C 1-4 alkyl and R¹⁵ is C 1-4 alkyl or phenyl(C 1-4 alkyl)), (17) C 1-4 alkylthio , (18) C 1-4 alkylsulfinyl, (19) C 1-4 alkylsulfonyl, (20) ethynyl, (21) hydroxymethyl, (22) tri(C 1-4 alkyl)silylethynyl or (23) acetyl; l, m and n each independently is 1 or 2; with a proviso that (1) when Y is a single bond, -CyA-(R²)l is neither cyclopentyl nor trifluorophenyl, (2) when Z is vinylene or ethynylene, CyB is not bonded to Z via a nitrogen atom, (3) when CyA is CyA-(7) (4- to 7-membered monocyclic unsaturated, partially saturated or saturated hetero ring containing one or two oxygen atoms), CyB is neither pyridine ring nor thiophene ring and (4) when A is (2)-O-R⁰ or -S(O)ₚ-R⁰ or (3)-NR¹⁶R¹⁷, Y is not a single bond); or aminoquinazoline derivatives mentioned in the Japanese Laid-Open Patent Publication Hei-07/188,214 (which is herein incorporated by reference) represented by the following formula (XCVII): 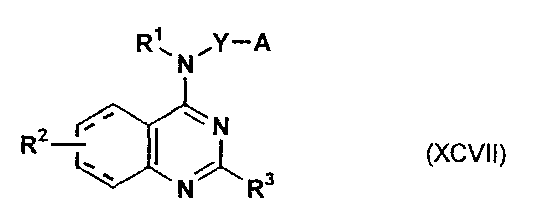 (where R¹ is hydrogen or C 1-4 alkyl; R² is C 1-4 alkyl - ethynyl; R³ is imidazolyl or pyridyl; Y is C 2-6 alkylene; A is (1) -O-R⁴-OH or (2) -S-R⁴-OH (where R⁴ is C 2-6 alkylene), and the following formula (XCVIII): 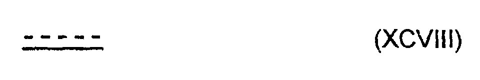 represents a simgle bond or a double bond);
19) purin-6-ones mentioned in the International Publication WO 94/00,453 (which is herein incorporated by reference) represented by the following formula (XCIX): 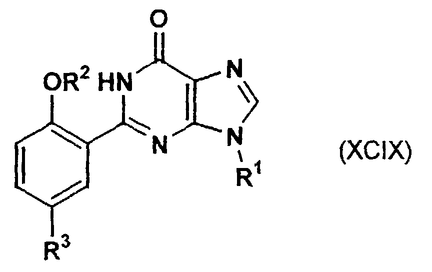 (where R¹ is C 1-4 alkyl; R² is C 2-4 alkyl; R³ is H or SO₂NR⁴R⁵); R⁴ and R⁵ form pyrrolidino, piperidino, morpholino or 4-N-(R⁶)-1-piperazinyl group together with a nitrogen bonded thereto; and R⁶ is H or C 1-3 alkyl); or purin-6-ones mentioned in the Japanese Laid-Open Patent Publication Hei-09/124,648 (which is herein incorporated by reference) represented by the following formula (C): 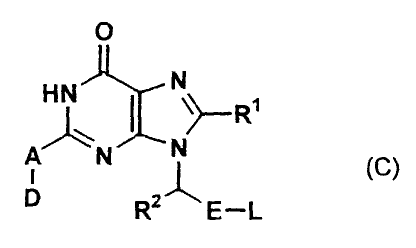 (where R¹ is hydrogen or straight or branched alkyl having up to 8 carbon atoms; R² is straight or branched acyl having up to 4 carbon atoms or straight or branched alkyl having up to 8 carbon atoms which may be substituted with hydroxyl, azido or a group of the formula -NR³R⁴ or -OSO₂R⁵ where R³ and R⁴ may be same or different and each is cycloalkyl having 3-6 carbons, hydrogen, formyl or straight or branched alkyl having up to 6 carbons which may be substituted with hydroxyl, carbonyl, straight or branched alkoxy or alkoxycarbonyl having up to 6 carbons or a group of the formula -(CO)ₐ-NR⁶R⁷ where a is 0 or 1; R⁶ and R⁷ are same or different and each is hydrogen, formyl, hydroxyl, phenyl or straight or branched alkyl having up to 6 carbons which may be substituted with hydroxyl or straight or branched alkoxy having up to 5 carbons; R³ and/or R⁴ are/is straight or branched alkoxycarbonyl having up to 6 carbons, carboxyl or straight or branched acyl having up to 6 carbons which may be substituted with hydroxyl or straight or branched alkoxy having up to 4 carbons; or R³ and/or R⁴ are/is a group of the formula -(CO)_{b}-T-NR⁸R⁹, -CO₂R¹¹ or -SO₂NR¹²R¹³ where b has the same meaning as the above "a" and may be same therewith or different therefrom; T is straight or branched alkyl having up to 5 carbons or, when b is not 0, it may be a linkage; R⁸ and R⁹ have the same meanings as the above R⁶ and R⁷ and may be same therewith or different therefrom; R¹⁰ is 5- to 7-membered saturated, partially unsaturated or unsaturated hetero ring containing up to three hetero atoms selected from a group of S, N and/or O which may be substituted, via said N functional group, with straight or branched alkyl, alkoxy or alkoxycarbonyl (each having up to 4 carbons), carboxyl, benzyloxycarbonyl or hydroxyl; R¹¹ is straight or branched alkyl having up to 5 carbons, benzyl or phenyl; R¹² and R¹³ may be same or different and each is hydrogen, phenyl or straight or branched alkyl having up to 6 carbons or R³ and R⁴ may contain, together with nitrogen, up to 3 hetero atom from a group of N, S and/or O or a group -NR¹⁴, forming 5- or 6-membered saturated, partially unsaturated or unsaturated hetero ring which, in some cases, may be substituted with carbonyl, with straight or branched alkoxycarbonyl having up to 5 carbons or with straight or branched alkyl having up to 5 carbons and a part thereof may be substituted with hydroxyl, carboxyl or straight or branched acyl, alkoxy or alkoxycarbonyl (each having up to 6 carbons) where R¹⁴ is hydrogen, carbonyl or straight or branched alkoxycarbonyl having up to 5 carbons; R⁵ is phenyl or straight or branched alkyl having up to 5 carbons; A is straight or branched alkylene or alkenylene each having up to 6 carbons; D and L may be same or different and each is aryl having 6-10 carbons or 5- to 7-membered aromatic hetero ring containing three hetero atoms from a group of S, N and/or O which may, in some cases, constitute a benzo-fusion; each of them may be substituted, to an extent of up to 3, with same or different halogen, hydroxyl, nitro, trifluoromethyl, carboxyl or straight or branched alkyl, alkoxy or alkoxycarbonyl (each having up to 6 carbons) or a group of the formula -(V)_{c}-NR¹⁵R¹⁶ and/or -OR¹⁷ where c is 0 or 1; V is a group of the formula -CO or -SO₂; R¹⁵ and R¹⁶ may be same or different and each has the same meaning as the above-mentioned R³ and R⁴; R¹⁷ is hydrogen, straight or branched alkenyl having up to 8 carbons or straight or branched alkyl having up to 8 carbons which may be substituted, to an extent of up to 3, with same or different hydroxyl, carboxyl or straight or branched alkoxycarbonyl having up to 5 carbons and/or said ring may be substituted with aryl having 6-10 carbons or with 5- to 7-membered aromatic hetero ring containing 3 hetero atoms from a group of S, N and/or O and, in some cases, being in a state of benzo-fusion; a part of each of them may be substituted, to an extent of up to 2, with same or different halogen, hydroxyl, nitro or trifluoromethyl, with straight or branched alkyl, alkoxy or alkoxycarbonyl each having up to 5 carbons or with a group of the formula (V')d-NR¹⁸R¹⁹; where d has the same meaning as the above-mentioned "a" and may be same therewith or different therefrom; R¹⁸ and R¹⁹ have the same meanings as the above-mentioned R³ and R⁴ and may be same therewith or different therefrom; V' has the same meaning with the above-mentioned V and may be same therewith or different therefrom; and/or a ring system mentioned for D may be substituted with a straight or branched acyl having up to 5 carbons or, in some cases, may be substituted with hydroxyl, with straight or branched alkoxy having up to 5 carbons or with a group of the formula -NR²⁰R²¹ where R²⁰ and R²¹ may be same or different and have the same meanings as the above-mentioned R³ and R⁴; D is a group represented by the following formula (CI): 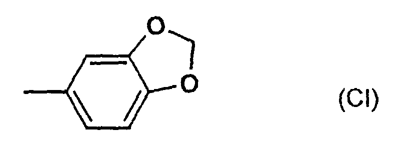 and E is a group of the formula -CH₂-Y-Z- where Y is a linkage or oxygen, sulfur or -NH; and Z is straight or branched alkylene chain having up to 5 carbons);
20) pyrazolo[3,4-d]pyrimidin-4-one derivatives mentioned in U.S. Patent No. 5,294,612 (which is herein incorporated by reference) represented by the following formula (CII): 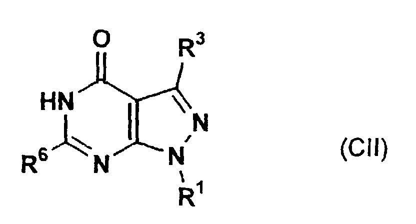 (where R¹ is hydrogen, alkyl C 4-7 cycloalkyl, C 1-10 alkyl or hydroxyl-substituted C 4-7 cycloalkyl, 2- or 3-tetrahydrofuranyl, 3-tetrahydrothienyl, 1,0-oxide, C 4-7 cycloalkyl C 1-10 alkyl, carboxy C 1-10 alkyl, dialkylamino C 1-10 alkyl, phenyl C 1-4 lower alkyl, or phenyl C 1-4 lower alkyl where 2-, 3- or 4-position of the phenyl ring is/are substituted with one or two (same or different) substituents selected from the group consisting of amino, halogen, C 1-10 alkyl, carboxyl, carbo C 1-4 lower alkoxy, carbamoyl, NHSO₂-(quinolinyl), nitro and cyano; R³ is C 1-4 lower alkyl, phenyl C 1-4 lower alkyl, lower alkoxyphenyl C 1-4 lower alkyl, di-C 1-4 lower alkoxyphenyl C 1-4 lower alkyl, pyridyl C 1-4 lower alkyl, C 4-7 cycloalkyl C 1-4 lower alkyl, phenylamino, di-C 1-10 alkylamino, halogen, trifluoromethyl, C 1-4 lower alkylthio, cyano or nitro; and R⁶ is a two-ring consisting of 5- or 6-membered hetero ring containing 1 or 2 nitrogen and 9- or 10-membered hetero ring containing 1 to 2 nitrogen and carbon wherein same or different substituent(s) each being selected from the group consisting of C 1-4 lower alkyl, halogen, C 1-4 lower alkoxy, C 4-7 cycloalkyloxy, 4-morpholinyl, C 1-4 lower alkoxy C 1-4 lower alkoxy, hydroxy, imidazolyl, oxo or 4-morpholinyl C 1-4 lower alkoxy is/are bonded to suitable carbon atom or wherein substituent being selected from the group consisting of C 1-4 lower alkyl, C 2-4 lower alkanoyl and trifluoroacetyl is bonded to suitable nitrogen atom);
21) pyridopyrimidinone derivatives mentioned in the International Publication WO 94/05,561 (which is herein incorporated by reference) represented by the following formula (CIII): 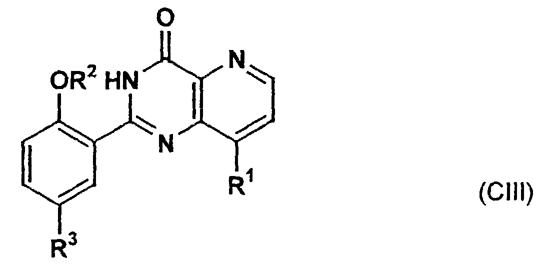 (where R¹ is H, C 1-4 alkyl, CN or CONR⁴R⁵; R² is C 2-4 alkyl; R³ is SO₂NR⁶R⁷, NO₂, NH₂, NHCOR⁸, NHSO₂R⁶ or N(SO₂R⁸)₂; R⁴ and R⁵ each independently is selected from H and C 1-4 alkyl; R⁶ and R⁷ each independently is selected from H and C 1-4 alkyl which may be substituted with CO₂R⁹, OH, pyridyl, 5-isoxazolin-3-onyl, morpholino or 1-imidazolidin-2-onyl or they may form, together with nitrogen atom bonded thereto, pyrrolidino, piperidino, morpholino, 1-pyrazolyl or 4-(NR¹⁰)-1-piperazinyl where those groups may be substituted with one or two substituents selected from C 1-4 alkyl, CO₂R⁹, NH₂ and OH; R⁸ is C 1-4 alkyl or pyridyl; R⁹ is H or C 1-4 alkyl; and R¹⁰ is H, C 1-4 alkyl or (hydroxy)C 2-3 alkyl);
22) 2-benzyl-polycyclic guanine derivatives mentioned in the International Publication WO 94/19,351 (which is herein incorporated by reference) represented by the following formula (CIV): 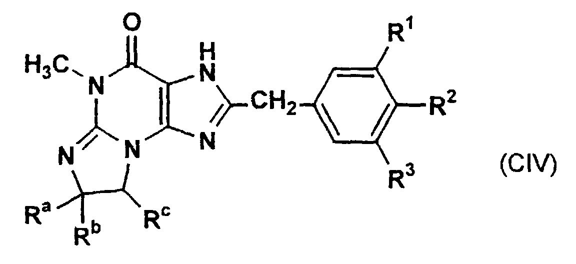 (where R¹, R² and R³ each independently is selected from the group consisting of hydrogen, lower alkyl, lower alkoxy, halogen, hydroxy(di-lower alkyl)amino, 4-morpholinyl, 1-pyrrolisinyl, 1-pyrolyl, -CF₃, -OCF₃, phenyl and methoxypheny; or R¹ and R² may form methylenedioxy together; or R¹ and R² may form a benzene ring together with carbon atom bonded thereto; R^{a} is hydrogen while R^{b} and R^{c} may form a saturated ring having 5 carbon atoms together with the carbon bonded thereto; or R^{a} is lower alkyl while R^{b} is hydrogen or lower alkyl and R^{c} is hydrogen; or R^{a}, R^{b} and the carbon bonded thereto may form a saturated ring having 5-7 carbon atoms while R^{c} is hydrogen; or R^{a} is hydrogen while R^{b}, R^{c} and carbon bonded thereto may form a tetrahydrofuran ring; or R^{a}, R^{b} and carbon bonded thereto and R^{b}, R^{c} and carbon bonded thereto each form a saturated ring having 5-7 carbon atoms);
23) amilino- or pyrazylamino-cyclobuten-1,2-dione derivatives mentioned in the International Publication WO 94/29,277 (which is herein incorporated by reference) represented by the following formula (CV): 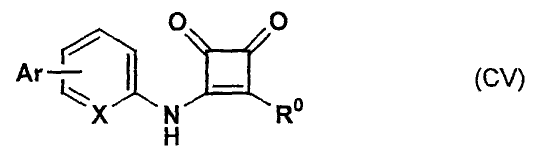 (where Ar is optionally-substituted aryl or heteroaryl ring selected from phenyl, naphthyl, pyridyl, pyrimidyl, pyridazinyl, pyrazinyl, imidazolyl, thienyl, oxazolyl, benzimidazolyl, benzoxazolyl, indolyl or thianaphthenyl; X is CH or N; and R⁰ is NR¹R² or hydrogen where R¹ and R² each independently is hydrogen or C 1-6 alkyl);
24) fluorenone derivatives mentioned in the Japanese Laid-Open Patent Publication Hei-07/61,949 (which is herein incorporated by reference) represented by the following formula (CVI): 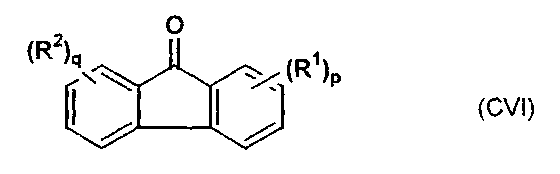 (where R¹ is hydrogen, hydroxyl, lower alkenyl, lower alkyl, halogen, lower alkoxy, lower alkylthio, lower alkanoyloxy, lower alkenyloxy, a group -ANR⁸R⁹ (where R⁸ and R⁹ are same or different and each independently is hydrogen, lower alkyl, lower alkoxycarbonyl-substituted lower alkyl, pyrimidinyl or pyrazinyl; or R⁸ and R⁹ may be bonded each other with or without nitrogen or oxygen forming a 5- or 6-membered saturated hetero ring together with nitrogen bonded thereto; said hetero ring may have a group selected from the group consisting of lower alkyl and lower alkoxycarbonyl as substituent; and A is lower alkylene), imidazolyl-substituted lower alkyl, lower alkoxy-substituted lower alkyl, hydroxyl-substituted lower alkoxy lower alkoxy-substituted lower alkyl or tri(lower alkyl)-substituted ammonium-substituted lower alkyl; R² is hydrogen, hydroxyl, lower alkenyl, lower alkyl, halogen, lower alkoxy, lower alkanoyloxy, lower alkenyloxy, a group -ANR⁸R⁹ (meanings of A, R⁸ and R⁹ are the same as those defined above), imidazolyl-substituted lower alkyl, lower alkoxy-substituted lower alkyl, pyridylthio-substituted lower alkyl, phenylthio-substituted lower alkyl where the phenyl ring may have lower alkoxy as substituent, benzimidazolylthio-substituted lower alkyl, imidazolylthio-substituted lower alkyl, lower alkanoyl, cycloalkylthio-substituted lower alkyl, cyano-substituted lower alkyl or tri(lower alkyl)substituted ammonium-substituted lower alkyl; p and q each is an integer of 1-4; and R¹ and R² each may be same or different);
25) 4-aminopyrimidine derivatives mentioned in the Japanese Laid-Open Patent Publication Hei-07/89,958 (which is herein incorporated by reference) represented by the following formula (CVII): 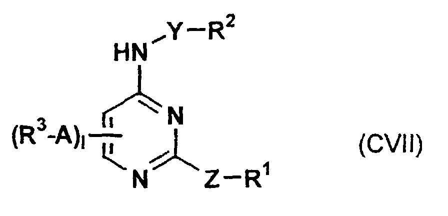 (where A is a single bond, C 1-4 alkylene or C 1-4 oxyalkylene; Y is a single bond, C 1-4 alkylene, C 1-4 alkyleneoxy, C 1-4 alkoxyphenylene or phenyl-(C 1-4) alkylene; Z is a single bond or vinylene; R¹ is 4- to 15-membered hetero ring containing one or two nitrogen atoms; R² is (1) 4- to 15-membered hetero ring containing one or two nitrogen atoms, one or two oxygen atoms or one sulfur atom, (2) C 4-15 carbon ring, (3) C 1-4 alkoxy, (4) hydroxy (C 1-4) alkoxy or (5) hydroxyl; R³ is (1) 4- to 15-membered hetero ring having one or two nitrogen atoms, one oxygen atom, one sulfur atom or one nitrogen atom and one sulfur atom, (2) C 4-15 carbon ring, (3) a group represented by the formula CH₂=CX- (where X is hydrogen or halogen) or (4) hydrogen; l is 1 or 2; the hetero ring represented by R¹ may be substituted with one or two C 1-4 alkyl, C 1-4 alkoxy, halogen, trifluoromethyl or nitro; the hetero ring represented by R² may be substituted with one or two C 1-4 alkyl, C 1-4 alkoxy, halogen, trifluoromethyl or nitro or a group represented by the formula -COOR¹⁰ (where R¹⁰ is hydrogen or C 1-4 alkyl); the cyclic group represented by R³ may be substituted with one or two C 1-4 alkyl, C 1-4 alkoxy, halogen, trifluoromethyl or nitro, cyano, ethynyl or a group represented by the formula
   -SONR⁷R⁸ (where R⁷ and R⁸ each is hydrogen or C 1-4 alkyl; when Y is a single bond, R² is not hydroxyl; when Z is vinylene, R¹ is bonded via nitrogen atom); and represented by the following formula (CVIII): 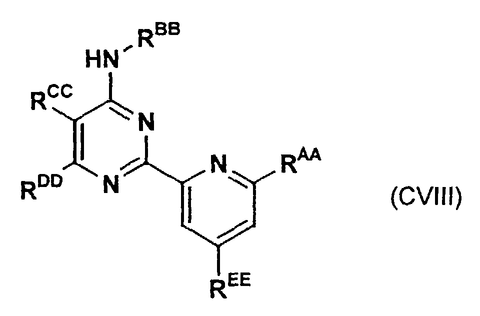 (where R^{AA} is methyl or n-propyl; R^{BB} is cyclopentyl, cyclohexyl, 2-hydroxyethyl, methoxyethyl, 2-(1-piperidinyl)ethyl, or phenyl or benzyl which may be substituted with one or two methyl, methoxy, chloro, nitro or trifluoromethyl; R^{CC} is hydrogen or methyl; R^{DD} is methyl, n-propyl, isopropyl or benzyl; and R^{EE} is hydrogen or methyl);
26) imidazoquinazoline derivatives mentioned in the International Publication WO 95/06,648 (which is herein incorporated by reference) represented by the following formula (CIX): 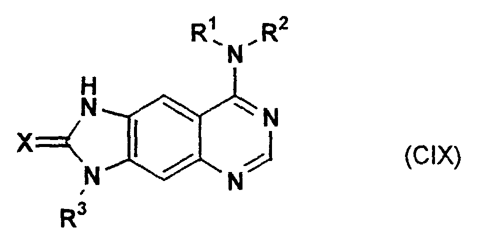 (where R¹ and R² are same or different and each is hydrogen, lower alkyl (said lower alkyl may be substituted with one to three (same or different) cycloalkyl, hydroxy, lower alkoxy, carboxy, lower alkoxycarbonyl, amino, monoalkyl-substituted amino, dialkyl-substituted amino, nitro, halogen or alicyclic heterocyclic group (said alicyclic heterocyclic group may be substituted with one to three (same or different) lower alkyl, aralkyl, aryl which may be substituted with 1-3 lower alkoxy or aromatic heterocyclic group)), cycloalkyl, bicycloalkyl, benzocycloalkyl (said benzocycloalkyl may be substituted with one to three (same or different) lower alkyl, hydroxy, lower alkoxy, carboxy, lower alkoxycarbonyl, amino, monoalkyl-substituted amino, dialkyl-substituted amino, nitro, sulfonamido, halogen or trifluoromethyl), lower alkenyl, aryl (said aryl may be substituted with one to three (same or different) lower alkyl, hydroxy, lower alkoxy, carboxy, lower alkoxycarbonyl, amino, monoalkyl-substituted amino, dialkyl-substituted amino, nitro, sulfonamido, halogen or trifluoromethyl), aromatic heterocyclic-substituted alkyl (said aromatic heterocyclic moiety may be substituted with one to three (same or different) lower alkyl, hydroxy, lower alkoxy, carboxy, lower alkoxycarbonyl, amino, monoalkyl-substituted amino, dialkyl-substituted amino, dialkyl-substituted amino, nitro, sulfonamido, halogen or trifluoromethyl while alkyl moiety may be substituted with aryl), aromatic heterocyclic group (said aromatic heterocyclic group may be substituted with one to three (same or different) lower alkyl, hydroxy, lower alkoxy, carboxy, lower alkoxycarbonyl, amino, monoalkyl-substituted amino, dialkyl-substituted amino, nitro, sulfonamido, halogen or trifluoromethyl) or aralkyl (in said aralkyl, an aryl moiety may be substituted with one to three (same or different) lower alkyl, lower alkoxy, dialkyl-substituted amino, halogen or trifluoromethyl); or R¹ and R² together may form a heterocyclic group containing N (said heterocyclic group may be substituted with one to three (same or different) lower alkyl, aryl or aralkyl); R³ is hydrogen, lower alkyl (said lower alkyl may be substituted with one to three (same or different) cycloalkyl, hydroxy, lower alkoxy, carboxy, lower alkoxycarbonyl, amino, monoalkyl-substituted amino, dialkyl-substituted amino, nitro, halogen or alicyclic heterocyclic group (said alicyclic heterocyclic group may be substituted with one to three (same or different) lower alkyl, aralkyl, aryl (which may be substituted with one to three, same or different, lower alkoxy) or aromatic heterocyclic ring)), cycloalkyl, lower alkenyl, aryl (said aryl may be substituted with one to three (same or different) lower alkyl, hydroxy, lower alkoxy, carboxy, lower alkoxycarbonyl, amino, monoalkyl-substituted amino, dialkyl-substituted amino, nitro, sulfonamido, halogen or trifluoromethyl), aromatic heterocyclic-substituted alkyl (said aromatic heteroyclic moiety may be substituted with one to three (same or different) lower alkyl, hydroxy, lower alkoxy, carboxy, lower alkoxycarbonyl, amino, monoalkyl-substituted amino, dialkyl-substituted amino, nitro, sulfonamido, halogen, or trifluoromethyl while alkyl moiety may be substituted with aryl), aromatic heterocyclic group (said aromatic heterocyclic group may be substituted with one to three (same or different) lower alkyl, hydroxy, lower alkoxy, carboxy, lower alkoxycarbonyl, amino, monoalkyl-substituted amino, dialkyl-substituted amino, nitro, sulfonamido, halogen or trifluoromethyl), or aralkyl (in said araylkyl, aryl moiety may be substituted with one to three (same or different) lower alky, lower alkoxy, dialkyl-substituted amino, halogen or trifluoromethyl); and X is O or S) or pharmacologically acceptable salts thereof; or imidazoquinazoline derivatives mentioned in the International Publication WO 96/26,940 (which is herein incorporated by reference) represented by the following formula (CX): 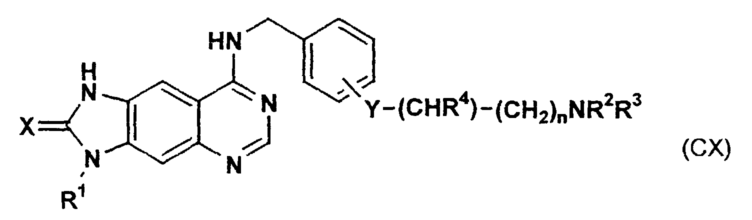 (where R¹ is hydrogen, substituted or unsubstituted lower alkyl, cycloalkyl, lower alkenyl, substituted or unsubstituted aralkyl, substituted or unsubstituted aryl, substituted or unsubstituted heteroarylalkyl or substituted or unsubstituted heteroaryl; R² and R³ are same or different and each is hydrogen, substituted or unsubstituted lower alkyl, substituted or unsubstituted aralkyl, substituted or unsubstituted aryl, substituted or unsubstituted heteroarylalkyl or substituted or unsubstituted heteroaryl; or R² and R³ together may form a substituted or unsubstituted heterocyclic group containing N; R⁴ is hydrogen or substituted or unsubstituted lower alkyl; X is O or S; Y is a single bond or O; and n is 0, 1, 2 or 3);
27) quinazoline derivatives mentioned in the Japanese Laid-Open Patent Publication Hei-07/126,255 (which is herein incorporated by reference) represented by the following formula (CXI): 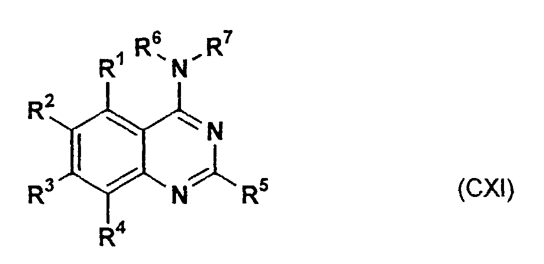 (where R¹, R², R³, R⁴ and R⁵ are same or different and each is hydrogen, halogen, lower alkyl or lower alkoxy; R⁶ and R⁷ are same or different and each is hydrogen, lower alkyl, hydroxyalkyl, lower alkoxyalkyl, cyanoalkyl, heteroarylalkyl, cycloalkyl, cycloalkylalkyl or optionally protected carboxyalkyl; or R⁶ and R⁷ may form a ring together with nitrogen atom bonded thereto; and said ring may be substituted);
28) anthranilic acid derivatives mentioned in the Japanese Laid-Open Patent Publication Hei-08/188,563 (which is herein incorporated by reference) represented by the following formula (CXII): 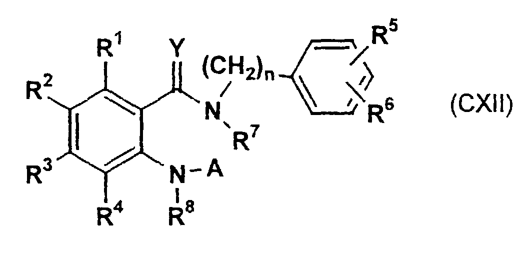 (where R¹, R², R³ and R⁴ are same or different and each is hydrogen, halogen, hydroxy, optionally halogen-substituted lower alkyl, optionally halogen-substituted lower alkoxy, nitro, hydroxyalkyl, cyano, a group represented by the following formula (CXIII): 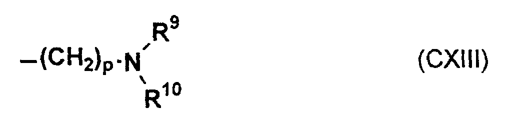 (where R⁹ and R¹⁰ are same or different and each is hydrogen, optionally halogen-substituted lower alkyl, arylalkyl, heteroarylalkyl, acyl or optionally protected carboxyl; R⁹ and R¹⁰ may form a ring together with nitrogen atom bonded thereto; said ring may be substituted; and p is 0 or an integer of 1-6), optionally substituted tetrazolyl, optionally protected carboxyl, optionally substituted carbamoyl, optionally substituted pyrazolyl, optionally substituted imidazolyl or a group represented by the following formula (CXIV): 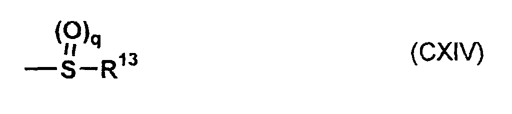 (where R¹³ is hydrogen or optionally halogen-substituted lower alkyl; and q is 0 or an integer of 1-2); adjacent two substituents selected from R¹, R², R³ and R⁴ may form a ring together with carbon atom bonded thereto; R⁵ and R⁶ are same or different and each is hydrogen, halogen, hydroxy, cyano, optionally halogen-substituted lower alkyl or optionally halogen-substituted lower alkoxy; R⁵ and R⁶ may form a cycloalkyl ring, oxolane ring, 1,3-dioxolane ring or 1,4-dioxolane ring together with carbon atom bonded thereto; W is a group represented by the formula -N= or the formula -CH=; R⁷ and R⁸ are same or different and each is hydrogen or optionally halogen-substituted lower alkyl; R¹ and R⁷ may form a ring together with carbon bonded thereto, optionally containing nitrogen, oxygen or sulfur where said ring may be substituted; A is hydrogen, optionally halogen-substituted lower alkyl or a group represented by the following formula (CXV):

   ―X-(CH₂)ₘ-Z (CXV)

   (where X is a group represented by the formula -CO-, -CS-, -CH₂- or -S(O)₂); Z is hydroxy, optionally halogen-substituted lower alkoxy, cyano, halogen, optionally protected carbamoyl, optionally substituted aryl, optionally substituted aryloxy, optionally substituted heteroaryl, optionally substituted heteroarylalkyloxy, a group represented by the formula -NR¹¹R¹² (where R¹¹ and R¹² are same or different and each is hydrogen, optionally halogen-substituted lower alkyl, optionally substituted arylalkyl, optionally substituted heteroarylalkyl, acyl, optionally protected carboxyl, or optionally substituted carbamoyl; R¹¹ and R¹² may form a ring together with nitrogen bonded thereto; and said ring may be substituted); or optionally-substituted cycloalkyl; and in is 0 or an integer of 1-6; Y is oxygen atom or sulfur atom; and n is 0 or an integer of 1-6);
29) benzofuro[3,2-d]pyrimidin-4-one derivatives mentioned in the Japanese Laid-Open Patent Publication Hei-07/267,961 (which is herein incorporated by reference) represented by the following formula (CXVI): 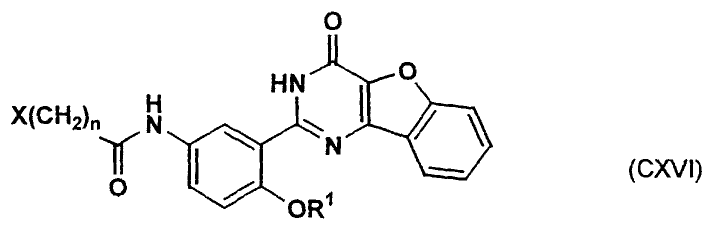 (where R¹ is alkyl having 1-4 carbons; n is 0-4; X is halogen, carboxyl, phenoxy, (1-methyl-1H-imidazol-2-yl)thio or R²R³N; R² and R³ may be same or different and each is hydrogen, halogenated alkyl having 1-4 carbons, alkyl having 1-4 carbons or hydroxyalkyl having 1-4 carbons or may form piperidino, morpholino, pyrrolidino, 4-hydroxypiperidino, 4-(2-hydroxyethyl)piperazino, 3-hydroxypyrrolidino or 1,2,3,4-tetrahydroisoquinolidino as R²R³N);
30) MS-681 mentioned in the Japanese Laid-Open Patent Publication Hei-07/285,993 (which is herein incorporated by reference) represented by the following formula (CXVII): 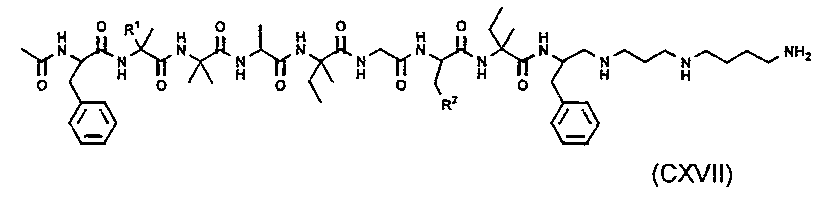 (where R¹ is methyl or ethyl; and R² is phenyl or 3-indolyl);
31) thieno[3,2-d]pyrimidin-4-one derivatives mentioned in the Japanese Laid-Open Patent Publication Hei-07/330,777 (which is herein incorporated by reference) represented by the following formula (CXVIII): 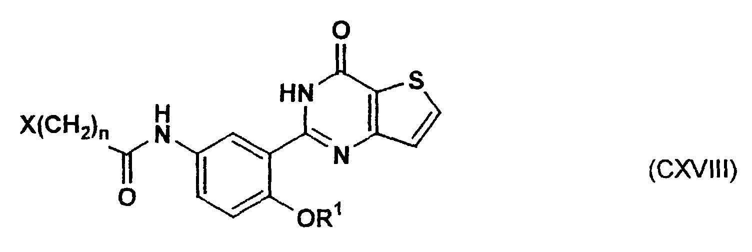 (where R¹ is alkyl having 1-4 carbons; n is 0 or 1; X is halogen, cycloalkyl having 3-6 carbons, 2-pyridyl, 2-furyl, alkoxy having 1-4 carbons, phenoxy or R²R³N; R² and R³ may be same or different and each is hydrogen, alkyl having 1-4 carbons, cycloalkyl having 3-6 carbons, hydroxyalkyl having 2-6 carbons, phenyl, picolyl, 3,4-methylenedioxybenzyl, 2-morpholinoethyl, 2-pyrrolidinoethyl, 2-piperidinoethyl or 2-(1-methyl-1H-pyrrol-2-yl)ethyl or may form morpholino, pyrrolidino, piperidino, 3-hydroxypyrrolidino, 4-hydroxypiperidino, 4-carbethoxypiperidino, 4-methylpiperazino, 4-(2-methoxyphenyl)piperazino, thiomorpholino or thiazolino as R²R³N); or thienopyrimidin-4-one derivatives mentioned in the Japanese Laid-Open Patent Publication Hei-08/143,571 (which is herein incorporated by reference) represented by the following formula (CXIX): 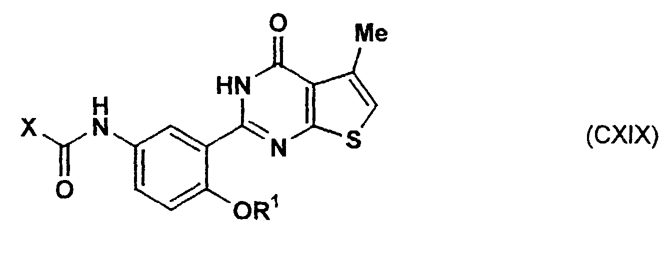 (where R¹ is alkyl having 1-4 carbons; X is phenoxy, morpholino, piperidino, pyrrolidino, 4-carbethoxypiperidino, 4-(2-hydroxyethyl)piperazino or R²R³N where R² and R³ are same or different and each is hydrogen, alkyl having 1-4 carbons or hydroxyalkyl having 2-4 carbons);
32) phthalazine derivatives mentioned in the International Publication WO 96/05,176 (which is herein incorporated by reference) represented by the following formula (CXX): 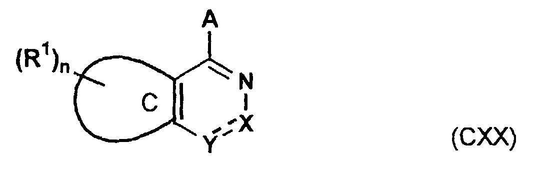 (where ring C is 5- to 6-membered ring which may have hetero atom; n is 0 or an integer of 1-4; R¹ is halogen, optionally substituted lower alkyl, optionally substituted lower alkoxy, optionally substituted lower cycloalkyl, nitro, cyano, a group represented by the formula -NR²R³ (where R² and R³ are same or different and each is hydrogen, optionally substituted lower alkyl, acyl, optionally substituted lower arylalkyl or optionally substituted lower heteroarylalkyl; or R² and R³ may form a ring together with nitrogen bonded thereto; and said ring may be substituted), a group represented by the formula -O-R⁹ (where R⁹ is hydrogen, optionally substituted lower alkyl, acyl, optionally substituted lower arylalkyl or optionally substituted lower heteroarylalkyl), a group represented by the formula -S-R¹⁰ (where R¹⁰ is hydrogen, optionally substituted lower alkyl, acyl, optionally substituted arylalkyl or optionally substituted heteroarylalkyl), a group represented by the following formula (CXXI): 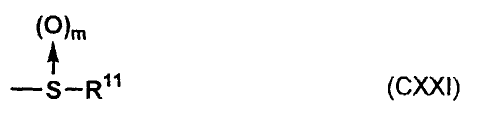 (where R¹¹ is hydrogen, lower alkyl or amino; and m is 0 or an integer of 1-2), optionally protected carboxyl; when n is 2-4, each R¹ may have the above substituent independently; A is hydrogen, halogen, a group represented by the formula -NR⁴R⁵ (where R⁴ and R⁵ are same or different and each is hydrogen, optionally substituted lower alkyl, acyl, optionally substituted arylalkyl or optionally substituted heteroarylalkyl; or R⁴ and R⁵ may form a ring together with nitrogen bonded thereto; and said ring may be substituted), optionally substituted aryl, optionally substituted heteroaryl, optionally substituted arylalkyl or optionally substituted heteroarylalkyl; X is a group represented by the formula -NR⁶ (where R⁶ is hydrogen, optionally substituted lower alkyl, optionally substituted arylalkyl or optionally substituted heteroarylalkyl) or a group represented by the formula -N=; Y is a group represented by the formula -CO- or a group represented by the formula -CB= (where B is hydrogen, halogen or a group represented by the formula -NR⁷R⁸ (where R⁷ and R⁸ are same or different and each is hydrogen, optionally substituted lower alkyl, acyl, optionally substituted arylalkyl or optionally substituted heteroarylalkyl; or R⁷ and R⁸ may form a ring together with nitrogen bonded thereto; and said ring may be substituted), a group represented by the formula -O-R¹² (where R¹² is hydrogen, optionally substituted lower alkyl, acyl, optionally substituted arylalkyl or optionally substituted heteroarylalkyl), a group represented by the formula -S-R¹³ (where R¹³ is hydrogen, optionally substituted lower alkyl, acyl, optionally substituted arylalkyl or optionally substituted heteroarylalkyl), optionally substituted aryl, optionally substituted heteroaryl, optionally substituted arylalkyl or optionally substituted heteroarylalkyl)); the following formula (CXXII): 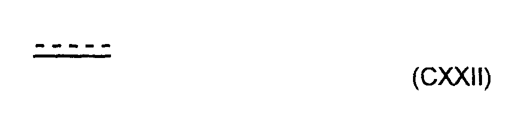 represents a double bond or a single bond except the case where n is 0 when the ring C is a benzene ring);
33) FR 901526 mentioned in the Japanese Laid-Open Patent Publication Hei-08/59,681 (which is herein incorporated by reference) and having the physicochemical properties as shown in the following Table 1.
   (A) Properties of the substance
      Acidic substance
   (B) Melting point
      229-233 °C (decomposition)
   (C) Infrared absorption spectrum
      γmax(KBr): 3270, 1620, 1570, 1370, 1280 cm⁻¹
   (D) Solubility in solvents
      soluble in dimethyl sulfoxide, methanol and acetone but insoluble in water
   (E) Color reaction
      positive to cerium sulfate reaction, ferric chloride reaction and iodine vapor reaction while negative to ninhydrin reaction, Molisch's reaction and Ehrlich's reaction
34) pyrazoloquinoline derivatives mentioned in the International Publication WO 96/28,446 (which is herein incorporated by reference) represented by the following formula (CXXIII): 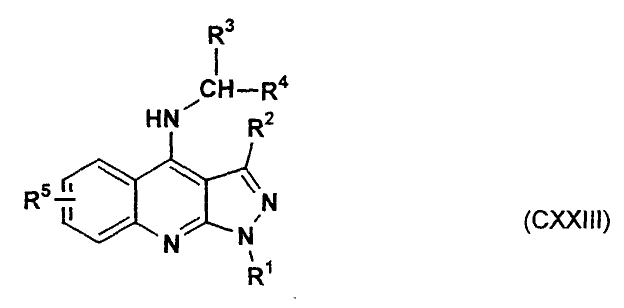 (where R¹ is lower alkyl, phenyl lower alkyl or cycloalkyl; R² is hydrogen or lower alkyl; R³ is hydrogen, lower alkyl or hydroxy lower alkyl; R⁴ is cycloalkyl (or cycloalkyl which is substituted with one or two, same or different, substituents selected from the group consisting of lower alkoxycarbonyl, carboxy, lower alkylthio lower alkoxycarbonyl, hydroxy lower alkyl, hydroxy, oxo, lower alkoxy, lower alkyl and halogen); R⁵ is one to three, same or different, substituents selected from hydrogen, lower alkyl, hydroxy, di-lower alkylamino lower alkoxy, carboxy lower alkoxy, lower alkoxycarbonyl lower alkoxy, nitro, polyhydroxy lower alkoxy, amino, epoxy lower alkoxy, carboxy, lower alkanoylamino, lower alkoxycarbonyl, pyridinyl, 4-morpholinyl lower alkoxy, lower alkylsulfonyl, cyano, 1-imidazolyl, halogen, di-lower alkylaminosulfonyl, oxadiazolyl (or oxadiazolyl where some suitable carbon atoms are substituted with lower alkyl), lower alkylsulfinyl, 1-pyrazolyl (or 1-pyrazolyl where some suitable carbon atoms are substituted with lower alkyl), trifluoromethylsulfonyl, lower alkenyl, lower alkyl and lower alkynyl);
35) qunazolin-4(3H)-one derivatives mentioned in the Japanese Laid-Open Patent Publication Hei-08/104,679 (which is herein incorporated by reference) represented by the following formula (CXXIV): 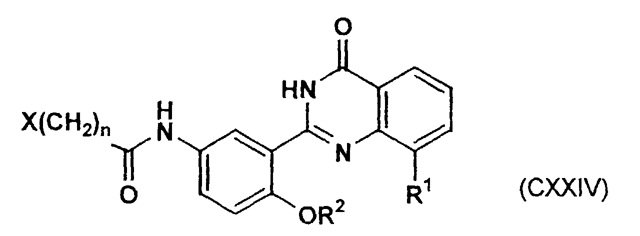 (where R¹ is hydrogen, methyl or methoxy; R² is alkyl having 1-4 carbons; n is 0 or 1; and X is halogen, phenoxy or R³R⁴N where R³R⁴N is morpholino, piperidino, pyrrolidino, 3-hydroxypyrrolidino, 4-hydroxypiperidino, 4-carbethoxypiperidino, thiomorpholino, hexamethylene-imino or bis(2-hydroxyethyl)amino;
36) 4-aminopyrimidine derivatives mentioned in U.S. Patent No. 5,525,604 (which is herein incorporated by reference) represented by the following formula (CXXV): 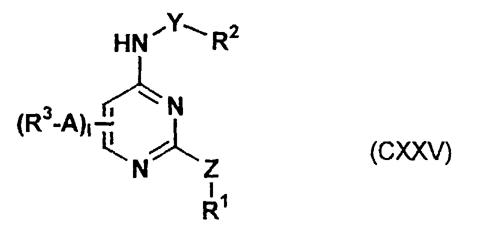 (where A is a single bond, C 1-4 alkylene or C 1-4 oxyalkylene; Y is a single bond, C 1-4 alkylene, C 1-4 alkyleneoxy, C 1-4 alkoxyphenylene or phenyl (C 1-4) alkylene; Z is a single bond or vinylene; R¹ is a hetero ring selected from the group consisting of pyrrole, pyridine, azepine, imidazole, pyrazole, pyrimidine, pyrazine, pyridazine, benzimidazole, quinoline, isoquinoline and partially saturated or saturated ring thereof; R² is (1) a hetero ring selected from the group consisting of pyrrole, pyridine, azepine, imidazole, pyrazole, pyrimidine, pyrazine, pyridazine, benzimidazole, quinoline, isoquinoline, furan, pyran, dioxole, dioxin, benzofuran, benzopyran, benzodioxole, beuzodioxin, thiophene, thioine, benzothiophene, benzothioine and partially saturated or saturated ring thereof, (2) C 4-15 carbon ring, (3) C 1-4 alkoxy, (4) hydroxy (C 1-4 alkyl) or (5) hydroxy; when R¹ is pyridine or pyridine which is substituted with one or two C 1-4 alkyl, C 1-4 alkoxy, halogen, trifluoromethyl or nitro, then R² is selected from benzodioxole, benzodioxole which is substituted with one or two C 1-4 alkyl, C 1-4 alkoxy, halogen, trifluoromethyl, nitro and the formula -COOR¹⁰ (where R¹⁰ is selected from hydrogen, benzodioxole substituted with C 1-4 alkyl and C 1-4 alkoxy); R³ is (1) hetero ring selected from the group consisting of pyrrole, pyridine, azepine, imidazole, pyrazole, pyrimidine, pyrazine, pyridazine, benzimidazole, quinoline, isoquinoline, furan, pyran, benzofuran, benzopyran, thiophene, thioine, benzothiophene, benzothioine, thiazole, isothiazole, thiazine, benzothiazole, benzoisothiazine, benzothiazine and partially saturated or saturated ring thereof; (2) C 4-15 carbon ring, (3) the formula CH₂=CH(X) (where X is halogen) or (4) hydrogen; l is 1 or 2; the ring represented by R¹ may be substituted with one or two C 1-4 alkyl, C 1-4-alkoxy, halogen, trifluoromethyl or nitro; the ring represented by R² may be substituted with one or two C 1-4 alkyl, C 1-4 alkoxy, halogen, trifluoromethyl, nitro or a formula -COOR¹⁰ (where R¹⁰ is hydrogen, C 1-4 alkyl or a ring represented by R³ substituted with one or two C 1-4 alkyl, C 1-4 alkoxy, halogen, trifluoromethyl, nitro, cyano, ethynyl or the formula -SONR⁷R⁸ (where R⁷ and R⁸ each independently is hydrogen or C 1-4 alkyl); when Y is a single bond, R² is not hydrogen; and when R¹ is not bonded via nitrogen, then Z is vinylene);
37) 2,8-disubstituted quinazolinone derivatives mentioned in the Japanese Laid-Open Patent Publication Hei-08/253,457 (which is herein incorporated by reference) and represented by the following formula (CXXVI): 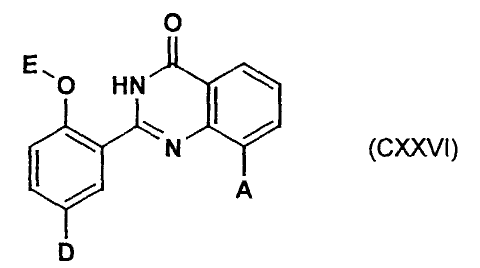 (where A is oxiranyl which may be substituted with straight or branched alkyl having up to eight carbons (which may be further substituted with phenyl) or a group represented by the following formula (CXXVII): 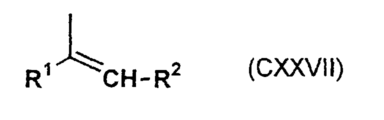 or by the following formula (CXXVIII): 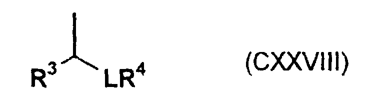 or by the following formula (CXXIX): 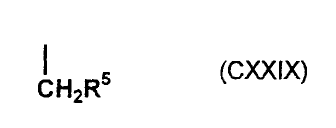 where R¹ is hydrogen or straight or branched alkyl having up to 6 carbons; R² is optionally phenyl-substituted straight or branched alkyl having up to 8 carbons; R³ is a straight or branched alkyl having up to 5 carbons or a group of the formula -OR⁶ where R⁶ is hydrogen, protected hydroxy or straight or branched alkyl having up to 5 carbons; R⁴ is optionally phenyl-substituted straight or branched alkyl having 2-10 carbons; L is a group of the formula -CO-, -CH(OH), -CH₂, -CH(N₃)or -CH(OSO₂R⁷) where R⁷ is straight or branched alkyl having up to 4 carbons or phenyl; R⁵ is optionally phenyl-substituted straight or branched alkyl having 3-8 carbons, benzyl or 2-phenylethyl; D is hydrogen or a group of the formula -SO₂-NR⁸R⁹ where R⁸ and R⁹ are same or different and each is hydrogen, phenyl, optionally hydroxyl-substituted straight or branched alkyl having up to 6 carbons, or 5-to 6-membered saturated heterocyclic group, together with a nitrogen atom and being able to have up to 2 hetero atoms from the group consisting of S, N and/or O and, further, free N-functional group, said heterocyclic group being optionally substituted with straight or branched alkyl having up to 6 carbons which may be further substituted with hydroxyl; and E is straight or branched alkyl having up to 8 carbons); 2,8-disubstituted quinazolinone derivatives mentioned in the Japanese Laid-Open Patent Publication Hei-08/253,457;
38) 2,9-disubstituted purin-6-one derivatives mentioned in the Japanese Laid-Open Patent Publication Hei-08/231,545 (which is herein incorporated by reference) represented by the following formula (CXXX): 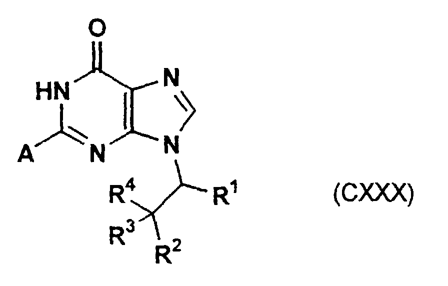 (where R¹ is a straight or branched alkyl optionally substituted with phenyl which may further be substituted with halogen, nitro, cyano or a straight or branched alkyl having up to 6 carbons; R² is hydrogen, hydroxyl or azide, or a straight or branched alkyl having up to 6 carbons, or a group of the formula -O-SO₂R⁵ where R⁵ is straight or branched alkyl having up to 4 carbons or phenyl; R³ is hydrogen or R² and R³ may form a group of the formula =O together; R⁴ is hydrogen or a straight or branched alkyl having up to 4 carbons; and A is a group having the following formula (CXXXI): 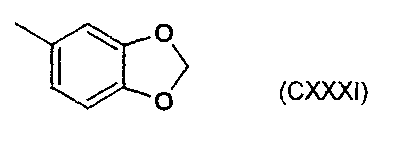 or a straight or branched alkyl having up to 20 carbons, or cycloalkyl having 3-7 carbons, or, in each case of being substituted with halogen, trifluoromethyl, carboxyl, nitro, cyano or phenyl, it is phenyl which is substituted (up to twice; same or different) with alkoxycarbonyl, alkoxy or straight or branched alkyl having up to 5 carbons and/or said ring may further be substituted with a straight or branched alkoxy having up to 5 carons);
39) 9-substituted 2-(2-n-alkoxyphenyl)-purin-6-one derivatives mentioned in the Japanese Laid-Open Patent Publication Hei-08/231,546 (which is herein incorporated by reference) represented by the following formula (CXXXII): 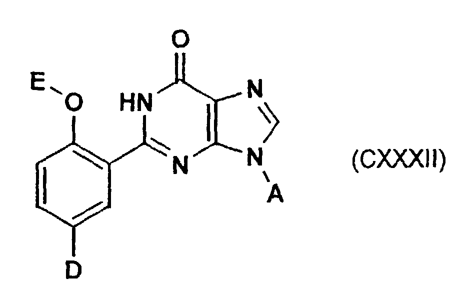 (where A is a group which is represented by the following formula (CXXXIII): 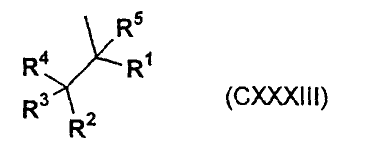 or by the following formula (CXXXIV): 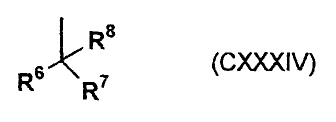 or by the following formula (CXXXV):

   ―(CH₂)ₐ-CH₃ (CXXXV)

   where a is 9, 10, 11, 12, 13, 14 or 15; R¹ is optionally phenyl-substituted straight or branched alkyl having 2-10 carbons; said phenyl may be further substituted with halogen, nitro or cyano, or with straight or branched alkyl having up to 6 carbons, or with a group of the formula -SO₂-NR⁹R¹⁰ where R⁹ and R¹⁰ are same or different and each is hydrogen, phenyl or optionally hydroxyl-substituted straight or branched alkyl having up to 6 carbons or, together with the nitrogen atom, may form a 5- to 6-membered saturated heterocyclic ring which may further contain up to 2 hetero atoms selected from S, N and/or O and may, in some cases, contain free N-functional group and, further, it may be substituted with optionally hydroxyl-substituted straight or branched alkyl having up to 6 carbons and/or may further be substituted with alkyl or, in some cases, with a group of the formula NR¹¹R¹² where R¹¹ and R¹² have the same meaning as the above R⁹ and R¹⁰ although same therewith or different therefrom; R² is hydrogen, azide, straight or branched alkyl having up to 6 carbons or a group of the formula -OR¹³, O-SO₂R¹⁴ or -NR¹⁵R¹⁶ where R¹³ is hydrogen, protected hydroxyl, straight or branched acyl having up to 6 carbons, benzoyl or straight or branched alkyl having up to 6 carbons which, in some cases, may be substituted with carboxyl, straight or branched alkoxycarbonyl having up to 6 carbons or a group of the formula -CO-NR¹⁷R¹⁸ where R¹⁷ and R¹⁸ may be same or different and each is hydrogen or straight or branched alkyl having up to 4 carbons; R¹⁴ is straight or branched alkyl having up to 4 carbons or phenyl; R¹⁵ and R¹⁶ may be same or different and each is hydrogen, protected amino, straight or branched alkyl or acyl having up to 6 carbons in each case, formyl, benzoyl or a group of the formula -SO₂R¹⁹ where R¹⁹ has the same meaning as R¹⁴ though may be same therewith or different therefrom; R³ is hydrogen or R² and R³ may form a group of the formula =O or =N-OR²⁰ together where R²⁰ is hydrogen or straight or branched alkyl having up to 6 carbons which may, in some cases, be substituted with phenyl or a group of the formula -NR²¹R²² where R²¹ and R²² may be same or different and each is hydrogen, phenyl or straight or branched alkyl having up to 6 carbons; R⁴ is hydrogen or straight or branched alkyl having up to 4 carbons; R⁵ and R⁸ may be same or different and each is hydrogen or straight or branched alkyl having up to 3 carbons; R⁶ is hydrogen or straight or branched alkyl having up to 5 carbons which may be substituted with hydroxyl; R⁷ is straight or branched alkyl having 2 to 8 carbons which is substituted with a group of the formula -NR²³R²⁴ where R²³ and R²⁴ may be same or different and each is hydrogen or straight or branched alkyl having up to 5 carbons which may be substituted with hydroxyl or with phenyl where said phenyl may be further substituted with a group of the formula -SO₂-NR²⁵R²⁶ where R²⁵ and R²⁶ have the same meanings as the above R⁹ and R¹⁰; D is hydrogen or a group of the formula -SO₂-NR²⁷R²⁸ where R²⁷ and R²⁸ may be same or different and have the same meanings as the above R⁹ and R¹⁰ though may be same therewith or different therefrom; and E is straight or branched alkyl having up to 8 carbons);
40) pyrazolopyrimidin-4-one derivatives mentioned in U.S. No. Patent 5,541,187 represented by the following formula (CXXXVI): 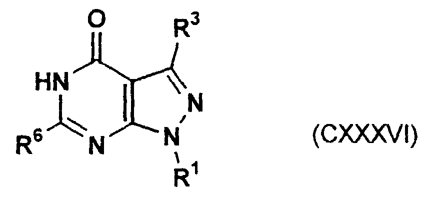 (where R¹ is phenyl lower alkyl where 2-, 3- or 4-position of the phenyl ring is substituted with one or two (same or different) substituents selected from the group consisting of hydrogen, alkyl, cycloalkyl, alkyl- or hydroxyl-substituted cycloalkyl, 2- or 3-tetrahydrofuranyl, 3-tetrahydrothienyl 1,1-dioxide, cycloalkylalkyl, carboxyalkyl, carbo lower alkoxyalkyl, dialkylaminoalkyl, phenyl lower alkyl, amino, halogen, alkylcarboxyl, carbo lower alkoxy, carbamoyl, NHSO₂-(quinolinyl), nitro and cyano; R³ is hydrogen, lower alkyl, phenyl lower alkyl, lower alkoxyphenyl lower alkyl, di-lower alkoxyphenyl lower alkyl, pyridyl lower alkyl, cycloalkyl lower alkyl, phenylamino, dialkylamino, halogen, trifluoromethyl, lower alkylthio, cyano or nitro; R⁶ is a hetero 5- or 6-membered ring containing 1 or 2 nitrogens where one or two substituents selected from the group consisting of lower alkyl, halogen, lower alkoxy, cycloalkyloxy, 4-morpholinyl, lower alkoxy lower alkoxy, hydroxy, imidazolyl, oxo or 4-morpholinyl lower alkoxy are bonded to suitable carbon atom(s) or where a substituent selected from lower alkyl, lower alkanoyl and trifluoroacetyl is bonded to a suitable nitrogen atom); pyrazolopyrimidin-4-one derivatives mentioned in the International Publication WO 96/28,429 (which is herein incorporated by reference) represented by the following formula (CXXXVII): 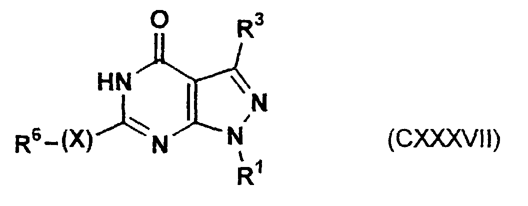 (where R¹ is tert-butyl or cyclopentyl; R³ is methyl, ethyl or phenylmethyl; X is -CH₂-, -O- or -NH-; R⁶ is phenyl, or phenyl which is substituted with one to three (same or different) substituents selected from the group consisting of lower alkyl, hydroxy, halogen, carboxy lower alkyl, 4-morpholinyl lower alkoxy, 5-tetrazolyl lower alkoxy, di-lower alkylamino, trifluoromethyl, nitro, amino, lower alkylsulfonylamino, di-lower alkylamino lower alkylphenylcarbonyloxy and 1-imidazolyl; and, when X is -CH₂-, R⁶ may be 2-, 3- or 4-pyridinyl, 1-pyrrolyl, 1-benzimidazolyl, 1,2,3,4-tetrahydro-2-isoquinolinyl, 1,2,3,4-tetrahydro-1-quinolinyl, hydroxy, 1-imidazolyl, 1-lower alkyl-2-, 3-, 4- or 5-pyrrolyl, 1-pyrazolyl, 3-, 4- or 5-isoxazolyl (or 3-, 4- or 5-isoxazolyl where a suitable carbon atom is substituted with lower alkyl), 2-thieny or 3-thienyl); or pyrazolopyrimidin-4-one derivatives mentioned in the International Publication WO 96/28,448 (which is herein incorporated by reference) represented by the following formula (CXXXVIII): 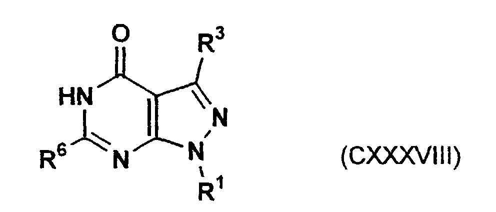 (where R¹ is tert-butyl or cyclopentyl; R³ is lower alkyl or phenyl lower alkyl; R⁶ is phenyl, or phenyl which is substituted with one to three (same or different) substituents selected from the group consisting of lower alkyl, lower alkoxy, hydroxy, 1-imidazolyl, lower alkenyloxy, di-lower alkylamino lower alkyl, 4-morpholinyl lower alkoxy, lower alkoxycarbonyl lower alkoxy, carboxy lower alkoxy, trifluoromethyl, 1-piperidinyl lower alkoxy, 1-pyrrolidinyl lower alkoxy, nitro, halo, amino, -(CH₂)₂O-, lower alkylsulfonylamino, lower alkoxy lower alkoxy, lower alkenyl, di-lower alkylamino, -OCH(CH₃)CH₂-, 4-morpholinylcarbonyl lower alkoxy, 4-thiomorpholinyl lower alkoxy, pyridinyl lower alkoxy, 1-lower alkyl-3-hexahydroazepinyloxy or 1-lower alkyl-4-piperidinyloxy);
41) heterocyclic compounds mentioned in the Japanese Laid-Open Patent Publication Hei-08/269,060 (which is herein incorporated by reference) represented by the following formula (CXXXIX): 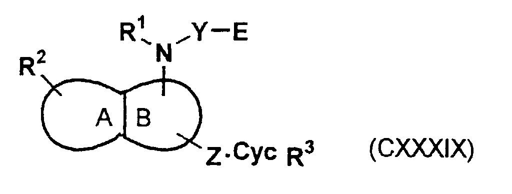 (where the following formula (CXL): 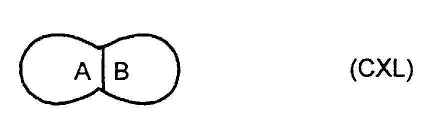 is a nitrogen-containing hetero ring optionally selected from the group consisting of the following formula (CXLI): 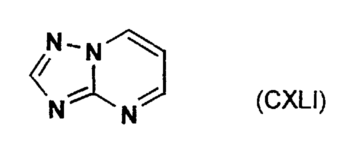 the following formula (CXLII): 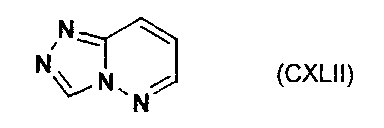 the following formula (CXLIII): 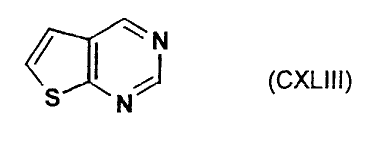 the following formula (CXLIV): 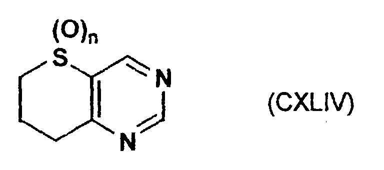 and the following formula (CXLV): 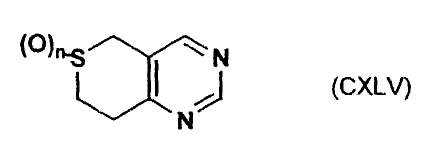 where n is 0 or an integer of 1 or 2; Y is a single bond or C 1-6 alkylene; Z is a single bond, C 1-2 alkylene or vinylene; E is (i) 4- to 15-membered monocyclic or dicyclic unsaturated, partially saturated or completely saturated hetero ring containing 1 or 2 nitrogen atoms, 1 or 2 oxygen atoms or 1 sulfur atom as the hetero atom, (ii) 4- to 15-membered monocyclic or dicyclic unsaturated or partially saturated carbon ring, or (iii) -OR⁴ where R⁴ is hydrogen, C 1-4 alkyl, or C 1-4 alkyl substituted with one hydroxyl group; Cyc is 5- to 7-membered monocyclic unsaturated, partially saturated or completely saturated hetero ring having 1 to 2 nitrogen atoms as the hetero atom or 5- to 7-membered monocyclic unsaturated or partially saturated carbon ring; R¹ is hydrogen or C 1-4 alkyl; R² is hydrogen, C 1-4 alkyl, C 1-4 alkoxy or halogen; R³ is hydrogen, C 1-4 alkyl, C 1-4 alkoxy or -COOR⁵ where R⁵ is hydrogen or C 1-4 alkyl; and, (1) when Z is vinylene, Cyc ring does not bond to Z via nitrogen atom in the Cyc ring and (2) when E is -OR⁴, Y is not a single bond);
42) pyrido[3,2-e]pyrazinone derivatives mentioned in the Japanese Laid-Open Patent Publication Hei-08/269,059 (which is herein incorporated by reference) represented by the following formula (CXLVI): 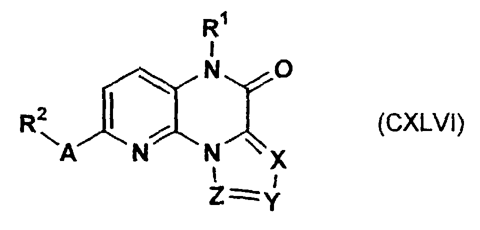 (where A is CH₂, NR³ or O; X, Y and Z are CR⁴ where at least one of X, Y and Z should be N; R¹ may be H (only when A is NR³) , C 1-10 optionally branched alkyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2), C 1-10 optionally branched alkenyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2), C 1-10 optionally branched alkynyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2) or C 5-7 cycloalkyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2); R² may be H, C 1-10 optionally branched alkyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryloxy, amino, substituted amino as well as with halogen, NO₂, CN, C=OR⁵ or S(O)ₙR⁶ where n is 0 to 3), C 1-10 optionally-branched alkenyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2), C 1-10 optionally branched alkynyl (which may be substituted with one to several hydroxy, C 1-6 alkyloxy, C 1-6 alkenyloxy, C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2) or C 5-7 cycloalkyl (which may be substituted with one to several hydroxy, C 1-6 alkyloxy-C 1-6 alkenyloxy-C 1-6 alkynyloxy, optionally substituted aryl, optionally substituted aryloxy, optionally substituted heteroaryl, optionally substituted heteroaryloxy, amino, substituted amino as well as with halogen, NO₂, CN, C=OR⁵ or S(O)ₙR⁶ where n is 0 to 2); R³ is H or C 1-6 alkyl; R⁴ is H, C 1-6 alkyl (optionally branched) or halogen; R⁵ is H, C 1-6 alkyl (optionally branched), phenyl, OH, C 1-6 alkyloxy (optionally branched), aryloxy (optionally branched) or amino (optionally branched); and R⁶ is H, C 1-6 alkyl, aryl (optionally substituted), OH, C 1-6 alkyloxy, aryloxy (optionally substituted) or amino (optionally substituted));
43) indole derivatives mentioned in the International Publication WO 96/32,379 (which is herein incorporated by reference) represented by the following formula (CXLVII): 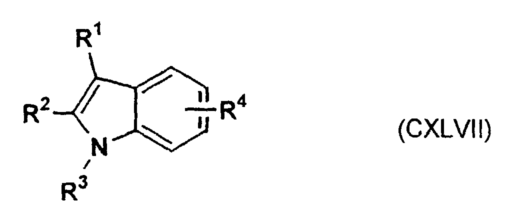 (where R¹ is hydrogen, halogen, nitro, carboxy, protected carboxy, acyl, cyano, hydroxyimino (lower) alkyl, optionally oxo-substituted lower alkyl, or lower alkyl optionally substituted with protected carboxy, carboxy or hydroxy; R² is hydrogen, halogen, lower alkyl, acyl, or lower alkyl optionally substituted with protected carboxy, carboxy, lower alkoxy or hydroxy; R³ is (1) oxo, (2) halogen, aryl, lower alkoxy, lower alkylenedioxy, cyano, nitro, carboxy, protected carboxy, acyl, or amino which may be substituted with acyl or protected carboxy, and (3) lower alkenyl or lower alkyl which may be substituted with one or more substituents selected from the group consisting of optionally halogen-substituted hetero ring; R⁴ is carboxy, protected carboxy, acyl, cyano, halogen, hetero ring, amino which may be substituted with acyl or with protected carboxy, or lower alkyl which may be substituted with protected carboxy, carboxy or acyl; and R¹ and R² may form a 4- to 7-membered carbon ring (which may be substituted with oxo) together with carbon atom bonded thereto);
44) 1H-pyrazolo[3,4-d]pyrimidin-4-one derivatives mentioned in the Japanese Laid-Open Patent Publication Hei-08/253,484 (which is herein incorporated by reference) represented by the following formula (CXLVIII): 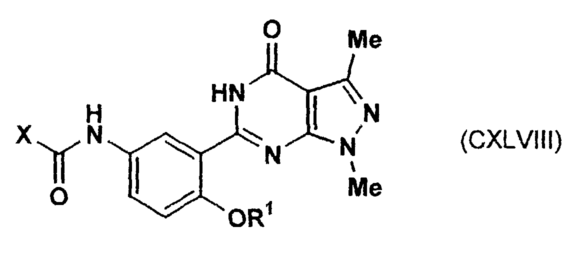 (where R¹ is alkyl having 1-4 carbon atoms; X is phenoxy or R²R³N where R² and R³ are same or different and each is hydrogen or hydroxyalkyl having 2-4 carbon atoms or, as R²R³N, it represents morpholino, piperidino, etc.);
45) 4-(arylaminomethylene)-2,4-dihydro-3-pyrazolone derivatives mentioned in the Japanese Laid-Open Patent Publication Hei-08/311,035 (which is herein incorporated by reference) represented by the following formula (CXLIX): 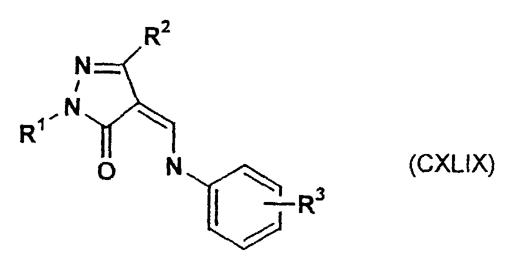 (where R¹ is benzyl, phenyl, phenyl which may be substituted with 1 to 3 amino, halogen, NO₂, CN, acyl, AO-, N,N-dialkylcarbamoyl, A-O-CO-NH-, SO₂NR⁴R⁵ (where R⁴ and R⁵ are H, C 1-6 alkyl, etc.), tetrazolyl, phospho, etc., or pyridyl; R² is C 1-5 alkyl, alkoxycarbonyl-C 1-5 alkyl, etc.; R³ is H, C 1-5 alkyl, etc.; and A is C 1-6 alkyl which may be substituted with fluorine or chlorine);
46) benzimidazole derivatives mentioned in the Japanese Laid-Open Patent Publication Hei-09/77,764 or in the International Publication WO 97/24,334 (which is herein incorporated by reference) represented by the following formula (CL): 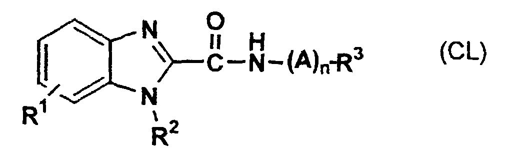 (where R¹ is hydrogen or halogen; R² is phenyl lower alkyl; R³ is a heterocyclic group selected from the group consisting of indolyl, indolinyl, 1H-indazolyl, 2(1H)-quinolinyl, 3,4-dihydro-2(1H)-quinolinyl and 3,4-dihydro-1,4(2H)-benzoxazinyl; A is lower alkylene; and n is 0 or 1); or benzimidazole derivatives that are recited in claims 13 - 19 of International Patent Publication WO 97/24334 (which is herein incorporated by reference) and which are represented by the following formula (CLI): 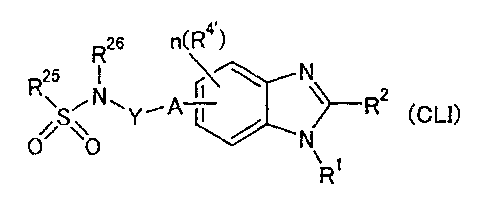 (where R¹ is a hydrogen atom, an arylsulfonyl group or a lower alkyl group, said lower alkyl group being optionally substituted either by an aryl group optionally substituted by one or two groups selected from the group consisting of a halogen atom, a haloaryl group, a lower alkyl group, a halo-lower alkyl group, a lower alkoxy group, a nitro group, an amino group, a cyano group, an aryl group, an aryl lower alkyloxy group, an arylsulfonyl lower alkyl group, an aryl lower alkyl group, a haloaryl lower alkyloxy group, an arylsulfonylamino group, an arylcarbonylamino group, an arylcarbonyl group, an arylalkenyl group, a cyanoaryl group and a heterocyclic group or by a heterocyclic group; R² is a hydrogen atom, a lower cycloalkyl group, a hydroxy group, a hydroxy lower alkyl group, a lower alkoxy group, a mercapto group, a lower alkylthio group, an amino group, a lower alkylamino group, a carboxy group, an aryl group or a lower alkyl group, said lower alkyl group being optionally substituted by a halogen atom, a lower alkoxy group, a cyano group, a halocarbonyl group, an aryl group or a heterocyclic group; R²⁵ is an alkyl group with up to 8 carbon atoms, a lower cycloalkyl group, a halo-lower alkyl group, a tri-lower alkylsilyl lower alkyl group, a lower alkoxy lower alkyl group, a lower alkylthio lower alkyl group, an aryl group, a heterocyclic group, an aryl lower alkyl group or a hydroxy lower alkyl group, said aryl group being optionally substituted by a halogen atom, a lower alkyl group, a halo-lower alkyl group, a lower alkoxy group or a nitro group; R²⁶ is a hydrogen atom or a lower alkyl group, provided that when said R²⁵ and R²⁶ are a lower alkyl group, they may bind together to form a ring; Y is a carbonyl group or a lower alkylene group; A is a single bond or represents a lower alkylene group or a lower alkenylene group; R⁴' is an optionally halogen substituted hydrocarbyl group; n represents an integer of 0 to 3), the following formula (CLII): 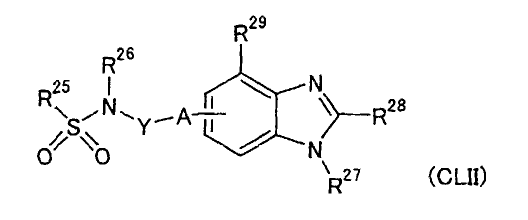 (where R²⁷ is a hydrogen atom, an alkyl group with up to 7 carbon atoms, a halo-lower alkyl group, an arylsulfonyl group, an aryl lower alkyl group, a heterocyclic lower alkyl group or a halo-heterocyclic lower alkyl group, the aromatic group in said aryl lower alkyl group being optionally substituted by one or two halogen atoms, lower alkyl groups, halo-lower alkyl groups, cyanoaryl groups, amino groups, lower alkoxy groups, nitro groups, cyano groups, aryl groups, haloaryl groups, arylsulfonyl lower alkyl groups, arylsulfonylamino groups, aryl lower alkyloxy groups, aryl lower alkyl groups, heterocyclic groups, aryloxy groups, arylcarbonyl groups, arylcarbonylamino groups, or aryl lower alkyloxy groups substituted by one or two halogen atoms; R²⁸ is a hydrogen atom, an alkyl group with up to 7 carbon atoms, a halo-lower alkyl group, a lower alkoxy lower alkyl group, a lower cycloalkyl group, an aryl group, an aryl lower alkyl group, a lower alkylamino group, a lower alkoxy group, a lower alkylthio group, a hydroxy group, a mercapto group, an amino group or a carboxyl group; R²⁵ is an alkyl group with up to 8 carbon atoms, a halo-lower alkyl group, a tri-lower alkylsilyl lower alkyl group, a lower alkoxy lower alkyl group, a lower alkylthio lower alkyl group, an aryl group, a heterocyclic group, an aryl lower alkyl group or a hydroxy lower alkyl group, said aryl group being optionally substituted by a halogen atom, a lower alkyl group, a halo lower alkyl group, a lower alkoxy group or a nitro group; R²⁶ is a hydrogen atom or a lower alkyl group, provided that when said R²⁵ and R²⁶ are a lower alkyl group, they may bind together to form a ring; Y is a carbonyl group or a lower alkylene group; A is a single bond or represents a lower alkylene group or a lower alkenylene group; R²⁹ is a hydrogen atom or a lower alkyl group), the following formula (CLIII): 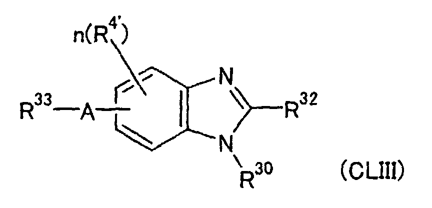 (where R³⁰ is a hydrogen atom, a lower alkyl group, an optionally substituted benzyl group represented by the following formula (CLIV): 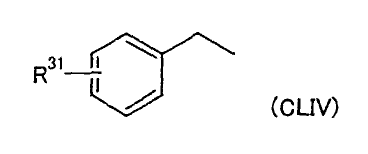 (where R³¹ is a hydrogen atom, a cyanoaryl group, an amino group, a lower alkoxy group, a nitro group, a cyano group, an aryl group, a haloaryl group, an arylsulfonyl lower alkyl group, an arylsulfonylamino group, an aryl lower alkyloxy group, an aryl lower alkyl group, a heterocyclic group or an aryloxy group), an aryl lower alkyloxy group optionally substituted by one or two halogen atoms, an arylsulfonyl group, a heterocyclic lower alkyl group, an arylcarbonylamino group, an arylcarbonyl group, an arylalkenyl group or a lower alkylene dioxyaryl group, said benzyl group being optionally further substituted by a lower alkyl group at α-site; R³² is a hydrogen atom, a lower alkyl group, a halo-lower alkyl group, a lower cycloalkyl group, an aryl group, an aryl lower alkyl group, a lower alkylamino group, a lower alkoxy group, a lower alkylthio group, a lower alkoxy lower alkyl group or a heterocyclic lower alkyl group; R³³ is a carboxyl group, a lower alkoxycarbonyl group, a (2-cyanoaryl)oxycarbonyl group or a substituent represented by the following formula (CLV): 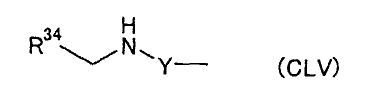 (where Y is a carbonyl group or a lower alkylene group; R³⁴ is an optionally substituted aryl group or a lower alkyl group, an aryl group or a heterocyclic group that are optionally substituted by a heterocyclic group); A is a single bond or represents a lower alkylene group or a lower alkenylene group; R⁴' is an optionally halogen substituted hydrocarbyl group; n is an integer of 0 - 3, provided that when R³⁰ is a hydrogen atom, n is 0), the following formula (CLVI): 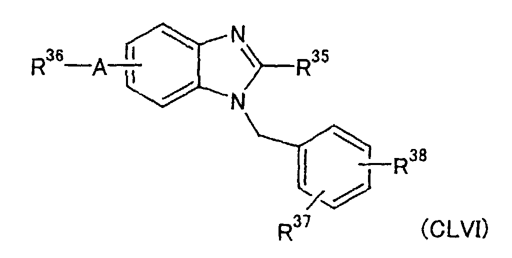 (where R³⁵ is a hydrogen atom, an aryl group, a lower alkoxy lower alkyl group, a lower alkyl group or an aryl lower alkyl group; R³⁶ is a carboxyl group, a lower alkoxycarbonyl group, a heterocyclic lower alkylamino group or a heterocyclic lower alkylcarbamoyl group; R³⁷ and R³⁸ are each independently a hydrogen atom, a halogen atom, a lower alkyl group, a halo-lower alkyl group, an aryl group, an aryl lower alkyl group or an aryl lower alkyloxy group; A is a single bond or represents a lower alkylene group or a lower alkenylene group; when R³⁵ is a lower alkyl group, A represents a lower alkylene group or a lower alkenylene group), the following formula (CLVII): 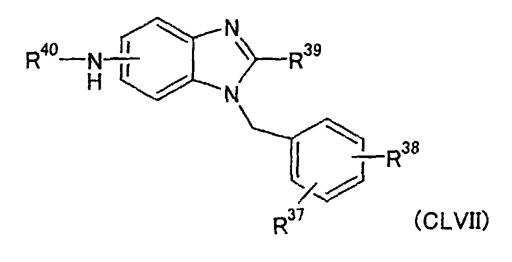 (where R³⁷ and R³⁸ are each independently a hydrogen atom, a halogen atom, a lower alkyl group, a halo-lower alkyl group, an aryl group, an aryl lower alkyl group or an aryl lower alkyloxy group; R³⁹ is a lower alkyl group; R⁴⁰ is a hydrogen atom, a lower alkoxycarbonyl group, a lower alkanoyl group, a lower alkanesulfonyl group or a carbamoyl group), the following formula (CLVIII): 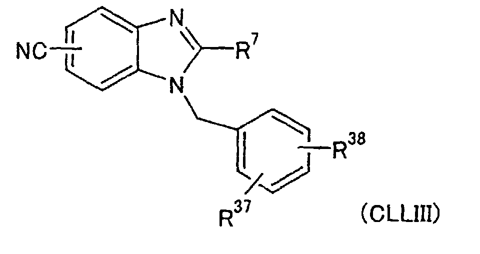 (where R³⁷ and R³⁸ are each independently a hydrogen atom, a halogen atom, a lower alkyl group, a halo-lower alkyl group, an aryl group, an aryl lower alkyl group or an aryl lower alkyloxy group; R⁷ is a lower alkyl group or a lower cycloalkyl group), and the following formula (CLIX): 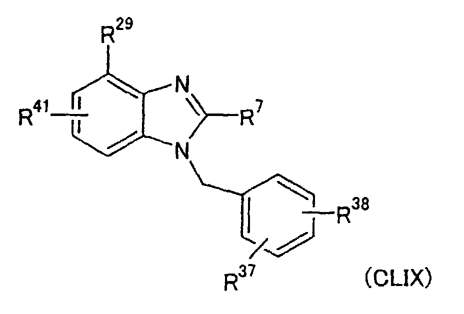 (where R³⁷ and R³⁸ are each independently a hydrogen atom, a halogen atom, a lower alkyl group, a halo-lower alkyl group, an aryl group, an aryl lower alkyl group or an aryl lower alkyloxy group; R⁷ is a lower alkyl group or a lower cycloalkyl group; R⁴¹ is a 2-pyridylcarbamoyl group, a 2-carboxy-1-pyrrolidinocarbonyl group, an N-methyl-N-(2-pyridylmethyl)carbamoyl group, a homopiperidinocarbonyl group, a [2-(N-oxo)-pyridylmethyl]carbamoyl group, a 4-(dimethylamino)benzylcarbamoyl group, a piperonylcarbamoyl group, an N-methyl-N-(2-pyridyl)carbamoyl group, a thiomorpholinocarbonyl group, a halosulfonyl group, an aminosulfonyl group, an acylaminosulfonyl group, a lower alkoxycarbonyl group or a carboxyl group; R²⁹ is a hydrogen atom or a lower alkyl group, provided that when R⁴¹ is a lower alkylcarbonyl group or a carboxyl group, R²⁹ is a lower alkyl group) or pharmaceutically acceptable salts of said benzimidazole derivatives, with the compound according to claim 20 being preferred;
47) the compounds that are described in International Patent Publication WO 98/06722 (which is herein incorporated by reference) and which are represented by the following formula (CLX): 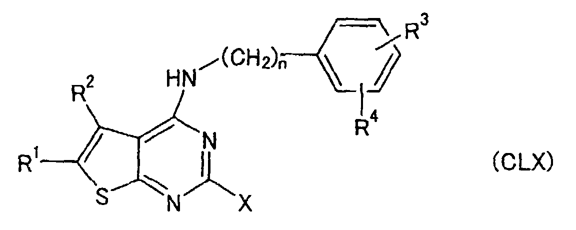 (where R¹ and R², when taken independently of each other, represent H, A, OA, alkenyl, alkynyl, NO₂, CF₃ or a halogen atom, provided that the remaining R¹ or R² is not H; R¹ and R² taken together represent an alkylene with 3 - 5 carbon atoms; R³ and R⁴, when taken independently of each other, represent H, A, OA, a halogen atom, NO₂, NH₂, NHA or NAA'; R³ and R⁴ taken together represent -O-CH₂-CH₂-, -O-CH₂- or -O-CH₂-CH₂-O-; A and A', when taken independently of each other, represent an alkyl group with 1 - 6 carbon atoms; X represents 5 - 7 unsaturated hetero rings that are either unsubstituted or mono-, di-or tri-substituted by A, a halogen atom or CF₃, provided that the hetero ring contains 1 - 4 nitrogen atoms, oxygen atoms and/or sulfur atoms and that the additional remaining CH₂ groups in the hetero ring may be substituted by NH, NA, S or O and are bound by N or C; X represents a fluorine atom, a chlorine atom, a bromine atom or an iodine atom; n represents 0, 1, 2 or 3) or pharmacologically acceptable salts of said compounds;
48) imidazoquinazoline derivatives that are described in International Patent Publication WO 98/08848 (which is herein incorporated by reference) and which are represented by the following formula (CLXI): 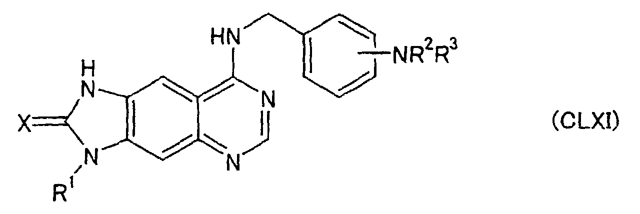 (where R¹ represents hydrogen, a substituted or unsubstituted lower alkyl, a substituted or unsubstituted cycloalkyl, a substituted or unsubstituted bicycloalkyl, a substituted or unsubstituted tricycloalkyl, a substituted or unsubstituted benzocycloalkenyl, a substituted or unsubstituted lower alkenyl, a substituted or unsubstituted aralkyl, a substituted or unsubstituted aryl or a substituted or unsubstituted heteroaryl; R² represents hydrogen, a substituted or unsubstituted lower alkyl, a substituted or unsubstituted cycloalkyl, a substituted or unsubstituted bicycloalkyl, a substituted or unsubstituted tricycloalkyl, a substituted or unsubstituted benzocycloalkenyl, a substituted or unsubstituted lower alkenyl, a substituted or unsubstituted aralkyl, a substituted or unsubstituted aryl or a substituted or unsubstituted heteroaryl; R³ represents hydrogen, a substituted lower alkyl, a substituted or unsubstituted cycloalkyl, a substituted or unsubstituted bicycloalkyl, a substituted or unsubstituted tricycloalkyl, a substituted or unsubstituted benzocycloalkenyl, a substituted or unsubstituted lower alkenyl, a substituted or unsubstituted aralkyl, a substituted or unsubstituted aryl or a substituted or unsubstituted heteroaryl; R² and R³, when taken together, may represent a substituted or unsubstituted heterocyclic group that is formed in the presence of N; X represents O or S).

The thirteenth aspect of the present invention is a composition for remedy of erectile dysfunction by percutaneous administration wherein the composition contains at least one of the compounds having an inhibitory action to cyclic GMP phosphodiesterase and salts or solvates thereof as an effective component.

The fourteenth aspect of the present invention is a composition for remedy of erectile dysfunction by percutaneous administration wherein the composition contains at least one of the compounds represented by the above-mentioned formula (XXV) and salts or solvates thereof as an effective component. The preferred compound is sildenafil.

The fifteenth aspect of the present invention is a composition for remedy of erectile dysfunction by percutaneous administration which is at least one of 5-[2-ethoxy-5-(4-methylpiperazin-1-ylsulfonyl)phenyl)-1-methyl-3-propyl-6,7-dihydro-1H-pyrazolo[4,3-d]pyrimidin-7-one and salts thereof.

The sixteenth aspect of the present invention is a composition for remedy of erectile dysfunction by percutaneous administration wherein the composition contains at least one of the following compounds having an inhibitory action to cGMP-PDE and salts or solvates thereof:
1) 2-piperazinyl-6,7-dimethoxyquinazoline derivatives mentioned in the Japanese Laid-Open Patent Publication Sho-52/100,479;
2) heterocyclopyrimidine derivatives mentioned in the Japanese Laid-Open Patent Publication Sho-53/103,497;
3) 5-substituted compounds of pyrazolo[4,3-d]pyrimidin-7-ones mentioned in the Japanese Laid-Open Patent Publication Sho-61/236,778;
4) griseol derivatives mentioned in the Japanese Laid-Open Patent Publication Sho-62/30,796 or Sho-62/30,782;
5) KS 506 mentioned in the Japanese Laid-Open Patent Publication Hei-02/1,463;
6) 1,4-dihydropyridine derivatives mentioned in the Japanese Laid-Open Patent Publication Hei-02/223,580;
7) pyrazolopyrimidinone derivatives mentioned in the International Publication WO 93/06,104 or WO 93/07,149;
8) nitrogen-containing heterocyclic compounds mentioned in the International Publication WO 93/07,124 or the Japanese Laid-Open Patent Publication Hei-07/10,843;
9) quinazolinone derivatives mentioned in the International Publication WO 93/12,095;
10) purin-6-ones mentioned in the International Publication WO 94/00,453 or the Japanese Laid-Open Patent Publication Hei-09/124,648;
11) pyrazolo[3,4-d]pyrimidin-4-one derivatives mentioned in the U.S. Patent No. 5,294,612;
12) pyridopyrimidinone derivatives mentioned in the International Publication WO 94/05,561;
13) imidazoquinazoline derivatives mentioned in the International Publication WO 95/06,648 or WO 96/26,940;
14) quinazoline derivatives mentioned in the Japanese Laid-Open Patent Publication Hei-07/126,255;
15) anthranilic acid derivatives mentioned in the Japanese Laid-Open Patent Publication Hei-08/188,563;
16) benzofuro[3,2-d]pyrimidin-4-one derivatives mentioned in the Japanese Laid-Open Patent Publication Hei-07/267,961;
17) MS-681 mentioned in the Japanese Laid-Open Patent Publication Hei-07/285,993;
18) thieno[3,2-d]pyrimidine-4-one derivatives mentioned in the Japanese Laid-Open Patent Publication Hei-08/143,571;
19) phthalazine derivatives mentioned in the International Publication WO 96/05,176;
20) quinazolin-4(3H)-one derivatives mentioned in the Japanese Laid-Open Patent Publication Hei-08/104,679;
21) pyrazolopyrimidin-4-one derivatives mentioned in the U.S. Patent No. 5,541,187;
22) pyrido[3,2-e]pyrazinone derivatives mentioned in the Japanese Laid-Open Patent Publication Hei-08/269,059; and
23) 1H-pyrazolo[3,4-d]pyrimidin-4-one derivatives mentioned in the Japanese Laid-Open Patent Publication Hei-08/253,484.

General formulae for the above-mentioned compounds are the same as those mentioned in the twelfth aspect of the present invention.

The seventeenth aspect of the present invention is a composition for intranasal administration wherein the composition contains at least one of the compounds having an inhibitory action to cyclic GMP phosphodiesterase and salts or solvates thereof as an effective component.

The eighteenth aspect of the present invention is a composition for intranasal administration wherein the composition contains at least one of the compounds represented by the formula (XXV) and salts or solvates thereof as an effective component. The preferred compound is sildenafil.

The nineteenth aspect of the present invention is a composition for intranasal administration which is at least one of 5-[2-ethoxy-5-(4-methylpiperazin-1-ylsulfonyl)phenyl]-1-methyl-3-propyl-6,7-dihydro-1H-pyrazolo[4,3-d]pyrimidin-7-one and salts thereof.

The twentieth aspect of the present invention is a composition for intranasal administration wherein the composition contains at least one of the compounds mentioned hereunder having an inhibitory action to cGMP-PDE and salts or solvates thereof:
1) 2-piperazinyl-6,7-dimethoxyquinazoline derivatives mentioned in the Japanese Laid-Open Patent Publication Sho-52/100,479;
2) heterocyclopyrimidine derivatives mentioned in the Japanese Laid-Open Patent Publication Sho-53/103,497;
3) 5-substituted compounds of pyrazolo[4,3-d]pyrimidin-7-ones mentioned in the Japanese Laid-Open Patent Publication Sho-61/236,778;
4) grizeol derivatives mentioned in the Japanese Laid-Open Patent Publication Sho-62/30,796 or Sho-62/30,782;
5) purinone derivatives mentioned in the Japanese Laid-Open Patent Publication Sho-63/196,585 or Hei-02/88,577;
6) KS 506 mentioned in the Japanese Laid-Open Patent Publication Hei-02/1,463;
7) phenylpyridone derivatives mentioned in the Japanese Laid-Open Patent Publications Hei-01/311,067 or Hei-03/145,466;
8) 1,4-dihydropyridine derivatives mentioned in the Japanese Laid-Open Patent Publication Hei-02/223,580;
9) condensed pyrimidine derivatives mentioned in the Japanese Laid-Open Patent Publication Hei-02/42,079 or Hei-02/56,484;
10) pyrimidopyrimidine derivatives mentioned in the Japanese Laid-Open Patent Publication Hei-02/40,388 or Hei-03/261,785;
11) quinazolinone derivatives mentioned in the Japanese Laid-Open Patent Publication Hei-02/193,983 or International Publication WO 93/12,095;
12) phenylpyrimidinone derivatives mentioned in the Japanese Laid-Open Patent Publication Hei-02/295,977 or Hei-02/295,978;
13) polycyclic guanine derivatives mentioned in the International Publication WO 91/19,717;
14) pyrazolopyrimidinone derivatives mentioned in the International Publication WO 93/06,104 or WO 93/07,149;
15) nitrogen-containing heterocyclic compounds mentioned in the International Publication WO 93/07,124 or the Japanese Laid-Open Patent Publication Hei-07/10,843;
16) benzimidazole derivatives mentioned in the Japanese Laid-Open Patent Publication Hei-05/222,000;
17) WS 63967 mentioned in the Japanese Laid-Open Patent Publication Hei-05/301,857;
18) 4-aminoquinazoline derivatives mentioned in any one of the Japanese Laid-Open Patent Publications Hei-06/192,235, Hei-08/99,962 and Hei-07/188,214, and U.S. Patents No. 5,436,233 and No. 5,439,895;
19) purin-6-ones mentioned in the International Publication WO 94/00,453 or the Japanese Laid-Open Patent Publication Hei-09/124,648;
20) pyrazolo[3,4-d]pyrimidin-4-one derivatives mentioned in the U.S. Patent No. 5,294,612;
21) pyridopyrimidinone derivatives mentioned in the International Publication WO 94/05,561;
22) 2-benzyl-polycyclic guanine derivatives mentioned in the International Publication WO 94/19,351;
23) amilino- or pyrazylamino-cyclobuten-1,2-dione derivatives mentioned in the International Publication WO 94/29,277;
24) fluorenone derivatives mentioned in the Japanese Laid-Open Patent Publication Hei-07/61,949;
25) 4-Aminopyrimidine derivatives mentioned in the Japanese Laid-Open Patent Publication Hei-07/89,958;
26) imidazoquinazoline derivatives mentioned in the International Publication WO 95/06,648 or WO 96/26,940;
27) quinazoline derivatives mentioned in the Japanese Laid-Open Patent Publication Hei-07/126,255;
28) anthranilic acid derivatives mentioned in the Japanese Laid-Open Patent Publication Hei-08/188,563;
29) benzofuro[3,2-d]pyrimidin-4-one derivatives mentioned in the Japanese Laid-Open Patent Publication Hei-07/267,961;
30) MS-681 mentioned in the Japanese Laid-Open Patent Publication Hei-07/285,993;
31) thieno[3,2-d]pyrimidin-4-one derivatives mentioned in the Japanese Laid-Open Patent Publication Hei-07/330,777 or Hei-08/143,571;
32) phthalazine derivatives mentioned in the International Publication WO 96/05,176;
33) FR 901526 mentioned in the Japanese Laid-Open Patent Publication Hei-08/59,681;
34) pyrazoloquinoline derivatives mentioned in the International Publication WO 96/28,446;
35) quinazolin-4(3H)-one derivatives mentioned in the Japanese Laid-Open Patent Publication Hei-08/104,679;
36) 4-aminopyrimidine derivatives mentioned in the U.S. Patent No. 5,525,604;
37) 2,8-disubstituted quinazolinone derivatives mentioned in the Japanese Laid-Open Patent Publication Hei-08/253,457;
38) 2,9-disubstituted purin-6-one derivatives mentioned in the Japanese Laid-Open Patent Publication Hei-08/231,545;
39) 9-substituted 2-(2-n-alkoxyphenyl)-purin-6-one derivatives mentioned in the Japanese Laid-Open Patent Publication Hei-08/231,546;
40) pyrazolopyrimidin-4-one derivatives mentioned in any one of the U.S. Patent No. 5,541,187 and the International Publications WO 96/28,429 and WO 96/28,448;
41) heterocyclic compounds mentioned in the Japanese Laid-Open Patent Publication Hei-08/269,060;
42) pyrido[3,2-e]pyrazinone derivatives mentioned in the Japanese Laid-Open Patent Publication Hei-08/269,059;
43) indole derivatives mentioned in the International Publication WO 96/32,379;
44) 1H-pyrazolo[3,4-d]pyrimidin-4-one derivatives mentioned in the Japanese Laid-Open Patent Publication Hei-08/253,484;
45) 4-(arylaminomethylene)-2,4-dihydro-3-pyrazolone derivatives mentioned in the Japanese Laid-Open Patent Publication Hei-08/311,035;
46) benzimidazole derivatives mentioned in the Japanese Laid-Open Patent Publication Hei-09/77,764 or the International Publication WO 97/24,334.
47) thienopyrimidine derivatives mentioned in the International Publication WO 98/06722; and
48) imidazoquinazoline derivatives mentioned in the International Publication WO 98/08848.

The general chemical formulae set forth above are the same as can be found in the description of the twelfth embodiment of the invention.

In its twenty-first embodiment, the present invention relates to the use of the compounds of the above formula (I), or salts or solvates thereof for preparing the intranasal compositions or the pharmaceutical compositions to be used as therapeutics of erectile dysfunction or female sexual dysfunction that are recited in the first to the eighth embodiments of the invention.

The twenty-second aspect of the present invention is a method for the therapy of erectile dysfunction or female sexual dysfunction which comprises administrating a composition containing a compound represented by the above formula (I) which is mentioned in the above first to eighth embodiments of the present invention or a salt or solvate thereof.

Embodiments of the present invention further include the use of a compound represented by the above-mentioned formulae (XXV) to (CL) or a salt or solvate thereof for preparing a composition mentioned in the above ninth to twentieth embodiments of the present invention and also include a method for the therapy of erectile dysfunction or female sexual dysfunction which comprises administrating a composition containing a compound represented by the above-mentioned formulae (XXV) to (CL) or a salt or solvate thereof.

### BRIEF DESCRIPTION OF THE DRAWINGS

Fig. 1 shows the structural formulae of the intermediates obtained in several examples of the invention;
Fig. 2 shows the structural formulae of the intermediates obtained in other examples of the invention;
Fig. 3 shows the structural formulae of the intermediates obtained in yet other examples of the invention; and

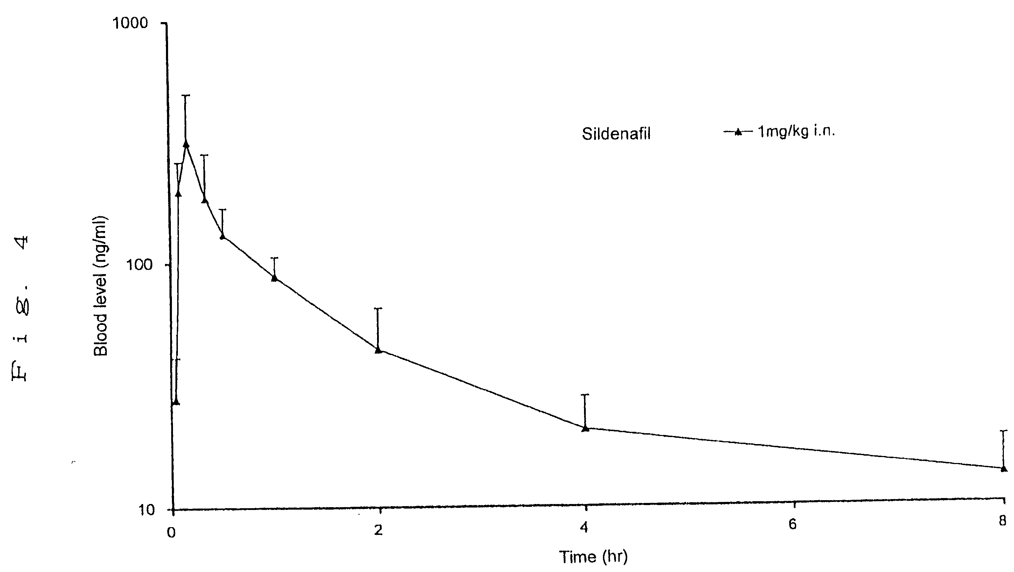

Fig. 4 is a graph showing the profile of blood level of sildenafil after its administration into the nasal cavity.

### Embodiment of the Invention

The present invention will now be described below in detail.

The pyridocarbazole derivatives which form the effective component of the composition of the invention have the respective position numbers shown in the following diagram; R¹ is bound in 1-, 2- or 3-position; R² or R³ is bound in 8-, 9-, 10- or 11-position; R⁴ is bound in 5-position; and R⁵ is bound in 6-position:

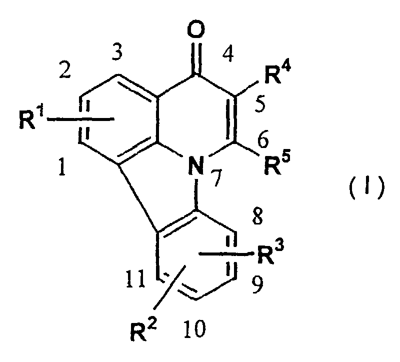

The compounds which form the effective component of the composition of the invention are represented by the formula (I) set forth above. In the formula, R¹ represents a hydrogen atom, a halogen atom, a cyano group, an optionally protected carboxyl group, an optionally protected carboxymethyl group, an alkoxycarbonyl group having 1 - 4 carbon atoms, a carbamoyl group, an acetylamino group, a 3-carboxy-1-propenyl group, a 2-hydroxypentyloxy group, a 2,2-diethoxyethoxy group, an optionally protected hydroxyl group, an optionally protected mercapto group, a straight- or branched-chain alkanoyloxy group having 1 - 4 carbon atoms, a carbonyloxy group substituted with a phenyl group or a pyridyl group, a straight- or branched-chain alkyl group having 1 - 4 carbon atoms which may be substituted with one hydroxyl group, an amino group which may be mono- or disubstituted with an alkyl group having 1 - 4 carbon atoms, an alkylthio group having 1 - 3 carbon atoms which may be monosubstituted with any group selected from the group consisting of a hydroxyl group, a carboxyl group, a phenyl group and a pyridyl group, or represented by the following formula (XXI):

―O―(CH₂)ₙ―Z (XXI)

(where Z is selected from the group consisting of a hydrogen atom, a carboxyl group, an alkoxy group having 1 or 2 carbon atoms which may be substituted with one hydroxyl group, an alkoxycarbonyl group having 1 - 6 carbon atoms, a carbamoyl group which may be mono- or disubstituted with a hydroxymethyl group or an alkyl group having 1 or 2 carbon atoms, an alkanoyl group having 1 - 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 - 4 carbon atoms or an alkoxycarbonyl group having 1 or 2 carbon atoms, a pyrazinyl group, a pyrimidinyl group, a furyl group, a thienyl group, an oxadiazolyl group and a 4-methoxyphenoxy group; n is 1 - 6).

More specifically, the term "halogen atom" refers to a fluorine atom, a chlorine atom or a bromine atom; the term "alkoxycarbonyl group having 1 - 4 carbon atoms" refers to a methoxycarbonyl group, an ethoxycarbonyl group, a n-propoxycarbonyl group, an i-propoxycarbonyl group, a cyclopropoxycarbonyl group, a n-butoxycarbonyl group, a t-butoxycarbonyl group or the like; the term "optionally protected hydroxyl group" refers to a hydroxyl group, a trimethylsilyloxy group, a t-butyldimethylsilyloxy group, a methoxymethyloxy group or the like; the term "optionally protected mercapto group" refers to a phenylthio group, a benzylthio group or the like; the term "straight- or branched-chain alkanoyloxy group having 1 - 4 carbon atoms" refers to an acetoxy group, a propionyloxy group, a butyryloxy group, a pivaloyloxy group or the like; the term "carbonyloxy group substituted with a phenyl group or a pyridyl group" refers to a benzoyloxy group, a nicotinoyloxy group, an isonicotinoyloxy group or the like; the term "straight- or branched-chain alkyl group having 1 - 4 carbon atoms which may be substituted with one hydroxyl group" refers to a methyl group, an ethyl group, a n-propyl group, an i-propyl group, a n-butyl group, a t-butyl group, a 1-hydroxyethyl group, a 2-hydroxyethyl group, a 1-hydroxypropyl group or the like; the term "amino group which may be mono- or disubstituted with an alkyl group having 1 - 4 carbon atoms" refers to a methylamino group, a dimethylamino group, an ethylamino group, a diethylamino group, a n-propylamino group, a n-butylamino group or the like; the term "alkylthio group having 1 - 3 carbon atoms which may be monosubstituted with any group selected from the group consisting of a hydroxyl group, a carboxyl group, a phenyl group and a pyridyl group" refers to a methylthio group, an ethylthio group, a 3-hydroxypropylthio group, a carboxymethylthio group, a 3-pyridylmethylthio group or the like; the following formula (XXI):

―O―(CH₂)ₙ―Z (XXI)

(where Z is selected from the group consisting of a hydrogen atom, a carboxyl group, an alkoxy group having 1 or 2 carbon atoms which may be substituted with one hydroxyl group, an alkoxycarbonyl group having 1 - 6 carbon atoms, a carbamoyl group which may be mono- or disubstituted with a hydroxymethyl group or an alkyl group having 1 or 2 carbon atoms, an alkanoyl group having 1 - 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 - 4 carbon atoms or an alkoxycarbonyl group having 1 or 2 carbon atoms, a pyrazinyl group, a pyrimidinyl group, a furyl group, a thienyl group, an oxadiazolyl group and a 4-methoxyphenoxy group; n is 1 - 6) refers to a methoxy group, an ethoxy group, a n-propoxy group, a n-butoxy group, a n-pentyloxy group, a n-hexyloxy group, a carboxymethyloxy group, a 2-carboxyethyloxy group, a 3-carboxypropyloxy group, a methoxymethoxy group, an ethoxymethoxy group, a 2-methoxyethoxy group, a 2-ethoxyethoxy group, a 2-(2-hydroxyethoxy)ethoxy group, a methoxycarbonylmethyloxy group, an ethoxycarbonylmethyloxy group, a n-propoxycarbonylmethyloxy group, an i-propoxycarbonylmethyloxy group, a n-butoxycarbonylmethyloxy group, a t-butoxycarbonylmethyloxy group, a n-pentyloxycarbonylmethyloxy group, a n-hexyloxycarbonylmethyloxy group, a cyclopropyloxycarbonylmethyloxy group, a cyclohexyloxycarbonylmethyloxy group, a 2-(methoxycarbonyl)ethyloxy group, a 2-(ethoxycarbonyl)ethyloxy group, a 2-(n-propoxycarbonyl)ethyloxy group, a 2-(i-propoxycarbonyl)ethyloxy group, a 2-(n-butoxycarbonyl)ethyloxy group, a 2-(t-butoxycarbonyl)ethyloxy group, a 2-(n-pentyloxycarbonyl)ethyloxy group, a 2-(n-hexyloxycarbonyl)ethyloxy group, a 2-(cyclopropyloxycarbonyl)ethyloxy group, a 2-(cyclohexyloxycarbonyl)ethyloxy group, a 3-(methoxycarbonyl)propyloxy group, a 3-(ethoxycarbonyl)propyloxy group, a 3-(n-propoxycarbonyl)propyloxy group, a 3-(i-propoxycarbonyl)propyloxy group, a 3-(n-butoxycarbonyl)propyloxy group, a 3-(t-butoxycarbonyl)propyloxy group, a 3-(n-pentyloxycarbonyl)propyloxy group, a 3-(n-hexyloxycarbonyl)propyloxy group, a 3-(cyclopropyloxycarbonyl)propyloxy group, a 3-(cyclohexyloxycarbonyl)propyloxy group, a N-hydroxymethylcarbamoylmethyloxy group, a N-methylcarbamoylmethyloxy group, a N,N-dimethylcarbamoylmethyloxy group, a N-ethylcarbamoylmethyloxy group, a N,N-diethylcarbamoylmethyloxy group, a N-n-propylcarbamoylmethyloxy group, a N-n-butylcarbamoylmethyloxy group, a 3-hydroxy-2-oxopropyloxy group, a 4-hydroxy-3-oxobutyloxy group, a 5-hydroxy-4-oxopentyloxy group, a 4-hydroxy-2-oxobutyloxy group, a 5-hydroxy-2-oxopentyloxy group, a 6-hydroxy-2-oxohexyloxy group, a 5-mercapto-2-oxopentyloxy group, a 4-carboxy-1-piperidinylcarbonylmethyloxy group, a 4-methoxycarbonyl-1-piperidinylcarbonylmethyloxy group, a 4-ethoxycarbonyl-1-piperidinylcarbonylmethyloxy group, a 4-morpholylcarbonylmethyloxy group, a 2-hydroxyethyloxy group, a 3-hydroxypropyloxy group, a 4-hydroxybutyloxy group, a 2-mercaptoethyloxy group, a 3-mercaptopropyloxy group, a 4-mercaptobutyloxy group, a 2-aminoethyloxy group, a 3-aminopropyloxy group, a 4-aminobutyloxy group, a benzyloxy group, a 2-phenethyloxy group, a 3-phenylpropyloxy group, a 5-hydroxymethyl-3-pyridylmethyloxy group, a 5-acetoxymethyl-3-pyridylmethyloxy group, a 6-hydroxymethyl-2-pyridylmethyloxy group, a 6-acetoxymethyl-2-pyridylmethylhoxy group, a 5-methyl-3-pyridylmethyloxy group, a 6-methyl-2-pyridylmethyloxy group, a 5-ethyl-3-pyridylmethyloxy group, a 5-t-butyl-3-pyridylmethyloxy group, a 5-methoxycarbonyl-3-pyridylmethyloxy group, a 5-ethoxycarbonyl-3-pyridylmethyloxy group, a 2-pyrazinylmethyloxy group, a 2-pyrimidinylmethyloxy group, a 4-pyrimidinylmethyloxy group, a 5-pyrimidinylmethyloxy group, a 2-furylmethyloxy group, a 3-furylmethyloxy group, a 2-thienylmethyloxy group, a 3-thienylmethyloxy group, a 3-oxadiazolylmethyloxy group, a 2-(4-methoxyphenoxy)ethyloxy group, a 3-(4-methoxyphenoxy)propyloxy group, a 4-(4-methoxyphenoxy)butyloxy group, or the like.

Preferably, R¹ is substituted in 2-position and it represents a hydroxyl group, a methoxy group, a carboxymethyloxy group, a 2-carboxyethyloxy group, a 3-carboxypropyloxy group, a methoxycarbonylmethyloxy group, an ethoxycarbonylmethyloxy group, a n-propoxycarbonylmethyloxy group, an i-propoxycarbonylmethyloxy group, a n-butoxycarbonylmethyloxy group, a t-butoxycarbonylmethyloxy group, a N-hydroxymethylcarbamoylmethyloxy group, a N-ethylcarbamoylmethyloxy group, a 4-hydroxy-2-oxobutyloxy group, a 5-hydroxy-2-oxopentyloxy group, a 2-hydroxyethyloxy group, a 3-hydroxypropyloxy group, a 4-hydroxybutyloxy group, a 3-aminopropyloxy group, a 4-aminobutyloxy group, a benzyloxy group, a 5-hydroxymethyl-3-pyridylmethyloxy group, a 5-acetoxymethyl-3-pyridylmethyloxy group, a 6-hydroxymethyl-2-pyridylmethyloxy group, a 6-acetoxymethyl-2-pyridylmethyloxy group, a 5-methyl-3-pyridylmethyloxy group, a 6-methyl-2-pyridylmethyloxy group, a 2-pyridylmethyloxy group, a 3-pyridylmethyloxy group, a 4-pyridylmethyloxy group, a 2-pyrazinylmethyloxy group, a 2-pyrimidinylmethyloxy group, a 4-pyrimidinylmethyloxy group, a 5-pyrimidinylmethyloxy group.

More preferably, R¹ represents a hydroxyl group, a methoxy group, a carboxymethyloxy group, a 2-carboxyethyloxy group, a 3-carboxypropyloxy group, a N-hydroxymethylcarbamoylmethyloxy group, a N-ethylcarbamoylmethyloxy group, a 4-hydroxy-2-oxobutyloxy group, a 5-hydroxy-2-oxopentyloxy group, a 2-hydroxyethyloxy group, a 3-hydroxypropyloxy group, a 4-hydroxybutyloxy group, a benzyloxy group, a 2-pyridylmethyloxy group, a 3-pyridylmethyloxy group, a 4-pyridylmethyloxy group, a 2-pyrazinylmethyloxy group, a 2-pyrimidinylmethyloxy group, a 4-pyrimidinylmethyloxy group, a 5-pyrimidinylmethyloxy group.

In the formula (I), R² represents a hydrogen atom, a halogen atom, an optionally protected hydroxyl group, an optionally protected mercapto group, an optionally protected amino group, a cyano group, a nitro group, a trifluoromethyl group, a trifluoromethoxy group, an optionally protected carboxyl group, a 4-morpholylacetyl group, a straight- or branched-chain alkanoyloxy group having 1 - 4 carbon atoms, a straight- or branched-chain alkanoyl group having 1 - 4 carbon atoms, a straight- or branched-chain alkyl group having 1 - 4 carbon atoms, an alkylthio group having 1 - 3 carbon atoms which may be monosubstituted with any group selected from the group consisting of a hydroxyl group, a carboxyl group, a phenyl group and a pyridyl group or a straight- or branched-chain alkoxy group having 1 - 4 carbon atoms which may be substituted with one alkoxycarbonyl group having 1 - 4 carbon atoms.

More specifically, the "halogen atom" refers to a fluorine atom, a chlorine atom or a bromine atom; the "optionally protected hydroxyl group" refers to a hydroxyl group, a trimethylsilyloxy group, a t-butyldimethylsilyloxy group, a methoxymethyloxy group or the like; the "optionally protected mercapto group" refers to a phenylthio group, a benzylthio group or the like; the "straight- or branched-chain alkanoyloxy group having 1 - 4 carbon atoms" refers to an acetoxy group, a propiomethylthio group, a 3-pyridylmethylthio group or the like; the "straight- or branched-chain alkoxy group having 1 - 4 carbon atoms which may be substituted with one alkoxycarbonyl group having 1 - 4 carbon atoms" refers to a methoxy group, an ethoxy group, a n-propoxy group, an i-propoxy group, a n-butoxy group, a t-butoxy group, a methoxycarbonylmethyloxy group, an ethoxycarbonylmethyloxy group, a n-propoxycarbonylmethyloxy group, an i-propoxycarbonylmethyloxy group, a n-butoxycarbonylmethyloxy group, a t-butoxycarbonylmethyloxy group, a 2-(methoxycarbonyl)ethyloxy group, a 2-(ethoxycarbonyl)ethyloxy group, a 2-(n-propoxycarbonyl)ethyloxy group, a 2-(i-propoxycarbonyl)ethyloxy group, a 2-(n-butoxycarbonyl)ethyloxy group, a 2-(t-butoxycarbonyl)ethyloxy group, a 2-(n-pentyloxycarbonyl)ethyloxy group, a 3-(methoxycarbonyl)propyloxy group, a 3-(ethoxycarbonyl)propyloxy group, a 3-(n-propoxycarbonyl)propyloxy group, a 3-(i-propoxycarbonyl)propyloxy group, a 3-(n-butoxycarbonyl)propyloxy group, a 3-(t-butoxycarbonyl)propyloxy group, or the like.

Preferably, R² is substituted in 9- or 10-position and represents a hydrogen atom, a hydroxyl group, a fluorine atom, a chlorine atom, a bromine atom, a methoxy group, an ethoxy group, a n-propoxy group, an i-propoxy group, a n-butoxy group, a t-butoxy group or a trifluoromethyl group.

More preferably, R² is substituted in 9-position and represents a chlorine atom, a bromine atom or a trifluoromethyl group.

In the formula (I), R³ represents a hydrogen atom, a halogen atom, an optionally protected hydroxyl group or a straight- or branched-chain alkoxy group having 1 - 4 carbon atoms. More specifically, the "halogen atom" refers to a fluorine atom, a chlorine atom, a bromine atom or the like; the "optionally protected hydroxyl group" refers to a hydroxyl group, a trimethylsilyloxy group, a t-butyldimethylsilyloxy group, a methoxymethyloxy group or the like; the "straight- or branched-chain alkoxy group having 1 - 4 carbon atoms" refers to a methoxy group, an ethoxy group, a n-propoxy group, an i-propoxy group, a cyclopropoxy group, a n-butoxy group, a t-butoxy group, or the like.

Preferably, R³ represents a hydrogen atom, a hydroxyl group, a fluorine atom, a chlorine atom, a bromine atom, a methoxy group, an ethoxy group, a n-propoxy group, an i-propoxy group, a n-butoxy group or a t-butoxy group. More preferably, R³ represents a hydrogen atom.

Preferably, R² and R³ are not a hydrogen atom at the same time.

The preferred combinations of R² and R³ are such that R² is substituted in 9- or 10-position and represents a hydrogen atom, a halogen atom, a hydroxyl group, a trifluoromethyl group or a straight- or branched-chain alkoxy group having 1 - 4 carbon atoms and R³ is a hydrogen atom. More preferably, R² is a halogen atom or a trifluoromethyl group which are substituted in 9-position and R³ is a hydrogen atom.

In the formula (I), R⁴ represents a hydrogen atom, a halogen atom, an optionally protected carboxyl group, a phenoxy group, an anilino group, a N-methylanilino group, a 4-morpholylcarbonyl group, an alkyl group having 1 or 2 carbon atoms which may be substituted with a cyclic alkyl group having 3 - 6 carbon atoms, a benzyl group which may be mono- or disubstituted in the phenyl portion with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 - 4 carbon atoms, an acetylamino group, a carboxyl group and an amino group, a pyridylmethyl group which may be substituted with an alkyl group having 1 - 4 carbon atoms, a morpholylmethyl group, a triazolylmethyl group, a furylmethyl group, a thienylmethyl group, a pyrimidinylmethyl group, a pyrazinylmethyl group, a pyrrolylmethyl group, an imidazolylmethyl group, a quinolylmethyl group, an indolylmethyl group, a naphthylmethyl group, a benzoyl group, an α-hydroxybenzyl group or an alkoxycarbonyl group having 1 or 2 carbon atoms.

More specifically, the "halogen atom" refers to a fluorine atom, a chlorine atom, a bromine atom or the like; the "alkyl group having 1 or 2 carbon atoms which may be substituted with a cyclic alkyl group having 3 - 6 carbon atoms" refers to a methyl group, an ethyl group, a cyclopropylmethyl group, a cyclohexylmethyl group or the like; the "benzyl group which may be mono- or disubstituted in the phenyl portion with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 - 4 carbon atoms, an acetylamino group, a carboxyl group and an amino group" refers to a 2-fluorobenzyl group, a 2-chlorobenzyl group, a 2-bromobenzyl group, a 3-fluorobenzyl group, a 3-chlorobenzyl group, a 3-bromobenzyl group, a 4-fluorobenzyl group, a 4-chlorobenzyl group, a 4-bromobenzyl group, a 2-hydroxybenzyl group, a 3-hydroxybenzyl group, a 4-hydroxybenzyl group, a 2-mercaptobenzyl group, a 3-mercaptobenzyl group, a 4-mercaptobenzyl group, a 2-methoxybenzyl group, a 3-methoxybenzyl group, a 4-methoxybenzyl group, a 2-ethoxybenzyl group, a 3-ethoxybenzyl group, a 4-ethoxybenzyl group, a 2-methylthiobenzyl group, a 3-methylthiobenzyl group, a 4-methylthiobenzyl group, a 2-ethylthiobenzyl group, a 3-ethylthiobenzyl group, a 4-ethylthiobenzyl group, a 2-methoxycarbonylbenzyl group, a 3-methoxycarbonylbenzyl group, a 4-methoxycarbonylbenzyl group, a 2-ethoxycarbonylbenzyl group, a 3-ethoxycarbonylbenzyl group, a 4-ethoxycarbonylbenzyl group, a 2-t-butoxycarbonylbenzyl group, a 3-t-butoxycarbonylbenzyl group, a 4-t-butoxycarbonylbenzyl group, a 2-acetylaminobenzyl group, a 3-acetylaminobenzyl group, a 4-acetylaminobenzyl group, a 2-carboxybenzyl group, a 3-carboxybenzyl group, a 4-carboxybenzyl group, a 2-aminobenzyl group, a 3-aminobenzyl group, a 4-aminobenzyl group, a 2,3-difluorobenzyl group, a 2,4-difluorobenzyl group, a 2,5-difluorobenzyl group, a 3,4-difluorobenzyl group, a 3,5-difluorobenzyl group, a 2,3-dichlorobenzyl group, a 2,4-dichlorobenzyl group, a 2,5-dichlorobenzyl group, a 3,4-dichlorobenzyl group, a 3,5-dichlorobenzyl group, a 2,3-dibromobenzyl group, a 2,4-dibromobenzyl group, a 2,5-dibromobenzyl group, a 3,4-dibromobenzyl group, a 3,5-dibromobenzyl group, a 2,3-dihydroxybenzyl group, a 2,4-dihydroxybenzyl group, a 2,5-dihydroxybenzyl group, a 3,4-dihydroxybenzyl group, a 3,5-dihydroxybenzyl group, a 2,3-dimethoxybenzyl group, a 2,4-dimethoxybenzyl group, a 2,5-dimethoxybenzyl group, a 3,4-dimethoxybenzyl group, a 3,5-dimethoxybenzyl group, a 2,3-diethoxybenzyl group, a 2,4-diethoxybenzyl group, a 2,5-diethoxybenzyl group, a 3,4-diethoxybenzyl group, a 3,5-diethoxybenzyl group, a 2-fluoro-3-methoxybenzyl group, a 2-fluoro-4-methoxybenzyl group, a 2-floro-5-methoxybenzyl group, a 3-fluoro-4-methoxybenzyl group, a 3-fluoro-5-methoxybenzyl group, a 3-fluoro-2-methoxybenzyl group, a 4-fluoro-2-methoxybenzyl group, a 5-fluoro-2-methoxybenzyl group, a 4-fluoro-3-methoxybenzyl group, a 5-fluoro-3-methoxybenzyl group, or the like.; the "pyridylmethyl group which may be substituted with an alkyl group having 1 - 4 carbon atoms" refers to a 2-pyridylmethyl group, a 3-pyridylmethyl group, a 4-pyridylmethyl group, a 5-methyl-3-pyridylmethyl group, a 6-methyl-2-pyridylmethyl group or the like; the "alkoxycarbonyl group having 1 or 2 carbon atoms" refers to a methoxycarbonyl group, an ethoxycarbonyl group or the like.

Preferably, R⁴ represents a hydrogen atom, a methyl group, a 2-pyrimidinylmethyl group, a 4-pyrimidinylmethyl group, a 5-pyrimidinylmethyl group, a 2-pyridylmethyl group, a 3-pyridylmethyl group, a 4-pyridylmethyl group, a 5-methyl-3-pyridylmethyl group or a 6-methyl-2-pyridylmethyl group.

More preferably, R⁴ represents a methyl group, a 5-pyrimidinylmethyl group, a 2-pyridylmethyl group, a 3-pyridylmethyl group or a 4-pyridylmethyl group.

In the formula (I), R⁵ represents a hydrogen atom or a methyl group, preferably a hydrogen atom.

If R¹, R², R³ and R⁵ in the formula (I) are a hydrogen atom at the same time, R⁴ is a substituent other than a hydrogen atom, a benzyl group, a 4-diethylaminobenzyl group and a furylmethyl group.

The preferred combinations of the substituents are such that R¹ is substituted in 2-position and represents a hydroxyl group, a carboxymethyloxy group, a 2-carboxyethyloxy group, a 3-carboxypropyloxy group, a N-hydroxymethylcarbamoylmethyloxy group, a N-ethylcarbamoylmethyloxy group, a 4-hydroxy-2-oxobutyloxy group, a 5-hydroxy-2-oxopentyloxy group, a 2-hydroxyethyloxy group, a 3-hydroxypropyloxy group, a 4-hydroxybutyloxy group, a benzyloxy group, a 2-pyridinomethyloxy group, a 3-pyridinomethyloxy group, a 4-pyridinomethyloxy group, a 2-pyrazinylmethyloxy group, a 2-pyrimidinylmethyloxy group, a 4-pyrimidinylmethyloxy group or a 5-pyrimidinylmethyloxy group; R² is a chlorine atom, a bromine atom or a trifluoromethyl group which are substituted in 9-position; R³ is a hydrogen atom; R⁴ is a methyl group, a 5-pyrimidinylmethyl group, a 2-pyridylmethyl group, a 3-pyridylmethyl group or a 4-pyridylmethyl group; and R⁵ is a hydrogen atom.

In the formula (IV), R⁶ represents a hydrogen atom, a halogen atom, a cyano group, an optionally protected carboxyl group, an optionally protected carboxymethyl group, an alkoxycarbonyl group having 1 - 4 carbon atoms, a carbamoyl group, an acetylamino group, a 3-carboxy-1-propenyl group, an optionally protected hydroxyl group, an optionally protected mercapto group, a straight- or branched-chain alkyl group having 1 - 4 carbon atoms which may be substituted with one hydroxyl group, an amino group which may be mono- or disubstituted with an alkyl group having 1 - 4 carbon atoms, an alkylthio group having 1 - 3 carbon atoms or a straight-chain alkoxy group having 1 - 6 carbon atoms which may be substituted with a 4-methoxyphenoxy group; R⁷ represents a hydrogen atom, a halogen atom, an optionally protected hydroxyl group, an optionally protected mercapto group, an optionally protected amino group, a cyano group, a nitro group, a trifluoromethyl group, a trifluoromethoxy group, an optionally protected carboxyl group, a straight- or branched-chain alkanoyl group having 1 - 4 carbon atoms, a straight- or branched-chain alkyl group having 1 - 4 carbon atoms or a straight- or branched-chain alkoxy group having 1 - 4 carbon atoms; R⁸ represents a hydrogen atom, a halogen atom, an optionally protected hydroxyl group or a straight- or branched-chain alkoxy group having 1 - 4 carbon atoms; R¹⁰ is a hydrogen atom, a halogen atom, a phenoxy group, an α-hydroxybenzyl group, an anilino group, a N-methylanilino group, a methyl group or a halogenomethyl group. More specifically, the substituents R⁶, R⁷, R⁸ and R¹⁰ are expressed by the definitions given to the specific examples of the relevant substituents which are represented by R¹, R², R³ and R⁴, respectively, in the formula (I) and which the specifically described hereinabove.

Referring to the formula (XVI) which will be set forth later in connection with production of the claimed compounds, R⁹ represents a hydrogen atom or a methyl group and R¹¹ represents a hydrogen atom or a straight-or branched-chain alkyl group having 1 - 4 carbon atoms; more specifically, the "straight- or branched-chain alkyl group having 1 - 4 carbon atoms" refers to a methyl group, an ethyl group, a n-propyl group, an i-propyl group, a n-butyl group, a t-butyl group or the like.

In the formula (XIX), R¹² is a hydrogen atom, a methyl group, a cyclic alkyl group having 3 - 6 carbon atoms, a phenyl group which may be mono- or disubstituted with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 - 4 carbon atoms, an acetylamino group, a carboxyl group and an amino group, a pyridyl group which may be substituted with an alkyl group having 1 - 4 carbon atoms, a morpholyl group, a triazolyl group, a furyl group, a thienyl group, a pyrimidinyl group, a pyrazinyl group, a pyrrolyl group, an imidazolyl group, a quinolyl group, an indolyl group or a naphthyl group.

More specifically, the "cyclic alkyl group having 3 - 6 carbon atoms" refers to a cyclopropyl group, a cyclohexyl group or the like; the "phenyl group which may be mono- or disubstituted with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 - 4 carbon atoms, an acetylamino group, a carboxyl group and an amino group" refers to a 2-fluorophenyl group, a 2-chlorophenyl group, a 2-bromophenyl group, a 3-fluorophenyl group, a 3-chlorophenyl group, a 3-bromophenyl group, a 4-fluorophenyl group, a 4-chlorophenyl group, a 4-bromophenyl group, a 2-hydroxyphenyl group, a 3-hydroxyphenyl group, a 4-hydroxyphenyl group, a 2-mercaptophenyl group, a 3-mercaptophenyl group, a 4-mercaptophenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-n-propoxyphenyl group, a 3-n-propoxyphenyl group, a 4-n-propoxyphenyl group, a 2-i-propoxyphenyl group, a 3-i-propoxyphenyl group, a 4-i-propoxyphenyl group, a 2-n-butoxyphenyl group, a 3-n-butoxyphenyl group, a 4-n-butoxyphenyl group, a 2-t-butoxyphenyl group, a 3-t-butoxyphenyl group, a 4-t-butoxyphenyl group, a 2-methoxycarbonylphenyl group, a 3-methoxycarbonylphenyl group, a 4-methoxycarbonylphenyl group, a 2-ethoxycarbonylphenyl group, a 3-ethoxycarbonylphenyl group, a 4-ethoxycarbonylphenyl group, a 2-t-butoxycarbonylphenyl group, a 3-t-butoxycarbonylphenyl group, a 4-t-butoxycarbonylphenyl group, a 2-acetylaminophenyl group, a 3-acetylaminophenyl group, a 4-acetylaminophenyl group, a 2-carboxyphenyl group, a 3-carboxyphenyl group, a 4-carboxyphenyl group, a 2-aminophenyl group, a 3-aminophenyl group, a 4-aminophenyl group, a 2,3-difluorophenyl group, a 2,4-difluorophenyl group, a 2,5-difluorophenyl group, a 3,4-difluorophenyl group, a 3,5-difluorophenyl group, a 2,3-dichlorophenyl group, a 2,4-dichlorophenyl group, a 2,5-dichlorophenyl group, a 3,4-dichlorophenyl group, a 3,5-dichlorophenyl group, a 2,3-dibromophenyl group, a 2,4-dibromophenyl group, a 2,5-dibromophenyl group, a 3,4-dibromophenyl group, a 3,5-dibromophenyl group, a 2,3-dihydroxyphenyl group, a 2,4-dihydroxyphenyl group, a 2,5-dihydroxyphenyl group, a 3,4-dihydroxyphenyl group, a 3,5-dihydroxyphenyl group, a 2,3-dimethoxyphenyl group, a 2,4-dimethoxyphenyl group, a 2,5-dimethoxyphenyl group, a 3,4-dimethoxyphenyl group, a 3,5-dimethoxyphenyl group, a 2,3-diethoxyphenyl group, a 2,4-diethoxyphenyl group, a 2,5-diethoxyphenyl group, a 3,4-diethoxyphenyl group, a 3,5-diethoxyphenyl group, a 2-fluoro-3-methoxyphenyl group, a 2-fluoro-4-methoxyphenyl group, a 2-fluoro-5-methoxyphenyl group, a 3-fluoro-4-methoxyphenyl group, a 3-fluoro-5-methoxyphenyl group, a 3-fluoro-2-methoxyphenyl group, a 4-fluoro-2-methoxyphenyl group, a 5-fluoro-2-methoxyphenyl group, a 4-fluoro-3-methoxyphenyl group, a 5-fluoro-3-methoxyphenyl group, or the like; the "pyridyl group which may be substituted with an alkyl group having 1 - 4 carbon atoms" refers to a 2-pyridyl group, a 3-pyridyl group, a 4-pyridyl group, a 5-methyl-3-pyridyl group, a 6-methyl-2-pyridyl group, or the like. Preferably, R¹² represents a 2-pyrimidinyl group, a 4-pyrimidinyl group, a 5-pyrimidinyl group, a 2-pyridyl group, a 3-pyridyl group, a 4-pyridyl group, a 5-methyl-3-pyridyl group or a 6-methyl-2-pyridyl group. More preferably, R¹² represents a 5-pyrimidinyl group, a 2-pyridyl group, a 3-pyridyl group or a 4-pyridyl group.

Further, R¹³ in the formula (XX) represents an alkoxycarbonyl group having 1 - 4 carbon atoms, a 3-carboxy-1-propenyl group, a 2,2-diethoxyethyl group, a straight- or branched-chain alkanoyl group having 1 - 4 carbon atoms, a carbonyl group substituted with a phenyl group or a pyridyl group, or a group: -(CH₂)ₙ-Z (where Z represents a hydrogen atom, a carboxyl group, an alkoxy group having 1 or 2 carbon atoms which may be substituted with one hydroxyl group, an alkoxycarbonyl group having 1 - 6 carbon atoms, a carbamoyl group which may be mono- or disubstituted with a hydroxymethyl group or an alkyl group having 1 or 2 carbon atoms, an alkanoyl group having 1 - 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 - 4 carbon atoms or an alkoxycarbonyl group having 1 or 2 carbon atoms, a pyrazinyl group, a pyrimidinyl group, a furyl group, a thienyl group, an oxadiazolyl group or a 4-methoxyphenoxy group; n is 1 - 6).

More specifically, the "alkoxycarbonyl group having 1 - 4 carbon atoms" refers to a methoxycarbonyl group, an ethoxycarbonyl group, a n-propoxycarbonyl group, an i-propoxycarbonyl group, a cyclopropoxycarbonyl group, a n-butoxycarbonyl group, a t-butoxycarbonyl group, or the like; the "straight- or branched-chain alkanoyl group having 1 - 4 carbon atoms" refers to an acetyl group, a propionyl group, a butyryl group, a pivaloyl group, or the like; the "carbonyl group substituted with a phenyl group or a pyridyl group" refers to a benzoyl group, a nicotinoyl group, an isonicotinoyl group, or the like; the group: -(CH₂)ₙ-Z (where Z represents a hydrogen atom, a carboxyl group, an alkoxy group having 1 or 2 carbon atoms which may be substituted with one hydroxyl group, an alkoxycarbonyl group having 1 - 6 carbon atoms, a carbamoyl group which may be mono- or disubstituted with a hydroxymethyl group or an alkyl group having 1 or 2 carbon atoms, an alkanoyl group having 1 - 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 - 4 carbon atoms or an alkoxycarbonyl group having 1 or 2 carbon atoms, a pyrazinyl group, a pyrimidinyl group, a furyl group, a thienyl group, an oxadiazolyl group or a 4-methoxyphenoxy group; n is 1 - 6) refers to a methyl group, an ethyl group, a n-propyl group, an i-propyl group, a n-butyl group, a t-butyl group, a n-pentyl group, a n-hexyl group, a carboxymethyl group, a 2-carboxyethyl group, a 3-carboxypropyl group, a methoxymethyl group, an ethoxymethyl group, a 2-methoxyethyl group, a 2-ethoxyethyl group, a 2-(2-hydroxyethoxy)ethyl group, a methoxycarbonylmethyl group, an ethoxycarbonylmethyl group, a n-propoxycarbonylmethyl group, an i-propoxycarbonylmethyl group, a n-butoxycarbonylmethyl group, a t-butoxycarbonylmethyl group, a n-pentyloxycarbonylmethyl group, a n-hexyloxycarbonylmethyl group, a cyclopropyloxycarbonylmethyl group, a cyclohexyloxycarbonylmethyl group, a 2-(methoxycarbonyl)ethyl group, a 2-(ethoxycarbonyl)ethyl group, a 2-(n-propoxycarbonyl)ethyl group, a 2-(i-propoxycarbonyl)ethyl group, a 2-(n-butoxycarbonyl)ethyl group, a 2-(t-butoxycarbonyl)ethyl group, a 2-(n-pentyloxycarbonyl)ethyl group, a 2-(n-hexyloxycarbonyl)ethyl group, a 2-(cyclopropyloxycarbonyl)ethyl group, a 2-(cyclohexyloxycarbonyl)ethyl group, a 3-(methoxycarbonyl)propyl group, a 3-(ethoxycarbonyl)propyl group, a 3-(n-propoxycarbonyl)propyl group, a 3-(i-propoxycarbonyl)propyl group, a 3-(n-butoxycarbonyl)propyl group, a 3-(t-butoxycarbonyl)propyl group, a 3-(n-pentyloxycarbonyl)propyl group, a 3-(n-hexyloxycarbonyl)propyl group, a 3-(cyclopropyloxycarbonyl)propyl group, a 3-(cyclohexyloxycarbonyl)propyl group, a N-hydroxymethylcarbamoylmethyl group, a N-methylcarbamoylmethyl group, a N,N-dimethylcarbamoylmethyl group, a N-ethylcarbamoylmethyl group, a N,N-diethylcarbamoylmethyl group, a N-n-propylcarbamoylmethyl group, a N-n-butylcarbamoylmethyl group, a 3-hydroxy-2-oxopropyl group, a 4-hydroxy-3-oxobutyl group, a 5-hydroxy-4-oxopentyl group, a 4-hydroxy-2-oxobutyl group, a 5-hydroxy-2-oxopentyl group, a 6-hydroxy-2-oxohexyl group, a 5-mercapto-2-oxopentyl group, a 4-carboxy-1-piperidinylcarbonylmethyl group, a 4-methoxycarbonyl-1-piperidinylcarbonylmethyl group, a 4-ethoxycarbonyl-1-piperidinylcarbonylmethyl group, a 4-morpholylcarbonylmethyloxy group, a 2-hydroxyethyl group, a 3-hydroxypropyl group, a 4-hydroxybutyl group, a 2-mercaptoethyl group, a 3-mercaptopropyl group, a 4-mercaptobutyl group, a 2-aminoethyl group, a 3-aminopropyl group, a 4-aminobutyl group, a benzyl group, a 2-phenethyl group, a 3-phenylpropyl group, a 5-hydroxymethyl-3-pyridylmethyl group, a 5-acetoxymethyl-3-pyridylmethyl group, a 6-hydroxymethyl-2-pyridylmethyl group, a 6-acetoxymethyl-2-pyridylmethyl group, a 5-methyl-3-pyridylmethyl group, a 6-methyl-2-pyridylmethyl group, a 5-ethyl-3-pyridylmethyl group, a 5-t-butyl-3-pyridylmethyl group, a 5-methoxycarbonyl-3-pyridylmethyl group, a 5-ethoxycarbonyl-3-pyridylmethyl group, a 2-pirazinylmethyl group, a 2-pyrimidinylmethyl group, a 4-pyrimidinylmethyl group, a 5-pyrimidinylmethyl group, a 2-furylmethyl group, a 3-furylmethyl group, a 2-thienylmethyl group, a 3-thienylmethyl group, a 3-oxadiazolylmethyl group, a 2-(4-methoxyphenoxy)ethyl group, a 3-(4-methoxyphenoxy)propyl group, a 4-(4-methoxyphenoxy)butyl group, or the like.

Preferably, R¹³ represents a carboxymethyl group, a 2-carboxyethyl group, a 3-carboxypropyl group, a methoxycarbonylmethyl group, an ethoxycarbonylmethyl group, a n-propoxycarbonylmethyl group, an i-propoxycarbonylmethyl group, a n-butoxycarbonylmethyl group, a t-butoxycarbonylmethyl group, a N-hydroxymethylcarbamoylmethyl group, a N-ethylcarbamoylmethyl group, a 4-hydroxy-2-oxobutyl group, a 5-hydroxy-2-oxopentyl group, a 2-hydroxyethyl group, a 3-hydroxypropyl group, a 4-hydroxybutyl group, a 3-aminopropyl group, a 4-aminobutyl group, a benzyl group, a 5-hydroxymethyl-3-pyridylmethyl group, a 5-acetoxymethyl-3-pyridylmethyl group, a 6-hydroxymethyl-2-pyridylmethyl group, a 6-acetoxymethyl-2-pyridylmethyl group, a 5-methyl-3-pyridylmethyl group, a 6-methyl-2-pyridylmethyl group, a 2-pyridylmethyl group, a 3-pyridylmethyl group, a 4-pyridylmethyl group, a 2-pirazinylmethyl group, a 2-pyrimidinylmethyl group, a 4-pyrimidinylmethyl group or a 5-pyrimidinylmethyl group.

More preferably, R¹³ represents a carboxymethyl group, a 2-carboxyethyl group, a 3-carboxypropyl group, a N-hydroxymethylcarbamoylmethyl group, a N-ethylcarbamoylmethyl group, a 4-hydroxy-2-oxobutyl group, a 5-hydroxy-2-oxopentyl group, a 2-hydroxyethyl group, a 3-hydroxypropyl group, a 4-hydroxybutyl group, a benzyl group, a 2-pyridylmethyl group, a 3-pyridylmethyl group, a 4-pyridylmethyl group, a 2-pirazinylmethyl group, a 2-pyrimidinylmethyl group, a 4-pyrimidinylmethyl group or a 5-pyrimidinylmethyl group.

Throughout the specification, the number of carbon atoms indicated for the alkoxycarbonyl group, alkanoyloxy group or alkanoyl group refers to that of carbon atoms in the corresponding alkoxy, alkyl or alkyl portion.

Aside from the protective groups specifically mentioned herein for the optionally protected substituents, the following may be mentioned: protective groups for the hydroxyl group include alkyl-type protective groups such as a methyl group, a t-butyl group, a benzyl group, a trityl group and a methoxymethyl group, silyl-type protective groups such as a trimethylsilyl group and a t-butyldimethylsilyl group, acyl-type protective groups such as a formyl group, an acetyl group and a benzoyl group, and carbonate-type protective groups such as a methoxycarbonyl group and a benzyloxycarbonyl group; protective groups for the carboxyl group include ester-type protective groups such as a methyl group, an ethyl group, a t-butyl group, a benzyl group and a methoxymethyl group; protective groups for the amino group include alkyl-type protective groups such as a benzyl group, a trityl group and a methoxymethyl group, acyl-type protective groups such as a formyl group, an acetyl group and a benzoyl group, and carbamate-type protective groups such as a t-butoxycarbonyl group and a benzyloxycarbonyl group.

The compounds which form the effective component of the composition of the invention may form salts with inorganic or organic acids. Examples of such salts include inorganic acid salts such as hydrochlorides, sulfates and nitrates, and organic acid salts such as acetates, citrates, oxalates, maleates, tartrates, p-toluenesulfonates and methanesulfonates. Depending on the types of the substituents used, salts may be formed with inorganic or organic bases. Examples include salts with inorganic bases such as sodium carbonate and potassium carbonate, as well as salts with organic bases such as triethylamine, diethylamine and pyridine. These salts can be obtained in the usual manner, as by mixing an equivalent amount of the compound which forms the effective component of the composition of the invention with a solution containing an acid or base of interest and obtaining the desired salt by filtration or evaporating the solvent.

The compounds of the invention and the salts thereof can form solvates with water, ethanol ors glycerol and these solvates are also within the scope of the invention. It should be noted that the solvates of the invention are not limited thereto.

The compounds which form the effective component of the composition of the invention represented by the formula (I) can be produced by processes represented by the reaction schemes to be set forth below. The compounds shown in the Reaction Schemes 1 and 2 to be set forth below, the compounds represented by the formulae set forth herein, i.e. the formulae (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII) and (XXIV), as well as the definitions of R¹, R², R³, R⁴, R⁵, R⁶, R⁷, R⁸, R⁹, R¹⁰, R¹¹, R¹², R¹³, X and Z are respectively the same as already discussed above. The pyridocarbazole derivatives represented by the formula (I) or salts thereof which form the effective component of the composition of the invention can be produced in accordance with Process I within the scope of Reaction Scheme 1 from compounds of the formula (II), (V) or (IX) which can easily be prepared from either documented or commercial compounds, or from salts of such compounds. The compounds of the formula (I) can also be produced by Process 2 or 3.

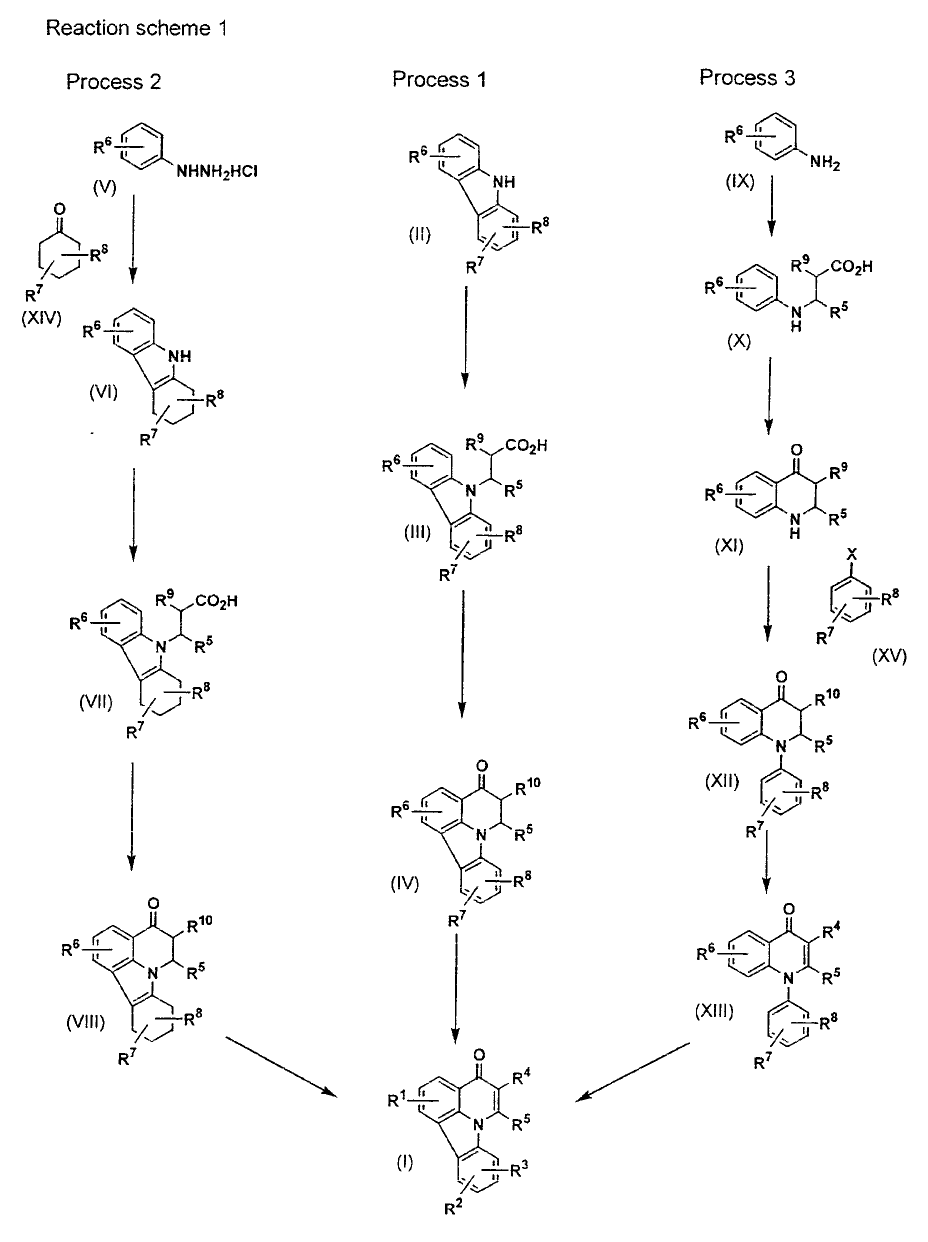

The processes for producing the compounds of the invention are described below in detail.

### (Process 1)

A carbazole derivative represented by the general formula (II) or a salt thereof: 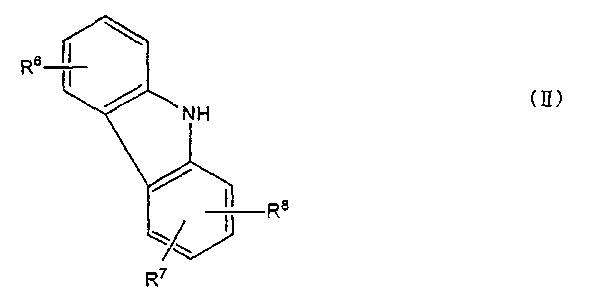 is reacted with a compound represented by the following general formula (XVI): 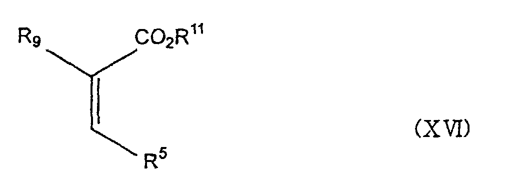 or the following general formula (XVII): 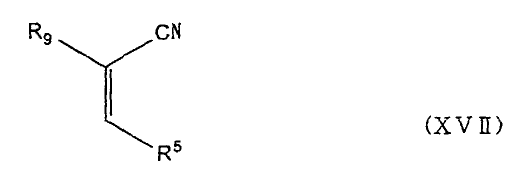 or the following general formula (XVIII) 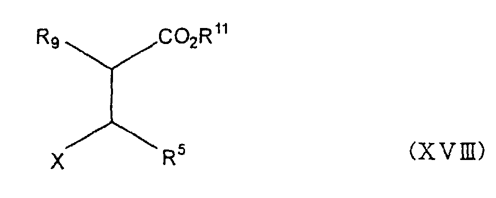 and, if necessary, hydrolysis is performed to yield a compound represented by the following general formula (III):

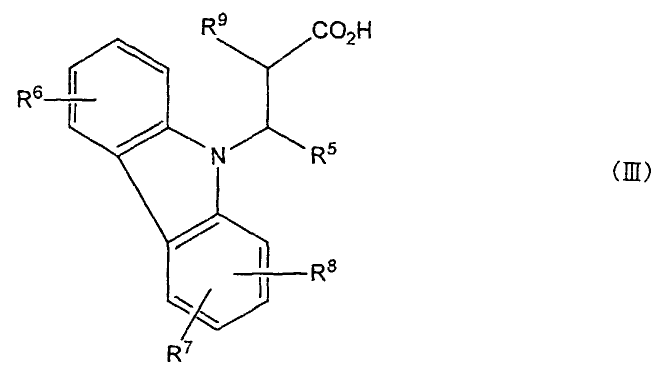

Stated more specifically, the compound of the formula (II) and the compound of the formula (XVI) or (XVII) are subjected to a Michael addition reaction in the presence or absence of copper acetate, N-benzyltrimethylammonium hydroxide (Triton B) or the like, preferably in the presence of Triton B, either without solvents or using a water, a ketone-based solvent such as acetone or methyl ethyl ketone or an ether-based solvent such as tetrahydrofuran (THF), dioxane or 1,2-dimethoxyethane (DME), preferably using acetone as a solvent at a temperature ranging from the one obtained by cooling with ice to the one where the reaction mixture is heated under reflux, preferably at room temperature for a sufficient time to ensure adequate progress of the reaction, specifically for 15 min to 1 h and, if necessary, hydrolysis is performed in either an acidic aqueous solution such as dilute hydrochloric acid or sulfuric acid or a basic aqueous solution such as dilute aqueous sodium hydroxide or potassium hydroxide, preferably in dilute aqueous hydrochloric acid or sodium hydroxide at a temperature ranging from room temperature to the one where the reaction mixture is heated under reflux, preferably at room temperature for a sufficient time to ensure adequate progress of the reaction, specifically from 15 min to 12 h; alternatively, the compound of the formula (II) and the compound of the formula (XVIII) are subjected to an addition reaction in the presence of an inorganic base such as potassium carbonate, cesium carbonate, calcium carbonate or sodium hydride or an organic base such as triethylamine, pyridine or N,N-dialkylaniline, preferably in the presence of sodium hydride using a polar solvent such as acetonitrile or dimethylformamide (DMF), a halogenated hydrocarbon solvent typified by chloroform or methylene chloride or an ether-based solvent typified by ether or tetrahydrofuran (THF), preferably using DMF as a solvent at a temperature ranging from room temperature to the one where the reaction mixture is heated under reflux, preferably at the temperature where heating under reflux is effected, for a sufficient time to ensure adequate progress of the reaction, specifically for 15 min to 3 h and, if necessary, hydrolysis is performed in either an acidic aqueous solution such as dilute hydrochloric acid or sulfuric acid or a basic aqueous solution such as dilute aqueous sodium hydroxide or potassium hydroxide, preferably in a dilute aqueous hydrochloric acid or sodium hydroxide solution at a temperature ranging from room temperature to the one where the reaction mixture is heated under reflux, preferably at room temperature for a sufficient time to ensure adequate progress of the reaction, specifically for 15 min to 12 h. By either method, the compound of the formula (III) can be produced.

Subsequently, the compound of the formula (III) is converted to an acid halide by reaction in the presence of a thionyl halide reagent such as thionyl chloride or thionyl bromide using a halogenated hydrocarbon solvent typified by chloroform or methylene chloride or an aromatic hydrocarbon-based solvent such as benzene or toluene, preferably using methylene chloride as a solvent at a temperature ranging from the one obtained by cooling with ice to the one where the reaction mixture is heated under reflux, preferably at room temperature for a sufficient time to ensure adequate progress of the reaction, specifically for 15 min to 1 h; thereafter, the resulting acid halide is subjected to Friedel-Crafts reaction in the presence of a Lewis acid such as aluminum chloride, tin chloride or zinc chloride either without solvents or using nitrobenzene, carbon disulfide or a halogenated hydrocarbon-based solvent such as methylene chloride, carbon tetrachloride or 1,2-dichloroethane, preferably using carbon disulfide or methylene chloride as a solvent at a temperature ranging from -78°C to the one where the reaction mixture is heated under reflux, preferably at room temperature for a sufficient time to ensure adequate progress of the reaction, specifically for 15 min to 3 h; alternatively, the acid halide is subjected to reaction in the presence of trifluoroacetic anhydride using an aromatic hydrocarbon-based solvent such as benzene, toluene or xylene, preferably using toluene as a solvent at a temperature ranging from room temperature to the one where the reaction mixture is heated under reflux, preferably at the temperature where heating under reflux is effected, for a sufficient time to ensure adequate progress of the reaction, specifically for 3 h to 10 h; alternatively, the acid halide is subjected to reaction in the presence of a phosphorylating agent such as phosphorus pentoxide, polyphosphoric acid or polyphosphate ester either without solvents or optionally using an aromatic hydrocarbon-based solvent such as benzene or toluene or a halogenated hydrocarbon-based solvent such as chlorobenzene, chloroform or methylene chloride, preferably using chloroform as a solvent at a temperature ranging from room temperature to the one where the reaction mixture is heated under reflux, preferably at the temperature where heating under reflux is effected, for a sufficient time to ensure adequate progress of the reaction, specifically for 1 h to 12 h; by either approach, a compound of the following general formula (IV) can be produced: 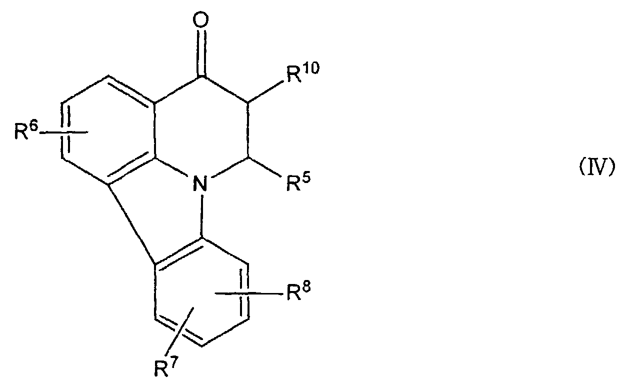 The above-described reaction for ring closure (cyclization) has such selectivity that on account of the difference in electronic environment between the substituents R⁶ and R⁷ (or R⁸) on the two benzene rings, cyclization favors the substituent which is relatively more effective electron donor. In order to achieve cyclization in the desired direction by taking advantage of this propensity, those substituents which can be changed or removed after cyclization can be used effectively. If the selectivity in cyclization is so low as to produce a mixture, purification may optionally be performed by separation through recrystallization or column chromatography.

If R⁶, R⁷ and R⁸ in the compound represented by the formula (IV) are groups included within the definitions of R¹, R² and R³ in the compound represented by the formula (I), R¹⁰ may be changed to R⁴ in the manner to be described below, whereby the compound of the formula (IV) is directly derivated to the compound of the formula (I) as shown in Process 1 under Reaction Scheme 1.

Subsequently, the compound represented by the formula (IV) is derivated to the compound represented by the formula (I) as set forth in Reaction Scheme 1 and the changes of substituents that are effected in the derivation are shown in Reaction Scheme 2 and described below in detail.

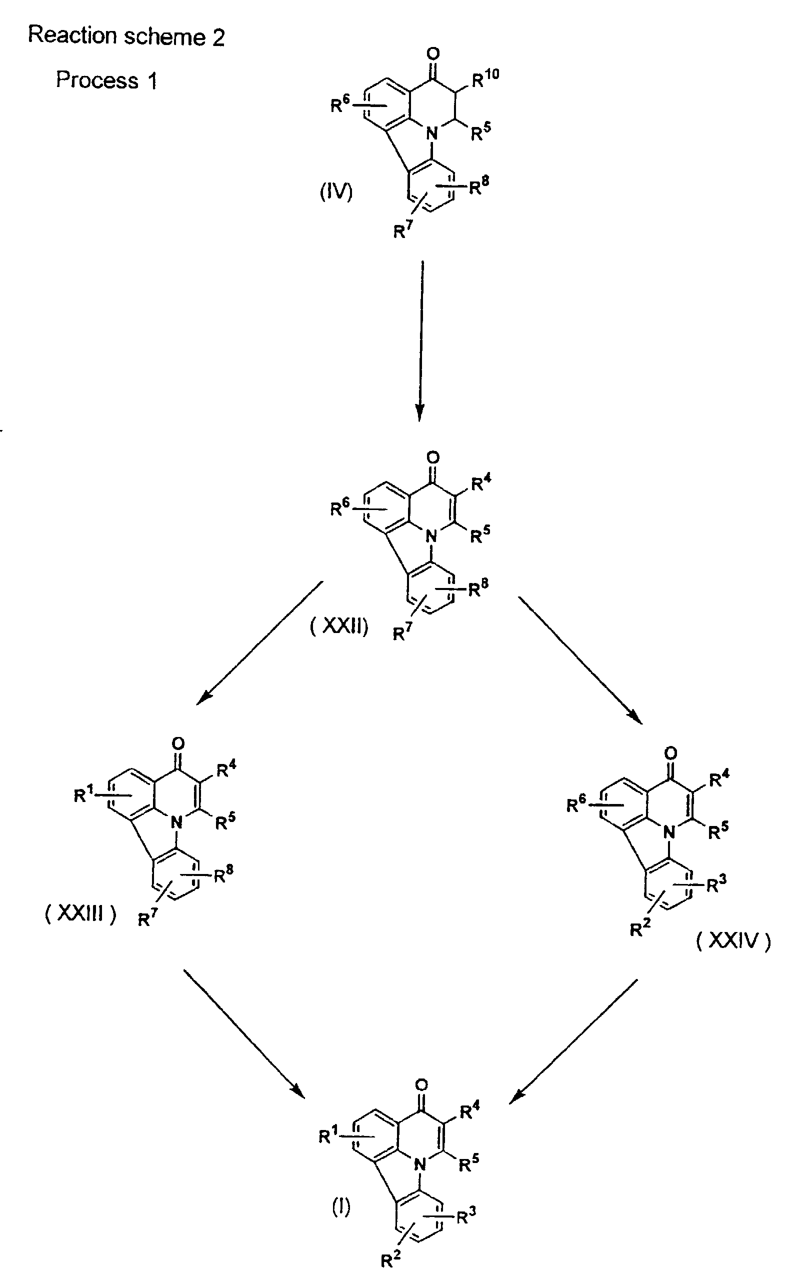

The compound of the formula (IV) is subjected to an aldole condensation reaction with aldehyde represented by R¹²-CHO (XIX), optionally in the presence of an inorganic base such as potassium hydroxide, sodium hydroxide or potassium carbonate or an organic base such as piperazine, piperidine, morpholine or n-BuLi, preferably in the presence of sodium hydroxide in an alcoholic solvent such as methanol or ethanol or an ether-based solvent such as ether, THF or dioxane, preferably using ethanol as a solvent at a temperature ranging from room temperature to the one where the reaction mixture is heated under reflux, preferably at room temperature for a sufficient time to ensure adequate progress of the reaction, specifically for 3 h to 12 h. The resulting compound is not isolated but dehydrated in situ to produce an enone which has the double bond subsequently isomerized in the ring, followed by oxidation in the manner described below. Alternatively, the reaction compound is isolated and subjected to oxidation reaction (dehydrogenation) in the presence of an oxidizing agent such as chloranil, dichlorodicyanobenzoquinone (DDQ) or 5% palladium on carbon, preferably DDQ, using an aromatic hydrocarbon-based nonpolar solvent such as benzene, toluene or xylene, an ether-based solvent such as THF, DME or dioxane or an alcoholic solvent such as ethylene glycol, preferably using dioxane as a solvent at a temperature ranging from room temperature to the one where the reaction mixture is heated under reflux, preferably at room temperature for a sufficient time to ensure adequate progress of the reaction, specifically from 1 h to 12 h; alternatively, the isolated reaction product is halogenated in the presence or absence of light, azobisisobutyronitrile (AIBN) or a peroxide such as benzoyl peroxide (BPO), preferably in their absence, using a suitable halogenating agent such as chlorine gas, bromine, copper bromide, N-bromosuccinimide (NBS), N-chlorosuccinimide (NCS), trihalogenomethanesulfonyl halogenide or trichlorobromomethane, preferably copper bromide, and also using a halogenated hydrocarbon solvent such as carbon tetrachloride, chloroform or methylene chloride, an aromatic hydrocarbon-based nonpolar solvent such as benzene or toluene, acetic acid or carbon disulfide solvent or an ester-based solvent such as ethyl acetate, preferably using chloroform or ethyl acetate as a solvent at a temperature ranging from room temperature to the one where the reaction mixture is heated under reflux, preferably at the temperature where heating under reflux is effected, for a sufficient time to ensure adequate progress of the reaction, specifically for 1 h to 12 h so as to yield a reactive derivative, which is thereafter subjected to the following replacement reaction with phenol, aniline, N-methylaniline, triazole, imidazole, morpholine or the like, optionally in the presence of an inorganic base such as potassium carbonate, cesium carbonate or calcium carbonate or an organic base such as triethylamine, pyridine or N,N-dialkylaniline, preferably cesium carbonate, and also optionally using a polar solvent such as acetonitrile or dimethylformamide (DMF), a halogenated hydrocarbon solvent typified by chloroform or methylene chloride or an ether-based solvent typified by ether or tetrahydrofuran (THF), preferably without using solvents, at a temperature ranging from room temperature to the one where the reaction mixture is heated under reflux, preferably at room temperature for a sufficient time to ensure adequate progress of the reaction, specifically for 30 min to 12 h; thereafter, the reaction product is oxidized (dehydrogenated) with an oxidizing agent such as chloranil or DDQ, preferably DDQ, using an aromatic hydrocarbon-based nonpolar solvent such as benzene, toluene or xylene or an ether-based solvent such as THF, DME or dioxane, preferably using dioxane as a solvent at a temperature ranging from room temperature to the one where the reaction mixture is heated under reflux, preferably at room temperature for a sufficient time to ensure adequate progress of the reaction, specifically for 1 h to 12 h; if desired, the replacement reaction may be bypassed and the reaction derivative obtained by halogenation is directly oxidized (dehydrogenated) under the conditions described above. In either way, the compound represented by the formula (XXII) can be produced.

Subsequently, the compound of the formula (XXII) may be subjected to substituent changes as required. If R⁶, R⁷ or R⁸ is a protected hydroxyl group, it is deprotected by treatment in an aqueous solution of hydrochloric acid or hydrofluoric acid, preferably in an aqueous solution of hydrochloric acid, at a temperature ranging from the one obtained by cooling with ice to the one where the reaction mixture is heated under reflux, preferably at room temperature for a sufficient time to ensure adequate progress of the reaction, specifically for 15 min to 12 h; if R⁶, R⁷ or R⁸ is a methoxy group, deprotection is performed by treatment in the presence of boron tribromide, aluminum chloride or hydrobromic acid, preferably in the presence of boron tribromide, using a halogenated hydrocarbon-based solvent such as methylene chloride or chloroform or acetic acid solvent, preferably using methylene chloride as a solvent at a temperature ranging from the one obtained by cooling with ice to the one where the reaction mixture is heated under reflux, preferably at room temperature for a sufficient time to ensure adequate progress of the reaction, specifically for 3 h to 24 h; if R⁶, R⁷ or R⁸ is a benzyloxy group, deprotection is performed by treatment in the presence of palladium and sodium acetate in acetic acid solvent at a temperature ranging from room temperature to the one where the reaction mixture is heated under reflux, preferably at the temperature where heating under reflux is effected, for a sufficient time to ensure adequate progress of the reaction, specifically for 1 h to 12 h; by either method of deprotection, the compound (XXII) can be converted to a hydroxy form. The compound represented by the formula (XXII) where R⁶ is a hydroxyl group is reacted with a reactive halogen derivative R¹³-X (XX) in the presence or absence of KI using an inorganic base such as potassium carbonate, cesium carbonate or calcium carbonate or an organic base such as triethylamine, pyridine or N,N-dialkylaniline, preferably using potassium carbonate, and also using a polar solvent such as acetonitrile, dimethylformamide (DMF) or dimethyl sulfoxide (DMSO) or an ether-based solvent such as THF, dioxane or DME, preferably using DMSO as a solvent at a temperature ranging from room temperature to 80°C, preferably at room temperature for a sufficient time to ensure adequate progress of the reaction, specifically for 1 h to 22 h so as to yield a compound represented by the general formula (XXIII). The compound of (XXII) may be reacted with acetyl chloride or a bromoacetic acid ester if R⁷ is a hydroxyl group, or with acetyl chloride if R⁸ is a hydroxyl group.

Alternatively, the compound represented by the general formula (XXII) may have substituents changed to suitable ones to yield a compound represented by the general formula (XXIV) and if R⁶ in this compound is a straight-chain alkyl group having 1 - 6 carbon atoms which may be substituted by a 4-methoxyphenoxy group, specifically exemplified by a 2-(4-methoxyphenoxy)ethyloxy group, a 3-(4-methoxyphenoxy)propyloxy group or a 4-(4-methoxyphenoxy)butyloxy group, deprotection may be performed in the presence of cerium ammonium nitrate (CAN) in acetonitrile either alone or in admixture with water, preferably using the mixture of acetonitrile and water as a solvent system, at a temperature ranging from the one obtained by cooling with ice to the one where the reaction mixture is heated under reflux, preferably at 0°C for a sufficient time to ensure adequate progress of the reaction, specifically for 15 min to 4 h so as to derivate a compound represented by the general formula (I), specifically one in which R¹ is a 2-hydroxyethyloxy group, a 3-hydroxypropyloxy group, a 4-hydroxybutyloxy group or the like.

Other substituent changes that can be effected in the compound of the formula (XXII) are as follows: if R⁶ or R⁷ is a halogen atom, they may be changed to an amino group by reaction in the presence of copper or copper iodide in aqueous ammonia at a temperature of 150 - 200°C, preferably at a temperature of 180 - 190°C for a sufficient time to ensure adequate progress of the reaction, specifically for 3 h to 12 h; alternatively R⁶ or R⁷ may be changed to a cyano group by reaction in the presence of copper cyanide in DMF at a temperature of 100 - 200°C, preferably at a temperature of 120 - 140°C for a sufficient time to ensure adequate progress of the reaction, specifically for 1 h to 12 h.

If R⁶ or R⁷ is a nitro group, they may be changed to an amino group by reaction in the presence of copper using dilute sulfuric acid as a solvent at a temperature ranging from room temperature to the one where the reaction mixture is heated under reflux, preferably at 50°C for a sufficient time to ensure adequate progress of the reaction, specifically for 30 min to 3 h.

If R⁶ or R⁷ is an amino group, they may be changed to a hydroxyl group by reaction in the presence of sodium nitrite using dilute sulfuric acid as a solvent at a temperature ranging from the one obtained by cooling with ice to the one where heating under reflux is effected, preferably at the temperature where heating under reflux is effected, for a sufficient time to ensure adequate progress of the reaction, specifically for 5 min to 3 h.

If R⁶ or R⁷ is an acetyl group, halogenation may be performed in the presence or absence of light, AIBN or a peroxide such as benzoyl peroxide (BPO), preferably in their absence using a suitable halogenating agent such as chlorine gas, bromine, copper bromide, N-bromosuccinimide (NBS), N-chlorosuccinimide (NCS), trihalogenomethanesulfonyl halogenide, trichlorobromomethane or phenyltrimethylammonium tribromide (PTT), preferably PTT, and also using a halogenated hydrocarbon solvent such as carbon tetrachloride, chloroform or methylene chloride, an aromatic hydrocarbon-based nonpolar solvent such as benzene or toluene, an ether-based solvent such as THF, dioxane or DME, acetic acid or carbon disulfide solvent, preferably using THF as a solvent at a temperature ranging from room temperature to the one where the reaction mixture is heated under reflux, preferably at the temperature where heating under reflux is effected, for a sufficient time to ensure adequate progress of the reaction, specifically for 1 h to 12 h; thereafter, the resulting halide is reacted with aniline, N-methylaniline, morpholine or the like using an inorganic base such as potassium carbonate, cesium carbonate, calcium carbonate or sodium hydrogencarbonate or an organic base such as triethylamine, pyridine or N,N-dialkylaniline, preferably using sodium hydrogencarbonate, and also using a polar solvent such as acetonitrile or dimethylformamide (DMF), a halogenated hydrocarbon solvent typified by chloroform or methylene chloride, an ether-based solvent typified by ether or THF or an alcoholic solvent such as methanol or ethanol, preferably using ethanol as a solvent at a temperature ranging from room temperature to the one where the reaction mixture is heated under reflux, preferably at the temperature where heating under reflux is effected, for a sufficient time to ensure adequate progress of the reaction, specifically for 1 h to 12 h.

If R⁶ or R⁷ is a halogen atom, dehalogenation may be performed in the presence of palladium using acetic acid as a solvent at a temperature ranging from room temperature to the one where the reaction mixture is heated under reflux, preferably at the temperature where heating under reflux is effected, for a sufficient time to ensure adequate progress of the reaction, specifically for 1 h to 12 h. The substituent changes described above may also be applied to R⁸.

If necessary, the compound of (XXIII) or (XXIV) may be subjected to further substituent changes so as to produce the compound of the formula (I) or a salt thereof:

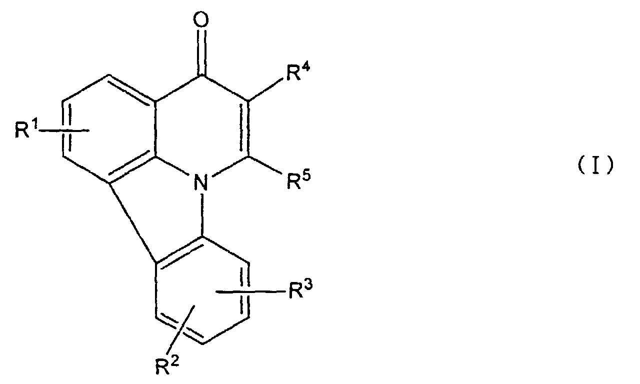

According to another method of producing the compound of the formula (I) or a salt thereof, R⁶, R⁷ and R⁸ in the compound of the formula (XXII) may be changed to other substituents by the same reactions as described above to prepare the compound represented by the formula (XXIV) which is then reacted with the reactive halogen derivative of the formula (XX) in the manner already described above.

### (Process 2)

Depending on the positions, types and number of substituents and the selectivity in ring closure (cyclization), the compound of the formula (I) may occasionally be synthesized more efficiently by Processes 2 and 3 than by Process 1.

A phenylhydrazine derivative represented by the following formula (V): 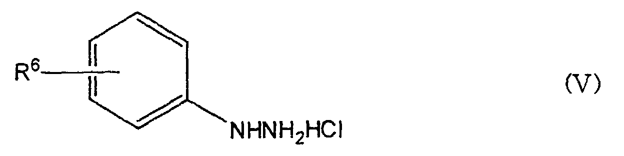 and a cyclohexanone derivative represented by the following formula (XIV) or a salt thereof: 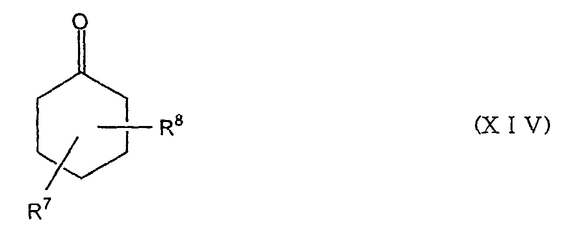 are subjected to the Fischer indole synthesis in the presence or absence of zinc chloride, a Lewis acid or a proton acid catalyst, preferably in their absence, using acetic acid as a solvent at a temperature ranging from room temperature to the one where the reaction mixture is heated under reflux, preferably at the temperature where heating under reflux is effected, for a sufficient time to ensure adequate progress of the reaction, specifically for 1 h to 3 h so as to yield a tetrahydrocarbazole derivative represented by the following general formula (VI):

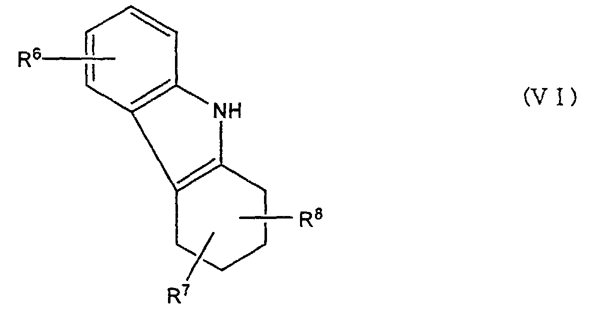

Subsequently, in the same manner as employed to achieve transformation from the formula (II) to (III) in Process 1, the compound (VI) is derivated to the following formula (VII): 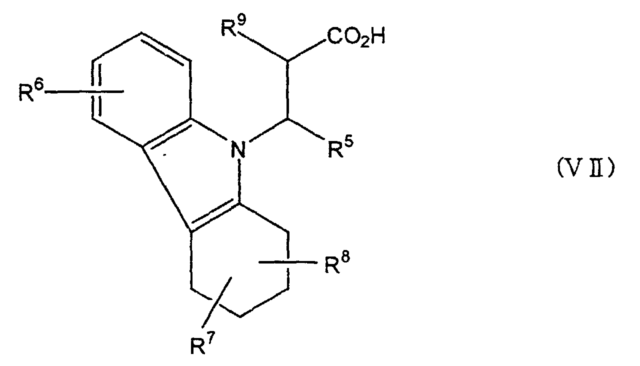 and, subsequently, in the same manner as employed to achieve transformation from the formula (III) to (IV) in Process 1, the compound (VII) is cyclized to give an intermediate of the following formula (VIII): 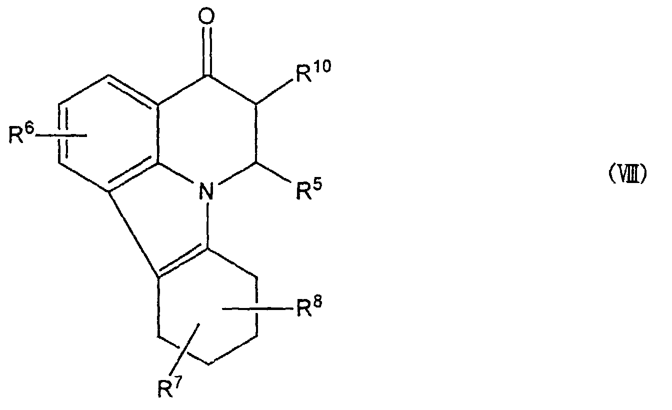 and the same procedure as employed to achieve transformation from the formula (IV) to (I) in Process 1 is repeated, followed by aromatization using DDQ to produce the compound represented by the following general formula (I) or a salt thereof:

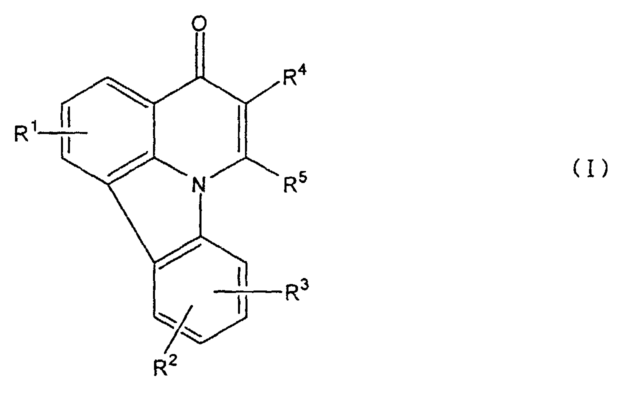

### (Process 3)

An aniline derivative represented by the following general formula (IX) or a salt thereof: 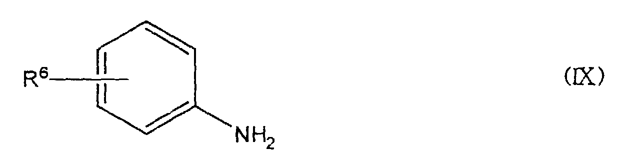 is subjected to the same procedure as employed to achieve transformation from the formula (II) to (III) in Process 1, thereby giving an intermediate of the following formula (X): 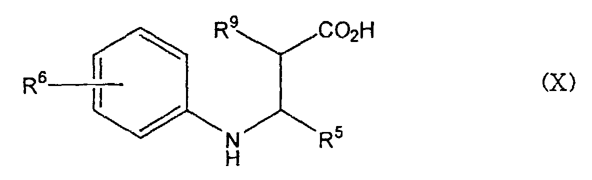 and the same procedure as employed to achieve transformation from the formula (III) to (IV) in Process 1 is repeated to perform cyclization, thereby yielding a compound of the following general formula (XI):

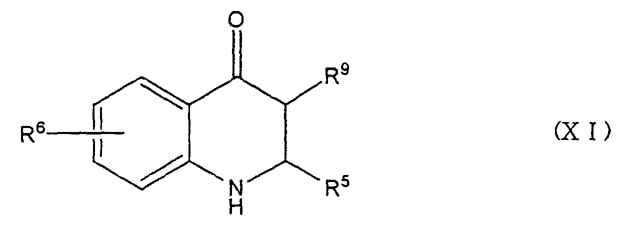

This compound and an aryl halide represented by the following general formula (XV) 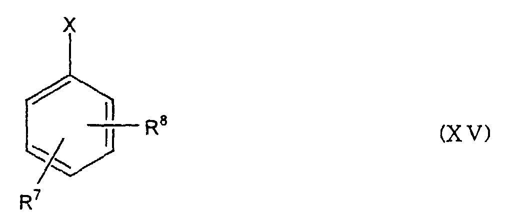 are subjected to Ullmann reaction in the presence of a copper powder, copper oxide or an iron powder, preferably in the presence of copper oxide, using an inorganic base such as potassium hydroxide or potassium carbonate or an alkali metal reagent such as sodium alkoxide or sodium hydroxide, preferably using potassium carbonate either without solvents or using a suitable high-boiling point solvent such as DMF, DMSO, DME, dibutyl ether, xylene, decalin or 1,3-dimethyl-2-imidazolidone (DMI), preferably in the absence of solvents, at 100 - 200°C, preferably at 180 - 190°C for a sufficient time to ensure adequate progress of the reaction, specifically for 1 h to 12 h, thereby introducing a desired substituted phenyl group to derivate a compound of the formula (XII): 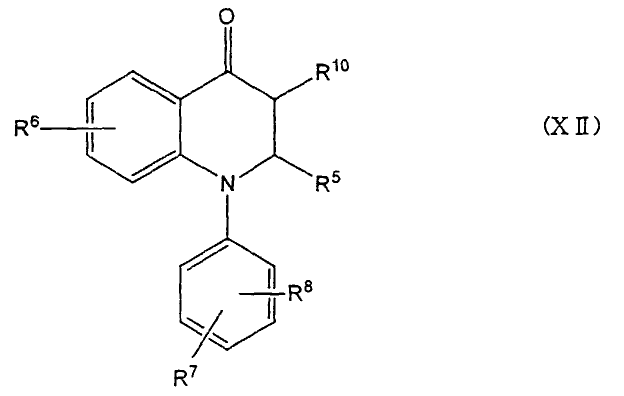 and the same procedure as employed to achieve transformation from the formula (IV) to (I) in Process 1 is repeated to give an intermediate compound of the formula (XIII): 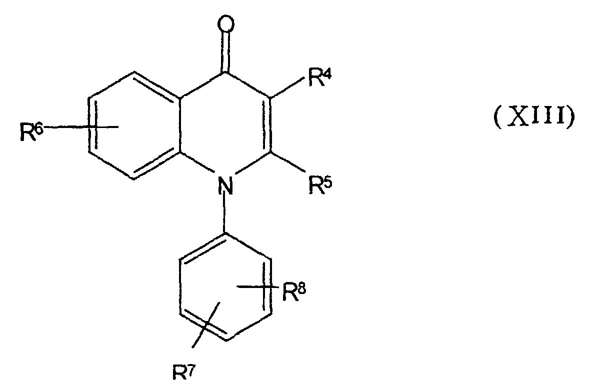 which is then subjected to an aromatic carbon-carbon bond formation in the presence of palladium acetate, a boron trifluoride acetic acid complex, palladium chloride or the like, preferably in the presence of palladium acetate, using a solvent such as acetic acid, trifluoroacetic acid or methanesulfonic acid, preferably using acetic acid as the solvent, at a temperature ranging from room temperature to the one where the reaction mixture is heated under reflux, preferably at the temperature where heating under reflux is effected, for a sufficient time to ensure adequate progress of the reaction, specifically for 1 h to 5 h, and the reaction product may optionally be subjected to the same reaction for substituent changes as in Process 1, thereby yielding the compound of the following general formula (I) or a salt thereof:

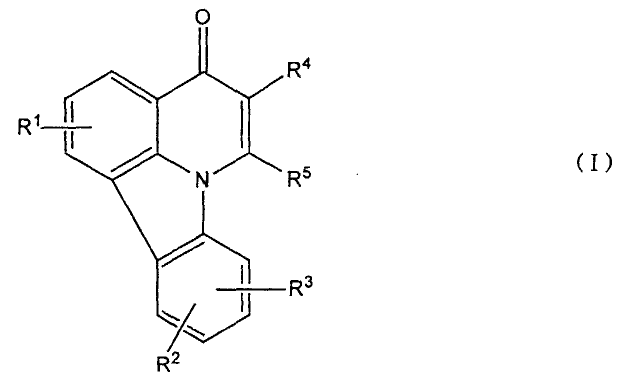

If the individual compounds synthesized by the processes described above contain reactive substituents such as a hydroxyl group, an amino group, a carboxyl group and a thiol group, they may be protected appropriately in the respective steps of reaction and later removed at appropriate stages. The methods of introducing and removing such protective groups may appropriately be selected in accordance with the types of the groups to be protected and the protective groups to be used and suitable methods may be found in the Overview in "Protective Groups in Organic Synthesis", 2nd Ed. 1991.

It should also be noted that the compounds prepared in the respective steps of each production process may have the functional groups optionally oxidized or reduced in the usual manner.

The compounds which form the effective component of the composition of the invention are represented by the above mentioned formula (XXV):
(where R¹ is H, C 1-3 alkyl, C 3-5 cycloalkyl or C 1-3 penfluoroalkyl; R² is H, OH, C 1-6 alkyl substituted with C 1-3 alkoxy or C 3-6 cycloalkyl or C 1-3 perfluoroalkyl; R³ is C 1-6 alkyl, C 3-6 alkenyl, C 3-6 alkynyl, C 3-7 cycloalkyl, C 1-6 perfluoroakyl or (C₃₋₆ cycloalkyl)-C₁₋₆ alkyl; R⁴ forms a pyrrolidinyl, piperidino, phorpholino or 4-N-(R⁶)-piperazinyl group together with a nitrogen atom to which R⁴ is bonded; R⁵ is H, C 1-4 alkyl, C 1-3 alkoxy, NR⁷R⁸ or CONR⁷R⁸; R⁶ is H, C 1-6 alkyl, (C₁₋₃ alkoxy)-C₂₋₆ alkyl, hydroxy C 2-6 alkyl, (R⁷R⁸N)-C 2-6 alkyl, (R⁷R⁸NCO)-C 1-6 alkyl, CONR⁷R⁸, CSNR⁷R⁸ or C(NH)NR⁷R⁸; and R⁷ and R⁸ each independently is H, C 1-4 alkyl, (C₁₋₃ alkoxy)-C₂₋₄ alkyl or hydroxy C 2-4 alkyl). The compounds which form the effective component of the composition of the invention represented by the formula (XXV) can be prepared according to the method described in Example 12 of the European Patent Publication No. 463,756.

Terms used in the present specification will be explained as hereunder.

Compounds having an inhibitory action to cGMP-PDE means the compounds having an inhibitory action to PDE of at least type V and their examples are the pyridocarbazole derivatives represented by the formula (I) in the specification of the present invention, the pyrazolopyridiminone derivatives represented by the formula (XXV) in the specification of the present invention, and the following compounds such as pyrazolopyridiminone derivatives in the European Patent Publication 463,756 or 526,004; purinone derivatives in the Japanese Laid-Open Patent Publication Sho-63/196,585 or Hei-02/88,577, the International Publication WO 94/00453 or the Japanese Laid-Open Patent Publication Hei-08/231,545, Hei-08/231,546 or Hei-09/124,648; quinazoline derivatives in the Japanese Laid-Open Patent Publication Sho-52/100,479, the International Publication WO95/06,648 or WO 96/26,940, or the Japanese Laid-Open Patent Publication Hei-08/104,679, Hei-07/126,255, Hei-06/192,235 or Hei-07/10,843; quinazolinone derivatives in the Japanese Laid-Open Patent Publication Hei-02/193,983, the International Patent Publication 93/12,095 or the Japanese Laid-Open Patent Publication Hei-08/253,457; pyrimidine derivatives in the Japanese Laid-Open Patent Publication Sho-53/103,497 or Sho-61/236,778, U.S. Patent No. 5,294,612, the Japanese Laid-Open Patent Publication Hei-02/42,079, Hei-02/56,484, Hei-02/40,388, Hei-03/261,785, Hei-07/89,958, Hei-07/267,961, Hei-07/330,777 or Hei-08/143,571, U.S. Patent No. 5,525,604 or 5,541,187, the International Publication WO 96/28,429 or WO 96/28,448 or the Japanese Laid-Open Patent Publication Hei-08/253,484; pyrimidinone derivatives in the Japanese Laid-Open Patent Publication Hei-02/295,978 or the International Publication WO 93/06,104, WO 93/07,149 or WO 94/05,561; griseol derivatives in the Japanese Laid-Open Patent Publication Sho-62/30,796; phenylpyridone derivatives in the Japanese Laid-Open Patent Publication Hei-01/311,067 or Hei-03/145,466; dihydropyridine derivatives in the Japanese Laid-Open Patent Publication Hei-02/223,580; polycyclic guanine derivatives in the International Publication WO 91/19,717 or WO 94/19,351; benzimidazole derivatives in the Japanese Laid-Open Patent Publication Hei-05/222,000 or the International Publication WO 97/24,334; fluorenone derivatives in the Japanese Laid-Open Patent Publication Hei-07/61,949; anthranilic acid derivatives in the Japanese Laid-Open Patent Publication Hei-08/188,563; pyridazine derivatives in the Japanese Laid-Open Patent Publication Hei-08/225,541; pyrazoloquinoline derivatives in the International Publication WO 96/28,446; pyridopyrazinone derivatives in the Japanese Laid-Open Patent Publication Hei-08/269,059; indole derivatives in the International Publication WO 96/32,379; dihydropyrazolone derivatives in the Japanese Laid-Open Patent Publication Hei-08/311,035; phthalazine derivatives in the International Publication WO 96/05,176; imidazoquinazoline derivatives in the International Publication WO 98/08848; and thienopyrimidine derivatives in the International Publication WO 98/06722 although the present invention is not limited thereto.

A composition for remedy of erectile dysfunction is administered not only at the stage of erectile dysfunction but also on a preventive sense to a patient having a history of erectile dysfunction.

A composition for intranasal administration includes all types of composition which is administered from nasal cavity whereby absorption from mucous membrane of nasal cavity or, in some cases, from bronchus or lung is expected and, therefore, a composition with an object of administration to digestive organs via nasal cavity is excluded. Examples of the common dosage form are liquid, diluted powder, capsule, lotion, gel, ointment and cream. In the case of liquid, it is preferred that pH is adjusted to 4-8 or, preferably, 5-7 and that osmotic pressure ratio is adjusted to about 1. In the case of diluted powder, it is preferred that more than 90% by weight of the particles are within a particle size of 10-250 µm. With regard to dosage form of those compositions, they are sucked through, dropped into or applied to nasal cavity using a suitable administering device such as quantitative sprayer (e.g., spray pump, aerosol, nebulizer or atomizer) or a dropping vessel (e.g., dropper or nasal dropper) to adhere to nasal mucous membrane whereby the effective component can be absorbed through the nasal mucous membrane.

Female sexual dysfunction means a disorder in sexual function including orgasm insufficiency related to clitoral disturbance.

### [Test Examples]

As hereunder, pharmacological actions, absorption, pharmacokinetics, toxicity, etc. of the representative compounds used as effective components in the compositions of the present invention will be mentioned although the present invention is never limited thereto.

### (Experiment 1)

### [PDE Inhibiting Activity]

### Method

On the basis of the method of Lugnier et al. (Biochem. Pharmacol., 35, 1743-1751, 1986), PDE was purified from the aorta in a dog. The canine aorta was minced and homogenized with a Waring Blender and a glass homogenizer in six volumes of a Tris-HCl buffer solution (pH 7.5, 20 mM) containing 2 mM magnesium acetate, 5 mM ethylenediaminetetraacetic acid (EDTA), 100 µg/mL of phenylmethylsulforyl fluoride and 15 mM 2-mercaptoethanol (2-ME), and centrifuged at 1200 x g for 30 min. The supernatant was separated and salted out with ammonium sulfate which was added to 45% saturation. The resulting precipitated fraction was resuspended in a Tris-HCl buffer solution (pH 7.5, 20 mM) containing 2 mM magnesium acetate and 1 mM 2-ME, dialyzed overnight and applied to a DEAE-trisacryl column (DEAE TRISACRYL M:IBF). By elution with a sodium chloride gradient (0 - 0.4 M), PDE types V and III were separated from the other isozymes. The supernatant fraction of 45% saturated ammonium sulfate was further mixed with ammonium sulfate to 65% saturation and salted out. The resulting precipitated fraction was similarly applied to the DEAE-trisacryl column and eluted by a sodium chloride gradient (0 - 0.4 M) so as to separate PDE type I.

The thus obtained PDE types V, III and I were measured for their activity in accordance with the method of Thompson et al. (Adv. Cyclic Nucleotide Res., 10, 69-72, 1979) and the method of Wells et al. (Biochim. Biophys. Acta, 384, 430-443, 1975). Specifically, a purified PDE sample was added to 50 mM Tris-HCl buffer solution (pH 7.5) containing 1 µM of substrate cGMP or cAMP (containing tritium-labelled cGMP or cAMP), 1 mM EGTA and 2 mM magnesium acetate. For PDE activity measurement, enzymatically produced 5' GMP or 5' AMP was further hydrolyzed into guanosine or adenosine with snake venom and separated from the substrate by means of an ion-exchange resin (Dowex 1-X2), followed by measuring with a scintillation counter. The activity of each test compound was determined as a percentage of the PDE activity measured when it was added as a dimethyl sulfoxide solution (DMSO) and its IC₅₀ (50% inhibition concentration) was calculated by the probit method. The final concentration of DMSO was adjusted to be no more than 2% in consideration of the effect on PDE activity. The results are shown in Table 1 below.

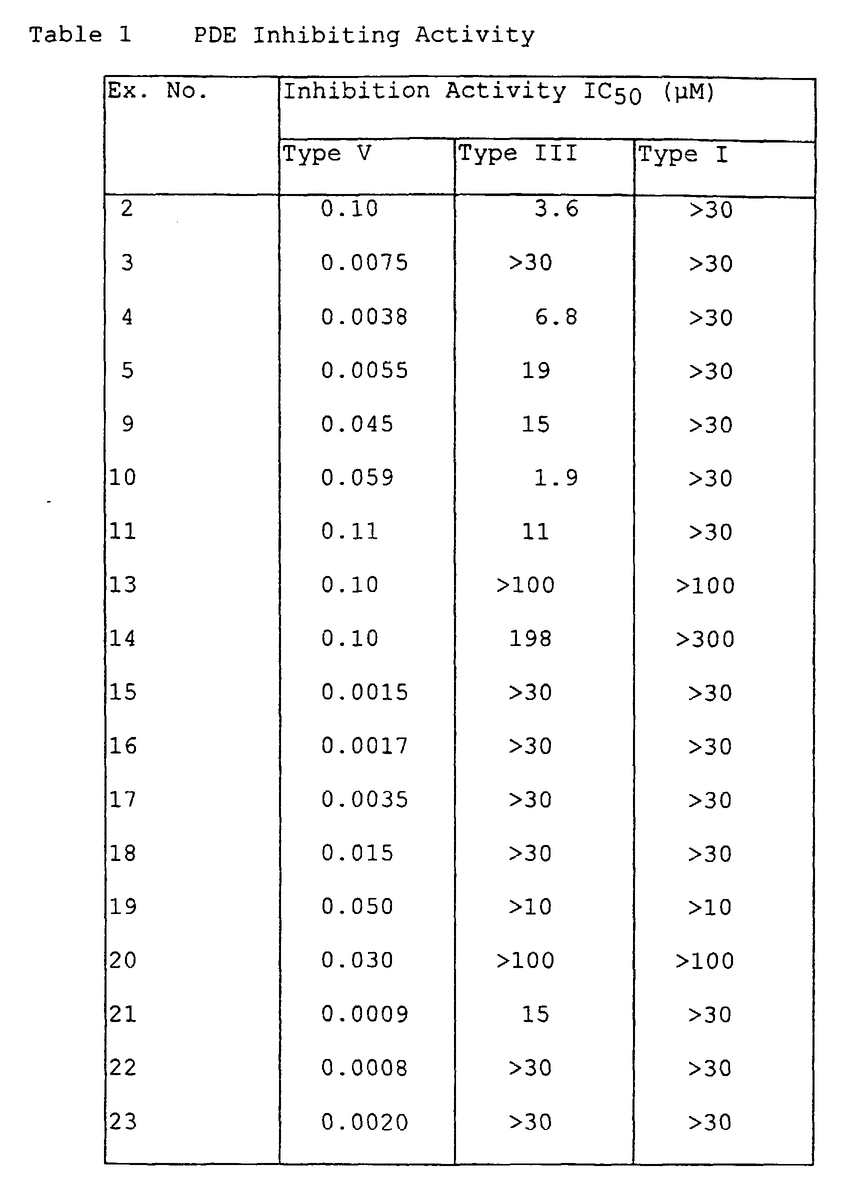

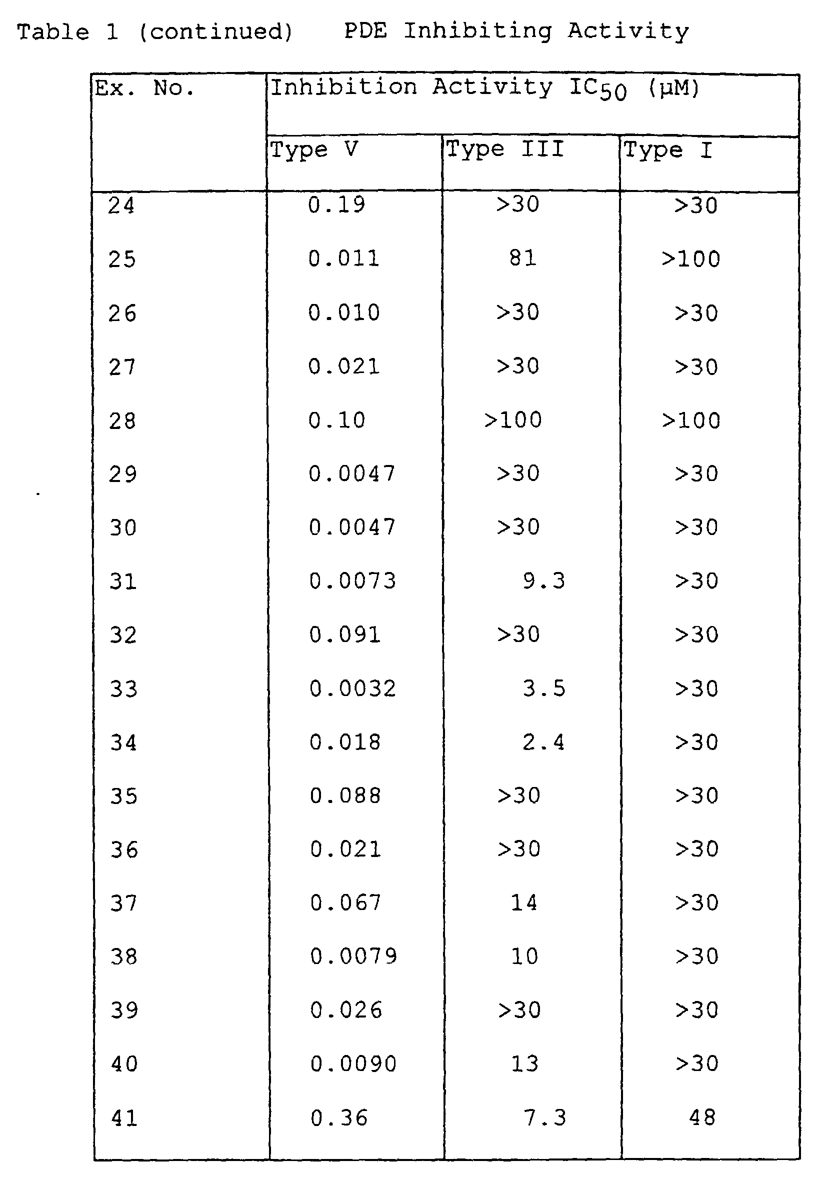

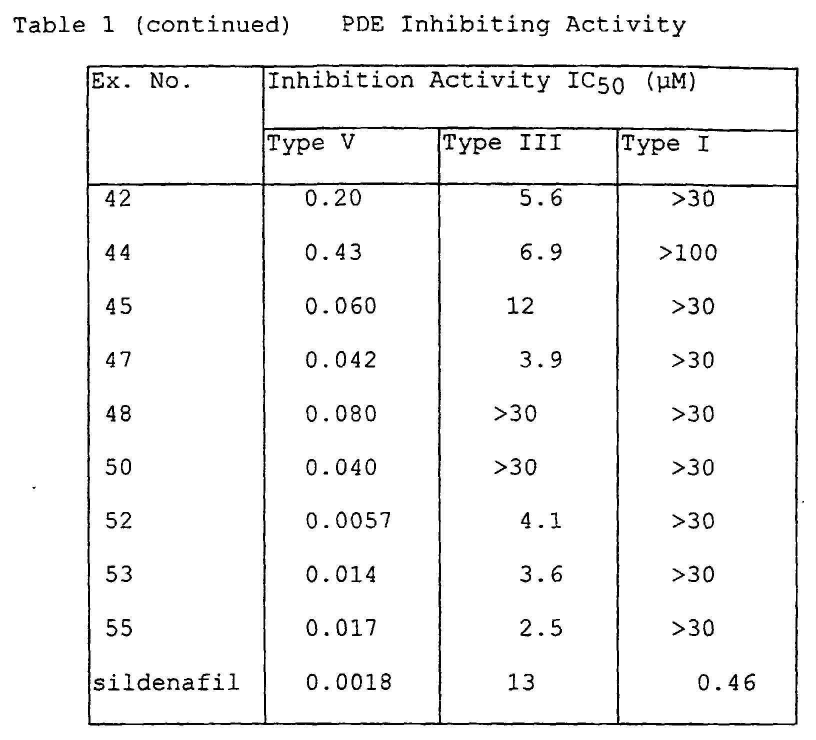

Each of the compounds forming the effective component of the composition of the invention was found to have a marked PDE type V inhibitory action and high selectivity in enzyme inhibition.

### (Experiment 2)

### [Action for enhancing the relaxation of smooth muscle of cavernous body of rabbits]

Rabbits were anesthetized by an intravenous injection of 40-50 mg/kg of pentobarbital (Nembutal™ manufactured by Dainippon Pharmaceutical Co., Ltd.), blood was drained out by incision of carotid artery, and penis was taken out and placed in an ice-cooled nutrient solution (Krebs bicarbonate containing 118 mM of NaCl, 4.7 mM of KCl, 1.2 mM of KH₂PO₄, 1.2 mM of MgSO₄.7H₂O, 1.5 mM of CaCl₂, 25 mM of NaHCO₃, 11 mM of glucose and 0.023 mM of EDTA·2Na). After the tissues around the albuginea were incised in a nutrient solution, the albuginea was incised to prepare cavernous body of the penis. The cavernous body was cut and both ends of the strips of about 1 cm length were ligated by nylon yarn. One end was fixed to a supporting rod, transferred into a 20-ml Magnus tube which was filled with the nutrient solution, passed with a mixed gas of 95% oxygen and 5% carbon dioxide and kept at 37 °C while another end of the strip was connected to an isometric transducer (TB-611T; NIHON KOHDEN CORP.) placed on the upper area and a stress load of about 2 g was applied. After stabilization of 60 minutes or longer, strips were contracted with 10 µM of phenylephrine in the presence of 5 µM of guanetidine and 1 µM of atropine and, after stabilization, 1 µM or 100 µM of nitroprusside was added whereby relaxation by nitroprusside was ascertained. After washing, a sample or solvent (DMSO with a final concentration of 0.1%) was added and, after about 20 minutes, tension was generated again by 10 µM of phenylephrine and the relaxation by 1 µM or 100 µM of nitroprusside was observed by the same manner as above. Rate of relaxation by 1 µM of nitroprusside after addition of the sample was compared with that after addition of the solvent and the increasing rate was calculated. The rate of relaxation was calculated in such a manner that the relaxation of tension level before addition of nitroprusside was defined as 0% relaxation and the relaxation by 100 µM of nitroprusside was defined as 100% relaxation. The result is given in Table 2.

**Table 2**

| Enhancing Action to Smooth Muscle relaxation of Cavernous Body of Rabbits | | |
|---|---|---|
| Example No. | Final Added Amount (nM) | Enhancing Rate of Relaxation (%) |
| Examples 52 | 100 | 90.8 |
| | 1000 | 190.9 |
| Sildenafil | 1000 | 189.1 |

The compounds forming the effective component of the composition of the present invention showed an enhancing action to smooth muscle relaxation of cavernous body of rabbits whereby it was found to exhibit an effect for the remedy of erectile dysfunction.

### (Experiment 3)

### [Absorption upon Intranasal Administration to Rats]

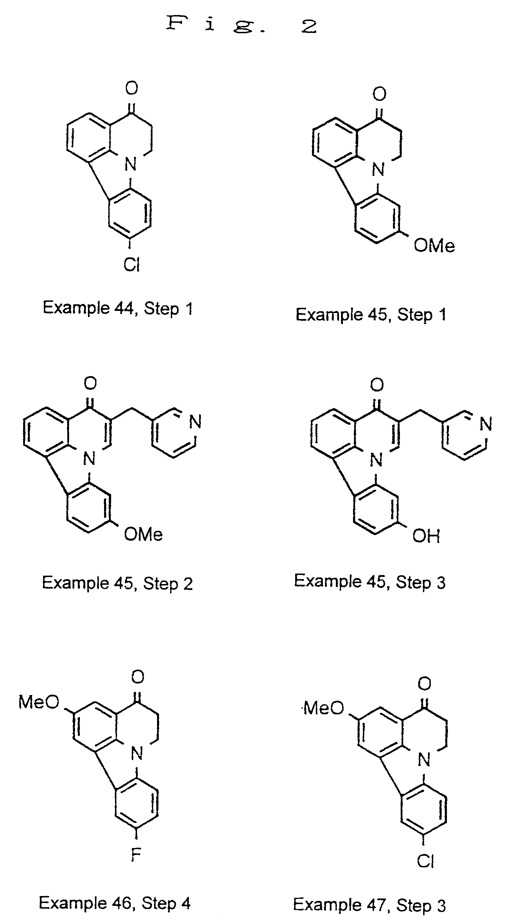

Male wistar rats (eight weeks age) were anesthetized by intraperitoneal injection of 1 g/kg of urethane. Neck was incised to expose a trachea and polyethylene tube (SP 120; Natsume Seisakusho) was inserted into the trachea. Then a part of esophagus was opened by cutting and a polyethylene tube of the same diameter was inserted to the direction of choana and the end was tightly sealed with absorbent cotton and adhesive. In order to prevent a leakage of drug solution from nasopalatine canal, said canal of upper chin opening to mouth was closed by synthetic adhesive. The drug was dissolved in a 0.1% tartaric acid solution and the drug of 500 µl/kg was administered by a micropipette from outer nasal cavity followed by closing both outer nasal cavities immediately. As a control, the rat to which the drug was intravenously injected was prepared. After the injection; aliquot of blood was collected from time to time from jugular vein using a heparin-treated syringe and centrifuged to give plasma. Certain amount of the resulting plasma was collected, extracted with tert-butyl-methyl-ether three times, and the organic phase was evaporated to dryness and dissolved in the initial solvent for HPLC. A certain amount thereof was injected into an HPLC connected to an ultraviolet absorption detector and the drug concentration in the plasma was measured by reversed phase column. Time for arriving the maximum blood concentration (Tmax), mean residence time (MRT) and mean absorption time (MAT) were calculated from the measured value. The result is given in FIG. 2 and in Table 3.

**Table 3**

| Absorptions by Intranasal Administration | | | | |
|---|---|---|---|---|
| Example No. | Dose (mg/kg) | Tmax (hr) | MRT (hr) | MAT (hr) |
| Example 52 | 0.3 | 0.2-0.5 | 2-3 | 1-2 |
| Sildenafil | 1.0 | 0.2-0.5 | 2-3 | 1-2 |

In both of the compound of Example 52 and sildenafil, Tmax was same or shortened, MRT was same and bioavailability was enhanced (as high as about 100%) as compared with oral administration. Thus, as a result of intranasal administration, quick and efficient absorption was noted, the affection by first-pass effect was not observed and an appropriate sustained time was resulted.

Further, according to the observation during administration of the drug, there was no irritation and, throughout the period of the experiment, no abnormal change was noted in nasal mucous membrane.

### (Experiment 4)

### [Absorption by Percutaneous Administration in Rats]

Abdominal skin of hairless rats (eight weeks age, Nippon SLC) was wiped with 70% ethanol, and 0.25 ml/kg of a drug solution (40 mg/ml DMSO) was applied to an area of 8 cm² (2 x 4 cm) and the applied area was coated with a film coating agent (Permiaid S, Nitto Medical) and fixed with an adhesive bandage (Elastopore, Nichiban). After application of the drug solution, aliquot of blood was collected from time to time using a heparin-treated syringe from jugular vein and centrifuged to give plasma. Aliquot plasma was extracted with tert-butyl-methyl-ether three times, and the organic phase was evaporated to dryness and dissolved in the initial solvent for HPLC. A certain amount thereof was injected into a HPLC connected to an ultraviolet absorption detector and the drug concentration in the plasma was measured by means of a reversed phase column. Pharmacokinetic parameters were calculated from the measured data by the same manner as in Experiment 3. The result is given in Table 4.

**Table 4**

| Absorptions by Percutaneous Administration | | | | |
|---|---|---|---|---|
| Example No. | Dose (mg/kg) | Tmax (hr) | MRT (hr) | MAT (hr) |
| Example 52 | 10 | 1.0 | 8.5 | 7.9 |
| Sildenafil | 10 | 1.0 | 14.7 | 14.3 |

In both of the compound of Example 52 and sildenafil, Tmax and MAT were elongated and MRT was elongated as well as compared with intranasal administration. Thus, as a result of percutaneous administration, slow and sustained absorption was noted.

Further, according to the observation during administration of the drug, there was no irritation and, throughout the period of the experiment, no abnormal change was noted in the skin to which the drug was applied.

### (Experiment 5)

### [Toxicity Test]

Toxicity of the compounds forming the effective component of the composition of the present invention was checked. To male wistar rats (four weeks age) were orally administered 100 mg/kg/day of the compounds of Examples 3, 15, 31, 34, 37, 38, 44 and 52 for four days. There was no dead case until the next morning of the completion of the administration and no abnormal change was noted in body weight and general symptoms. With regard to sildenafil, clinical administration has been conducted already and no serious side effect was reported (cf. Drugs of the Future, volume 22, pages 138-143, 1997).

From the above facts, it has been demonstrated that the compounds represented by the above formulae (I) and (XXV) which are effective components of the composition of the present invention have significant inhibitory action to PDE of type V and very high selectivity in enzyme inhibition. It has been further demonstrated that the compounds represented by the above formulae (I) and (XXV) which are effective components of the composition of the present invention and salts thereof have an action of enhancing the relaxation of smooth muscle of cavernous body.

Moreover, the compounds which form the effective component of the composition of the present invention are absorbed by both intranasal and percutaneous administration routes and, in the case of intranasal administration, prompt absorption, appropriately-acting pharmaceutical effect and good availability are resulted while, in the case of percutaneous administration, gradual and sustained absorption is resulted.

In the meanwhile, the compounds which form the effective component of the composition of the present invention did not show side effect upon administration and, in the toxicity test, no abnormality was noted at all whereby the compounds were ascertained to be highly safe.

Accordingly, the compounds represented by the above formula (I) which form the effective component of the composition of the present invention had a significant inhibitory action to PDE of type V and a very high selectivity to enzyme inhibition and showed an action of enhancing the relaxation of smooth muscle of cavernous body of rabbits whereby it can be used in a composition for remedy of erectile dysfunction. In addition, it is useful as a composition for the remedy of female sexual dysfunction.

What is more, if the PDE type V inhibitor having an enzyme inhibiting action with adequately high selectivity is not used, other drugs administered either alone or in combination with cardiovascular drugs such as nitrates or hypotensives may cause an excessive drop in blood pressure as a potentially life-threatening side effect. The compounds of the above formula (I) which form the effective component of the composition of the invention are potent and have an extremely high selectivity for PDE type V enzyme inhibition, with weak hypotensive action. Therefore, if administered either alone or together with nitrates, the compounds will cause less side effects due to the lowering of blood pressure. As a further advantage, the compounds do not enhance the actions of other hypotensives and may be used with them with high safety. They do not cause nasal congestion or rubor, either.

Therefore, the PDE type V inhibitor of the above formula (I) in the composition of the invention is useful as a therapeutic of erectile dysfuntion or female sexual dysfunction that does not cause any side effects on the heart, that will cause less side effects due to the lowering of blood pressure even if it is administered in combination with nitrates, and that does not enhance the actions of other hypotensives but may be used with them with high safety.

The pharmaceuticals of the invention are administered in the form of pharmaceutical compositions.

The pharmaceutical compositions of the invention may contain at least one of the compounds having a cGMP-PDE inhibitory action, and salts or solvates thereof and they are prepared by being combined with pharmaceutically acceptable additives. More specifically, excipients (e.g., lactose, sucrose, mannitol, crystalline cellulose and silicic acid), binders [e.g., crystalline cellulose, sugars (e.g., mannitol, sucrose, sorbitol, erythritol and xylitol), dextrin, hydroxypropyl cellulose, hydroxymethyl cellulose (HPMC), polyvinyl pyrrolidone (PVP) and macrogol], lubricants (e.g., magnesium stearate, calcium stearate and talc), coloring agents, flavoring agents, disintegrants (e.g., corn starch and carboxymethyl cellulose), antiseptics (benzalkonium chloride, paraoxybenzoate), isotonic agents (e.g., glycerol, sodium chloride, calcium chloride, mannitol, grape sugar), pH adjusting agent (sodium hydroxide, potassium hydroxide, sodium carbonate, hydrochloric acid, sulfuric acid, buffers such as phosphoric acid buffer), stabilizers (e.g., sugar, sugar alcohol and xanthan gum), dispersants, antioxidants [e.g., ascorbic acid, butyl hydroxyanisole (BHA), propyl gallate and dl-α-tocopherol], buffering agents, preservatives (e.g., parabens such as methyl paraben and propyl paraben, benzyl alcohol and benzalkonium chloride), fragrances (e.g., vanillin, 1-menthol and rose oil), solubilizers (e.g., polyoxyethylene cured castor oil, polysorbate 80, polyethylene glycol, phospholipid), absorption accelerators (e.g., sodium glycocholate, sodium edetate, sodium caprylate, acyl carnitines, limonene), suspending or emulsifying agents, and other common suitable additives or solvents may be combined appropriately with the compounds which form the effective component of the composition of the invention into various dosage forms. Suitable dosage forms include tablets, capsules, granules, powders, suppositories, vaginal suppositories, sublingual preparations, buccal preparations, intraoral disintegrable preparations, chewable tablets, troches, jelly preparations, pastes preparations, patches to be applied to the oral mucosa, syrups (oral liquids and emulsions), inhalants, external applications (e.g., ointments, creams, jellies and gels), paints (e.g., tapes, patches and cataplasms), injections, intranasal preparations (e.g., liquids and powders), etc. These may be administered to the patient either orally or parenterally (e.g., by intravenous, intra-arterial, subcutaneous, intramuscular, intrarectal, intravaginal, intranasal or percutaneous route or by transmucomembranous route such as through the oral mucomembranous or penile mucomembrane).

Preferably, the composition can be administered to patients by means of preparations for oral administration, intranasal administration, percutaneous administration or transmucomembranous administration. Among them, preparation for intranasal administration is particularly preferred. In administering the composition of the present invention, there are a method where the composition is given at one draft when necessary and another method where predetermined amount is given per day and that is continued for certain period. In the former case, intranasal administration or administration through the oral mucosa is particularly preferred and the intended effect is anticipated to develops in several to about thirty minutes after the administration. In the case of administration for certain continuous period, oral or percutaneous administration is preferred. Especially in the case of percutaneous administration, absorption of the drug is resulted gradually and continuously and it is possible to achieve a state of ready-for-erection whereby natural erection can be resulted by sexual stimulation.

Dose of the effective component of the composition of the present invention for adults per day is usually from 0.1 mg to 2.5 g, preferably from 0.5 mg to 1.0 g or, more preferably, from 1 mg to 250 mg although that may be appropriately increased or decreased depending upon symptom, age, administering method and administering route. It is also possible that the daily dose is administered at a time or on a divided manner (2 to 6 times a day) by oral, intranasal or percutaneous route as well as by means of transmucomembranous administration, of intravenous dripping injection or of continuous administration.

In the case of intranasal administration, the composition is sucked by, dropped on or applied to nasal cavity using an appropriate administering device such as a quantitative sprayer (e.g., spray pump, aerosol, nebulizer or atomizer) or a dropping vessel (e.g., dropper or nasal dropping pipette) to adhere to nasal mucous membrane whereby the effective component is absorbed through nasal mucous membrane. In the case of liquid preparation for intranasal administration, it is administered to one of or both nostrils at a dose of 20-300 µl, preferably 50-200 µl or, more preferably, 100-150 µl per nostril at a time for one to six times a day. In the case of diluted powder preparation for intranasal administration, a capsule filled with the powder was placed on a particular sprayer equipped with a needle to pierce the needle whereby very small holes are formed on both upper and lower parts of the capsule and then air is blown thereinto using a rubber ball blower or the like so that the powder is sprayed out. As such, the powder can be administered to one of or both nostrils at a dose of 1-20 mg or preferably 5-10 mg per nostril at a time for one to six times a day. When viscosity is relatively high such as in the case of gel for intranasal administration, it is also possible that the preparation is directly applied to nasal cavity from a tube or that a certain amount is taken into a device which can be inserted followed by administering into nasal cavity.

In the case of a patient suffering from nasal congestion or nasal inflammation, it is not possible to afford a suitable absorption as compared with the administered dose and, therefore, the preparation may be administered together with or in a form of compounded preparation with vasoconstrictor drugs such as epinephrine, naphazoline nitrate, tramazoline hydrochloride and tetrazoline; antiallergic drugs such as sodium cromoglicate and ketotifen fumarate; suppressor for secretion of nasal mucus such as flutropium bromide and ipratropium bromide; and steroids such as prednisolone, flunisolide, fluticasone propionate, beclomethasone propionate, sodium dexamethasone metasulfobenzoate and sodium dexamethasone phosphate.

In the case of percutaneous administration, it can be conducted in a form of ointment, cream, gel or a sticking agent such as tape, patch and cataplasma. It is also possible to utilize absorption accelerators or drug delivery system such as iontophoresis. A long-acting effect is resulted by exchanging once a day or every several days and, therefore, sticking agent is preferred.

Intraoral administration as an example of transmucomembranous administration may be performed with various dosage forms including sublingual preparations, buccal preparations, intraoral disintegrable preparations, chewable tablets, troches, jelly preparations, pastes preparations and patches to be applied to the oral mucosa. Preferred dosage forms are sublingual preparations and intraoral disintegrable preparations, with the latter being particularly preferred. If desired, these dosage forms may be used in combination with absorption accelerators, pH adjusting agents, fragrances, flavoring agents, disintegrants or the like.

As one of the means for transmucomembranous administration, the preparation may be applied, for example, to the inner side of condom whereby the drug can be absorbed from penis.

### Examples

The following examples are provided for the purpose of further illustrating the present invention but are in no way to be taken as limiting.

NMR measurements were performed with JEOL JNM-EX270 FT-NMR (product of JEOL Ltd.) or JEOL JNM-LA300 FT-NMR (product of JEOL Ltd.; the data taken with this model are preceded by an asterisk); IR measurements with HORIBA FT-200 (product of HORIBA Ltd.); and m.p. measurements with Mettler FP-80, FP-82, FP-81HT or FP-90 (each produced by Mettler Instruments AG). In the following examples, the yield of each "titled compound" is parenthesized in both absolute and relative terms.

### Example 1: Synthesis of 10-bromo-2-methoxy-5-(3-pyridylmethyl)-4H-pyrido[3,2,1-jk]carbazol-4-one

### [Step 1] Synthesis of 3-bromo-6-methoxycarbazole

Sodium (23.3 g) was added in small portions to anhydrous methanol (260 ml) to form a solution at room temperature. Subsequently, anhydrous dimethylformamide (1,400 ml), copper iodide (117 g) and 3,6-dibromocarbazole (100 g) were added successively and the mixture was heated under reflux for 2 hours under an argon atmosphere. The reaction mixture was filtered through Celite while hot and left to cool, followed by addition of water (2 L) and extraction with methylene chloride. The methylene chloride layer was successively washed with water, 1N hydrochloric acid, water and saturated aqueous solution of sodium chloride, dried over anhydrous sodium sulfate and the solvent was evaporated under reduced pressure. The residue was purified by silica gel column chromatography (eluent: hexane/methylene chloride = 1:1) to obtain the titled compound (33.4 g; 39%).
melting point: 149.9-151.1 °C
IR spectrum (KBr tab.) ν cm⁻¹: 3390, 2900, 1491, 1205, 1169,806
NMR spectrum (DMSO-d₆) δ ppm:11.23(1H,s), 8.36(1H,s), 7.78(1H,d,J=2.0Hz), 7.48-7.40(3H,m), 7.07(1H,dd,J=8.8,1.5Hz), 3.84(3H,s)

### [Step 2] Synthesis of 3-bromo-6-methoxycarbazole-N-β-propionic acid

The compound (30 g) obtained in Step 1 was suspended in acsetone (80 ml) and the suspension was cooled to 0 °C in an ice bath, followed by addition of methyl acrylate (25 ml) and then dropwise addition of Triton B (10 ml). The ice bath was removed and the mixture was stirred for 1 hour at room temperature and thereafter the solvent was evaporated under reduced pressure. The resulting residue was suspended in methanol (30 ml) and sodium hydroxide (10 g) dissolved in water (60 ml) was added dropwise at room temperature and the mixture was refluxed for 20 minutes. The solvent was evaporated under reduced pressure and thereafter water and ether were added to separate the mixture into the aqueous and the organic phase. The aqueous layer was rendered acidic by addition of 4 N hydrochloric acid and the resulting precipitate was dissolved in ethyl acetate; the solution was washed with water and saturated aqueous solution of sodium chloride, dried over anhydrous sodium sulfate and the solvent was evaporated under reduced pressure. The residual crude crystals were washed with hexane/ether and recovered by filtration to obtain the titled compound (33.6 g; 88%).
melting point: 149.7-152.1 °C
IR spectrum (KBr tab.) ν cm⁻¹: 3425, 2920, 1705, 1697, 1491, 1298, 802
NMR spectrum (DMSO-d₆) δ ppm: 8.38(1H,d, J=1.5Hz), 7.81(1H,d,J=2.4Hz), 7.59-7.49(3H,m), 7.11(1H,dd,J=8.8,2.4Hz), 4.57(2H,t,J=6.8Hz), 3.84(3H,s), 2.67(2H,t,J=6.8Hz)

### [Step 3] Synthesis of 10-bromo-5,6-dihydro-2-methoxy-4H-pyrido[3,2,1-jk]carbazol-4-one

The compound (24 g) obtained in Step 2 was suspended in anhydrous chloroform (500 ml) and PPE (118 g) dissolved in anhydrous chloroform (350 ml) was added at room temperature and the resulting mixture was heated under reflux for 1 hour under an argon atmosphere. After allowing to cool, the mixture was poured into 1 N sodium hydroxide (500 ml) and extracted with chloroform. The chloroform layer was washed with saturated aqueous solution of sodium chloride, dried over anhydrous sodium sulfate and the solvent was evaporated under reduced pressure. The residue was purified by silica gel flash column chromatography (eluent: hexane/methylene chloride = 1:3), and the crude purification product was washed with methanol and recovered by filtration to obtain the titled compound (17.1 g; 75%).
melting point: 175.2-176.1 °C
IR spectrum (KBr tab.) ν cm⁻¹: 2910, 1672, 1497, 1479, 797
NMR spectrum (DMSO-d₆) δ ppm: 8.49(1H,s), 8.14(1H,d,J=2.4Hz), 7.64(2H,bs), 7.36(1H,d,J=2.0Hz), 4.54(2H,t,J=7.1Hz), 3.88(3H,s), 3.13(2H,t, J=7.1Hz)

### [Step 4] Synthesis of 10-bromo-2-methoxy-5-(3-pyridylmethyl)-4H-pyrido[3,2,1-jk]carbazol-4-one

The compound (27 g) obtained in Step 3 was suspended in ethanol (600 ml) and pyridine-3-aldehyde (15 ml) and sodium hydroxide (20 g) which was dissolved in water (100 ml) were added to the suspension at room temperature and the mixture was stirred for 12 hours at room temperature. About one half of the solvent was evaporated under reduced pressure and the precipitated crystals were recovered by filtration and successively washed with water, ethanol and ether to obtain the titled compound (30 g; 87%).

### Example 2: Synthesis of 10-bromo-2-hydroxy-5-(3-pyridylmethyl)-4H-pyrido[3,2,1-jk]carbazol-4-one

10-bromo-2-methoxy-5-(3-pyridylmethyl)-4H-pyrido[3,2,1-jk]carbazol-4-one (10.2 g) obtained in Example 1 was suspended in anhydrous methylene chloride (1000 ml), and boron tribromide (25 g) was added dropwise at room temperature. The reaction mixture was stirred at room temperature for 12 hours, and poured into ice water (500 ml). To this mixture was added saturated aqueous solution of sodium carbonate until the termination of foaming. The crystals precipitated were recovered by filtration. The thus obtained crude crystals were washed in a mixture of methylene chloride and methanol to obtain the titled compound (6.4 g, 65%).

### Example 3: Synthesis of 10-bromo-2-t-butoxycarbonylmethyloxy-5-(3-pyridylmethyl)-4H-pyrido[3,2,1-jk]carbazol-4-one

10-bromo-2-hydroxy-5-(3-pyridylmethyl)-4H-pyrido[3,2,1-jk]carbazol-4-one (4.4 g) obtained in Example 2 was suspended in dimethyl sulfoxide (250 ml), and to the suspension was added potassium carbonate (4.5 g). The mixture was stirred at room temperature for 30 minutes, and t-butyl bromoacetate (2.1 ml) was added. The mixture was stirred at room temperature for 12 hours, and the reaction mixture was poured into ice water (300 ml) and extracted with methylene chloride. The methylene chloride layer was washed with saturated aqueous solution of sodium chloride, and dried over anhydrous sodium sulfate, and the solvent was evaporated under reduced pressure. The residue was purified by silica gel flash column chromatography (eluent: methylene chloride containing 3% methanol) to produce the titled compound (3.4 g, 63%).

### Example 4: Synthesis of 10-bromo-2-i-propoxycarbonylmethyloxy-5-(3-pyridylmethyl)-4H-pyrido[3,2,1-jk]carbazol-4-one

10-bromo-2-hydroxy-5-(3-pyridylmethyl)-4H-pyrido[3,2,1-jk]carbazol-4-one (3.6 g) obtained in Example 2 was suspended in dimethyl sulfoxide (200 ml), and to the suspension was added potassium carbonate (2.5 g), and the mixture was stirred at room temperature for 30 minutes. i-propyl bromoacetate (1.4 ml) was added, and the mixture was stirred at room temperature for 12 hours. The reaction mixture was poured into ice water (300 ml) and extracted with ethyl acetate. The ethyl acetate layer was washed with saturated aqueous solution of sodium chloride, and dried over anhydrous sodium sulfate, and the solvent was evaporated under reduced pressure. The residue was purified by silica gel flash column chromatography (eluent: methylene chloride containing 3% methanol), and the crude product was washed with ether and a small amount of methanol, and recovered by filtration to obtain the titled compound (2.6 g, 59%).

### Example 5: Synthesis of 10-bromo-2-ethoxycarbonylmethyloxy-5-(3-pyridylmethyl)-4H-pyrido[3,2,1-jk]carbazol-4-one

10-bromo-2-hydroxy-5-(3-pyridylmethyl)-4H-pyrido[3,2,1-jk]carbazol-4-one (13.9 g) obtained in Example 2 was suspended in dimethyl sulfoxide (500 ml), and to the suspension was added potassium carbonate (9.5 g), and the mixture was stirred at room temperature for 30 minutes. Ethyl bromoacetate (4.2 ml) was added, and the mixture was stirred at room temperature for 12 hours. The reaction mixture was poured into ice water (500 ml) and precipitated crystals were recovered by filtration. The thus obtained crude crystals were purified by silica gel flash column chromatography (eluent: methylene chloride containing 3% methanol), and the crude product was re-precipitated from chloroform-hexane and recovered by filtration to obtain the titled compound (9.0 g, 53%).

### Example 6: Synthesis of 10-bromo-2-carboxymethyloxy-5-(3-pyridylmethyl)-4H-pyrido[3,2,1-jk]carbazol-4-one

10-bromo-2-ethoxycarbonylmethyloxy-5-(3-pyridylmethyl)-4H-pyrido[3,2,1-jk]carbazol-4-one (2 g) obtained in Example 5 was suspended in ethanol (100 ml), and to the suspension was added 1N aqueous solution of sodium hydroxide (20 ml), and the mixture was stirred at room temperature for 12 hours. After evaporating the solvent under reduced pressure, 4N sodium hydroxide and methylene chloride were added to the residue for phase separation. To the aqueous layer was added 4N hydrochloric acid to a pH of 6, and the precipitated crystals were recovered by filtration to obtain the titled compound (1.8 g, 96%).

### Example 7: Synthesis of 10-bromo-2-n-propoxycarbonylmethyloxy-5-(3-pyridylmethyl)-4H-pyrido[3,2,1-jk]carbazol-4-one

The procedure of Example 3 was repeated by using 10-bromo-2-hydroxy-5-(3-pyridylmethyl)-4H-pyrido[3,2,1-jk]carbazol-4-one (250 mg) and n-propyl bromoacetate (0.1 ml) to obtain the titled compound (170 mg, 55%).

### Example 8: Synthesis of 10-bromo-2-n-pentyloxycarbonylmethyloxy-5-(3-pyridylmethyl)-4H-pyrido[3,2,1-jk]carbazol-4-one

10-bromo-2-carboxymethyloxy-5-(3-pyridylmethyl)-4H-pyrido[3,2,1-jk]carbazol-4-one (300 mg) obtained in Example 6 was suspended in anhydrous benzene (10 ml), and to the suspension was added thionyl chloride (0.95 ml) at room temperature. The mixture was heated under reflux under an argon atmosphere for 3 hours and allowed to cool. The solvent was evaporated under reduced pressure, and after adding anhydrous benzene (5 ml), the solvent was evaporated again. The resulting residue was dissolved in anhydrous methylene chloride (3 ml), and the solution was added dropwise to a solution of 1-pentanol (0.065 ml) and triethylamine (0.18 ml) dissolved in methylene chloride (30 ml) under cooling with ice, and the solution was stirred for 20 minutes. Water was added to the reaction mixture, and the mixture was extracted with methylene chloride. The methylene chloride layer was repeatedly washed with saturated aqueous solution of sodium chloride, and dried over anhydrous sodium sulfate. The residual crude crystals were washed with ether, and recovered by filtration to obtain the titled compound (200 mg, 58%).

### Example 9: Synthesis of 10-bromo-2-cyclohexyloxycarbonyl-methyloxy-5-(3-pyridylmethyl)-4H-pyrido[3,2,1-jk]carbazol-4-one

In accordance with Example 8, the titled compound (120 mg; 34%) was prepared from 10-bromo-2-carboxymethyloxy-5-(3-pyridylmethyl)-4H-pyrido[3,2,1- jk]carbazol-4-one (300 mg) and cyclohexanol (0.063 ml).

### Example 10: Synthesis of 10-bromo-5-(3-pyridylmethyl)-2-(3-pyridylmethyloxy)-4H-pyrido[3,2,1-jk]carbazol-4-one

10-bromo-2-hydroxy-5-(3-pyridylmethyl)-4H-pyrido[3,2,1-jk]carbazol-4-one (3.9 g) obtained in Example 2 was suspended in dimethyl sulfoxide (230 ml), and to the suspension was added potassium carbonate (4.0 g), and the mixture was stirred at room temperature for 30 minutes. 3-picolylchloride hydrochloride (1.9 g) was added and the mixture was stirred at room temperature for 12 hours. The reaction mixture was poured into ice water (500 ml), and the precipitated crystals were recovered by filtration. The resulting crude crystals were washed with methanol and recovered by filtration to obtain the titled compound (2.9 g, 61%).

### Example 11: Synthesis of 2-benzyloxy-10-bromo-5-(3-pyridylmethyl)-4H-pyrido[3,2,1-jk]carbazol-4-one

10-bromo-2-hydroxy-5-(3-pyridylmethyl)-4H-pyrido[3,2,1-jk]carbazol-4-one (2.0 g) obtained in Example 2 was suspended in a mixed solvent of anhydrous dimethylformamide (64 ml) and anhydrous tetrahydrofuran (120 ml), and sodium hydride (60%, 260 mg) was added to the suspension under cooling with ice. Benzylbromide (400 mg) was added dropwise and the mixture was stirred at room temperature for 12 hours. A small amount of methanol was added to the reaction mixture, and the mixture was extracted with ethyl acetate. The ethyl acetate layer was successively washed with 1N aqueous solution of sodium hydroxide and saturated aqueous solution of sodium chloride, dried over anhydrous sodium sulfate, and the solvent was evaporated under reduced pressure. The residue was purified by silica gel column chromatography (eluent: methylene chloride containing 3% methanol) to produce the titled compound (1.7 g, 67%).

### Example 12: Synthesis of 2-acetoxy-10-bromo-5-(3-pyridylmethyl)-4H-pyrido[3,2,1-jk]carbazol-4-one

10-bromo-2-hydroxy-5-(3-pyridylmethyl)-4H-pyrido[3,2,1-jk]carbazol-4-one (200 mg) obtained in Example 2 was suspended in pyridine (5 ml), and to the suspension was added acetic anhydride (0.14 ml), and the mixture was stirred at room temperature for 2 hours. A small amount of methanol was added dropwise to the reaction mixture, and the solvent was evaporated under reduced pressure. The residual crude crystals were successively washed with methanol and ether to produce the titled compound (100 mg; 46%).

### Example 13: Synthesis of 10-bromo-2-methoxy-5-methyl-4H-pyrido[3,2,1-jk]carbazol-4-one

### [Step 1] Synthesis of 3-bromo-6-methoxycarbazole-N-α-methyl-β-propionic acid

3-bromo-6-methoxycarbazole (20 g) produced in Example 1, Step 1 was dissolved in anhydrous tetrahydrofuran (200 ml), and to the solution were added methyl methacrylate (77.6 ml), then Triton B (0.7 ml). The mixture was heated under reflux under an argon atmosphere for 2 hours, and the solvent was evaporated under reduced pressure. The resulting residue was suspended in methanol (60 ml), and sodium hydroxide (6.4 g) dissolved in water (80 ml) was added dropwise at room temperature, and the mixture was stirred at room temperature for 12 hours. The solvent was evaporated under reduced pressure, and water and ether were added for phase separation. The aqueous layer was rendered acidic by addition of 4N hydrochloric acid, and ethyl acetate was added for further phase separation. The ethyl acetate layer was washed with water and saturated aqueous solution of sodium chloride, dried over anhydrous sodium sulfate, and the solvent was evaporated under reduced pressure. The residual crude crystals were washed with hexane and ether and recovered by filtration to obtain the titled compound (25.0 g, 95%).
melting point: 183.4-186.0 °C
IR spectrum (KBr tab.) ν cm⁻¹: 3420, 2950, 1697, 1491, 800
NMR spectrum (DMSO-d₆) δ ppm: 8.38(1H,s), 7-80(1H,s), 7.57-7.49(3H,m), 7.15-7.08(1H,m), 4.58(1H,m), 4.34(1H,m), 3.84(3H,s), 3.07-2.88(1H,m), 1.04(3H,d,J=6.4Hz)

### [Step 2] Synthesis of 10-bromo-5,6-dihydro-2-methoxy-5-methyl-4H-pyrido[3,2,1-jk]carbazol-4-one

The compound (30 g) obtained in Step 1 was suspended in toluene (1200 ml), and diphosphorus pentaoxide (20 g) was added to the suspension. The mixture was heated under reflux under an argon atmosphere for 12 hours (during which diphosphorus pentaoxide (20 g) was supplemented twice). After allowing to cool, the reaction mixture was poured into ice water (1000 ml), filtered through Celite to remove the floating material, and extracted with ethyl acetate. The ethyl acetate layer was washed with saturated aqueous solution of sodium chloride, dried over anhydrous sodium sulfate, and the solvent was evaporated under reduced pressure. The residue was purified by silica gel column chromatography (eluent: hexane/ethyl acetate = 5:1), and the crude purification product was recrystallized from ethanol and recovered by filtration to obtain the titled compound (16.0 g, 56%).
melting point: 163.8-166.6 °C
IR spectrum (KBr tab.) ν cm⁻¹: 1687, 1672, 1497, 1479, 1444, 1194, 797
NMR spectrum (DMSO-d₆) δ ppm: 8.47(1H,s), 8.12(1H,d,J=1.5Hz), 7.76-7.58(2H,m), 7.36(1H,d, J=1.5Hz), 4.77(1H,dd,J=11.8,6.5Hz), 4.06(1H,dd,J=11.8,11.8Hz), 3.87(3H,s),3.32-3.23(1H,m), 1.28(3H,d,J=6.5Hz)

### [Step 3] Synthesis of 10-bromo-2-methoxy-5-methyl-4H-pyrido[3,2,1-jk]carbazol-4-one

The compound (5.9 g) obtained in Step 2 was dissolved in anhydrous dioxane (400 ml), and DDQ (5.8 g) was added at room temperature. The mixture was heated under reflux under an argon atmosphere for 23 hours (during which DDQ (2 g) was supplemented twice). After allowing to cool, the reaction mixture was added to 1N aqueous solution of sodium hydroxide (500 ml), and extracted with ethyl acetate. The ethyl acetate layer was successively washed with 1N aqueous solution of sodium hydroxide and saturated aqueous solution of sodium chloride, dried over anhydrous sodium sulfate, and the solvent was evaporated under reduced pressure. The resulting crude crystals were washed with methanol (60 ml) at an elevated temperature and recovered by filtration to obtain the titled compound (4.8 g, 82%).

### Example 14: Synthesis of 10-bromo-2-hydroxy-5-methyl-4H-pyrido[3,2,1-jk]carbazol-4-one

10-bromo-2-methoxy-5-methyl-4H-pyrido[3,2,1-jk]carbazol-4-one (4.8 g) obtained in Example 13 was suspended in anhydrous methylene chloride (400 ml), and boron tribromide (25 g) was added dropwise to the suspension at room temperature. The mixture was stirred at room temperature for 12 hours and the reaction mixture was poured into ice water (1500 ml) and the crystals precipitated were recovered by filtration. The resulting crude crystals were washed with ether and recovered by filtration to obtain the titled compound (4.6 g, constant amount).

### Example 15: Synthesis of 10-bromo-2-t-butoxycarbonylmethyloxy-5-methyl-4H-pyrido[3,2,1-jk]carbazol-4-one

10-bromo-2-hydroxy-5-methyl-4H-pyrido[3,2,1-jk]carbazol-4-one (250 mg) obtained in Example 14 was suspended in dimethyl sulfoxide (10 ml), and potassium carbonate (210 mg) was added to the suspension. The mixture was stirred at room temperature for 30 minutes and t-butyl bromoacetate (0.13 ml) was added to the mixture. The mixture was stirred for 2.5 hours at room temperature, and the reaction mixture was poured into ice water (50 ml) and extracted with ethyl acetate. The ethyl acetate layer was washed with saturated aqueous solution of sodium chloride, and dried over anhydrous sodium sulfate, and the solvent was evaporated under reduced pressure. The residue was purified by silica gel column chromatography (eluent: methylene chloride containing 2% methanol), and the crude purification product was washed with ether and recovered by filtration to obtain the titled compound (130 mg, 39%).

### Example 16: Synthesis of 10-bromo-5-methyl-2-i-propoxycarbonylmethyloxy-5-(3-pyridylmethyl)-4H-pyrido[3,2,1-jk]carbazol-4-one

10-bromo-2-hydroxy-5-methyl-4H-pyrido[3,2,1-jk]carbazol-4-one (250 mg) obtained in Example 14 was suspended in dimethyl sulfoxide (10 ml), and potassium carbonate (210 mg) was added to the suspension. The mixture was stirred at room temperature for 30 minutes and i-propyl bromoacetate (0.12 ml) and potassium iodide (1 grain) were added successively, and the mixture was stirred at room temperature for 12 hours. The reaction mixture was poured into ice water (50 ml) and extracted with ethyl acetate. The ethyl acetate layer was washed with saturated aqueous solution of sodium chloride, and dried over anhydrous sodium sulfate, and the solvent was evaporated under reduced pressure. The residue was purified by silica gel flash column chromatography (eluent: ethyl acetate), and the crude purification product was washed with ether and recovered by filtration to obtain the titled compound (220 mg, 67%).

### Example 17: Synthesis of 10-bromo-2-ethoxycarbonylmethyloxy-5-methyl-4H-pyrido[3,2,1-jk]carbazol-4-one

10-bromo-2-hydroxy-5-methyl-4H-pyrido[3,2,1-jk]carbazol-4-one (400 mg) obtained in Example 14 was suspended in dimethyl sulfoxide (10 ml), and potassium carbonate (0.34 g) was added to the suspension. The mixture was stirred at room temperature for 30 minutes and ethyl bromoacetate (0.16 ml) was added. The mixture was stirred at room temperature for 12 hours, and the reaction mixture was poured into ice water (50 ml). The crystals precipitated were recovered by filtration. The resulting crude crystals were successively washed with water, ethanol and ether, and recovered by filtration to obtain the titled compound (360 mg, 71%).

### Example 18: Synthesis of 10-bromo-2-(2-oxopentyloxy)-5-methyl-4H-pyrido[3,2,1-jk]carbazol-4-one

10-bromo-2-hydroxy-5-methyl-4H-pyrido[3,2,1-jk]carbazol-4-one (250 mg) obtained in Example 14 was suspended in dimethyl sulfoxide (10 ml), and potassium carbonate (210 mg) was added to the suspension. The mixture was stirred at room temperature for 30 minutes and 1-bromo-2-pentanone (188 mg) was added to the mixture. The mixture was stirred at room temperature for 12 hours, and the reaction mixture was poured into ice water (500 ml), and extracted with ethyl acetate. The ethyl acetate layer was washed with saturated aqueous solution of sodium chloride, and dried over anhydrous sodium sulfate, and the solvent was evaporated under reduced pressure. The residue was purified by silica gel flash column chromatography (eluent: methylene chloride containing 2% methanol) to obtain the titled compound (150 mg, 48%).

### Example 19: Synthesis of 10-bromo-2-methoxy-4H-pyrido[3,2,1-jk]carbazol-4-one

10-bromo-5,6-dihydro-2-methoxy-4H-pyrido[3,2,1-jk]carbazol-4-one (3.6 g) obtained in Example 1, Step 3 was dissolved in anhydrous dioxane (300 ml), and DDQ (3.0 g) was added at room temperature. The mixture was heated under reflux under an argon atmosphere for 5 hours. After allowing to cool, the reaction mixture was added to 1N aqueous solution of sodium hydroxide (500 ml), and extracted with ethyl acetate. The ethyl acetate layer was successively washed with 1N aqueous solution of sodium hydroxide and saturated aqueous solution of sodium chloride, dried over anhydrous sodium sulfate, and the solvent was evaporated under reduced pressure. The residue was purified by silica gel column chromatography (eluent: methylene chloride containing 3% methanol) to obtain the titled compound (2.9 g, 79%).

### Example 20: Synthesis of 10-bromo-2-hydroxy-4H-pyrido[3,2,1-jk]carbazol-4-one

10-bromo-2-methoxy-4H-pyrido[3,2,1-jk]carbazol-4-one (3.2 g) obtained in Example 19 was suspended in anhydrous methylene chloride (500 ml), and boron tribromide (25 g) was added dropwise at room temperature. The mixture was stirred at room temperature for 12 hours and the reaction mixture was poured into ice water (1 L) and the crystals precipitated were recovered by filtration. The methylene chloride layer of the filtrate was washed with saturated aqueous solution of sodium chloride, dried over anhydrous sodium sulfate, and the solvent was evaporated under reduced pressure. The two types of crystals thus obtained were combined, and the above-described procedure was repeated. The reaction mixture was poured into ice water (1 L), and the crystals precipitated were recovered by filtration. The crude crystals obtained were washed with a mixed solution of chloroform and methanol at an elevated temperature and recovered by filtration to obtain the titled compound (2.4 g, 78%).

### Example 21: Synthesis of 10-bromo-2-t-butoxycarbonylmethyloxy-4H-pyrido[3,2,1-jk]carbazol-4-one

10-bromo-2-hydroxy-4H-pyrido[3,2,1-jk]carbazol-4-one (2.5 g) obtained in Example 20 was suspended in dimethyl sulfoxide (120 ml), and potassium carbonate (2.2 g) was added to the suspension. The mixture was stirred at room temperature for 30 minutes and t-butyl bromoacetate (1.4 ml) was added to the mixture. The mixture was stirred at room temperature for 12 hours, and the reaction mixture was poured into ice water (900 ml), and the crystals precipitated were recovered by filtration. The crude crystals were purified by silica gel flash column chromatography (eluent: methylene chloride containing 2% methanol) to obtain the titled compound (1.9 g, 68%).

### Example 22: Synthesis of 10-bromo-2-i-propoxycarbonylmethyloxy-4H-pyrido[3,2,1-jk]carbazol-4- one

10-bromo-2-hydroxy-4H-pyrido[3,2,1-jk]carbazol-4-one (3 g) obtained in Example 20 was suspended in dimethyl sulfoxide (120 ml), and potassium carbonate (2.6 g) was added to the suspension. The mixture was stirred at room temperature for 30 minutes and i-propyl bromoacetate (1.4 ml) and potassium iodide (1 grain) were successively added to the mixture. The mixture was stirred at room temperature for 12 hours, and the reaction mixture was poured into ice water (500 ml), and the crystals precipitated were recovered by filtration. The crude crystals were purified by silica gel flash column chromatography (eluent: methylene chloride containing 1% methanol) to obtain the titled compound (2.0 g, 51%).

### Example 23: Synthesis of 10-bromo-2- ethoxycarbonylmethyloxy-4H-pyrido[3,2,1-jk]carbazol-4-one

10-bromo-2-hydroxy-4H-pyrido[3,2,1-jk]carbazol-4-one (400 mg) obtained in Example 20 was suspended in dimethyl sulfoxide (10 ml), and potassium carbonate (0.34 g) was added to the suspension. The mixture was stirred at room temperature for 30 minutes and ethyl bromoacetate (0.15 ml) was added to the mixture. The mixture was stirred at room temperature for 12 hours, and the reaction mixture was poured into ice water (50 ml), and the crystals precipitated were recovered by filtration. The crude crystals obtained were successively washed with water, ethanol and ether, and recovered by filtration to obtain the titled compound (410 mg, 84%).

### Example 24: Synthesis of 10-bromo-2-carboxymethyloxy-4H-pyrido[3,2,1-jk]carbazol-4-one

10-bromo-2-ethoxycarbonylmethyloxy-4H-pyrido[3,2,1-jk]carbazol-4-one (200 mg) obtained in Example 23 was suspended in a mixed solution of ethanol (10 ml) and methylene chloride (10 ml), and 1N aqueous solution of sodium hydroxide (1 ml) was added to the suspension. The mixture was stirred at room temperature for 10 minutes, and the solvent was evaporated under reduced pressure. To the residue was added water and 1N hydrochloric acid to pH 1, and the crystals precipitated were recovered by filtration to obtain the titled compound (160 mg, 84%).

### Example 25: Synthesis of 10-bromo-2-(3-pyridylmethyloxy)-4H-pyrido[3,2,1-jk]carbazol-4-one

10-bromo-2-hydroxy-4H-pyrido[3,2,1-jk]carbazol-4-one (300 mg) obtained in Example 20 was suspended in dimethyl sulfoxide (20 ml), and potassium carbonate (400 mg) was added to the suspension. The mixture was stirred at room temperature for 30 minutes and 3-picolylchloride hydrochloride (180 mg) was added and the mixture was stirred at room temperature for 12 hours. The reaction mixture was poured into ice water (100 ml), and the precipitated crystals were recovered by filtration. The resulting crude crystals were successively washed with methanol and ether and recovered by filtration to obtain the titled compound (200 mg, 52%).

### Example 26: Synthesis of 2-benzyloxy-10-bromo-4H-pyrido[3,2,1-jk]carbazol-4-one

In accordance with Example 11, the titled compound (563 mg; 74%) was prepared from the 10-bromo-2-hydroxy-4H-pyrido[3,2,1-jk]carbazol-4-one (590 mg) that was obtained in Example 20.

### Example 27: Synthesis of 10-bromo-2-(1,2,4-oxadiazol-3-yl)methyloxy-4H-pyrido[3,2,1-jk]carbazol-4-one

In accordance with Example 10, the titled compound (37 mg; 12%) was prepared from the 10-bromo-2-hydroxy-4H-pyrido[3,2,1-jk]carbazol-4-one (250 mg) that was obtained in Example 20 and 3-(chloromethyl)-1,2,4-oxadiazole (113 mg).

### Example 28: Synthesis of 2-acetoxy-10-bromo-4H-pyrido[3,2,1-jk]carbazol-4-one

In accordance with Example 12, the titled compound (163 mg; 72%) was prepared from the 10-bromo-2-hydroxy-4H-pyrido[3,2,1-jk]carbazol-4-one (200 mg) that was obtained in Example 20.

### Example 29: Synthesis of 10-bromo-2-(2-oxopentyloxy)-4H-pyrido[3,2,1-jk]carbazol-4-one

10-bromo-2-hydroxy-4H-pyrido[3,2,1-jk]carbazol-4-one (250 mg) obtained in Example 20 was suspended in dimethyl sulfoxide (10 ml), and potassium carbonate (210 mg) was added to the suspension. The mixture was stirred at room temperature for 30 minutes and 1-bromo-2-pentanone (170 mg) was added to the mixture. The mixture was stirred at room temperature for 3 hours, and the reaction mixture was poured into ice water and extracted with ethyl acetate. The ethyl acetate layer was washed with saturated aqueous solution of sodium chloride, dried over anhydrous sodium sulfate, and the solvent was evaporated under reduced pressure. The residue was purified by silica gel flash column chromatography (eluent: methylene chloride containing 2% methanol) to obtain the titled compound (130 mg, 41%).

### Example 30: Synthesis of 9-bromo-2-methoxy-5-(3-pyridylmethyl)-4H-pyrido[3,2,1-jk]carbazol-4-one

### [Step 1] Synthesis of 4'-bromo-2'-nitrophenylbenzoquinone

4-bromo-2-nitroaniline (10 g) was suspended in a mixed solution of conc. hydrochloric acid (120 ml) and water (22 ml), and the suspension was heated on a hot water bath for dissolution of the content. After the complete dissolution, the solution was cooled to 10 °C, and stirred for 30 minutes. Sodium nitrite (5.3 g) dissolved in water (15 ml) was added dropwise to the solution maintained at a temperature not exceeding 10 °C and the insoluble content was separated by using glass wool filter. The filtrate was slowly added dropwise to the suspension of sodium hydrogencarbonate (56.8 g) and benzoquinone (5.6 g) in water (56.7 ml), and the crystals precipitated were recovered by filtration. The resulting crude crystals were washed with ethanol and recovered by filtration to obtain the titled compound (8.6 g, 60%).
melting point: 164.1-168.7 °C
IR spectrum (KBr tab.) ν cm⁻¹: 1664, 1651, 1603, 1524, 1354, 1101, 918
NMR spectrum (*DMSO-d₆) δ ppm: 8.40(1H,d,J=2.0Hz), 8.14(1H,dd,J=8.1,2.0Hz), 7.59(1H,d,J=8.1Hz), 7.13(1H,s),7.03(2H,s)

### [Step 2] Synthesis of 2'-amino-4'-bromophenylhydroquinone

The compound (8.5 g) obtained in Step 1 was suspended in 3N hydrochloric acid (213 ml), and tin chloride dihydrate (25 g) was added to the suspension. The mixture was heated to 90 °C on a hot water bath, stirred for 2 hours, allowed to cool, and poured into water (300 ml). The mixture was adjusted to pH 7 by addition of 3N aqueous solution of sodium hydroxide and extracted with ethyl acetate. The ethyl acetate layer was washed with saturated aqueous solution of sodium chloride, and dried over anhydrous sodium sulfate, and the solvent was evaporated under reduced pressure. The resulting crude purification product was washed with ether and recovered by filtration to obtain the titled compound (4.8 g, 61%).
melting point: 203.4-206.5 °C
IR spectrum (KBr tab.) ν cm⁻¹: 3388, 1616, 1506, 1479, 1406, 1244, 1211, 779
NMR spectrum (*DMSO-d₆) δ ppm: 8.87(1H,s), 8.80(1H,s), 6.90(1H,d,J=2.0Hz), 6.84(1H,d,J=8.5Hz), 6.74(1H,d,J=2.0Hz), 6.71(1H,d,J=1.4Hz), 6.58(1H,dd,J=8.5,3.0Hz), 6.46(1H,d,J=3.0Hz), 4.87(2H,s)

### [Step 3] Synthesis of 2-bromo-6-hydroxycarbazole

The compound (14 g) obtained in Step 2 was dissolved in methanol, and silica gel (90 g) was added to the solution. The solvent was evaporated under reduced pressure, and the residue was stirred at 90 °C on a hot water bath for 10 hours, and extracted with methanol. The silica gel used for the reaction was separated by filtration, and the solvent was evaporated from the filtrate under reduced pressure. The residue was purified by silica gel column chromatography (eluent: hexane/ethyl acetate = 1:1) to obtain the titled compound (5.0 g, 38%).
melting point: 248.4-252.2 °C
IR spectrum (KBr tab.) ν cm⁻¹: 3402, 1608, 1583, 1458, 1178, 812, 609
NMR spectrum (*DMSO-d₆) δ ppm: 11.06(1H,s), 9.02(1H,s), 7.95(1H,d,J=8.2Hz), 7.58(1H,d,J=1.7Hz), 7.42(1H,d,J=2.4Hz), 7.31(1H,d,J=8.7Hz), 7.20(1H,dd,J=8.2,1.7Hz), 6.92(1H,dd,J=8.7, 2.4Hz)

### [Step 4] synthesis of 2-bromo-6-methoxycarbazole

The compound (6 g) obtained in Step 3 was dissolved in acetone (180 ml), and potassium hydroxide (1.3 g) was added to the solution at room temperature. Dimethyl sulfate (2.2 ml) was then added dropwise to the solution. After stirring at room temperature for 2 hours, the solvent was evaporated under reduced pressure and the residue was extracted with water and ethyl acetate. The ethyl acetate layer was washed with saturated aqueous solution of sodium chloride, and dried over anhydrous sodium sulfate, and the solvent was evaporated under reduced pressure. The residue was purified by silica gel flash column chromatography (eluent: hexane/ethyl acetate = 4:1) to obtain the titled compound (3.2 g, 51%).
melting point: 138.6-142.6 °C
IR spectrum (KBr tab.) ν cm⁻¹: 3336, 1495, 1219, 1201, 804
NMR spectrum (*DMSO-d₆) δ ppm: 11.19(1H,s), 8.04(1H,d,J=8.3Hz), 7.69(1H,d,J=2.5Hz), 7.61(1H,d, J=1.7Hz), 7.40(1H,d,J=8.7Hz), 7.23(1H,dd, J=8.3,1.7Hz), 7.04(1H,dd,J=8.7,2.5Hz), 3.83(3H,s)

### [Step 5] Synthesis of 2-bromo-6-methoxycarbazole-N-β-propionic acid

The compound (2.8 g) obtained in Step 4 was suspended in acetone (50 ml), and to the suspension were added dropwise methyl acrylate (1.8 ml), then Triton B (0.6 ml) at room temperature. After stirring for 40 minutes, the solvent was evaporated under reduced pressure. The resulting residue was dissolved in methanol (50 ml), and to the solution was added dropwise sodium hydroxide (0.9 g) dissolved in water (10 ml) at room temperature, and the mixture was stirred at room temperature for 70 minutes. The solvent was evaporated under reduced pressure, and the residue was adjusted to pH 3 by adding 1N hydrochloric acid and extracted with ethyl acetate. The ethyl acetate layer was washed with saturated aqueous solution of sodium chloride, and dried over anhydrous sodium sulfate, and the solvent was evaporated under reduced pressure. The residue was washed with hexane and recovered by filtration to obtain the titled compound (3.2 g, 91%).
melting point: 169.0-171.9 °C
IR spectrum (KBr tab.) ν cm⁻¹: 1693, 1489, 1483, 1290, 1225, 1209, 874
NMR spectrum (*DMSO-d₆) δ ppm: 12.39(1H,s), 8.08(1H,d,J=8.2Hz), 7.86(1H,d,J=1.6Hz), 7.74(1H,d,J=2.6Hz), 7.55(1H,d,J=8.9Hz), 7.29(1H,dd,J=8.2,1.6Hz), 7.12(1H,dd,J=8.9,2.6Hz), 4.58(2H,t,J=6.8Hz), 3.85(3H,s),2.71(2H,t, J=6.8Hz)

### [Step 6] Synthesis of 9-bromo-5,6-dihydro-2-methoxy-4H-pyrido[3,2,1-jk]carbazol-4-one

The compound (2.9 g) obtained in Step 5 was suspended in anhydrous chloroform (100 ml), and to the suspension was added PPE (21.6 g) dissolved in anhydrous chloroform (100 ml) at room temperature. The mixture was heated under reflux under an argon atmosphere for 1 hour, allowed to cool, poured into water (300 ml), and extracted with chloroform. The chloroform layer was washed with saturated aqueous solution of sodium chloride, and dried over anhydrous sodium sulfate, and the solvent was evaporated under reduced pressure. The residue was purified by silica gel column chromatography (eluent: hexane/ethyl acetate = 9:1) to obtain the titled compound (1.7 g, 63%).
melting point: 174.9-178.8 °C
IR spectrum (KBr tab.) ν cm⁻¹: 1666, 1479, 1298, 1223, 1201, 1032, 797
NMR spectrum (*DMSO-d₆) δ ppm: 8.17(1H,d,J=8.4Hz), 8.10(1H,d,J=2.4Hz), 7.95(1H,d,J=1.8Hz), 7.40(1H,dd,J=8.4,1.8Hz), 7.36(1H,d, J=2.4Hz), 4.56(2H,t,J=7.1Hz), 3.88(3H,s), 3.12(2H,t,J=7.1Hz)

### [Step 7] Synthesis of 9-bromo-2-methoxy-5-(3-pyridylmethyl)-4H-pyrido[3,2,1-jk]carbazol-4-one

The compound (3.7 g) obtained in Step 6 was suspended in ethanol (210 ml), and to the suspension were added pyridine-3-aldehyde (1.7 ml) and sodium hydroxide (3.6 g) dissolved in water (20 ml) at room temperature. The mixture was stirred at room temperature for 12 hours, and approximately half of the solvent was evaporated under reduced pressure. The crystals precipitated were recovered by filtration and successively washed with water, ethanol and ether to obtain the titled compound (4.2 g, 90%).

### Example 31: Synthesis of 9-bromo-2-hydroxy-5-(3-pyridylmethyl)-4H-pyrido[3,2,1-jk]carbazol-4-one

9-bromo-2-methoxy-5-(3-pyridylmethyl)-4H-pyrido[3,2,1-jk]carbazol-4-one (700 mg) obtained in Example 30 was suspended in anhydrous methylene chloride (70 ml), and to the suspension was added dropwise a solution of boron tribromide in methylene chloride (1M; 10 ml) at room temperature. The reaction mixture was stirred at room temperature for 12 hours, and poured into ice water (100 ml). To this mixture was added saturated aqueous solution of sodium carbonate until the termination of foaming. The crystals precipitated were recovered by filtration. The thus obtained crude crystals were successively washed with ethanol and ether to obtain the titled compound (450 mg, 67%).

### Example 32: Synthesis of 9-bromo-2-t-butoxycarbonylmethyloxy-5-(3-pyridylmethyl)-4H-pyrido[3,2,1-jk]carbazol-4-one

9-bromo-2-hydroxy-5-(3-pyridylmethyl)-4H-pyrido[3,2,1-jk]carbazol-4-one (200 mg) produced in Example 31 was suspended in dimethyl sulfoxide (8 ml). After adding potassium carbonate (136 mg), the mixture was stirred at room temperature for 30 minutes, and t-butyl bromoacetate (0.09 ml) was added. After stirring at room temperature for 2 hours, the reaction mixture was poured into ice water (20 ml) and the crystals precipitated were recovered by filtration. The thus obtained crude crystals were successively washed with water, ethanol and ether and recovered by filtration to obtain the titled compound (144 mg, 56%).

### Example 33: Synthesis of 9-bromo-2-carboxymethyloxy-5-(3-pyridylmethyl)-4H-pyrido[3,2,1-jk]carbazol-4-one

9-bromo-2-t-butoxycarbonylmethyloxy-5-(3-pyridylmethyl)-4H-pyrido[3,2,1-jk]carbazol-4-one (57 mg) produced in Example 32 was dissolved in acetic acid (0.5 ml) and 48% HBR (0.5 ml), and the solution was stirred at 60°C for 1 hour and allowed to cool. Saturated aqueous solution of sodium hydrogencarbonate was added to pH 7, and the crystals precipitated were recovered by filtration, successively washed with water, ethanol and ether, and recovered by filtration to obtain the titled compound (40 mg, 79%).

### Example 34: Synthesis of 9-bromo-5-(3-pyridylmethyl)-2-(3-pyridylmethyloxy)-4H-pyrido[3,2,1-jk]carbazol-4-one

9-bromo-2-hydroxy-5-(3-pyridylmethyl)-4H-pyrido[3,2,1-jk]carbazol-4-one (200 mg) produced in Example 31 was suspended in dimethyl sulfoxide (8 ml). After adding potassium carbonate (204 mg), the mixture was stirred at room temperature for 30 minutes, and 3-picolylchloride (0.09 ml) was added. After stirring at room temperature for 12 hours, the reaction mixture was poured into ice water (20 ml) and the crystals precipitated were recovered by filtration. The thus obtained crude crystals were successively washed with water, ethanol and ether and recovered by filtration to obtain the titled compound (177 mg, 72%).

### Example 35: Synthesis of 2-(5-acetoxymethyl-3-pyridylmethyloxy)-9-bromo-5-(3-pyridylmethyl)-4H-pyrido[3,2,1-jk]carbazol-4-one

### [Step 1] Synthesis of pyridine-3,5-dicarboxylic acid dimethyl ester

Pyridine-3,5-dicarboxylic acid (8.3 g) was suspended in dry methanol (60 ml); thionyl chloride (11 ml) was added dropwise at room temperature and the mixture was heated under reflux for 1.5 h in an argon atmosphere. After cooling, the solvent was distilled off under reduced pressure and extraction was conducted with water-ethyl acetate. The ethyl acetate layer was washed with saturated sodium hydrogencarbonate and saturated saline and dried with anhydrous sodium sulfate; thereafter, the solvent was distilled off under reduced pressure to yield the titled compound (7.5 g; 78%).
Melting point: 83.5 - 84.5 °C
IR spectrum (KBr tab.) ν cm⁻¹: 1734, 1603, 1315, 1269, 1240, 995, 746
NMR spectrum (*DMSO-d₆) δ ppm: 9.30(2H, s), 8.66(1H, s), 3.93(6H, s).

### [Step 2] Synthesis of pyridine-3,5-dimethanol monoacetate

The compound (11.9 g) obtained in Step 1 was dissolved in dry ether (300 ml) and the solution was cooled to 0 °C on an ice bath; thereafter, lithium aluminum hydride (6 g) was added in small amounts and the temperature of the solution was elevated slowly to room temperature, at which it was stirred for 12 h. The solution was again cooled on an ice bath and methanol (400 ml) was added, with the solvent being then distilled off under reduced pressure. The residue was purified by silica gel column chromatography (eluting solvent: 3% methanol containing chloroform) and the crude product was crystallized with mixed solvent of hexane and ether to give a crude crystal. The crystal obtained (3.4 g) was suspended in pyridine (10 ml) and acetyl chloride (1.8 ml) was added dropwise at room temperature. Thereafter, the solvent was distilled off under reduced pressure and the residue was purified by silica gel column chromatography (eluting solvent: 3% methanol containing chloroform); the crude product was washed with methanol and recovered by filtation to yield the titled compound (1 g; 23%).
Melting point: 152.2 - 130.9 °C
IR spectrum (KBr tab.) ν cm⁻¹: 3305, 2740, 2700, 1747, 1566, 1230, 1072
NMR spectrum (*DMSO-d₆) δ ppm: 8.79(1H, s), 8.74(1H, s), 8.37(1H, s), 5.25(2H, s), 4.68(2H, s), and 2.11(3H,s)

### [Step 3] Synthesis of 3-acetoxymethyl-5-chloromethylpyridine

The compound (500 mg) obtained in Step 2 was suspended in dry benzene (8 ml). Thionyl chloride (0.2 ml) was added dropwise at room temperature and the mixture was stirred for 15 min at room temperature. The solvent was distilled off under reduced pressure to yield the titled compound (540 mg; 83%).
IR spectrum (neat) ν cm⁻¹: 3396, 3367, 1716, 1633, 1562, 1385, 1227, 1057
NMR spectrum (*DMSO-d₆) δ ppm: 8.76 (1H, d, J = 1.8 Hz), 8.70 (1H, d, J = 1.8 Hz), 8.16 (1H, s), 5.20 (2H, s), 4.90 (2H, s), 2.10 (3H, s)

### [Step 4] Synthesis of 2-(5-acetoxymethyl-3-pyridylmethyloxy)-10-bromo-5-(3-pyridylmethyl)-4H-pyrido[3,2,1-jk]carbazol-4-one

In accordance with Example 34, the titled compound (145 mg; 69%) was prepared from 9-bromo-2-hydroxy-5-(3-pyridylmethyl)-4H-pyrido[3,2,1-jk]carbazol-4-one (145 mg) and the compound (105 mg) obtained in Step 3.

### Example 36: Synthesis of 9-bromo-2-(5-hydroxymethyl-3-pyridylmethyloxy)-5-(3-pyridylmethyl)-4H-pyrido[3,2,1-jk]carbazol-4-one

2-(5-acetoxymethyl-3-pyridylmethyloxy)-9-bromo-5-(3-pyridylmethyl)-4H-pyrido[3,2,1-jk]carbazol-4-one (200 mg) produced in Example 35 was suspended in methanol (10 ml), and to the suspension was added a solution of sodium hydroxide (85 mg) in water (0.8 ml). The mixture was stirred at room temperature for 10 minutes, and the crystals precipitated were recovered by filtration and successively washed with methanol and ether to obtain the titled compound (170 mg, 92%).

### Example 37: Synthesis of 9-bromo-2-(N-ethylcarbamoylmethyloxy)-5-(3-pyridylmethyl)-4H-pyrido[3,2,1-jk]carbazol-4-one

The procedure of Example 34 was repeated by using 9-bromo-2-hydroxy-5-(3-pyridylmethyl)-4H-pyrido[3,2,1-jk]carbazol-4-one (300 mg) and N-ethyl-2-chloroacetamide (153 mg) to obtain the titled compound (230 mg, 63%).

### Example 38: Synthesis of 9-bromo-2-(3-hydroxypropyloxy)-5-(3-pyridylmethyl)-4H-pyrido[3,2,1-jk]carbazol-4-one

9-bromo-2-hydroxy-5-(3-pyridylmethyl)-4H-pyrido[3,2,1-jk]carbazol-4-one (400 mg) produced in Example 31 was suspended in dimethyl sulfoxide (40 ml). After adding potassium carbonate (540 mg), the mixture was stirred at room temperature for 30 minutes, and 3-bromo-1-propanol (0.3 ml) was added. After stirring at room temperature for 12 hours, the reaction mixture was poured into ice water (100 ml) and the crystals precipitated were recovered by filtration. The thus obtained crude crystals were successively washed with water, ethanol and ether and recovered by filtration to obtain the titled compound (177 mg, 72%).

### Example 39: Synthesis of 5-benzyl-9-bromo-2-methoxy-4H-pyrido[3,2,1-jk]carbazol-4-one

9-bromo-5,6-dihydro-2-methoxy-4H-pyrido[3,2,1-jk]carbazol-4-one (200 mg) obtained in Example 30, Step 6 was suspended in ethanol (12 ml), and benzaldehyde (103 mg) and sodium hydroxide (190 mg) dissolved in water (1 ml) were added to the suspension. The mixture was stirred at room temperature for 12 hours, and approximately half of the solvent was evaporated under reduced pressure. The crystals precipitated were recovered by filtration and successively washed with water, ethanol and ether to obtain the titled compound (217 mg, 87%).

### Example 40: Synthesis of 5-benzyl-9-bromo-2-hydroxy-4H-pyrido[3,2,1-jk]carbazol-4-one

5-benzyl-9-bromo-2-methoxy-4H-pyrido[3,2,1-jk]carbazol-4-one (137 mg) produced in Example 39 was dissolved in acetic acid (7 ml) and 48% HBr (7 ml), and the solution was stirred at room temperature for 30 hours. The reaction mixture was poured into water and the crystals precipitated were recovered by filtration and successively washed with water, ethanol and ether to obtain the titled compound (77 mg, 58%).

### Example 41: Synthesis of 2-hydroxy-5-(3-pyridylmethyl)-4H-pyrido[3,2,1-jk]carbazol-4-one

### [Step 1] Synthesis of 5,6-dihydro-2-methoxy-4H-pyrido[3,2,1-jk]carbazol-4-one

In accordance with the procedures of Steps 1, 2 and 3 in Example 1, the titled compound was obtained from 3-bromocarbazole that was prepared by the method described in a reference (*Kogyo Kagaku Zasshi*, vol. 70, 63 (1967)).
Melting point: 127.7 - 129.6 °C
IR spectrum (KBr tab.) ν cm⁻¹: 1653, 1479, 1460, 1392, 1140, 746
NMR spectrum (DMSO-d₆) δ ppm: 8.22(1H,d,J=7.3Hz), 8.07(1H,d,J=2.4Hz), 7.64(1H,d,J=8.3Hz), 7.52(1H,dd,J=8.3,7.3Hz), 7.34(1H,d,J=2.4Hz), 7.28-7.19(1H,m), 4.54(2H,t,J=7.0Hz), 3.89(3H,s), 3.13(2H,t,J=7.0Hz)

### [Step 2] Synthesis of 2-methoxy-5-(3-pyridylmethyl)-4H-pyrido[3,2,1-jk]carbazol-4-one

In accordance with Step 4 in Example 1, the titled compound (64 g; 61%) was prepared from the compound (2 g) obtained in Step 1.
Melting point: 192.9 - 193.7 °C
IR spectrum (KBr tab.) ν cm⁻¹: 1593, 1508, 1464, 1228, 752
NMR spectrum (DMSO-d₆) δ ppm: 9.14(1H,s), 8.64(1H,d,J=1.6Hz), 8.38(1H,dd,J=4.8,1.6Hz), 8.26(1H,d,J=7.6Hz), 8.18(1H,d,J=2.2Hz), 8.09(1H,d,J=7.6Hz), 7.77(1H,d,J=7.8Hz), 7.65(1H,t,J=7.6Hz), 7.54(1H,d,J=2.2Hz), 7.46(1H,t,J=7.6Hz), 7.28(1H,dd,J=7.8,4.8Hz), 3.95(3H,s), 3.91(2H,s)

### [Step 3] Synthesis of 2-hydroxy-5-(3-pyridylmethyl)-4H-pyrido[3,2,1-jk]carbazol-4-one

In accordance with Example 2, the titled compound (2.8 g; 62%) was prepared from the compound (5 g) obtained in Step 2.

### Example 42: Synthesis of 2-acetoxy-5-(3-pyridylmethyl)-4H-pyrido[3,2,1-jk]carbazol-4-one

In accordance with Example 12, the titled compound (510 mg; 89%) was prepared from the 2-hydroxy-5-(3-pyridylmethyl)-4H-pyrido[3,2,1-jk]carbazol-4-one (510 mg) that was obtained in Example 41.

### Example 43: Synthesis of 2-chloro-4H-pyrido[3,2,1-jk]carbazol-4-one

### [Step 1] Synthesis of 6-chloro-1,2,3,4-tetrahydrocarbazole

4-chlorophenylhydrazine hydrochloride (25 g) was suspended in acetic acid (120 ml), and cyclohexanone (14-5 ml) was added. The mixture was heated under reflux for 2 hours and cooled to 0 °C. The precipitated crystals were recovered by filtration, and washed with water and ethanol. The crude product was recrystallized from methanol to obtain the titled compound (12.4 g, 43%).
melting point: 146.3-146.4 °C
IR spectrum (KBr tab.) ν cm⁻¹: 3406,2939,1470,1439, 1057,800,592
NMR spectrum (DMSO-d₆) δ ppm: 10.84(1H,s), 7.33(1H,d,J=2.1Hz), 7.21(1H,d,J=8.5Hz), 6.96(1H,dd,J=8.5,2.1Hz), 2.73-2.57(4H,m), 1.84-1.76(4H,m)

### [Step 2] Synthesis of 6-chloro-1,2,3,4-tetrahydrocarbazole-N-β-propionic acid

The compound (10 g) produced in Step 1 was suspended in acetone (50 ml), and the suspension was cooled in an ice bath, and to the suspension were added methyl acrylate (8.8 ml) and then Triton B (2 ml). After stirring for 1 hour, the solvent was evaporated under reduced pressure. The thus obtained residue was suspended in methanol (20 ml), and sodium hydroxide (4.3 g) dissolved in water (50 ml) was added dropwise to the suspension at room temperature. The mixture was heated under reflux for 20 minutes. The solvent was evaporated under reduced pressure, and water and ether were added for phase separation. The aqueous layer was rendered acidic by addition of 4N hydrochloric acid, and the precipitate formed was dissolved in ethyl acetate. The solution was successively washed with water and saturated aqueous solution of sodium chloride, dried over anhydrous sodium sulfate, and the solvent was evaporated under reduced pressure. The residual crude crystals were washed with hexane and ether to obtain the titled compound (10.1 g, 75%).
melting point: 158.1-159.1 °C
IR spectrum (KBr tab.) ν cm⁻¹: 2935, 1711, 1471, 1446, 1290, 957, 797
NMR spectrum (DMSO-d₆) δ ppm: 12.4(1H,s),7.43-7.37(2H,m), 7.03(1H,dd,J=8.9,2.0Hz), 4.27(2H,t, J=5.4Hz), 2.73(2H,t,J=5.4Hz), 2.64-2.56(4H,m), 1.86-1.75(4H,m)

### [Step 3] Synthesis of 2-chloro-8,9,10,11-tetrahydro-4H-pyrido[3,2,1-jk]carbazol-4-one

The compound (10 g) obtained in Step 2 was suspended in anhydrous toluene (200 ml), and diphosphorus pentaoxide (51 g) was added to the suspension. The mixture was heated under reflux under an argon atmosphere for 3 hours. After allowing to cool, the reaction mixture was added to water, and the insoluble content was removed by filtration through Celite. The filtrate was extracted with ethyl acetate. The ethyl acetate layer was washed with saturated aqueous solution of sodium chloride, dried over anhydrous sodium sulfate, and the solvent was evaporated under reduced pressure. The residue was purified by silica gel column chromatography (eluent: ethyl acetate) to obtain the titled compound (1.47 g, 16%).
melting point: 255.7-258.2 °C
IR spectrum (KBr tab.) ν cm⁻¹: 2929, 1614, 1595, 1554, 1489, 1277, 1207, 824
NMR spectrum (DMSO-d₆) δ ppm: 8.33(1H,d,J=7.8Hz), 7.93(1H,d,J=1.7Hz), 7.75(1H,d,J=1.7Hz), 6.21(1H,d,J=7.8Hz), 2.93-2.80(2H,m), 2.72-2.61(2H,m), 1.93-1.80(4H,m)

### [Step 4] Synthesis of 2-chloro-4H-pyrido[3,2,1-jk]carbazol-4-one

The compound (0.8 g) obtained in Step 3 was dissolved in anhydrous dioxane (20 ml), and DDQ (1.48 g) was added at room temperature. The mixture was heated under reflux under an argon atmosphere for 6 hours. After allowing to cool, the reaction mixture was added to 1N sodium hydroxide and extracted with ethyl acetate. The ethyl acetate layer was washed with saturated aqueous solution of sodium chloride, dried over anhydrous sodium sulfate, and the solvent was evaporated under reduced pressure. The residue was purified by silica gel column chromatography (eluent: methylene chloride/ethyl acetate = 2:1) to obtain the titled compound (320 mg, 41%).

### Example 44: Synthesis of 10-chloro-4H-pyrido[3,2,1-jk]carbazol-4-one

### [Step 1] Synthesis of 10-chloro-5,6-dihydro-4H-pyrido[3,2,1-jk]carbazol-4-one

The procedure of Example 1, Steps 2 and 3 was repeated, and the titled compound was obtained from 3-chlorocarbazole prepared by the procedure described in Rec. Trav Chim, 73, 197 (1954).
melting point: 144.3-147.9 °C
IR spectrum (KBr tab.) ν cm⁻¹: 1682, 1489, 1346, 1333, 1219, 798
NMR spectrum (DMSO-d₆) δ ppm: 8.52-8.38(2H,m), 7.81(1H,dd,J=7.8,1.0Hz), 7.73(1H,d,J=8.8Hz), 7.57(1H,dd,J=8.8,2.4Hz), 7.33(1H,t,J=7.1Hz), 4.61(2H,t,J=7.1Hz), 3.14(2H,t, J=7.1Hz)

### [Step 2] Synthesis of 10-chloro-4H-pyrido[3,2,1-jk]carbazol-4-one

The procedure of Example 19 was repeated, and the titled compound (1 g, 34.0%) was obtained from the compound (3 g) produced in Step 1.

### Example 45: Synthesis of 9-acetoxy-5-(3-pyridylmethyl)-4H-pyrido[3,2,1-jk]carbazol-4-one

### [Step 1] Synthesis of 5,6-dihydro-9-methoxy-4H-pyrido[3,2,1-jk]carbazol-4-one

In accordance with the procedures of Step 4 in Example 30 and Steps 2 and 3 in Example 1, the titled compound was prepared from a commercially available 2-hydroxycarbazole.
Melting point: 115.4 - 117.5 °C
IR spectrum (KBr tab.) ν cm⁻¹: 1680, 1630, 1475, 1356, 1223, 1082, 743
NMR spectrum (DMSO-d₆) δ ppm: 8.27(1H,d,J=7.6Hz), 8.09(1H,d,J=8.7Hz), 7.68(1H,d,J=7.6Hz), 7.28-7.22(2H, m), 6.89(1H,dd,J=8.7,2.1Hz), 4.56(2H,t,J=7.0Hz), 3.90(3H, s), 3.12(2H,t,J=7.0Hz)

### [Step 2] Synthesis of 9-methoxy-5-(3-pyridylmethyl)-4H-pyrido[3,2,1-jk]carbazol-4-one

In accordance with the procedure of Step 4 in Example 1, the titled compound (1.52 g; 74%) was prepared from the compound (1.52 g) obtained in Step 1.
Melting point: 207.2 °C (decomposition)
IR spectrum (KBr tab.): ν cm⁻¹: 1624, 1612, 1514, 1470, 1244, 756
NMR spectrum (DMSO-d₆) δ ppm: 9.24(1H, s), 8.65(1H,d,J=1.5 Hz), 8.40-8.37(2H, m), 8.17(1H,d,J=8.5Hz), 8.01(1H,d,J=7.6Hz), 7.81(1H,d,J=2.3Hz), 7.80-7.75(1H, m), 7.65(1H,t),J=7.6 Hz), 7.31-7.27(1H, m), 7.07(1H,dd,J=8.5,2.3Hz), 3.95(3H, s), 3.91(2H, s)

### [Step 3] Synthesis of 9-hydroxy-5-(3-pyridylmethyl)-4H-pyrido[3,2,1-jk]carbazol-4-one

In accordance with Example 2, the titled compound (0.66 g; 49%) was prepared from the compound (1.4 g) obtained in Step 2.
Melting point: 335.0 °C (decomposition)
IR spectrum (KBr tab.) ν cm⁻¹: 3050, 1614, 1498, 1448, 1279, 1232, 827
NMR spectrum (DMSO-d₆) δ ppm: 10.25(1H, bs), 9.11(1H, s), 8.64(1H,d,J=2.0Hz), 8.41-8.28(2H, m), 8.05(1H,d, J=8.4Hz), 7.97(1H,dd,J=7.8,1.0Hz), 7.82-7.74(1H, m), 7.62(1H,t,J=7.8Hz), 7.47(1H,d,J=2.1Hz), 7.32-7.25(1H, m), 6.92(1H,dd,J=8.4,2.1Hz), 3.90(2H, s)

### [Step 4] Synthesis of 9-acetoxy-5-(3-pyridylmethyl)-4H-pyrido[3,2,1-jk]carbazol-4-one

In accordance with Example 12, the titled compound (151 mg; 67%) was prepared from the compound (200 mg) obtained in Step 3.

### Example 46: Synthesis of 10-fluoro-2-methoxy-5-(3-pyridylmethyl)-4H-pyrido[3,2,1-jk]carbazole-4-one

### [Step 1] Synthesis of 6-fluoro-1,2,3,4-tetrahydrocarbazole

The procedure of Example 43, Step 1 was repeated, and the titled compound (126 g, 83%) was obtained from commercially available 4-fluorophenylhydrazine (130 g).
melting point: 107.6 °C (dec)
IR spectrum (KBr tab.) ν cm⁻¹: 3408, 2931, 1583, 1483, 1446, 795
NMR spectrum (CDCl₃) δ ppm: 7.66(1H,bs), 7.17(1H,dd,J=8.9,4.3Hz), 7.09(1H,dd,J=9.6,2.6Hz), 6.88-6.80(1H,m), 2.74-2.64(4H,m), 1.96-1.82(4H,m)

### [Step 2] Synthesis of 3-fluorocarbazole

The compound (1 g) produced in Step 1 was dissolved in xylene (6 ml), and chloranil (1.3 g) was added. The mixture was heated under reflux in an argon atmosphere for 3 hours and allowed to cool. The reaction mixture was decanted, and the insoluble content was removed by filtration, and the filtrate was concentrated under reduced pressure. The residue was purified by silica gel flash column chromatography (eluent: hexane/ethyl acetate = 15:1) to obtain the titled compound (232 mg, 24%).
melting point: 203.2 °C (dec)
IR spectrum (KBr tab.) ν cm⁻¹: 3419, 1585, 1497, 1169, 746
NMR spectrum (DMSO-d₆) δ ppm: 11.29(1H,bs), 8.13(1H,d,J=7.8Hz), 7.95(1H,dd,J=9.5,2.7Hz), 7.53-7.37(3H,m), 7.30-7.11(2H,m)

### [Step 3] Synthesis of 3-bromo-6-fluorocarbazole

The compound (13.5 g) produced in Step 2 was dissolved in dimethylformamide (200 ml), and N-bromosuccinimide (14.2 g) dissolved in dimethylformamide (136 ml) was added dropwise to the solution in an ice bath. The mixture was stirred for 15 minutes, and the reaction mixture was poured into ice water, and extracted with ethyl acetate. The ethyl acetate layer was washed with saturated aqueous solution of sodium chloride, and dried over anhydrous sodium sulfate, and the solvent was evaporated under reduced pressure. The residue was purified by silica gel column chromatography (eluent: hexane/ethyl acetate = 8:1) to obtain the titled compound (16.8 g, 87%).
melting point: 158.0 °C (dec)
IR spectrum (KBr tab.) ν cm⁻¹: 3410, 1489, 1443, 1161, 810, 571
NMR spectrum (DMSO-d₆) δ ppm: 11.48(1H,s), 8.40(1H,d,J=2.0Hz), 8.03(1H,dd,J=9.5,2.7Hz), 7.55-7.45(3H,m), 7.32-7.24(1H,m)

### [Step 4] Synthesis of 5,6-dihydro-10-fluoro-2-methoxy-4H-pyrido[3,2,1-jk]carbazol-4-one

The procedure of Example 1, Steps 1, 2 and 3 was repeated to obtain the titled compound from the compound produced in Step 3.
melting point: 166.8-169.4 °C
IR spectrum (KBr tab.) ν cm⁻¹: 1672, 1483, 1290, 1190, 1124, 854, 783
NMR spectrum (DMSO-d₆) δ ppm: 8.09(1H,d,J=2.4Hz), 8.07(1H,d,J=2.4Hz), 7.66(1H,dd,J=8.8,4.4Hz), 7.42-7.35(2H,m), 4.54(2H,t,J=7.1Hz), 3.88(3H,s), 3.13(2H,t,J=7.1Hz)

### [Step 5] Synthesis of 10-fluoro-2-methoxy-5-(3-pyridylmethyl)-4H-pyrido[3,2,1-jk]carbazol-4-one

The procedure of Example 1, Step 4 was repeated to obtain the titled compound (2.28 g, 86%) from the compound (2 g) produced in Step 4.

### Example 47: Synthesis of 10-chloro-2-ethoxycarbonylmethyloxy-5-(3-pyridylmethyl)-4H-pyrido[3,2,1-jk]carbazol-4-one

### [Step 1] Synthesis of 3-methoxycarbazole

The procedure of Example 1, Step 1 was repeated to obtain the titled compound (76 g, 82%) from 3-bromocarbazole (116 g) prepared by the procedure described in *Kogyo Kagaku Zasshi (Journal of Industrial Chemistry)*, 70, 63, (1967).
melting point: 153.2-154.3 °C
IR spectrum (KBr tab.) ν cm⁻¹: 3406, 1497, 1460, 1171, 1034, 820, 748
NMR spectrum (DMSO-d₆) δ ppm: 11.03(1H,s), 8.09(1H,d,J=7.8Hz), 7.67(1H,d,J=2.3Hz), 7.45-7.31(3H,m), 7.13-7.08(1H,m), 7.02(1H,dd,J=8.8, 2.3Hz), 3.84(3H,s)

### [Step 2] Synthesis of 3-chloro-6-methoxycarbazole

The procedure of Example 46, Step 3 was repeated, and the titled compound (6.4 g, 17%) was obtained from the compound (33 g) produced in Step 1 and N-chlorosuccinimide (23.5 g).
melting point: 152.7-154.9 °C
IR spectrum (KBr tab.) ν cm⁻¹: 3415, 1491, 1462, 1223, 1205, 1169, 814
NMR spectrum (DMSO-d₆) δ ppm: 11.21(1H,s), 8.21(1H,d,J=2.0Hz), 7.75(1H,d,J=2.4Hz), 7.47-7.32(3H,m), 7.05(1H,dd,J=8.8,2.4Hz), 3.84(3H,s)

### [Step 3] Synthesis of 10-chloro-5,6-dihydro-2-methoxy-4H-pyrido[3,2,1-jk]carbazol-4-one

The procedure of Example 1, Steps 2 and 3 was repeated to obtain the titled compound from the compound produced in Step 2.
melting point: 162.2-168.2 °C
IR spectrum (KBr tab.) ν cm⁻¹: 1672, 1495, 1479, 1288, 1200, 798
NMR spectrum (DMSO-d₆) δ ppm: 8.36(1H,d,J=2.4Hz), 8.14(1H,d,J=2.3Hz), 7.68(1H,d,J=8.6Hz) 7.54(1H,dd,J=8.6,2.3Hz), 7.37(1H,d,J=2.4Hz), 4.55(2H,t,J=7.0Hz), 3.88(3H,s), 3.13(2H,t,J=7.0Hz)

### [Step 4] Synthesis of 10-chloro-2-methoxy-5-(3-pyridylmethyl)-4H-pyrido[3,2,1-jk]carbazol-4-one

In accordance with Step 4 in Example 1, the titled compound (850 mg; 69%) was prepared from the compound (950 mg) obtained in Step 3.
Melting point: 221.7 - 225.6 °C
IR spectrum (KBr tab.): ν cm⁻¹: 1608, 1579, 1506, 1477, 1433, 1423, 1333
NMR spectrum (DMSO-d₆) δ ppm: 9.14 (1H,s), 8.63(1H,d,J=0.8 Hz), 8.44(1H,d,J=1.0Hz), 8.38(1H,d,J=4.9Hz), 8.26(1H,d,J=1.0Hz) 8.11(1H,d,J=8.3Hz), 7.81-7.74(1H,m), 7.71(1H,dd,J=8.2,0.8Hz), 7.58(1H,d,J=1.0Hz), 7.28(1H,dd,J=8.2,4.9Hz), 3.95(3H,s), 3.90(2H,s)

### [Step 5] Synthesis of 10-chloro-2-hydroxy-5-(3-pyridylmethyl)-4H-pyrido[3,2,1-jk]carbazol-4-one

In accordance with Example 2, the titled compound (380 mg; 54%) was prepared front the compound (700 mg) obtained in Step 4.
Melting point: 300.0 °C <
IR spectrum (KBr tab.) ν cm⁻¹: 3317, 1581, 1510, 1454, 1425, 1392, 1331
NMR spectrum (DMSO-d₆) δ ppm: 10.15(1H, s), 9.12(1H, s), 8.68(1H, s), 8.47-8.36(2H, m), 8.10(1H,d,J=8.8Hz), 8.02(1H, s), 7.88(1H,d,J=7.6Hz), 7.72-7.64(1H, m), 7.54-7.48(1H, m), 7.39(1H,dd,J=7.6,4.4Hz), 3.91(2H, s)

### [Step 6] Synthesis of 10-chloro-2-ethoxycarbonylmethyloxy-5-(3-pyridylmethyl)-4H-pyrido[3,2,1-jk]carbazol-4-one

In accordance with Example 5, the titled compound (124 mg; 83%) was prepared from the compound (120 mg) obtained in Step 5.

### Example 48: Synthesis of 2,10-dichlor-4H-pyrido[3,2,1-jk]carbazol-4-one

### [Step 1] Synthesis of N-(4-chlorophenyl)-β-alanine

p-chloroaniline (200 g) was suspended in water (100 ml) and acrylic acid (54.1 ml) was added to the suspension. The mixture was heated under reflux for 2 hours under a nitrogen atmosphere. After allowing to cool, 2 N aqueous solution of sodium hydroxide (500 ml) was added, and the mixture was extracted with ether. The aqueous layer was adjusted to pH 3 with 1 N hydrochloric acid and extracted with ethyl acetate. The ethyl acetate layer was washed with saturated aqueous solution of sodium chloride, dried over anhydrous sodium sulfate, and the solvent was evaporated under reduced pressure. The titled compound (137.3g, 87%) was obtained without further purification.
melting point: 119.0-121.0 °C
IR spectrum (KBr tab.) ν cm⁻¹: 1707, 1599, 1508, 1435, 1329, 1219, 816
NMR spectrum (*DMSO-d₆) δ ppm: 7.08(2H,d,J=8.9Hz), 6.56(2H,d,J=8.9Hz), 5.83(1H,bs), 3.21(2H,t,J=6.8Hz), 2.50-2.45(2H,m)

### [Step 2] Synthesis of 6-chloro-2,3-dihydro-4(1H)-quinolinone

The compound (137 g) obtained in Step 1 was added to polyphosphoric acid (2147 g) and the mixture was heated at 120 to 130 °C for 1 hour with stirring in an oil bath. The reaction mixture was poured into ice water (4 L) and extracted with ethyl acetate. The ethyl acetate layer was washed with saturated aqueous solution of sodium chloride, dried over anhydrous sodium sulfate, and the solvent was evaporated under reduced pressure. The residue was purified by silica gel column chromatography (eluent: hexane/ethyl acetate = 1:2) to obtain the titled compound (83.5 g, 66%).
melting point: 124.8-129.8 °C
IR spectrum (KBr tab.) ν cm⁻¹: 3348, 1648, 1613, 1512, 1398, 1294, 1167, 814
NMR spectrum (*DMSO-d₆) δ ppm: 7.49(1H,d,J=2.6Hz), 7.29(1H,dd,J=9.6,2.6Hz), 7.02(1H,s), 6.80(1H,d,J=9.6Hz), 3.46-3.41(2H,m), 2.56-2.54(2H,m)

### [Step 3] Synthesis of 6-chloro-1-(4-chlorophenyl)-2,3-dihydro-4(1H)-quinolinone

The compound (9.02 g) obtained in Step 2, 1-chloro-4-iodobenzene (23.7 g), copper (II) oxide (1.04 g) and potassium carbonate (6.87 g) were mixed, and the mixture was heated at 180 to 190 °C for 6 hours with stirring in an oil bath under an argon atmosphere. The reaction mixture was poured into ice water and extracted with ether. Insoluble content was removed by filtration and the ether layer was washed with saturated aqueous solution of sodium chloride, dried over anhydrous sodium sulfate, and the solvent was evaporated under reduced pressure. The residue was purified by silica gel column chromatography (eluent: hexane/methylene chloride = 1:2) to obtain the titled compound (5.3 g, 36%).
melting point: 142.8-149.5 °C
IR spectrum (KBr tab.) ν cm⁻¹: 1677, 1490, 1475, 1209, 1166, 825
NMR spectrum (*DMSO-d₆) δ ppm: 7.82(1H,d,J=8.6Hz), 7.67(2H,d,J=2.7Hz), 7.41-7.39(1H,m), 7.37-7.36(1H,m), 7,33(1H,dd,J=9.1,2.7Hz), 6.62(1H,d,J=9.1Hz), 3.90(2H,t,J=6.9Hz), 2.80(2H,t,J=6.9Hz)

### [Step 4] Synthesis of 6-chloro-1-(4-chlorophenyl)-4(1H)-quinolinone

The compound (1 g) obtained in Step 3 was dissolved in ethylene glycol (10 ml). To the solution was added 5% palladium carbon (200 mg) and the mixture was heated under reflux for 30 minutes under an argon atmosphere. After allowing to cool, the insoluble content was removed by filtration and the filtrate was distilled to remove the solvent under reduced pressure. The residue was purified by silica gel column chromatography (eluent: hexane/ethyl acetate = 1:1) to obtain the titled compound (120 mg, 12%).
melting point: 236.3-237.5 °C
IR spectrum (KBr tab.) ν cm⁻¹: 1632, 1587, 1493, 1471, 1293, 825
NMR spectrum (DMSO-d₆) δ ppm: 8.14(1H,d,J=2.6Hz), 8.02(1H,d,J=7.6Hz), 7.75-7.72(2H,m), 7.67-7.63(3H,m), 7.05(1H,d,J=9.2Hz), 6.22(1H,d,J=7.6Hz)

### [Step 5] Synthesis of 2,10-dichlor-4H-pyrido[3,2,1-jk]carbazol-4-one

6-chloro-1-(4-chlorophenyl)-4(1H)-quinolinone (2 g) obtained in Step 4 was dissolved in acetic acid (150 ml) and to the solution were added a boron trifluoride-acetic acid complex (44 ml) and palladium diacetate (6.28 g). The mixture was heated under reflux for 1 hour under an argon atmosphere and allowed to cool. The insoluble content was removed by filtration and the solution was extracted with water and ethyl acetate. The ethyl acetate layer was washed with saturated aqueous solution of sodium chloride, dried over anhydrous sodium sulfate, and the solvent was evaporated under reduced pressure. The residue was purified by silica gel flash column chromatography (eluent: hexane/ethyl acetate = 1:2) to obtain the titled compound (40 mg, 2%).

### Example 49: Synthesis of 10-bromo-2-methyl-5-(3-pyridylmethyl)-4H-pyrido[3,2,1-jk]carbazol-4-one

### [Step 1] Synthesis of 6-methyl-1,2,3,4-tetrahydrocarbazole

The procedure of Example 43, Step 1 was repeated by using p-tolylhydrazine hydrochloride (25 g) to obtain the titled compound (27 g, 93%).
melting point: 147.3-150.6 °C
IR spectrum (KBr tab.) ν cm⁻¹: 3396, 2929, 1589, 1439, 1315, 797, 596
NMR spectrum (DMSO-d₆) δ ppm: 10.45(1H,s), 7.12(1H,s), 7.11(1H,d,J=7.8Hz), 6.78(1H,dd,J=7.8,1.5Hz), 2.69-2.65(2H,m), 2.60-2.50(2H,m), 2.34(3H,s), 1.81-1.78(4H,m)

### [Step 2] Synthesis of 3-methylcarbazole

The compound (20 g) obtained in Step 1 was dissolved in xylene (500 ml) and 10% palladium carbon (6 g) was added to the solution. The mixture was heated under reflux for 5 hours under an argon atmosphere. The reaction mixture was filtered while hot and the filtrate was distilled to remove the solvent under reduced pressure. The residue was purified by silica gel flash column chromatography (eluent: hexane/ethyl acetate = 20:1) to obtain the titled compound (7.5 g, 38%).
melting point: 206.2-209.5 °C
IR spectrum (KBr tab.) ν cm⁻¹: 3408, 1462, 1242, 806, 748, 729, 573
NMR spectrum (DMSO-d₆) δ ppm: 11.09(1H,s), 8.05(1H,d,J=7.8Hz), 7.89(1H,s), 7.44(1H,d,J=8.3Hz), 7.38-7.32(2H,m), 7.20(1H,dd,J=8.3,1.5Hz), 7.11(1H,t,J=6.8Hz), 2.46(3H,s)

### [Step 3] Synthesis of 3-bromo-6-methylcarbazole

The procedure of Example 46, Step 3 was repeated by using the compound (2 g) produced in Step 2 to obtain the titled compound (1.85 g, 64%).
melting point: 211.7-212.6 °C
IR spectrum (KBr tab.) ν cm⁻¹: 3394, 1491, 1444, 1296, 1240, 812, 569
NMR spectrum (DMSO-d₆) δ ppm: 11.29(1H,bs), 8.29(1H,d,J=2.0Hz), 7.95(1H,d,J=1.2Hz), 7.54-7.35(3H,m), 7.24(1H,dd,J=8.3,1.2Hz), 2.45(3H,s)

### [Step 4] Synthesis of 10-bromo-5,6-dihydro-2-methyl-4H-pyrido[3,2,1-jk]carbazol-4-one

The procedure of Example 1, Steps 2 and 3 was repeated by using the compound produced in Step 3 to obtain the titled compound.
melting point: 201.7-204.4 °C
IR spectrum (KBr tab.) ν cm⁻¹: 1670, 1597, 1498, 1477, 1281, 1221, 791
NMR spectrum (CDCl3) δ ppm: 8.17(1H,d,J=1.6Hz), 7.98(1H,s), 7.77-7.76(1H,m), 7.59(1H,dd,J=8.5,1.6Hz), 7.26(1H,s), 4.46(2H,t,J=7.1Hz), 3.15(2H,t,J=7.1Hz), 2.55(3H,s)

### [Step 5] Synthesis of 10-bromo-2-methyl-5-(3-pyridylmethyl)-4H-pyrido[3,2,1-jk]carbazol-4-one

The procedure of Example 1, Step 4 was repeated by using the compound (2 g) produced in Step 4 to obtain the titled compound (120 mg, 47%).

### Example 50: Synthesis of 10-bromo-2-methyl-4H-pyrido[3,2,1-jk]carbazol-4-one

The titled compound was obtained as a high polarity component in the reaction of Step 4 in Example 49.

### Example 51: Synthesis of 9-bromo-2-(3-hydroxypropyloxy)-5-(3-pyridylmethyl)-4H-pyrido[3,2,1-jk]carbazol-4-one hydrochloride

9-bromo-2-(3-hydroxypropyloxy)-5-(3-pyridylmethyl)-4H-pyrido[3,2,1-jk]carbazol-4-one (300 mg) obtained in Example 38 was suspended in methanol (10 ml) and a solution of hydrogen chloride in methanol (5 ml) was added under cooling with ice. The mixture was stirred for 5 minutes. The solvent was evaporated under reduced pressure, and the resulting crude crystals were washed with ether to obtain the titled compound (320 mg, 98%).

### Example 52: Synthesis of 9-bromo-2-(3-hydroxypropyloxy)-5-(3-pyridylmethyl)-4H-pyrido[3,2,1-jk]carbazol-4-one methanesulfonate

9-bromo-2-(3-hydroxypropyloxy)-5-(3-pyridylmethyl)-4H-pyrido[3,2,1-jk]carbazol-4-one (250 mg) obtained in Example 38 was suspended in methanol (200 ml) and to the suspension was added a solution of methanesulfonic acid (57 mg) in methanol (5 ml) at room temperature. The mixture was stirred for 30 minutes. The solvent was evaporated under reduced pressure, and the resulting crude crystals were successively washed with a small amount of methanol and ether to obtain the titled compound (270 mg, 89%).

### Example 53: Synthesis of 9-chloro-2-(3-hydroxypropyloxy)-5-(3-pyridylmethyl)-4H-pyrido[3,2,1-jk]carbazol-4-one

### [Step 1] Synthesis of 2-chloro-6-[3-(4-methoxyphenoxy)propyloxy]carbazole

2-chloro-6-hydroxycarbazole (14.2 g) prepared by the procedure described in *Justus Liebigs Ann. Chem*., 617, 54 (1958) was dissolved in methanol (140 ml) and to the solution was added 2 N solution of potassium hydroxide in methanol (36.7 ml). The mixture was stirred for 5 minutes at room temperature. After evaporating the solvent under reduced pressure, toluene (40 ml) was added and the solvent was evaporated again under reduced pressure. The resulting crystals were suspended in toluene (53 ml). To the suspension were added 3-(4-methoxyphenoxy)propylbromide (18.0 g) prepared by the procedure described in *Kokai Tokkyo Koho JP* 02193942, dissolved in toluene (18 ml), then 18-crown-6 (1.9 g) dissolved in toluene (18 ml). The mixture was heated under reflux for 1 hour and allowed to cool. 0.001 N hydrochloric acid (120 ml) was added, and the mixture was extracted with ethyl acetate. The ethyl acetate layer was washed with saturated aqueous solution of sodium chloride, dried over anhydrous sodium sulfate, and the solvent was evaporated under reduced pressure. The residue was dissolved in acetone, and re-precipitated from methanol/water (1:1), then dissolved in methylene chloride. After adding silica gel, the solvent was evaporated under reduced pressure, and the residue was eluted from the silica gel as the adsorbent with a solution of hexane/methylene chloride (1:1). The solvent was evaporated under reduced pressure to obtain the titled compound (15.7g, 56%).
melting point: 137.1-138.7 °C
IR spectrum (KBr tab.) ν cm⁻¹: 3396, 1508, 1456, 1294, 1201, 1031, 823
NMR spectrum (*DMSO-d₆) δ ppm: 11.21(1H,s), 8.12(1H,d,J=2.3Hz), 7.73(1H,d,J=2.3Hz), 7.47(1H,d,J=1.8Hz), 7.40(1H,d,J=8.7Hz), 7.12(1H,dd,J=8.3,1.8Hz), 7.06(1H,dd,J=8.7,2.3Hz), 6.95-6.82(4H,m), 4.21(2H,t,J=6.2Hz), 4.11(2H,t,J=6.1Hz), 3.69(3H,s), 2.25-2.13(2H,m)

### [Step 2] Synthesis of 2-chloro-6-[3-(4-methoxyphenoxy)propyloxy]carbazole-N-β-propionic acid

The compound (15.7 g) obtained in Step 1 was dissolved in acetone (630 ml), and to the solution were added dropwise methyl acrylate (4.6 g) and then Triton B (3.7 ml) under cooling with ice. After stirring for 1 hour, the solvent was evaporated under reduced pressure. The resulting residue was suspended in methanol (94 ml), and sodium hydroxide (3.3 g) dissolved in water (4.4 ml) was added dropwise to this suspension, and the mixture was stirred at 60°C for 30 minutes on a hot water bath. After evaporating the solvent under reduced pressure, the residue was decanted with ether and 1 N hydrochloric acid and ethyl acetate were added to the resulting crystals for phase separation. The ethyl acetate layer was washed with saturated aqueous solution of sodium chloride, dried over anhydrous sodium sulfate, and the solvent was evaporated under reduced pressure to obtain the titled compound (17.6 g, 94%).
melting point: 126.7-130.7 °C
IR spectrum (KBr tab.) ν cm⁻¹: 2937, 1699, 1514, 1489, 1238, 1072, 822
NMR spectrum (*DMSO-d₆) δ ppm: 12.34(1H,bs), 8.12(1H,d,J=8.4Hz), 7.77(1H,d,J=2.5Hz), 7.71(1H,d,J=1.6Hz), 7.54(1H,d,J=8.9Hz), 7.20-7.18(2H,m),6.97-6.80(4H,m), 4.58(2H,t,J=6.7Hz), 4.22(2H,t,J=6.2Hz), 4.11(2H,t,J=6.2Hz), 3.69(3H,s), 2.71(2H,t,J=6.7Hz), 2.26-2.13(2H,m)

### [Step 3] Synthesis of 9-chloro-5,6-dihydro-2-[3-(4-methoxyphenoxy)propyloxy]-4H-pyrido[3,2,1-jk]carbazol-4-one

The compound (14.6 g) obtained in Step 2 was suspended in anhydrous chloroform (400 ml), and to the suspension was added PPE (83.2 g) dissolved in anhydrous chloroform (400 ml) at room temperature, and the mixture was heated under reflux for 1.5 hours under an argon atmosphere and allowed to cool. The mixture was poured into water and extracted with methylene chloride. The methylene chloride layer was washed with saturated aqueous solution of sodium chloride, dried over anhydrous sodium sulfate, and the solvent was evaporated under reduced pressure. The residue was crystallized from acetone/methanol (1:1) and recovered by filtration. The crude crystals were dissolved in chloroform while hot and re-precipitated from methanol to obtain the titled compound (6.3 g, 38%).
melting point: 156.3-159.0 °C
IR spectrum (KBr tab.) ν cm⁻¹: 2953, 1680, 1506, 1443, 1228, 1065, 818
NMR spectrum (*DMSO-d₆) δ ppm: 8.20(1H,d,J=8.3Hz), 8.11(1H,d,J=2.2Hz), 7.80(1H,d,J=1.7Hz), 7.35(1H,d,J=2.2Hz), 7.25(1H,dd,J=8.3,1.7Hz), 6.94-6.77(4H,m), 4.54(2H,t,J=7.0Hz), 4.24(2H,t,J=6.1Hz), 4.10(2H,t,J=6.1Hz), 3.67(3H,s), 3.10(2H,t,J=7.0Hz), 2.24-2.12(2H,m)

### [Step 4] Synthesis of 9-chloro-2-[3-(4-methoxyphenoxy) propyloxy]-5-(3-pyridylmethyl)-4H-pyrido[3,2,1-jk]carbazol-4-one

The compound (6.3 g) obtained in Step 3 was suspended in ethanol (410 ml) and to the suspension were added pyridine-3-aldehyde (2.5 g) and sodium hydroxide (4.6 g) dissolved in water (25 ml). The mixture was stirred for 15 minutes at 60°C on a hot water bath. After evaporating the solvent under reduced pressure, the crystals were washed with water. The resulting crude crystals were dissolved in an aqueous solution of hot acetonitrile (hot acetonitrile:water = 20:1), and re-precipitated from water to obtain the titled compound (6.7 g, 87%).
melting point: 157.0-160.9 °C
IR spectrum (KBr tab.) ν cm⁻¹: 3431, 2933, 1605, 1508, 1462, 1232, 1064
NMR spectrum (*DMSO-d₆) δ ppm: 9.10(1H,s), 8.66-8.60(1H,m), 8.42-8.35(1H,m), 8.25(1H,d,J=1.8Hz), 8.22(1H,d,J=8.2Hz), 8.19(1H,d,J=2.2Hz), 7.79-7.70(1H,m), 7.53(1H,d,J=2.2Hz), 7.47(1H,dd,J=8.2,1.8Hz), 7.32-7.23(1H,m), 6.95-6.78(4H,m), 4.29(2H,t,J=6.2Hz), 4.11(2H,t,J=6.1Hz), 3.86(2H,s), 3.66(3H,s), 2.28-2.14(2H,m)

### [Step 5] Synthesis of 9-chloro-2-(3-hydroxypropyloxy)-5-(3-pyridylmethyl)-4H-pyrido[3,2,1-jk]carbazol-4-one

The compound (3.7 g) obtained in Step 4 was suspended in an aqueous solution of acetonitrile (400 ml, acetonitrile:water = 4:1), and to the suspension was added dropwise slowly CAN (11.5 g) dissolved in an aqueous solution of acetonitrile (40 ml, acetonitrile:water = 4:1) on an ice bath. After stirring for 15 minutes, 1N aqueous solution of sodium hydroxide was added. The precipitated crystals were recovered by filtration and washed with a small amount of water. The crude crystals were added to ethyl acetate. After stirring for 3 hours, the insoluble content was removed by filtration and the filtrate was successively washed with 10% aqueous solution of sodium sulfite, 1N aqueous solution of sodium hydroxide and saturated aqueous solution of sodium chloride, dried over anhydrous sodium sulfate, and the solvent was evaporated under reduced pressure. The resulting crystals were washed with ether and recovered by filtration to obtain the titled compound (1.3 g, 46%).

### Example 54: Synthesis of 9-chloro-2-(3-hydroxypropyloxy)-5-(3-pyridylmethyl)-4H-pyrido[3,2,1-jk]carbazol-4-one methanesulfonate

The procedure of Example 52 was repeated by using the compound (780 mg) produced in Example 53 to obtain the titled compound (696 mg, 73%).

### Example 55: Synthesis of 2-(3-hydroxypropyloxy)-5-(3-pyridylmethyl)-9-trifluoromethyl-4H-pyrido[3,2,1-jk]carbazol-4-one

The procedure of Example 53, Steps 1, 2, 3, 4 and 5 was repeated by using commercially available 4-trifluoromethyl-2-nitroaniline to obtain the titled compound.

### Example 56: Synthesis of 2-(3-hydroxypropyloxy)-5-(3-pyridylmethyl)-9-trifluoromethyl-4H-pyrido[3,2,1-jk]carbazol-4-one methanesulfonate

The procedure of Example 52 was repeated by using the compound (600 mg) produced in Example 55 to obtain the titled compound (693 mg, 94%).

### Example 57: Synthesis of sildenafil

The procedure of Example 12 in the *European Patent Publication* No. 463,756 was used to obtain the titled compound.

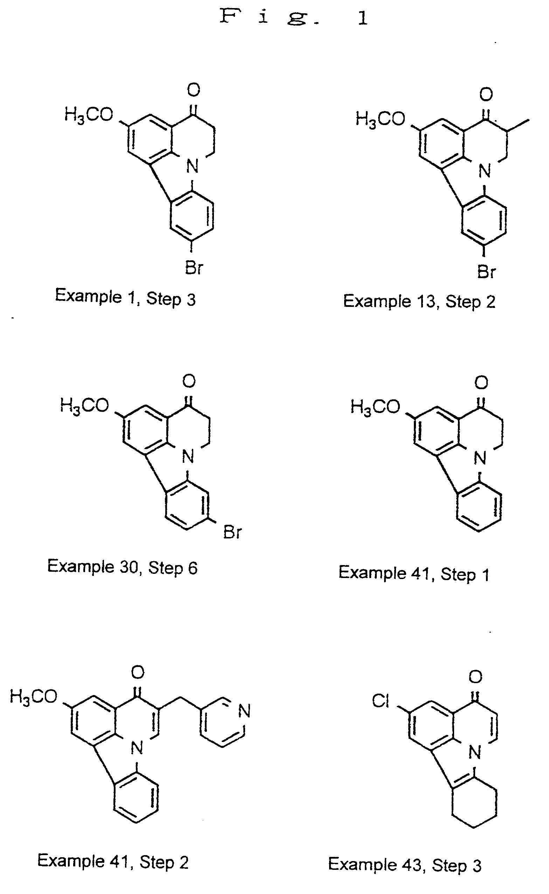

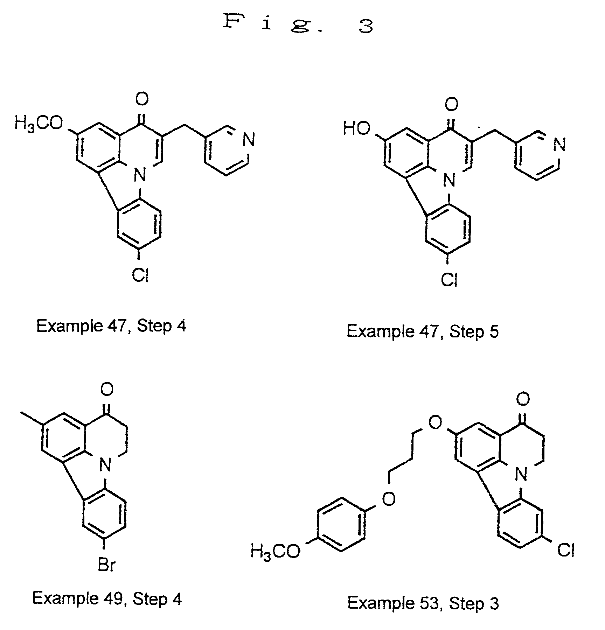

Physical data of the compounds of Examples 1 to 57 were shown in Table 5 and the structual formulae of the compounds of Examples 1 to 57 were shown in Tables 6 to 13. The structures of the intermediates in several Examples were shown in FIG. 1 to 3.

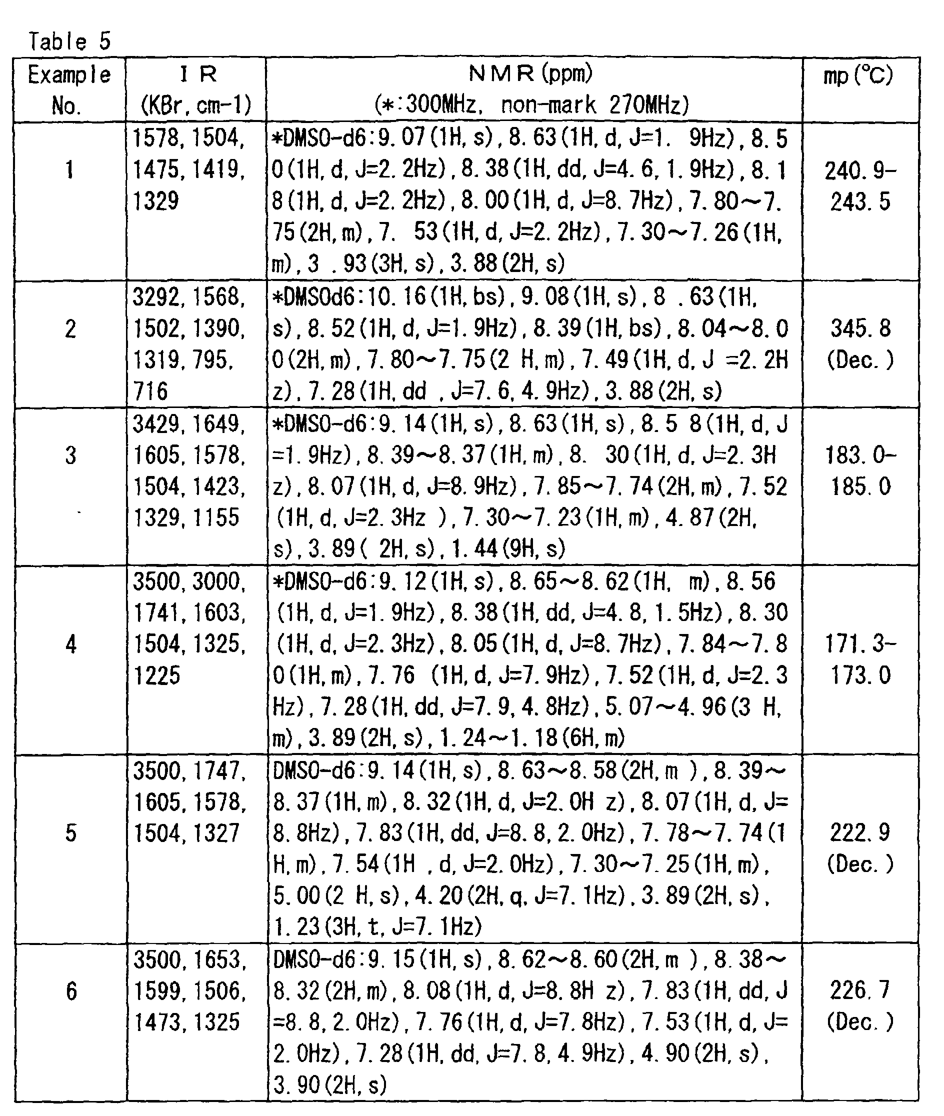

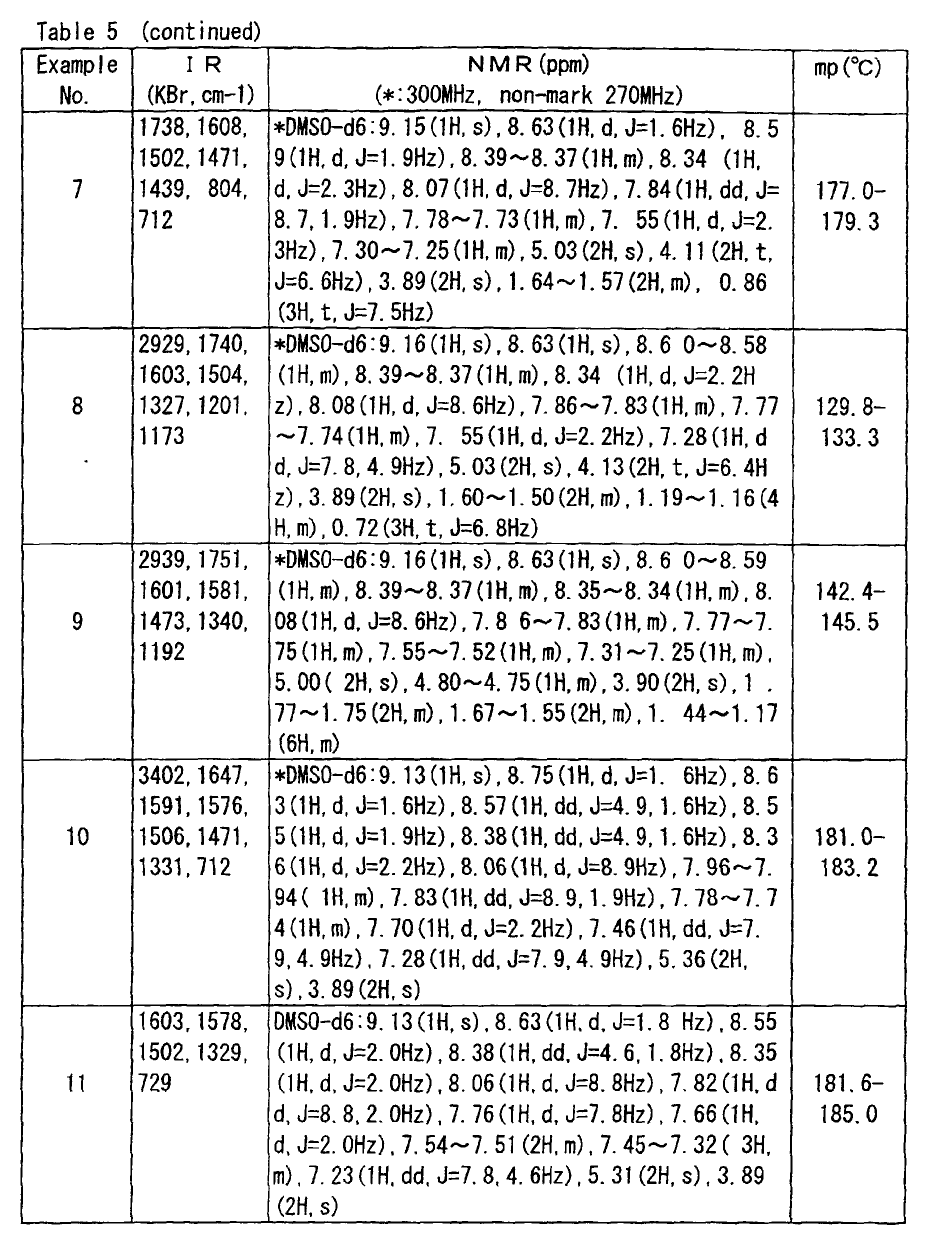

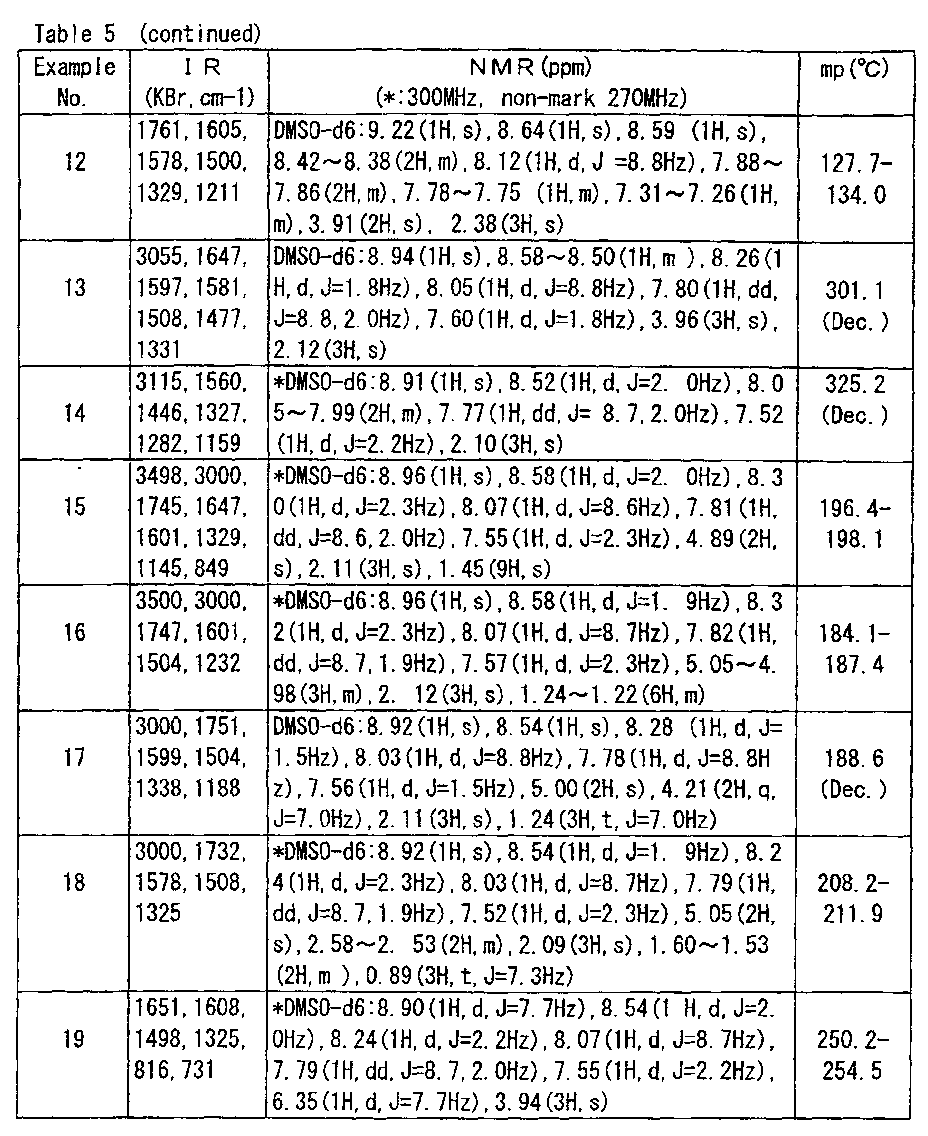

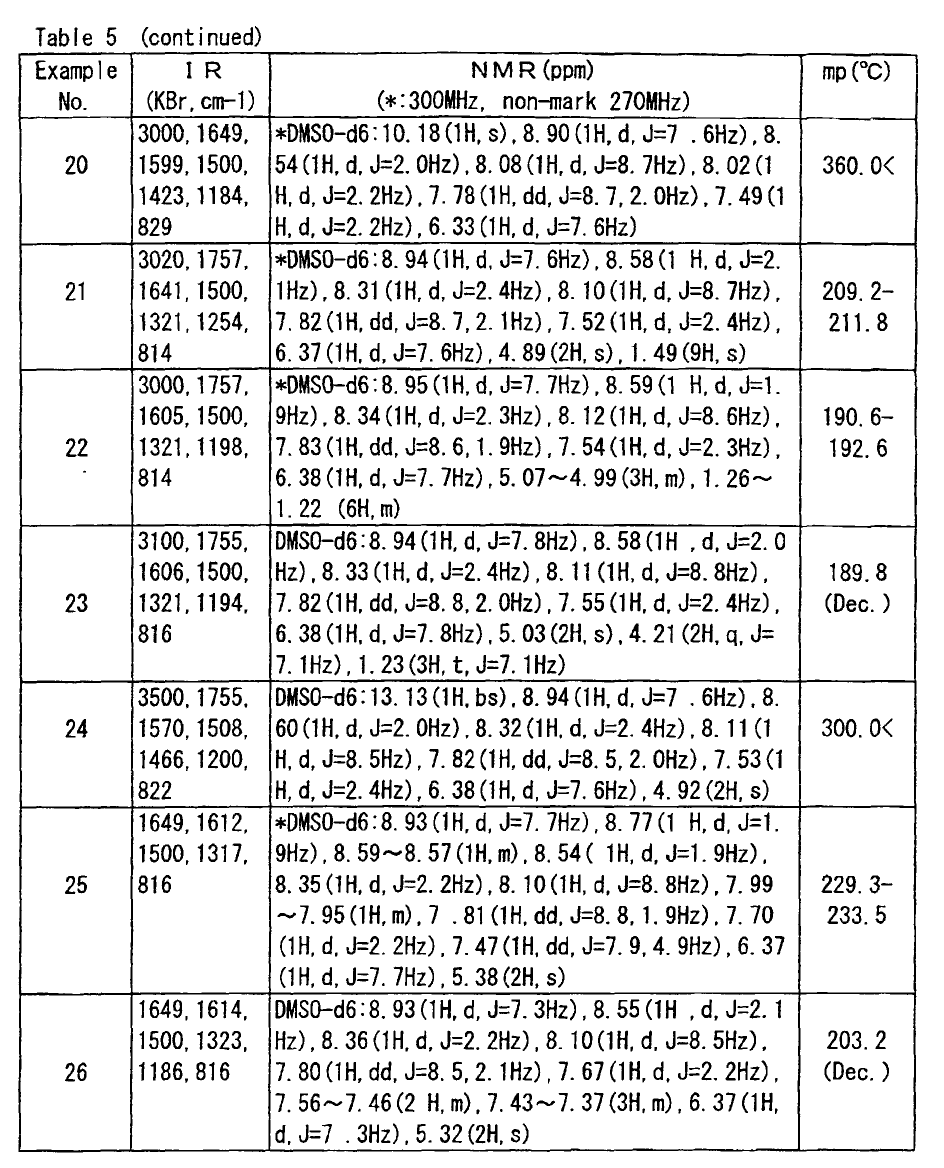

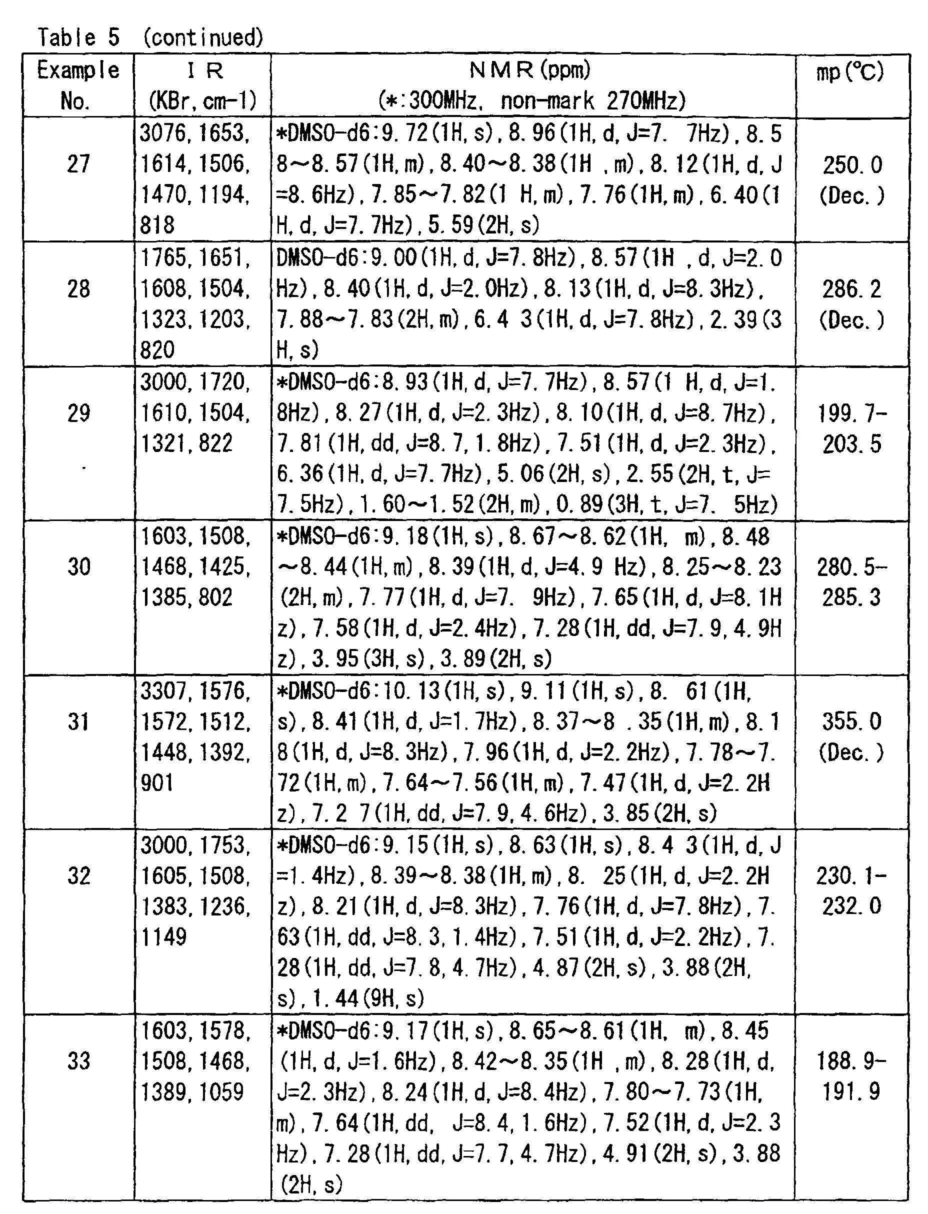

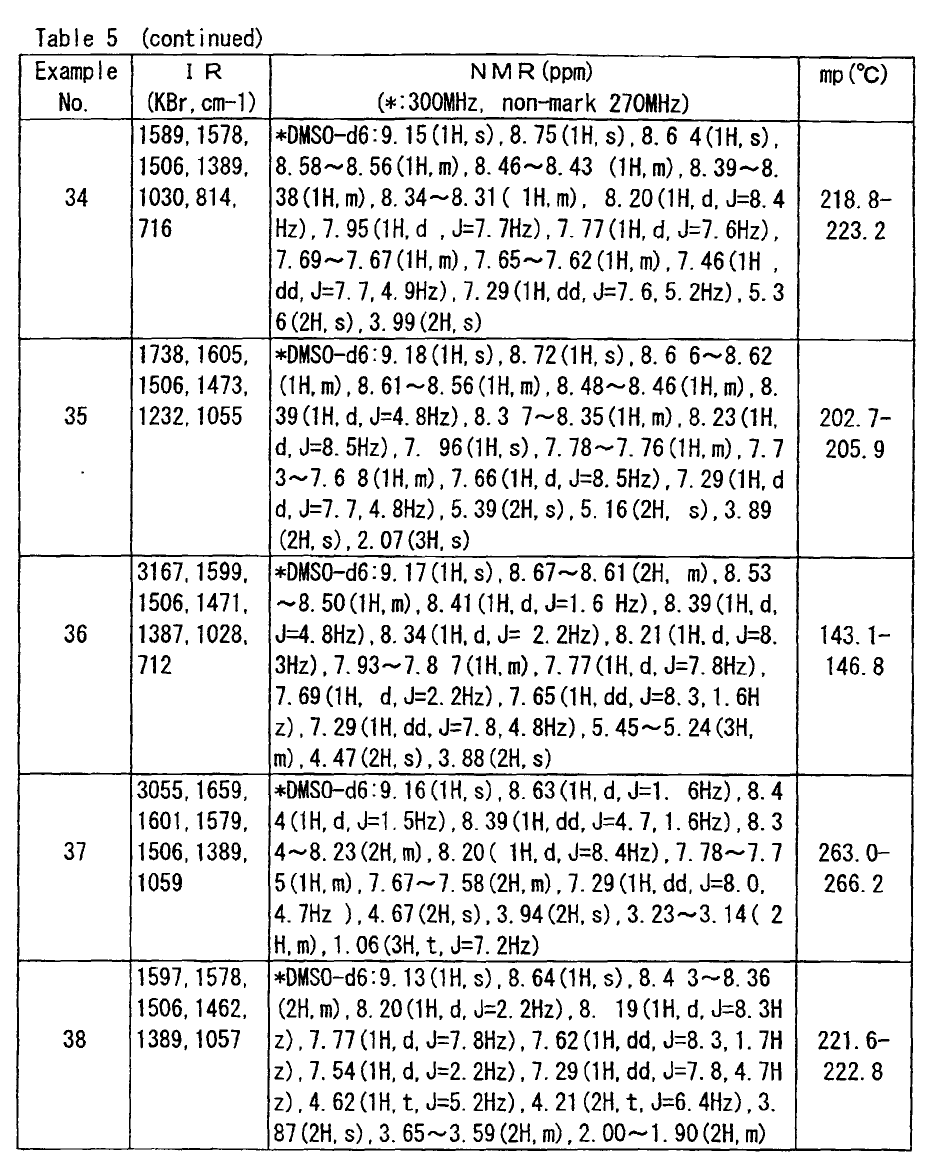

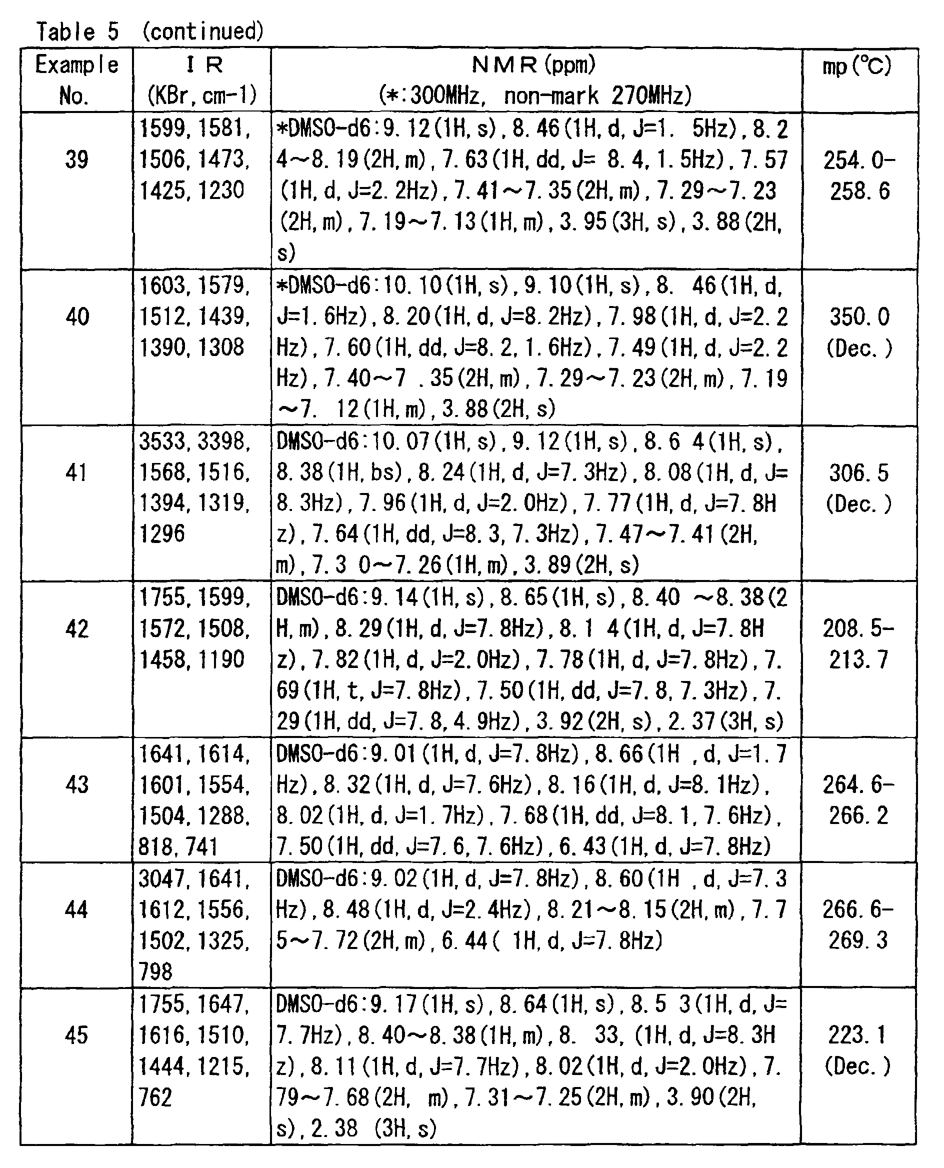

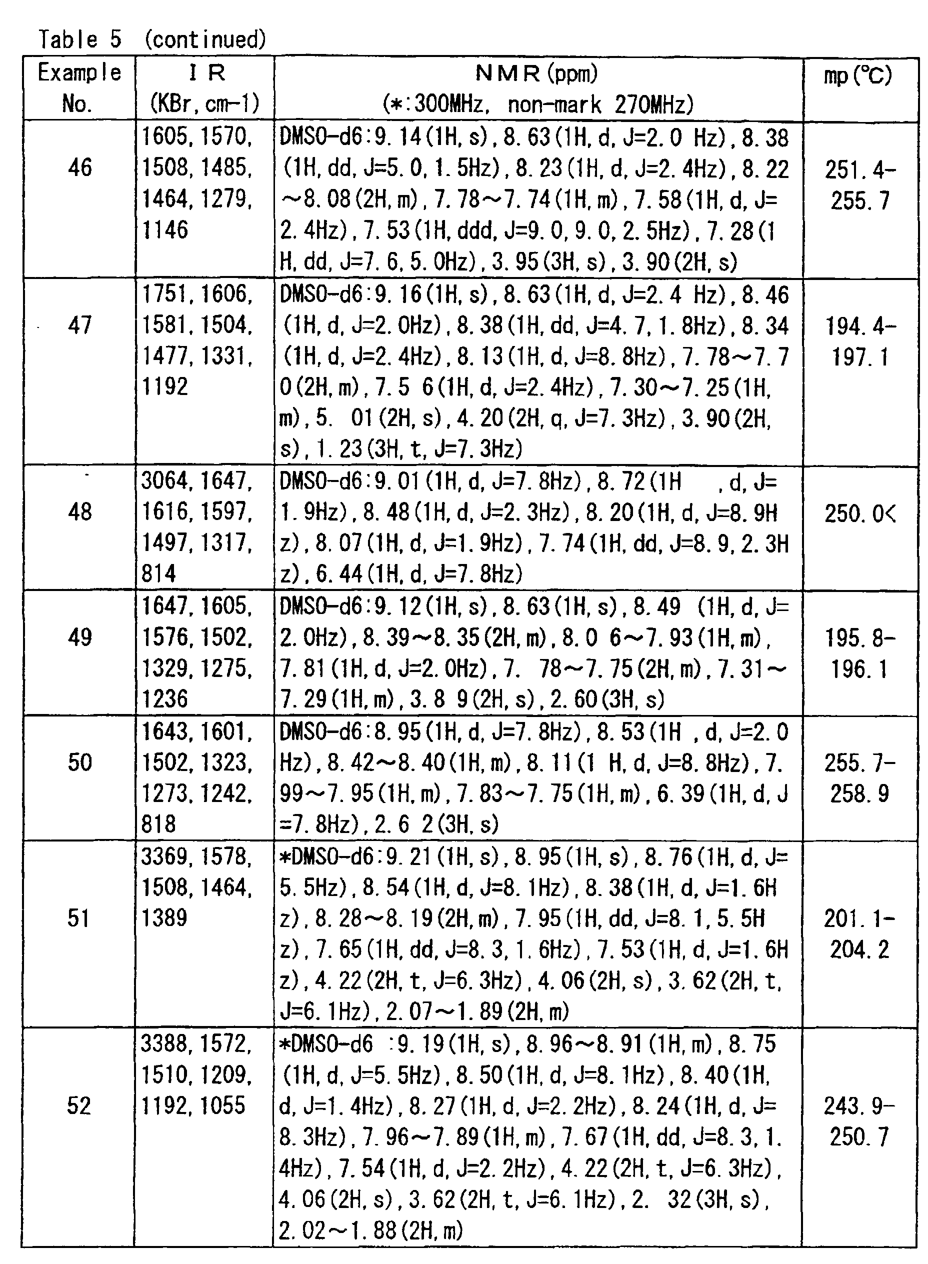

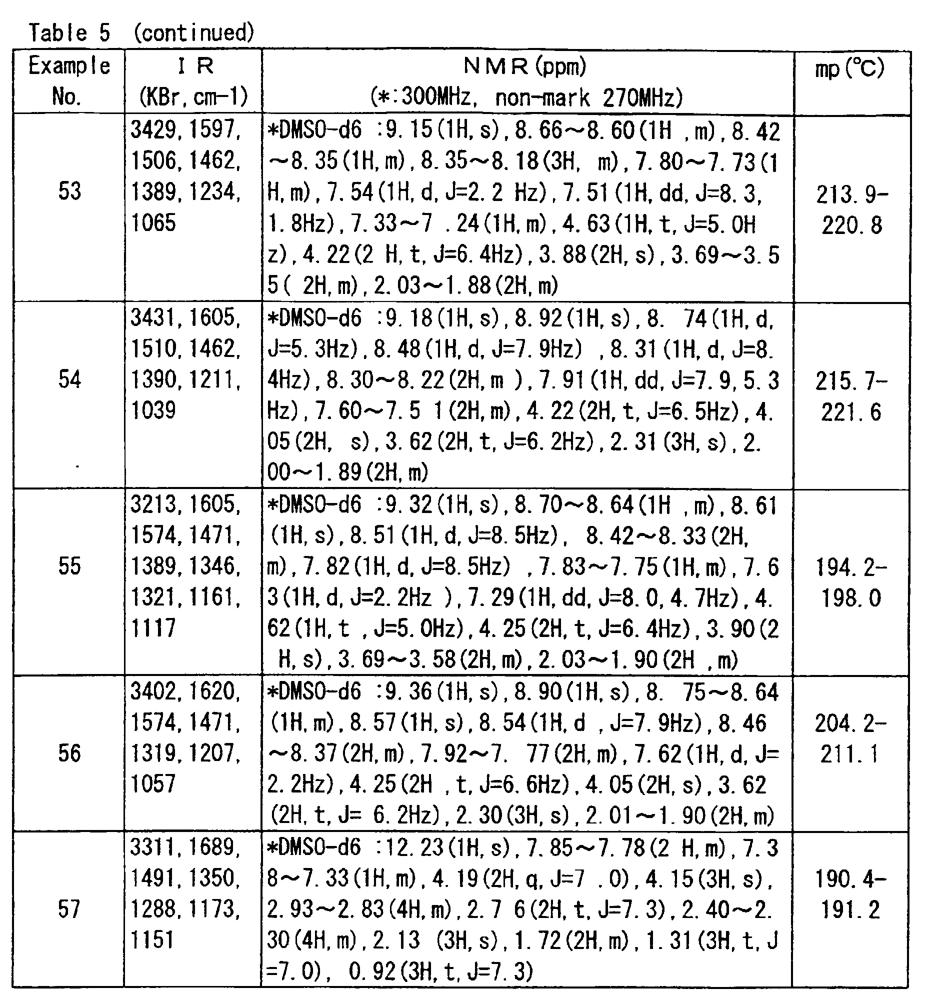

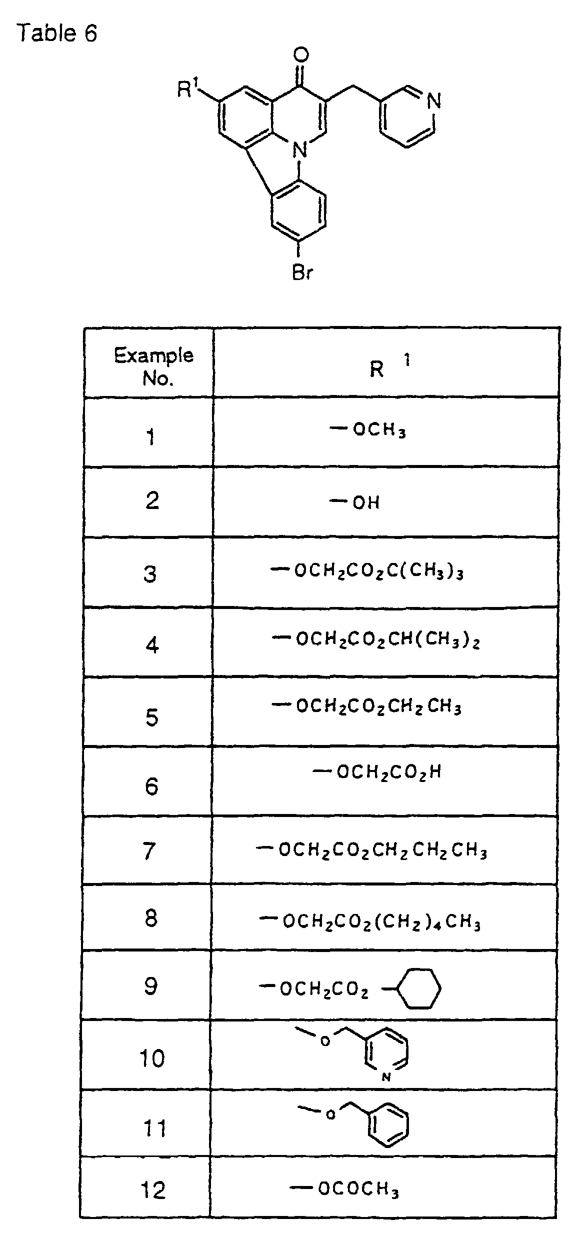

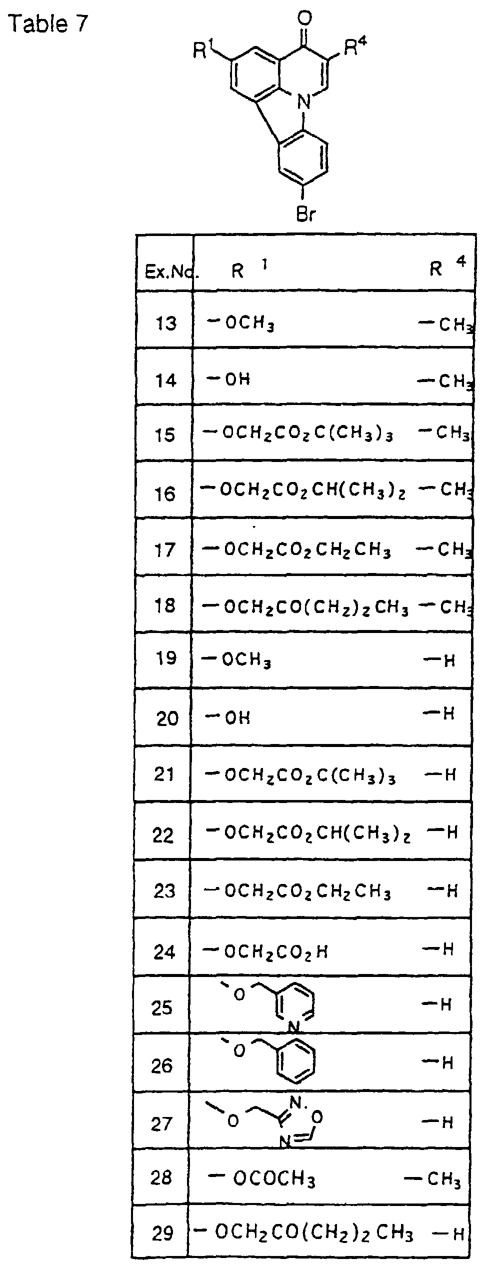

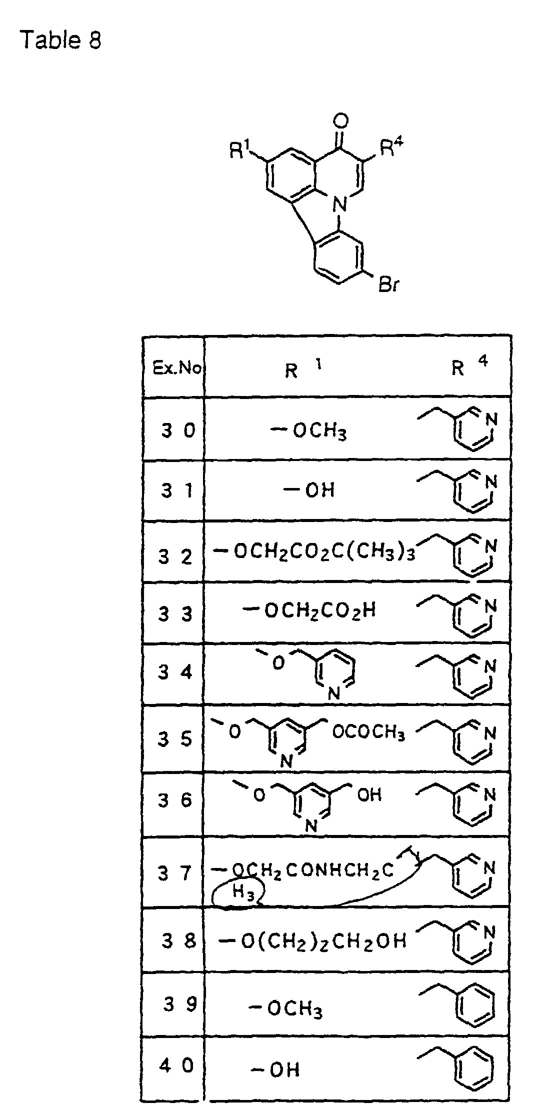

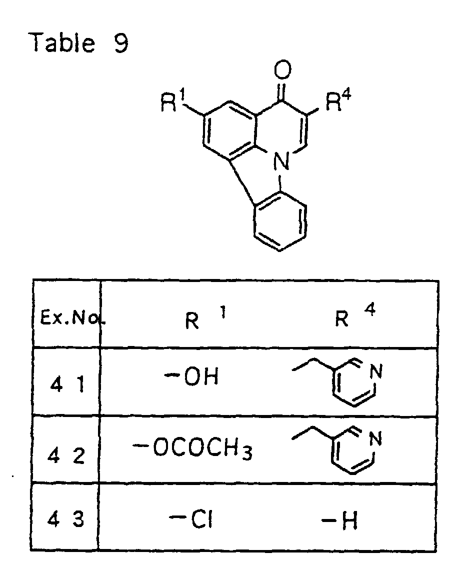

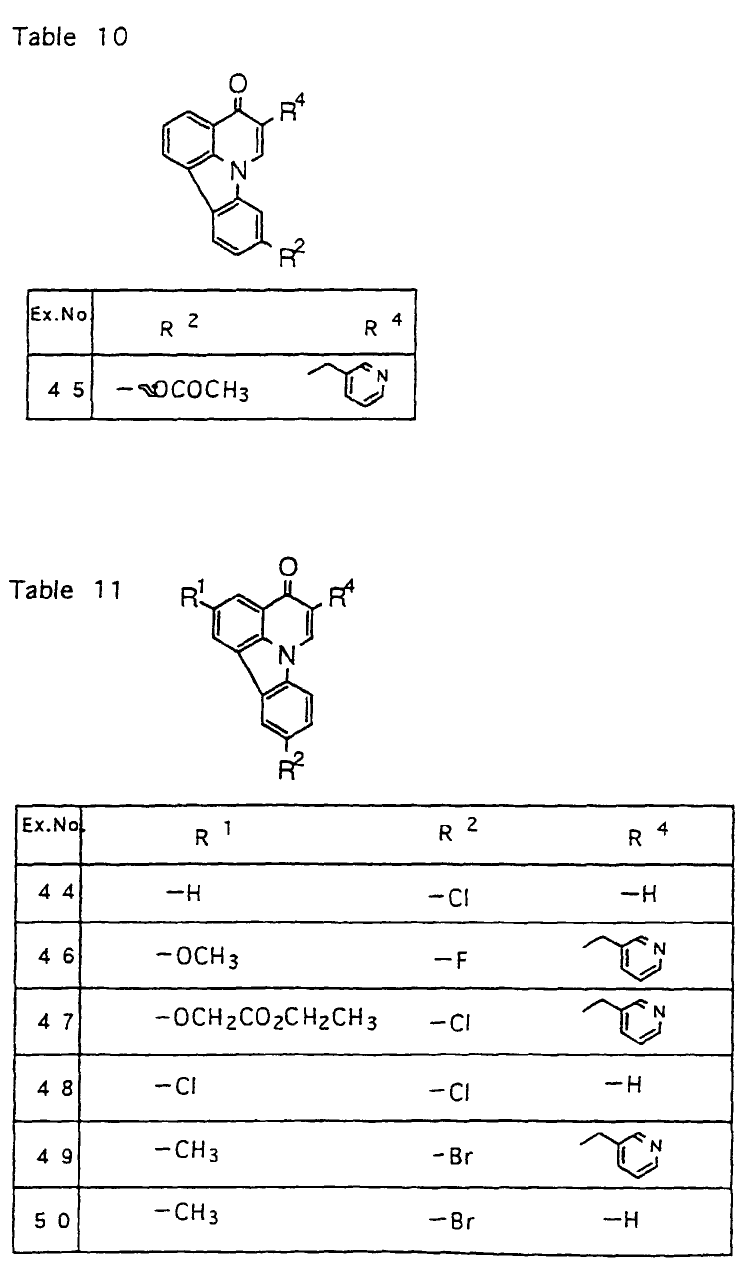

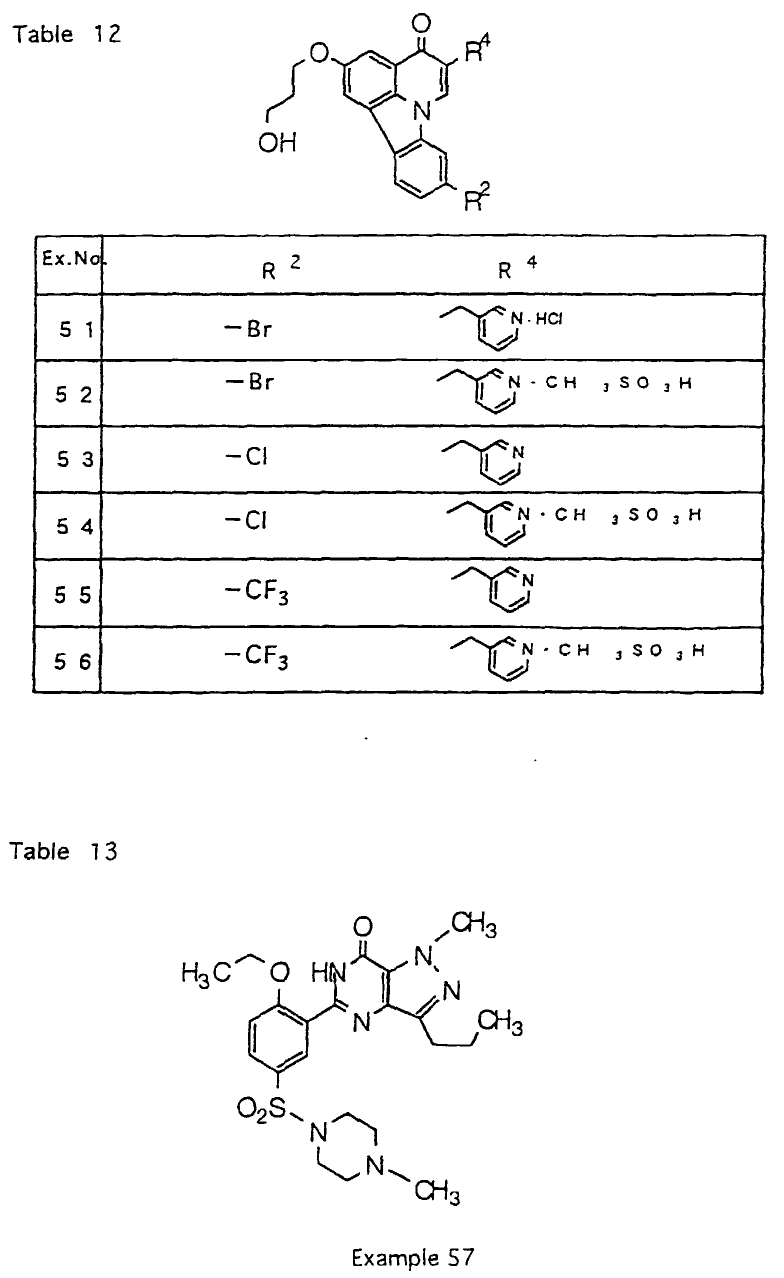

The following are non-limiting examples of the pharmaceutical formulations containing the compounds used in the invention.

### (Example of Preparation 1: Liquid Preparation for Intranasal Administration)

| | |
|---|---|
| Compound of Example 52 | 3 g |
| Citric acid | 6 g |
| Benzalkonium chloride | 50 mg |

The above components were weighed and homogeneously dissolved in pure water to make the total amount 100 ml. The solution was filtered through a filter of 0.2 µm and each 3.0 ml was charged in a quantitative sprayer capable of spraying 0.05-0.1 ml per stroke.

### (Example of Preparation 2: Liquid Preparation for Intranasal Administration)

The preparation was manufactured by the same manner as in Example of Preparation 1 except that the amount of the compound of Example 52 used was 2 g.

### (Example of Preparation 3: Liquid Preparation for Intranasal Administration)

| | |
|---|---|
| Sildenafil | 3 g |
| Citric acid | 6 g |
| Benzalkonium chloride | 50 mg |

The above components were weighed and made into a preparation by the same manner as in Example of Preparation 1.

### (Example of Preparation 4: Liquid Preparation for Intranasal Administration)

| | |
|---|---|
| Compound of Example 52 | 3 g |
| HCO-60 | 10 g |
| Beuzalkonium chloride | 50 mg |

The above components were weighed and pure water was added to make the total amount 100 ml. After that, the preparation was manufactured by the same manner as in Example of Preparation 1.

### (Example of Preparation 5: Liquid Preparation for Intranasal Administration)

| | |
|---|---|
| Compound of Example 34 | 3 g |

The above component was weighed and homogeneously dissolved in glycerol to make the total amount 100 ml.

### (Example of Preparation 6: Liquid Preparation for Intranasal Administration)

| | |
|---|---|
| Compound of Example 52 | 1 g |

The above component was weighed and homogeneously dissolved in glycerol to make the total amount 100 ml.

### (Example of Preparation 7: Liquid Preparation for Intranasal Administration)

| | |
|---|---|
| Compound of Example 54 | 2 g |

The above component was weighed and homogeneously dissolved in glycerol to make the total amount 100 ml.

### (Example of Preparation 8: Liquid Preparation for Intranasal Administration)

| | |
|---|---|
| Compound of Example 38 | 300 mg |
| Crystalline cellulose | 9.7 g |

The above components were weighed and homogeneously mixed. Each 20 mg of the mixture was charged in a hard gelatin capsule for attaching to a sprayer for nostril.

### (Example of Preparation 9: Diluted Powder for Intranasal Administration)

| | |
|---|---|
| Sildenafil | 300 mg |
| Crystalline cellulose | 9.7 g |

The above components were weighed and the preparation was manufactured by the same manner as in Example of Preparation 8.

### (Example of Preparation 10: Diluted Powder for Intranasal Administration)

| | |
|---|---|
| Compound of Example 52 | 200 mg |
| Hydroxypropylcellulose | 9.8 g |

The above components were weighed and the preparation was manufactured by the same manner as in Example of Preparation 8.

### (Example of Preparation 11: Diluted Powder for Intranasal Administration)

| | |
|---|---|
| Compound of Example 3 | 300 mg |
| Lactose | 9.7 g |

The above components were weighed and the preparation was manufactured by the same manner as in Example of Preparation 8.

### (Example of Preparation 12: Oily Ointment)

| | |
|---|---|
| Compound of Example 52 | 5 g |
| Propylene glycol | 50 g |
| Propylene glycol monostearate | 7.5 g |
| Diisopropyl adipate | 5 g |
| White vaseline | 32.5 g |

Compound of Example 52 was dissolved in propylene glycol and then other components were added thereto. The mixture was warmed at about 60 °C with vigorous stirring to mix homogeneously.

### (Example of Preparation 13: Oily Ointment)

| | |
|---|---|
| Compound of Example 52 | 5 g |
| Glycerol | 50 g |
| Propylene glycol monostearate | 7.5 g |
| Diisopropyl adipate | 5 g |
| White vaseline | 32.5 g |

The above components were weighed and the preparation was manufactured by the same manner as in Example of Preparation 12.

### (Example of Preparation 14: Cream Preparation)

| | |
|---|---|
| Compound of Example 52 | 1.2 g |
| Propylene glycol | 12 g |
| Stearyl alcohol | 20 g |
| White vaseline | 25 g |
| HCO-60 | 4 g |
| Glycerol monostearate | 1 g |
| Methyl paraben | 0.1 g |
| Propyl paraben | 0.1 g |

Stearyl alcohol, white vaseline, HCO-60 and glycerol monostearate were dissolved by heating at about 75 °C. In the meanwhile, compound of Example 52, methyl paraben and propyl paraben were dissolved in propylene glycol, 20 g of pure water was added thereto, and the mixture was heated at about 75 °C and added to the above-prepared mixture followed by stirring to give an emulsion. This was gradually cooled with stirring.

### (Example of Preparation 15: Water-Soluble Ointment)

| | |
|---|---|
| Compound of Example 52 | 5 g |
| Glycerol | 70 g |
| Starch | 10 g |
| Methyl paraben | 0.1 g |
| Propyl paraben | 0.1 g |

The above components were weighed and all components except starch were homogeneously mixed. Starch was added to the mixture little by little with stirring. Pure water was added to make the total amount 100 g.

### (Example of Preparation 16: Water-Soluble Gel Preparation)

| | |
|---|---|
| Compound of Example 52 | 10 g |
| Citric acid | 6 g |
| Hydroxypropylmethylcellulose 2208 indicated viscosity: 4000) | 1.5 g |
| Methyl paraben | 0.1 g |
| Propyl paraben | 0.1 g |

The above components were weighed and all components except hydroxypropylmethylcellulose 2208 were homogeneously mixed. The mixture was dissolved in about 70 g of pure water and then hydroxypropylmethylcellulose 2208 was added thereto little by little with stirring. To this was added pure water to make the total amount 100 g.

### (Example of Preparation 17: Water-Soluble Gel Preparation)

| | |
|---|---|
| Sildenafil | 10 g |
| Citric acid | 6 g |
| Hydroxypropylmethylcellulose 2208 (indicated viscosity: 4000) | 1.5 g |
| Methyl paraben | 0.1 g |
| Propyl paraben | 0.1 g |

The above components were weighed and the preparation was manufactured by the same manner as in Example of Preparation 16.

### (Example of Preparation 18: Tablets)

| | |
|---|---|
| Compound of Example 3 | 100 g |
| Lactose | 350 g |
| Potato starch | 120 g |
| Polyvinyl alcohol | 15 g |
| Magnesium stearate | 15 g |

The above components were weighed and compound of Example 3, lactose and potato starch were homogeneously mixed. Aqueous solution of polyvinyl alcohol was added to the above mixture and granules were prepared by a wet granulating method. The granules were dried, mixed with magnesium stearate and tabletted with compression to manufacture tablets each weighing 300 mg.

### (Example of Preparation 19: Capsules)

| | |
|---|---|
| Compound of Example 15 | 50 g |
| Lactose | 435 g |
| Magnesium stearate | 15 g |

The above components were weighed and homogenously mixed. Each 300 mg of the mixture was filled in a suitable hard capsule using a capsule filling device to manufacture capsule preparations.

### (Example of Preparation 20: Injection)

| | |
|---|---|
| Compound of Example 34 | 2 g |
| Propylene glycol | 200 g |
| Distilled water for injection | q.s. |

The above components were weighed and the compound of Example 34 was dissolved in propylene glycol. To this was added sterile water for injection to make the total amount 1,000 ml. This was sterilized by filtration and each 5 ml of it was charged in a 10-ml ampoule followed by sealing by fusion to manufacture injection preparations.

### (Example of Preparation 21: Suppository)

| | |
|---|---|
| Compound of Example 38 | 100 g |
| Polyethylene glycol 1500 | 180 g |
| Polyethylene glycol 4000 | 720 g |

The compound of Example 38 was well ground in a mortar and the resulting fine powder was made into suppositories (each weighing 1 g) by a fusion method.

### (Example of Preparation 22: Diluted Powder)

| | |
|---|---|
| Compound of Example 16 | 200 g |
| Lactose | 790 g |
| Magnesium stearate | 10 gs |

The above components were weighed and homogeneously mixed to manufacture a 20% powdery preparation.

### (Example of Preparation 23: Intraoral Disintegrable Drug)

| | |
|---|---|
| Compound of Example 52 | 50 g |
| Xylitol | 1100 g |
| Maltitol | 200 g |
| Corn starch | 626 g |
| Aspartame | 4 g |
| Gum arabic powder | 20 g |

The above components were weighed and mixed with a stirring granulator for 1 min. Then, 32 ml of water was added to the mixture, which was blended uniformly. The granulation was charged into a single-punch tablet machine and compressed at a pressure of 10 kg/cm² (force: 30 kg) with punches 20 mm in diameter having flat faces and bevel edges, thereby producing ca. 800 tablets.

### (Example of Preparation 24: Intraoral Disintegrable Drug)

A granulation was prepared with a fluid bed granulator using 2.67 kg of a 15% aqueous maltose solution for 8 kg of mannitol and dried. For the first one (1.0) kilogram of the aqueous maltose solution, coating with fine particles was performed at a spray pressure of 3.0 kg/cm² and granulation followed. The compound of Example 52 (0.9 g), gelatin (0.2 g) and mannitol (0.9 g) were weighed and mixed in a mortar. After mixing with 7 g of the already prepared mannitol granulation, the blend was charged into an oil press and compressed with punches 8 mm in diameter and 9.6 mm in radius at a pressure of 20 kg/cm² to yield tablets each weighing 300 mg.

### Industrial Applicability

The compounds having a pyridocarbazole skeleton that are used as an effective component in the compositions of the present invention have an extremely high selectivity for PDE type V enzyme inhibition and are weak in hypotensive action. Hence, they will cause reduced side effects due to the lowering of blood pressure even if they are administered either alone or in combination with nitrates. In addition, they do not enhance the actions of other hypotensives and may be used with them with high safety. What is more, the compounds used in the present invention proved effective in tests on a rabbit model with relaxed smooth muscles in the cavernous tissue. In view of their additional advantages (very low toxicity and reduced side effects), the compounds are useful as medicines either clinically or in animals and are expected to be particularly effective in the treatment of erectile dysfuntion or female sexual dysfunction.

The intranasal compositions of the invention achieve rapid absorption and appropriate efficacy retention times and exhibit high bioavailability since they are the avoidance of the first pass effect of the digestive tract and the liver. In addition, the compositions have no irritating effect on the nasal mucous membrane, nor do they cause congestion or engorgement; they are free from the potential side effects in the digestive organs and the one of prolonged erection. In sharp contrast with the injection into the cavernous tissue, the compositions can be administered by the patient himself with reduced burden on the both the patient and the doctor and are free from potential side effects such as damage or corporal fibrasis of penis. Unlike sildenafil which is administered perorally, the compositions of the invention are tree from the undesired effect of meals on drug absorption. As a further advantage, it is difficult to administer an overdose of the compositions at a time and the patient, even if he wants early or further manifestation of the efficacy, is unable to take in an excessive amount of the compositions; hence, high safety is ensured.

The percutaneous compositions of the invention provide slow and steady absorption and can sustain preparedness for erection such that spontaneous erection will occur in response to sexual stimulus. In addition, the compositions are the avoidance of the first pass effect of the digestive tract and the liver and are free from the potential side effects in the digestive organs and the one of prolonged erection. Compared to the injection into the cavernous tissue, the compositions that can be administered by the patient himself cause reduced burden on both the patient and the doctor and are free from potential side effects such as damage or corporal fibrosis. Unlike sildenafil which is administered perorally, the compositions are free from the undesired effect of meals on drug absorption. In addition, percutaneous administration is more convenient than administration by other routes.

The transmucosal compositions of the invention achieve rapid absorption and appropriate efficacy retention times and exhibit high bioavailability since they are the avoidance of the first pass effect of the digestive tract and the liver. In addition, the compositions are free from the potential side effects in the digestive organs and the one of prolonged erection. In sharp contrast with the injection into the cavernous tissue, the compositions can be administered by the patient himself with reduced burden on the both the patient and the doctor and are free from potential side effects such as damage or corporal fibrosis. Sublingual preparations, intraoral disintegrable preparations and the like can conveniently be administered without water or with a small amount of water and, hence, are suitable for patients who are permitted to drink only limited amounts of water. As a further advantage, the compositions can be dissolved and absorbed in the oral cavity so efficiently that they have a potential for quick action. Even patients with dysphagia have no difficulty in taking those drugs.

## Claims

1. A composition for remedy of erectile dysfunction wherein the composition contains at least one of the compounds represented by the following formula (I) and salts or solvates thereof as an effective component: 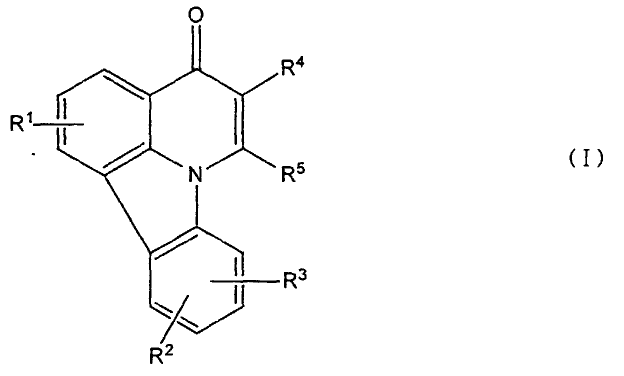 (where R¹ represents a hydrogen atom, a halogen atom, a cyano group, an optionally protected carboxyl group, an optionally protected carboxymethyl group, an alkoxycarbonyl group having 1 - 4 carbon atoms, a carbamoyl group, an acetylamino group, a 3-carboxy-1-propenyl group, a 2-hydroxypentyloxy group, a 2,2-diethoxyethoxy group, an optionally protected hydroxyl group, an optionally protected mercapto group, a straight- or branched-chain alkanoyloxy group having 1 - 4 carbon atoms, a carbonyloxy group substituted with a phenyl group or a pyridyl group, a straight- or branched-chain alkyl group having 1 - 4 carbon atoms which may be substituted with one hydroxyl group, an amino group which may be mono- or disubstituted with an alkyl group having 1 - 4 carbon atoms, an alkylthio group having 1 - 3 carbon atoms which may be monosubstituted with any group selected from the group consisting of a hydroxyl group, a carboxyl group, a phenyl group and a pyridyl group, or represented by the following formula (XXI):
- O - (CH₂)ₙ - Z (XXI)
(where Z is selected from the group consisting of a hydrogen atom, a carboxyl group, an alkoxy group having 1 or 2 carbon atoms which may be substituted with one hydroxyl group, an alkoxycarbonyl group having 1 - 6 carbon atoms, a carbamoyl group which may be mono- or disubstituted with a hydroxymethyl group or an alkyl group having 1 or 2 carbon atoms, an alkanoyl group having 1 - 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 - 4 carbon atoms or an alkoxycarbonyl group having 1 or 2 carbon atoms, a pyrazinyl group, a pyrimidinyl group, a furyl group, a thienyl group, an oxadiazolyl group and a 4-methoxyphenoxy group; n is 1 - 6); R² is selected from the group consisting of a hydrogen atom, a halogen atom, an optionally protected hydroxyl group, an optionally protected mercapto group, an optionally protected amino group, a cyano group, a nitro group, a trifluoromethyl group, a trifluoromethoxy group, an optionally protected carboxyl group, a 4-morpholylacetyl group, a straight- or branched-chain alkanoyloxy group having 1 - 4 carbon atoms, a straight- or branched-chain alkanoyl group having 1 - 4 carbon atoms, a straight- or branched-chain alkyl group having 1 - 4 carbon atoms, an alkylthio group having 1 - 3 carbon atoms which may be monosubstituted with any group selected from the group consisting of a hydroxyl group, a carboxyl group, a phenyl group and a pyridyl group and a straight- or branched-chain alkoxy group having 1 - 4 carbon atoms which may be substituted with one alkoxycarbonyl group having 1 - 4 carbon atoms; R³ is selected from the group consisting of a hydrogen atom, a halogen atom, an optionally protected hydroxyl group and a straight- or branched-chain alkoxy group having 1 - 4 carbon atoms; R⁴ is selected from the group consisting of a hydrogen atom, a halogen atom, an optionally protected carboxyl group, a phenoxy group, an anilino group, a N-methylanilino group, a 4-morpholylcarbonyl group, an alkyl group having 1 or 2 carbon atoms which may be substituted with a cyclic alkyl group having 3 - 6 carbon atoms, a benzyl group which may be mono- or disubstituted in the phenyl portion with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 - 4 carbon atoms, an acetylamino group, a carboxyl group and an amino group, a pyridylmethyl group which may be substituted with an alkyl group having 1 - 4 carbon atoms, a morpholylmethyl group, a triazolylmethyl group, a furylmethyl group, a thienylmethyl group, a pyrimidinylmethyl group, a pyrazinylmethyl group, a pyrrolylmethyl group, an imidazolylmethyl group, a quinolylmethyl group, an indolylmethyl group, a naphthylmethyl group, a benzoyl group, an α-hydroxybenzyl group and an alkoxycarbonyl group having 1 or 2 carbon atoms; R⁵ represents a hydrogen atom or a methyl group; when R¹, R², R³ and R⁵ are a hydrogen atom at the same time, R⁴ is not a hydrogen atom, a benzyl group, a 4-diethylaminobenzyl group or a furylmethyl group.)

2. A composition for remedy of female sexual dysfunction wherein the composition contains at least one of the compounds represented by the above formula (I) and salts or solvates thereof as an effective component.

3. A therapeutic composition according to claim 1 or 2 wherein the composition is administered intranasally.

4. A therapeutic composition according to claim 1 or 2 wherein the composition is administered percutaneously.

5. A therapeutic composition according to claim 1 or 2 wherein the composition is administered transmucosally.

6. A therapeutic composition according to any one of claims 1 to 5 wherein the compound represented by the formula (I) mentioned in claim 1 is at least one of 9-bromo-2-(3-hydroxypropyloxy)-5-(3-pyridylmethyl)-4H-pyrido[3,2,1-jk]carbazol-4-one and salts or solvates thereof.

7. A composition for intranasal administration wherein the composition contains at least one of the compounds represented by the formula (I) mentioned in claims 1 and salts or solvates thereof as an effective component.

8. A composition for intranasal administration according to claim 7 wherein the compound represented by the above formula (I) is at least one of 9-bromo-2-(3-hydroxypropyloxy)-5-(3-pyridylmethyl)-4H-pyrido[3,2,1-jk]carbazol-4-one and salts or solvates thereof.

9. A composition for remedy of erectile dysfunction by intranasal administration wherein the composition contains at least one of compounds having an inhibitory action to cyclic GMP phosphodiesterase, and salts or solvates thereof as an effective component.

10. A composition for remedy of erectile dysfunction by intranasal administration according to claim 9 wherein the compound having an inhibitory action to cyclic GMP phosphodiesterase is at least one of the compounds represented by the following formula (XXV) and salts or solvates thereof: 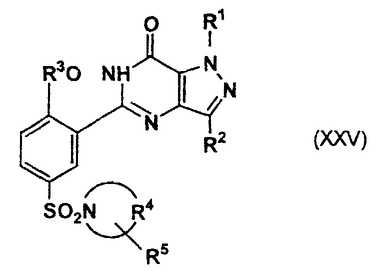 (where R¹ is a hydrogen atom, C₁₋₃ alkyl, C₃₋₅ cycloalkyl or C₁₋₃ perfluoroalkyl; R² is selected from the group consisting of H, OH, C₁₋₆ alkyl substituted with C₁₋₃ alkoxy or C₃₋₆ cycloalkyl and C₁₋₃ perfluoroalkyl; R₃ is selected from the group consisting of C₁₋₆ alkyl, C₃₋₆ alkenyl, C₃₋₆ alkynyl, C₃₋₇ cycloalkyl, C₁₋₆ perfluoroakyl and (C₃₋₆ cycloalkyl)-C₁₋₆ alkyl; R⁴ forms a group selected from the group consisting of pyrrolidinyl, piperidino, morpholino and 4-N-(R⁶)-piperazinyl together with a nitrogen atom to which R⁴ is bonded; R⁵ is selected from the group consisting of H, C₁₋₄ alkyl, C₁₋₃ alkoxy, NR⁷R⁸ and CONR⁷R⁸; R⁶ is selected from the group consisting of H, C₁₋₆ alkyl, (C₁₋₃ alkoxy)-C₂₋₆ alkyl, hydroxy C₂₋₆ alkyl, (R⁷R⁸N)-C₂₋₆ alkyl, (R⁷R⁸NCO)-C₁₋₆ alkyl, CONR⁷R⁸, CSNR⁷R⁸ and C(NH)NR⁷R⁸; and R⁷ and R⁸ each independently is selected from the group consisting of H, C₁₋₄ alkyl, (C₁₋₃ alkoxy)-C₂₋₄ alkyl and hydroxy C₂₋₄ alkyl).

11. A composition for remedy of erectile dysfunction by intranasal administration according to claim 9 wherein the compound having an inhibitory action to cyclic GMP phosphodiesterase is at least one of 5-[2-ethoxy-5-(4-methylpiperazin-1-ylsulfonyl)phenyl]-1-methyl-3-propyl-6,7-dihydro-1H-pyrazolo[4,3-d]pyrimidin-7-one and a salt thereof.

12. A composition for remedy of erectile dysfunction by intranasal administration according to claim 9 wherein the compound having an inhibitory action to cyclic GMP phosphodiesterase is at least one of the compounds mentioned hereunder and salts or solvates thereof:
1) 2-piperazinyl-6,7-dimethoxyquinazoline derivatives mentioned in the Japanese Laid-Open Patent Publication Sho-52/100,479;
2) heterocyclopyrimidine derivatives mentioned in the Japanese Laid-Open Patent Publication Sho-53/103,497;
3) 5-substituted compounds of pyrazolo[4,3-d]pyrimidin-7-ones mentioned in the Japanese Laid-Open Patent Publication Sho-61/236,778;
4) griseol derivatives mentioned in the Japanese Laid-Open Patent Publication Sho-62/30,796 or Sho-62/30,782;
5) purinone derivatives mentioned in the Japanese Laid-Open Patent Publication Sho-63/196,585 or Hei-02/88,577;
6) KS 506 mentioned in the Japanese Laid-Open Patent Publication Hei-02/1,463;
7) phenylpyridone derivatives mentioned in the Japanese Laid-Open Patent Publication Hei-01/311,067 or Hei-03/145,466;
8) 1,4-dihydropyridine derivatives mentioned in the Japanese Laid-Open Patent Publication Hei-02/223,580;
9) condensed pyrimidine derivatives mentioned in the Japanese Laid-Open Patent Publication Hei-02/42,079 or Hei-02/56,484;
10) pyrimidopyrimidine derivatives mentioned in the Japanese Laid-Open Patent Publication Hei-02/40,388 or Hei-03/261,785;
11) quinazolinone derivatives mentioned in the Japanese Laid-Open Patent Publication Hei-02/193,983 or International Publication WO 93/12,095;
12) phenylpyrimidinone derivatives mentioned in the Japanese Laid-Open Patent Publication Hei-02/295,977 or Hei-02/295,978;
13) polycyclic guanine derivatives mentioned in the International Publication WO 91/19,717;
14) pyrazolopyrimidinone derivatives mentioned in the International Publication WO 93/06,104 or WO 93/07,149;
15) nitrogen-containing heterocyclic compounds mentioned in the International Publication WO 93/07,124 or the Japanese Laid-Open Patent Publication Hei-07/10,843;
16) benzimidazole derivatives mentioned in the Japanese Laid-Open Patent Publication Hei-05/222,000;
17) WS 63967 mentioned in the Japanese Laid-Open Patent Publication Hei-05/301,857;
18) 4-aminoquinazoline derivatives mentioned in any of the Japanese Laid-Open Patent Publications Hei-06/192,235, Hei-08/99,962 and Hei-07/188,214 and U.S. Patents No. 5,436,233 and No. 5,439,895;
19) purin-6-ones mentioned in the International Publication WO 94/00,453 or the Japanese Laid-Open Patent Publication Hei-09/124,648;
20) pyrazolo[3,4-d]pyrimidin-4-one derivatives mentioned in the U.S. Patent No. 5,294,612;
21) pyridopyrimidinone derivatives mentioned in the International Publication WO 94/05,561;
22) 2-benzyl-polycyclic guanine derivatives mentioned in the International Publication WO 94/19,351;
23) amilino- or pyrazylamino-cyclobuten-1,2-dione derivatives mentioned in the International Publication WO 94/29,277;
24) fluorenone derivatives mentioned in the Japanese Laid-Open Patent Publication Hei-07/61,949;
25) 4-aminopyrimidine derivatives mentioned in the Japanese Laid-Open Patent Publication Hei-07/89,958;
26) imidazoquinazoline derivatives mentioned in the International Publication WO 95/06,648 or WO 96/26,940;
27) quinazoline derivatives mentioned in the Japanese Laid-Open Patent Publication Hei-07/126,255;
28) anthranilic acid derivatives mentioned in the Japanese Laid-Open Patent Publication Hei-08/188,563;
29) benzofuro[3,2-d]pyrimidin-4-one derivatives mentioned in the Japanese Laid-Open Patent Publication Hei-07/267,961;
30) MS-681 mentioned in the Japanese Laid-Open Patent Publication Hei-07/285,993;
31) thieno[3,2-d]pyrimidin-4-one derivatives mentioned in the Japanese Laid-Open Patent Publication Hei-07/330,777 or Hei-08/143,571;
32) phthalazine derivatives mentioned in the International Publication WO 96/05,176;
33) FR 901526 mentioned in the Japanese Laid-Open Patent Publication Hei-08/59,681;
34) pyrazoloquinoline derivatives mentioned in the International Publication WO 96/28,446;
35) quinazolin-4(3H)-one derivatives mentioned in the Japanese Laid-Open Patent Publication Hei-08/104,679;
36) 4-aminopyrimidine derivatives mentioned in the U.S. Patent No. 5,525,604;
37) 2,8-disubstituted quinazolinone derivatives mentioned in the Japanese Laid-Open Patent Publication Hei-08/253,457;
38) 2,9-disubstituted purin-6-one derivatives mentioned in the Japanese Laid-Open Patent Publication Hei-08/231,545;
39) 9-substituted 2-(2-n-alkoxyphenyl)-purin-6-one derivatives mentioned in the Japanese Laid-Open Patent Publication Hei-08/231,546;
40) pyrazolopyrimidin-4-one derivatives mentioned in any of the U.S. Patent No. 5,541,187, and the International Publications WO 96/28,429 and WO 96/28,448;
41) heterocyclic compounds mentioned in the Japanese Laid-Open Patent Publication Hei-08/269,060;
42) pyrido[3,2-e]pyrazinone derivatives mentioned in the Japanese Laid-Open Patent Publication Hei-08/269,059;
43) indole derivatives mentioned in the International Publication WO 96/32,379;
44) 1H-pyrazolo[3,4-d]pyrimidin-4-one derivatives mentioned in the Japanese Laid-Open Patent Publication Hei-08/253,484;
45) 4-(arylaminomethylene)-2,4-dihydro-3-pyrazolone derivatives mentioned in the Japanese Laid-Open Patent Publication Hei-08/311,035;
46) benzimidazole derivatives mentioned in the Japanese Laid-Open Patent Publication Hei-09/77,764 or the International Publication WO 97/24,334;
47) thienopyrimidine derivatives mentioned in the International Publication WO 98/06722; and
48) imidazoquinazoline derivatives mentioned in International Publication WO 98/08848.

13. A composition for remedy of erectile dysfunction by percutaneous administration wherein the composition contains at least one of the compounds having an inhibitory action to cyclic GMP phosphodiesterase and salts or solvates thereof as an effective component.

14. A composition for remedy of erectile dysfunction by percutaneous administration according to claim 13 wherein the compound having an inhibitory action to cyclic GMP phosphodiesterase is at least one of the compounds represented by the formula (XXV) mentioned in claim 10 and salts or solvates thereof.

15. A composition for remedy of erectile dysfunction by percutaneous administration according to claim 13, wherein the compound having a cyclic GMP phosphodiesterase inhibitory action is either 5-[2-ethoxy-5-(4-methylpiperazin-1-ylsulfonyl)phenyl]-1-methyl-3-propyl-6,7-dihydro-1H-pyrazolo[4,3-d]pyrimidin-7-one or a salt thereof or both.

16. A composition for remedy of erectile dysfunction by percutaneous administration according to claim 13 wherein the compound having a selective inhibitory action to cyclic GMP phosphodiesterase is at least one of the following compounds and salts thereof:
1) 2-piperazinyl-6,7-dimethoxyquinazoline derivatives mentioned in the Japanese Laid-Open Patent Publication Sho-52/100,479;
2) heterocyclopyrimidine derivatives mentioned in the Japanese Laid-Open Patent Publication Sho-53/103,497;
3) 5-substituted compounds of pyrazolo[4,3-d]pyrimidin-7-ones mentioned in the Japanese Laid-Open Patent Publication Sho-61/236,778;
4) griseol derivatives mentioned in the Japanese Laid-Open Patent Publication Sho-62/30,796 or Sho-62/30,782;
5) KS 506 mentioned in the Japanese Laid-Open Patent Publication Hei-02/1,463;
6) 1,4-dihydropyridine derivatives mentioned in the Japanese Laid-open Patent Publication Hei-02/223,580;
7) pyrazolopyrimidinone derivatives mentioned in the International Publication WO 93/06,104 or WO 93/07,149;
8) nitrogen-containing heterocyclic compounds mentioned in the International Publication WO 93/07,124 or the Japanese Laid-Open Patent Publication Hei-07/10,843;
9) quinazolinone derivatives mentioned in the International Publication WO 93/12,095;
10) purin-6-ones mentioned in the International Publication WO 94/00,453 or the Japanese Laid-Open Patent Publication Hei-09/124,648;
11) pyrazolo[3,4-d]pyrimidin-4-one derivatives mentioned in the U.S. Patent No. 5,294,612;
12) pyridopyrimidinone derivatives mentioned in the International Publication WO 94/05,561;
13) imidazoquinazoline derivatives mentioned in the International Publication WO 95/06,648 or WO 96/26,940;
14) quinazoline derivatives mentioned in the Japanese Laid-Open Patent Publication Hei-07/126,255;
15) anthranilic acid derivatives mentioned in the Japanese Laid-Open Patent Publication Hei-08/188,563;
16) benzofuro[3,2-d]pyrimidin-4-one derivatives mentioned in the Japanese Laid-Open Patent Publication Hei-07/267,961;
17) MS-681 mentioned in the Japanese Laid-Open Patent Publication Hei-07/285,993;
18) thieno[3,2-d]pyrimidin-4-one derivatives mentioned in the Japanese Laid-Open Patent Publication Hei-08/143,571;
19) phthalazine derivatives mentioned in the International Publication WO 96/05,176;
20) quinazolin-4(3H)-one derivatives mentioned in the Japanese Laid-Open Patent Publication Hei-08/104,679;
21) pyrazolopyrimidin-4-one derivatives mentioned in the U.S. Patent No. 5,541,187;
22) pyrido[3,2-e]pyrazinone derivatives mentioned in the Japanese Laid-Open Patent Publication Hei-08/269,059; and
23) 1H-pyrazolo[3,4-d]pyrimidin-4-one derivatives mentioned in the Japanese Laid-Open Patent Publication Hei-08/253,484.
